# EUROPEAN PATENT APPLICATION

(11) **EP 3 385 388 A1**
(43) Date of publication of application: **10.10.2018**
(21) Application number: 18172179.6
(22) Date of filing: 22.03.2012
(51) Int. Cl.: C12P 1/00

(54) **MICROBIAL PRODUCTION OF CHEMICAL PRODUCTS AND RELATED COMPOSITIONS, METHODS AND SYSTEMS**

(30) Priority: 22.03.2011 US 201161466363 P; 22.03.2011 US 201161466433 P; 26.09.2011 US 201161539378 P; 26.09.2011 US 201161539162 P
(62) Divisional of application: 12759932.2
(71) Applicant: OPX Biotechnologies Inc., Boulder, CO 80301 (US)
(72) Inventor: LYNCH, Michael, D., Boulder, CO 80301 (US); LIPSCOMB, Tanya, E.w., Boulder, CO 80301 (US); TRAHAN, Ashley, D., Boulder, CO 80301 (US); SINGH, Amar, Boulder, CO 80301 (US); WOLTER, Travis, Boulder, CO 80301 (US)
(74) Representative: Fox, Charlotte

(57) **Abstract**

Metabolically engineered microorganism strains are disclosed, such as bacterial strains, in which there is an increased utilization of malonyl-CoA for production of a chemical product. Such chemical products include polyketides, 3-hydroxypropionic acid, and various other chemical products described herein. Methods of production also may be applied to further downstream products, such as consumer products. In various embodiments, modifications to a microorganism and/or culture system divert, at least transiently, usage of malonyl-coA from the fatty acid biosynthesis pathway and thereby provides for usage of the malonyl-coA for a chemical product other than a fatty acid. In various embodiments, the fatty acid biosynthesis pathway is modulated to produce specific fatty acids or combinations of fatty acids.

## Description

### RELATED APPLICATIONS

This application claims priority to U.S. provisional patent applications US 61/466,363, filed on March 22, 2011; US 61/466,433, filed on March 22, 2011; US 61/539,162, filed September 26, 2011; and US 61/539,378, filed September 26, 2011; each of which are hereby incorporated by reference in their entirety.

### STATEMENT AS TO FEDERALLY SPONSORED RESEARCH

This invention was made with Government support under DE-AR0000088 awarded by the United States Department of Energy. The Government has certain rights in this invention.

### SEQUENCE LISTINGS

The instant application contains a Sequence Listing which has been submitted in ASCII format via EFS-Web and is hereby incorporated by reference in its entirety. Said ASCII copy, created on March 22, 2012, is named 34246760.txt and is 848 kbytes in size.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BACKGROUND OF THE INVENTION

There is increased emphasis on renewable production of fuels and industrial chemicals as spikes in petroleum costs occur and as petroleum finite resources are consumed. Notwithstanding advances in the general field, there remains a need to find metabolic solutions to renewable production so as to increase rate, productivity, yield, and overall cost-effectiveness for biosynthesis of various chemical products.

### SUMMARY OF THE INVENTION

Disclosed herein is a method for producing a chemical, the method comprising combining a carbon source, a microorganism, and a cell culture to produce the chemical, wherein a) said cell culture comprises an inhibitor of fatty acid synthase and/or the microorganism is genetically modified for reduced enzymatic activity in at least two of the microorganism's fatty acid synthase pathway enzymes, providing for reduced conversion of malonyl-CoA to fatty acids; and b) the microorganism additionally has one or more genetic modifications relating to a metabolic production pathway from malonyl-CoA to the chemical. In various embodiments, the at least two fatty acid synthase pathway enzymes with reduced enzymatic activity are an enoyl-coA reductase and a beta-ketoacyl-ACP synthase. In one example, the enoyl-coA reductase is fabI or a peptide of 80% or more homology to SEQ ID NO: 14. In addition, the beta-ketoacyl-ACP synthase may be selected from the group consisting of fabB, fabF, a peptide of 80% or more homology to SEQ ID NO: 9, and a peptide of 80% or more homology to SEQ ID NO: 8. In various emdodiments, a third fatty acid synthase pathway enzyme is modified for reduced enzymatic activity. For example, the enoyl-coA reductase is fabI or a peptide of 80% or more homology to SEQ ID NO: 14, the beta-ketoacyl-ACP synthase is fabB or a peptide of 80% homology or more to SEQ ID NO: 9, and the third fatty acid synthase pathway enzyme is fabF or a peptide of 80% homology or more to SEQ ID NO: 8.

In various embodiments, the third fatty acid synthase pathway enzyme is a malonyl-coA-ACP transacylase. For example, the malonyl-coA-ACP transacylase is modified fabD or a peptide of 80% or more homology to SEQ ID NO: 7. In various embodiments, the enoyl-coA reductase is fabI or a peptide of 80% or more homology to SEQ ID NO: 14, the beta-ketoacyl-ACP synthase is selected from the group consisting of fabB or a peptide of 80% homology or more to SEQ ID NO: 9, and fabF or a peptide of 80% homology or more to SEQ ID NO: 8, and the malonyl-coA-ACP transacylase is fabD or a peptide of 80% or more homology to SEQ ID NO: 7.

In various emdodiments, the at least two fatty acid synthase pathway enzymes with reduced enzymatic activity are an enoyl-coA reductase and a malonyl-coA-ACP transacylase. For example, the enoyl-coA reductase is fabI or a peptide of 80% or more homology to SEQ ID NO: 14. As an additional example, the malonyl-coA-ACP transacylase is fabD or a peptide of 80% or more homology to SEQ ID NO: 7.

Also disclosed are methods as described above, where the microorganism is E. coli. In any of the embodiments described herein, the method may be performed at above room temperature. Preferably, the method is performed at a temperature between 25°C and 50°C.

Also disclosed are methods as described herein, where the microorganism has one or more genetic modifications to increase levels of pantothenate. As an example, the one or more genetic modifications to increase levels of pantothenate is a modification of panE or a peptide of 80% or more homology to panE.

In various embodiments, the microorganism has one or more genetic modifications to increase pyruvate dehydrogenase activity. For example, the one or more genetic modifications to increase pyruvate dehydrogenase activity is a modification of aceE or a peptide of 80% or more homology to SEQ ID NO: 172.

In various embodiments, the increased pyruvate dehydrogenase activity is resistant to inhibition by elevated NADH levels. In addition, the microorganism may be further genetically modified to have reduced alcohol dehydrogenase activity. As an example, the microorganism is genetically modified to have a modification of adhE or a peptide of 80% or more homology to adhE.

Also disclosed are methods as described herein, wherein the microorganism is further genetically modified to have one or more genetic modifications relating to a metabolic production pathway from malonyl-CoA to the chemical, wherein the pathway uses NADH as a reducing agent and wherein there is a mutation or deletion of an alcohol dehydrogenase gene.

In various embodiments, the microorganism is further genetically modified to have modified malonyl-coA dependant acetoacetyl-coA synthase activity. For example, the modified malonyl-coA dependant acetoacetyl-coA synthase is npth07 or a peptide of 80% or more homology to npth07.

In various embodiments, the microorganism is further genetically modified to have altered elongase activity. For example, the microorganism has one or more modifications in the group consisting of elo1, elo2, and elo3, or a peptide of 80% or more homology to a peptide from SEQ ID NOs: 199-201.

Disclosed herein are methods where acetoacetyl-coA is converted to (S)-3-hydroxybutyryl-coA, (R)-3-hydroxybutyryl-coA, 3-hydroxymethylglutaryl-coA, crotonyl-coA, butyryl-coA, isobutyryl-coA, methacrylyl-coA, or 2-hydroxyisobutyryl-coA. The conversion occurs in one step or the conversion occurs in more than one step. In various methods acetoacetyl-coA is converted to a linear acyl-coA with a chain length from 4-18 carbons.

In various embodiments, chemicals produced according to the methods disclosed herein are selected from the group consisting of butanol, isobutanol, 3-hydroxypropionic acid, methacrylic acid, 2-hydroxyisobutyrate, butyrate, isobutyrate, acetoacetate, polyhydroxybutyrate, (S)-3-hydroxybutyrate, (R)-3-hydroxybutyrate, mevalonate, an isoprenoid, a fatty acid, a fatty aldehyde, a fatty alcohol, a fatty acid ester, phloroglucinol, resorcinol, an alkylresorcinol, tetracycline, erythromycin, avermectin, macrolides, Vancomycin-group antibiotics, and Type II polyketides. Preferably, the chemical is produced at a concentration higher than a concentration of said chemical produced by a wild type microorganism.

Also disclosed herein are methods for producing a C4-C18 fatty acid, said methods comprising combining a carbon source, a microorganism, and a cell culture to produce the C4-C18 fatty acid, wherein a) said cell culture comprises an inhibitor of fatty acid synthase and/or the microorganism is genetically modified for reduced enzymatic activity in at least one of the microorganism's native fatty acid synthase pathway enzymes, providing for reduced conversion of malonyl-CoA to fatty acyl-ACPs; and b) the microorganism additionally has one or more genetic modifications increasing fatty acid production. The C4-C18 fatty acid may be selected from the group consisting of C4, C6, C8, C10, C12, C14, C16 and C18 fatty acids. An additional step comprises isolating a C4-C18 fatty acid from said cell culture. In various embodiments, the microorganism synthesizes fatty acyl-coAs of 4-18 carbon chain length. Preferably, the microorganism expresses at least one heterologous elongase enzyme. The elongase enzyme may be a peptide with a sequence corresponding to the group consisting of elo1, elo2, and elo3, or a peptide of 80% or more homology to a peptide from SEQ ID NOs: 199-201.

Also disclosed herein are genetically modified microorganisms for use according to any of the methods described herein. The microorganisms may be yeast or bacteria. In various embodiments, the microorganism is E. coli.

Microorganisms comprising a sequence selected from the group consisting of SEQ ID NO: 1-215 or a sequence of 80% or more homology to SEQ ID NO: 1-215 are disclosed. Further disclosed are geneically modified microorganisms comprising at least one, two, three, four, five, six, seven, eight, nine, ten, or more sequences selected from group consisting of SEQ ID NO: 1-215 or sequences of 80% or more homology to SEQ ID NO: 1-215. In addition, microorganisms further genetically modified to induce enzyme expression from the yibD gene promoter with a decreased environmental phosphate concentration are disclosed. In various emdobiments, the microorganism is further genetically modified to maintain plasmids with expression of a gapA gene on the plasmid and a deletion of the gapA gene in the chromosome.

In various embodiments, microorganisms as described herein have a genotype with 10, 11, 12, 13, 14, 15, or more features selected from the group consisting of F-, Δ(araD-araB)567, ΔlacZ4787(::rrnB-3), LAM-, rph-1, Δ(rhaD-rhaB)568, hsdR514, ΔldhA:frt, ΔpflB:frt, ΔmgsA:frt, ΔpoxB:frt, Δpta-ack:frt, fabIts (S241F)-zeoR, fabB(ts), ΔfabF:frt, coaA*, fabD(ts), and ΔaceBAK:frt.

In various embodiments, microorganisms as described herein have a genotype with 10, 11, 12, 13, 14, 15, or more features selected from the group consisting of F-, Δ(araD-araB)567, ΔlacZ4787(::rrnB-3),LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, fabB(ts)-(A329V), ΔfabF:frt, coaA(R106A), fabD(ts)-(W257Q), ΔlacI:frt, ΔpuuC::T5-aceEF-lpd(E354K):loxP, ΔaceBAK:frt, lpd(E354K):loxP, ΔaldB:PyibD-T7pol:loxP, ΔadhE:frt, ΔaldA:cscBKA.

In various embodiments, the present invention provides for production of 3HP in cell culture with a plamid containing one or more genes as described herein. Accordingly, a mixture of 3HP and cell culture is disclosed, as well as mixtures of 3HP and cellular debris including plasmids or vectors containing such genes and genetic material.

### BRIEF DESCRIPTION OF DRAWINGS

The novel features of the invention are set forth with particularity in the claims. A better understanding of the features and advantages of the present invention will be obtained by reference to the following detailed description that sets forth illustrative embodiments, in which the principles of the invention are utilized, and the accompanying drawings of which
FIG. 1 Enzymatic conversion steps involved in production of malonyl-CoA.
FIG. 2A Metabolic pathways for production and utilization of malonyl-CoA.
FIGs. 2B A fatty acid biosynthesis initiation pathway.
FIG. 2C Two fatty acid biosynthesis initiation pathways.
FIG. 2D Superpathyway of fatty acid biosynthesis initiation (E. coli).
FIG. 3 Chemicals into which 3-HP may be converted for further commercial use.
FIGs. 4A-D Pathway involving production of coenzyme A.
FIG. 5 Chemical products produced from malonyl-CoA.
FIG. 6 Pathways toward production of fatty acid methyl esters.

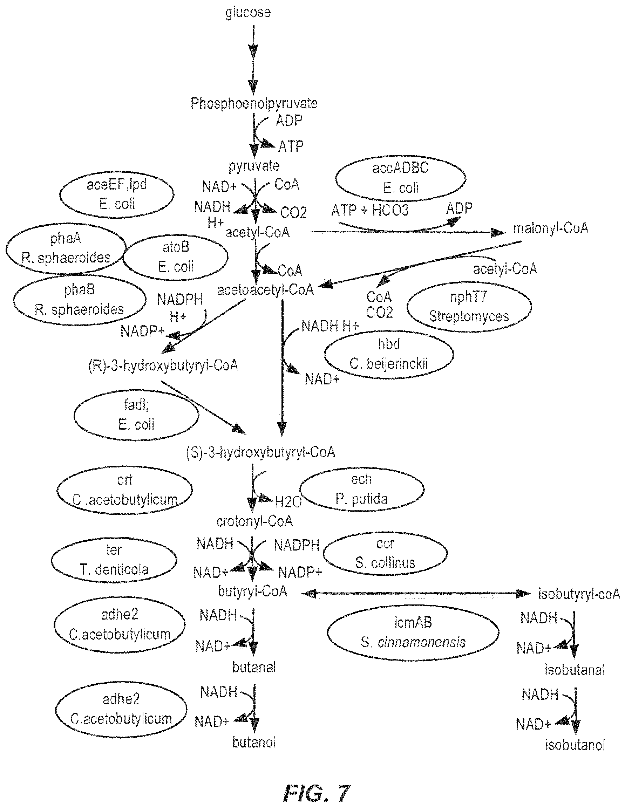

FIG. 7 Pathways toward production of butanol and isobutanol.

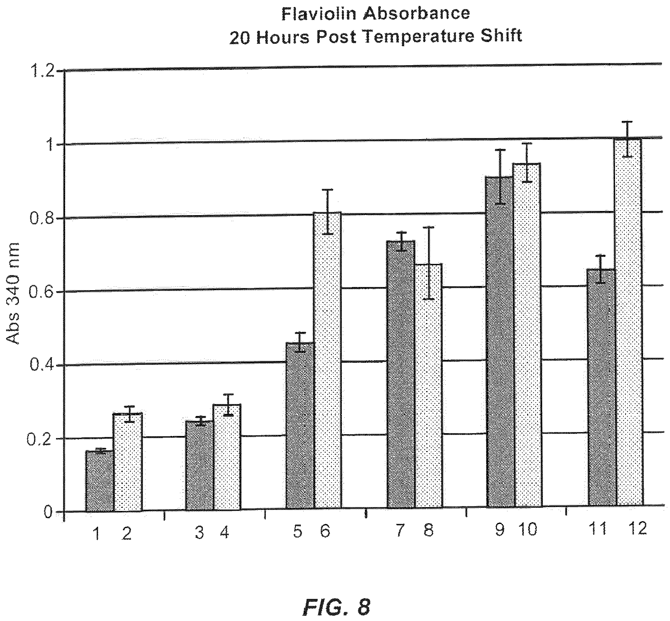

FIG. 8 Flaviolin asorbance at 24 hrs for FAS (Fatty Acid Synthesis pathway) mutants having a plasmid Ptrc-THNS comprising a nucleic acid sequence encoding THN synthase. Numbers along X-axis refer to strains.

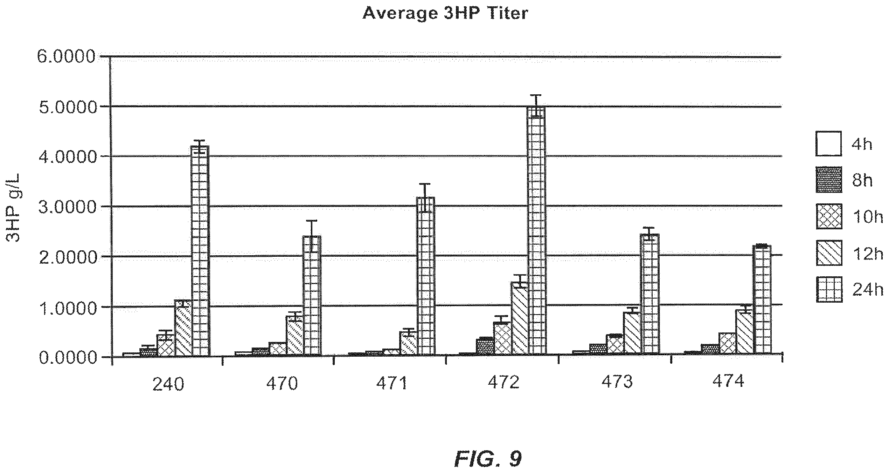

FIG. 9 3-HP Production by FAS mutant strains.

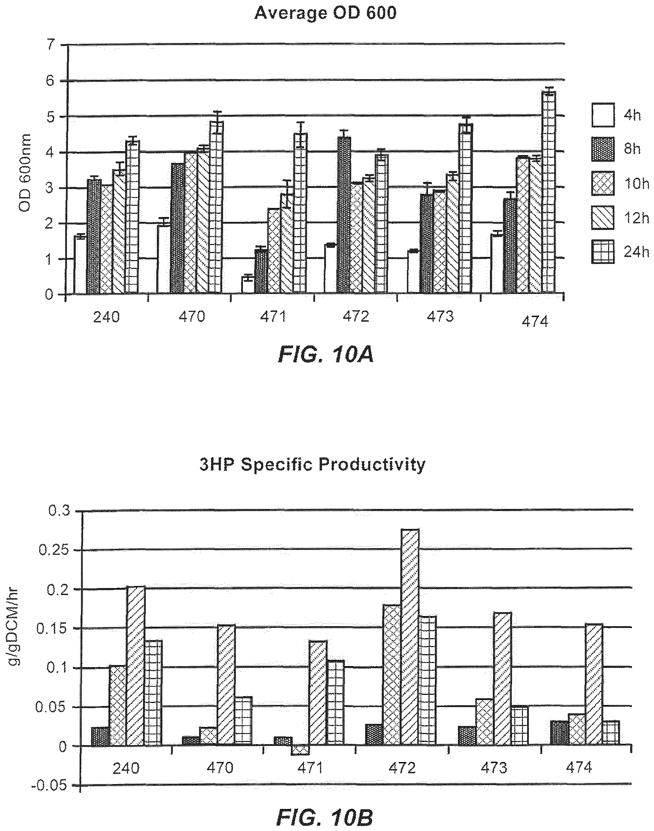

FIG.10A Growth of FAS mutant strains.
FIG. 10B Productivity of FAS mutant strains.

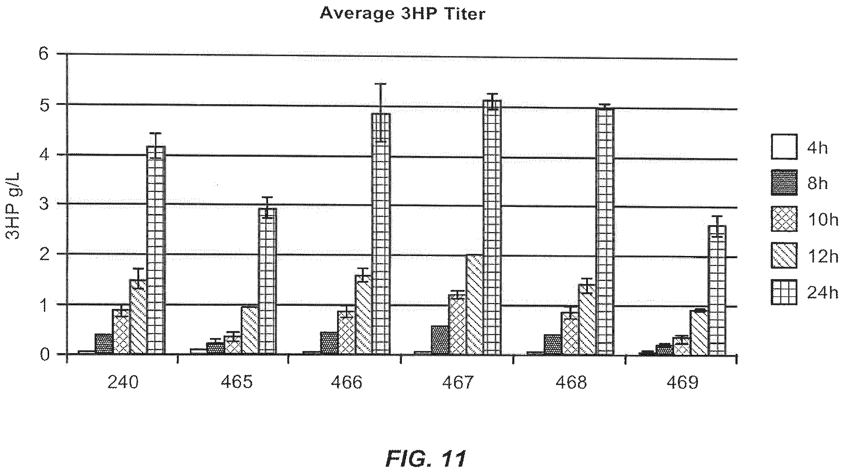

FIG. 11 Additional 3-HP Production of FAS mutants.

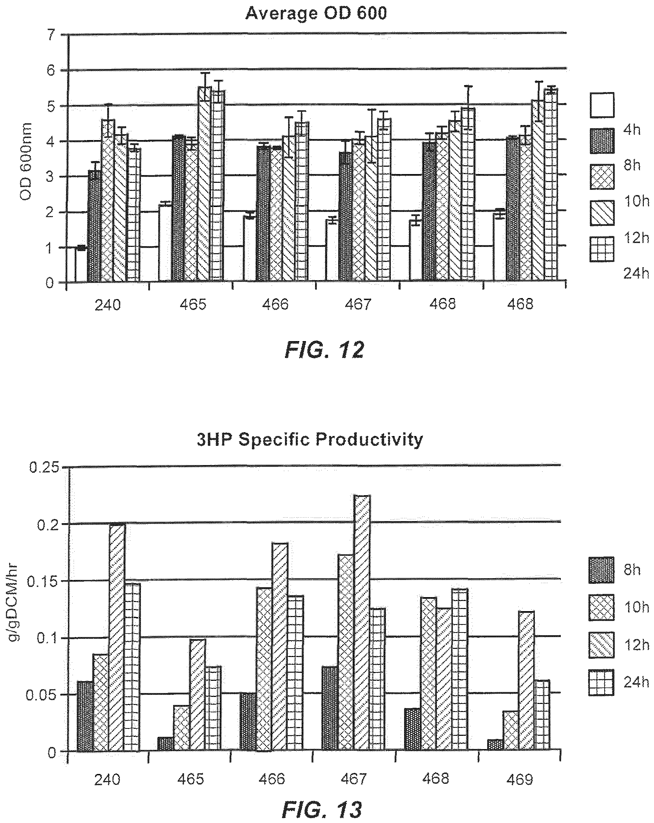

FIG. 12 Additional growth results of FAS mutants.
FIG.13 Additional Specific Productivity results of FAS mutants.

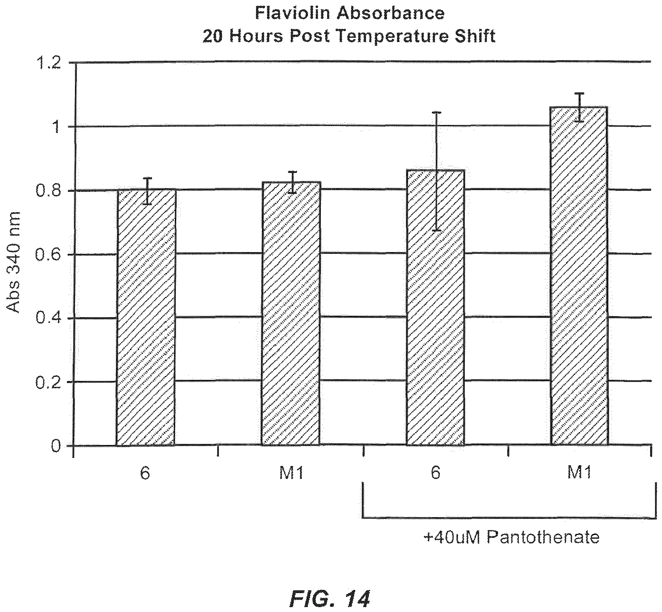

FIG.14 Flaviolin Absorbance results at 24 hours for FAS and CoA mutants

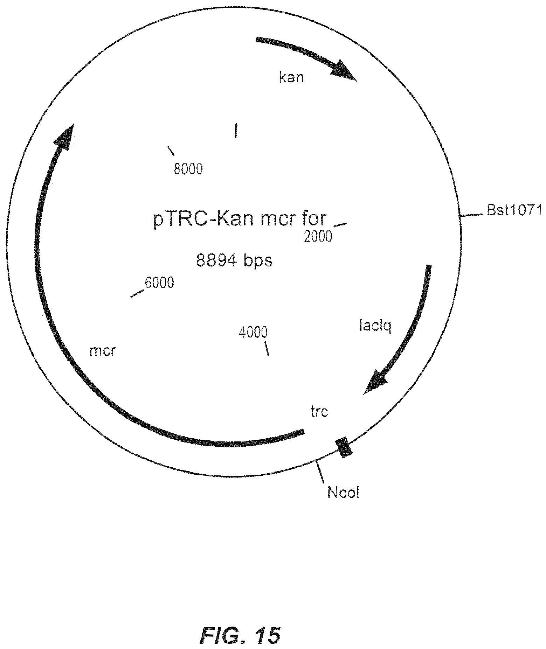

FIG. 15: Plasmid Map 1: Original pTrc-ptrc-MCR

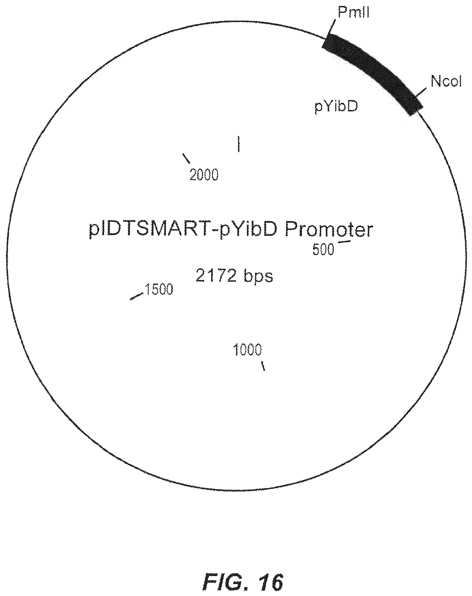

FIG 16: Plasmid Map 2: pIDTSMART-PyibD. Synthesized by Integrated DNA Technologies.

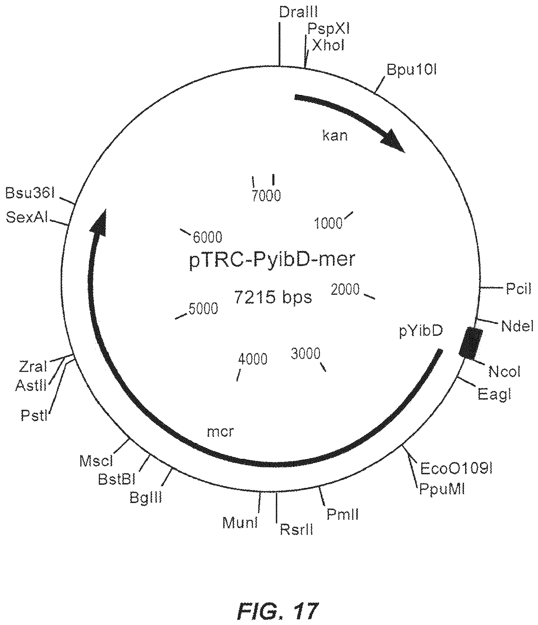

FIG. 17: Plasmid Map 3: New MCR construct. pTRC-PyibD-mcr (low phosphate induction) (SEQ ID NO:170).

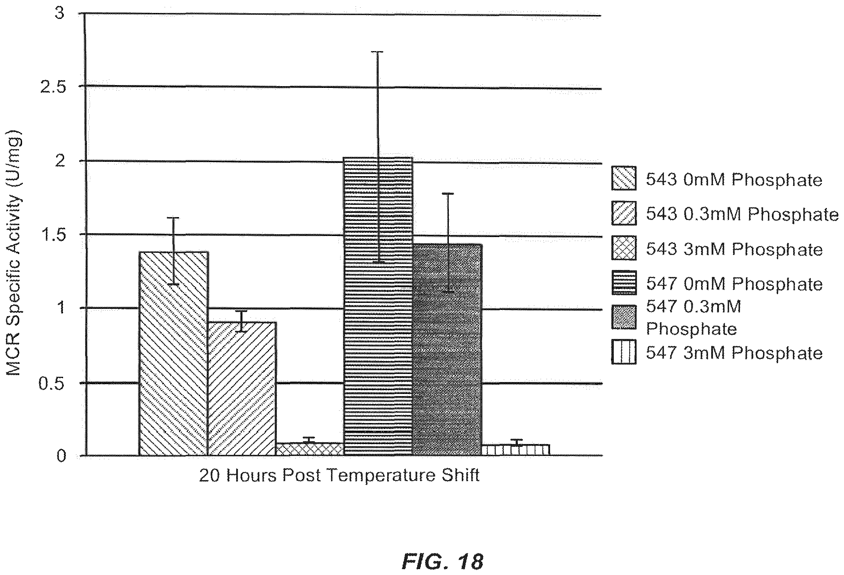

FIG. 18: mcr activity for PyibD-mcr measured at variable phosphate levels
FIG 19. Pyruvate dehydrogenase activity for various strains. Figure 19 shows that strains expressing one or two copies of the mutated lpdA gene (E354K) are less sensitive to inhibition by NADH compared to the wild type strains (BX775).

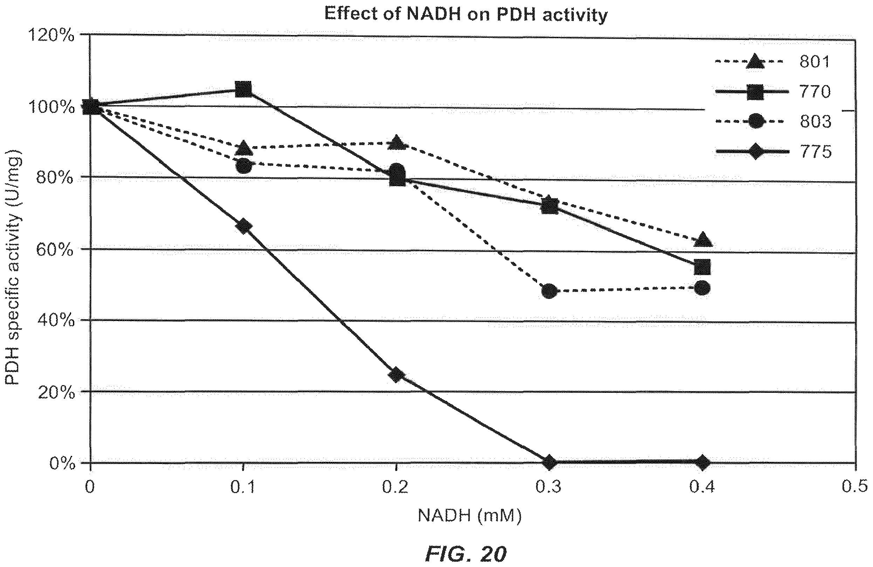

FIG. 20. Effect of NADH inhibition on pyruvate dehydrogenase activity.

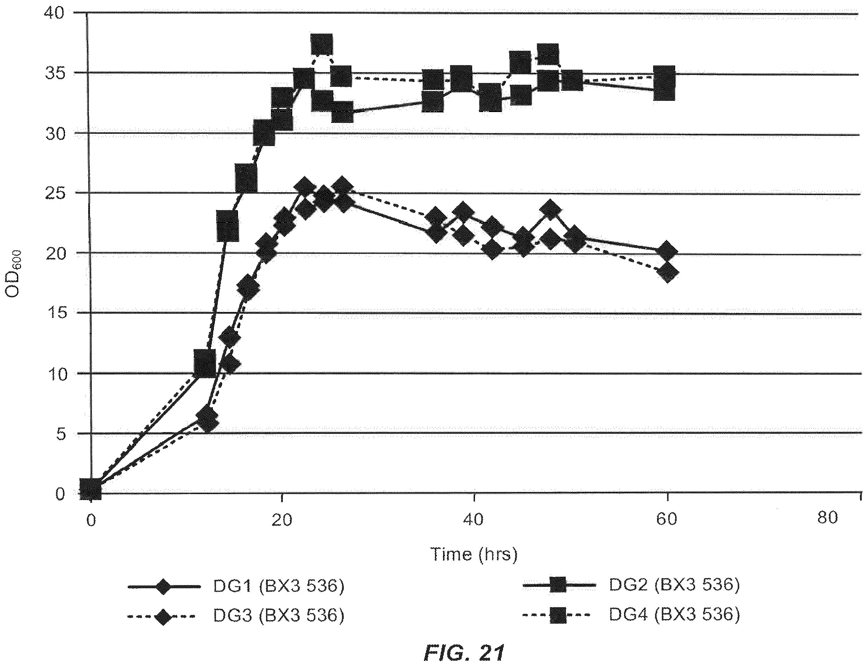

FIG 21. Growth profiles of 535 and 536 biocatalysts in fermentors

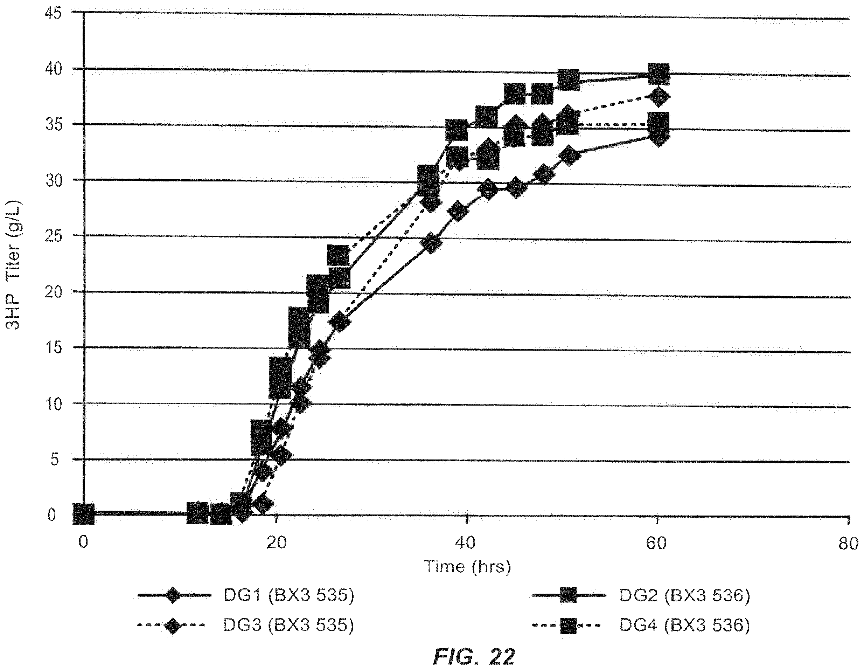

FIG. 22. 3-HP production from sucrose

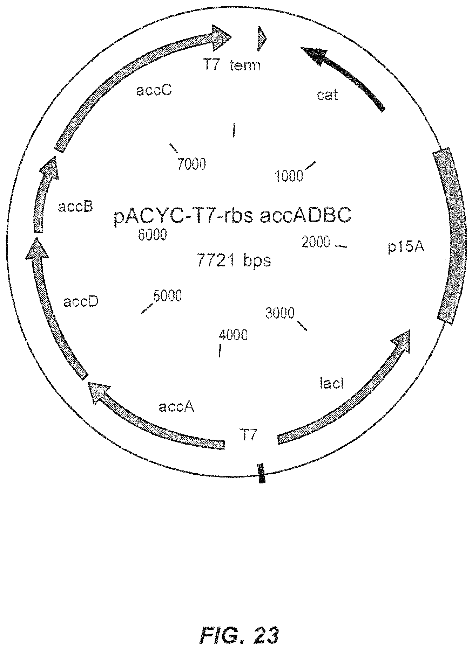

FIG. 23. Plasmid Map 4: pACYC-T7-rbs accADBC

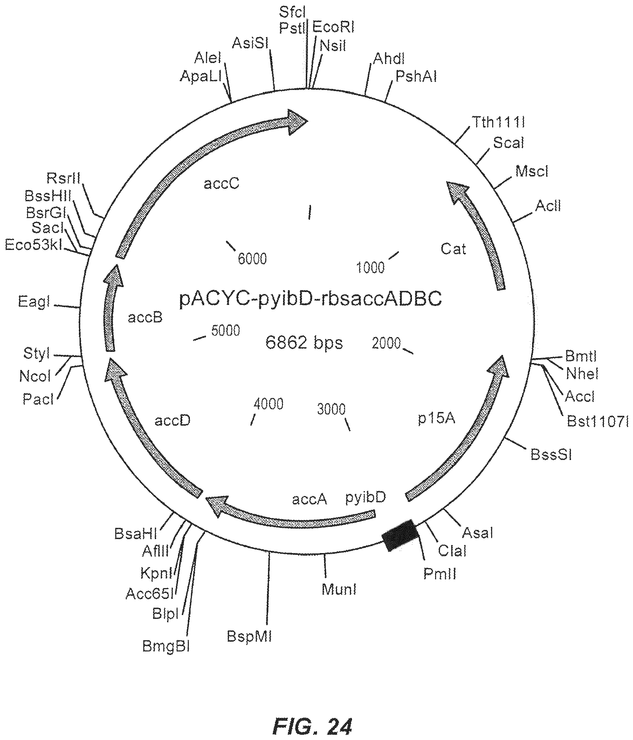

FIG. 24. Plasmid Map 5: map of pACYC-pyibD-rbsaccADBC

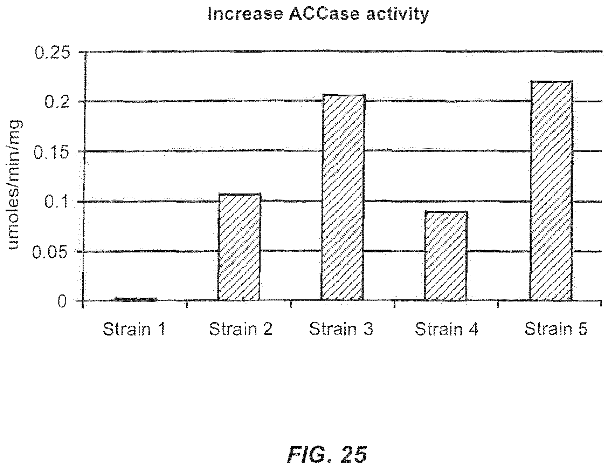

FIG. 25 shows increased ACCase activity for various strains.

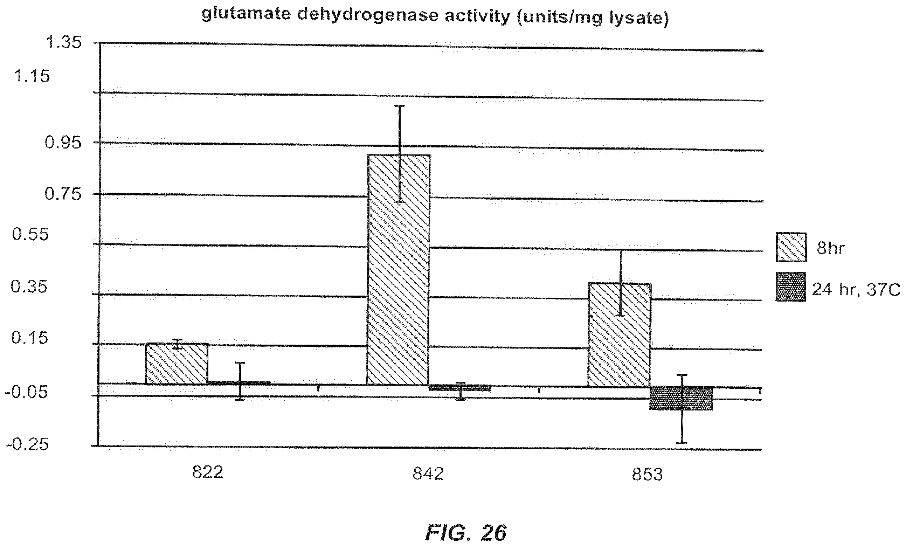

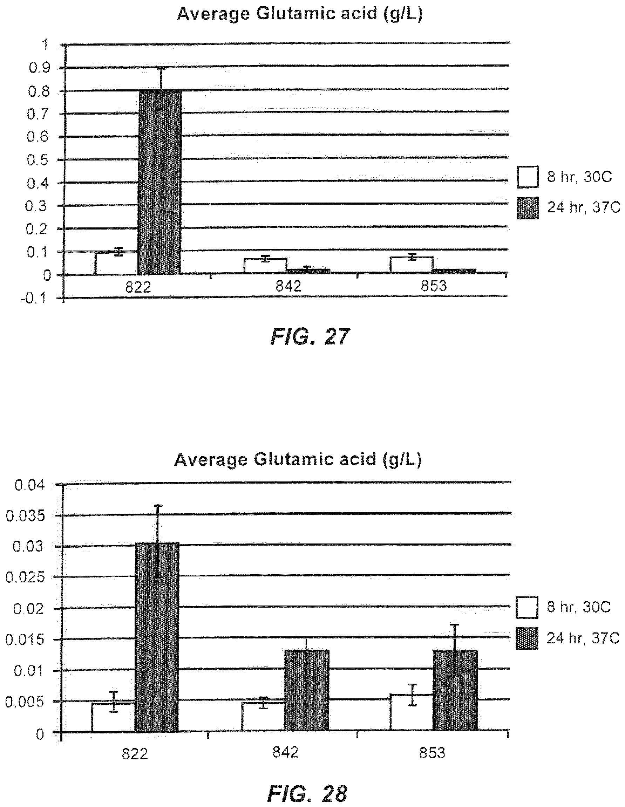

FIG. 26 shows glutmate dehydrogenase activity for strains with altered glutamate biosynthesis capabilities. In addition to the specific activity measurements, cultures grown at 30 degrees Celsius and shifted to 37 degree Celsius were also evaluated for their glutamate levels and there glutamine levels, a product derived from glutamate. Glutamate and glutamine levels can be determined in g/L using appropriate sensors such as those from YSI Incorporated using the manufactures instructions. Each sample was measured in triplicate. The glutamate and glutamine results are shown in Figures 27 and 28. These results show a significant decrease in glutamate and glutamine production from culture with strains carrying the *Psychrobacter sp. TAD1* gdh gene alone (853) or the gltB deletion and *Psychrobacter sp. TAD1* gdh gene in combination (842) upon shift to 37 Celsius when compared to the parent strain from which these two strains were derived (822).
FIG. 27: average glutamic acid production for strains with altered glutamate biosynthesis capabilities
FIG. 28: average glutamine production for strains with altered glutamate biosynthesis capabilities.
FIG. 29: Average specific productivity of 3-HP for a strain with altered glutamate biosynthesis
FIG. 30: Biochemical assays of the mcr from Sulfolobus tokodaii specific activity.

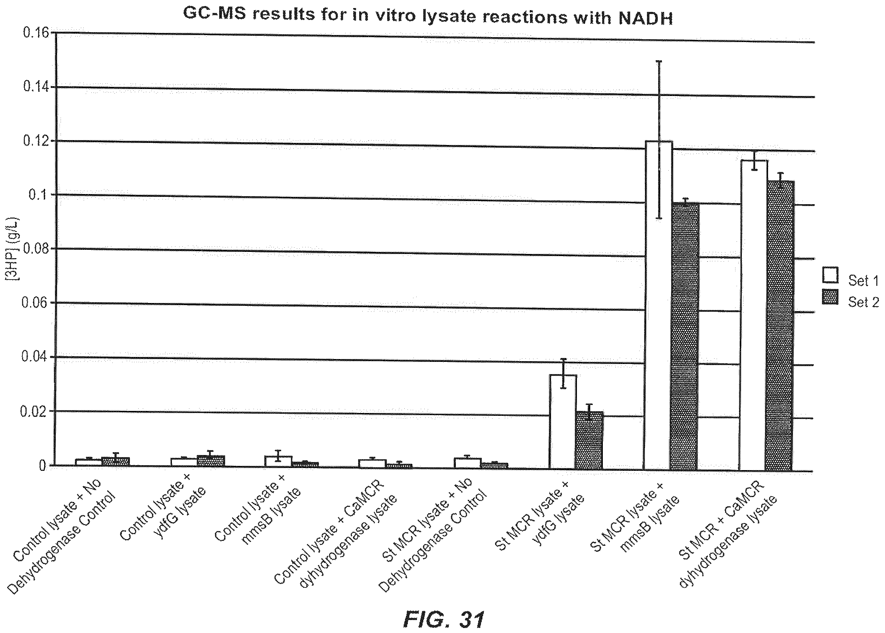

FIG. 31: GC-MS results for in vitro lysate reactions with NADH
FIG. 32: Flaviolin production measured in E. coli strains with fabI mutations complemented by *C*. *necator* fabI homologues

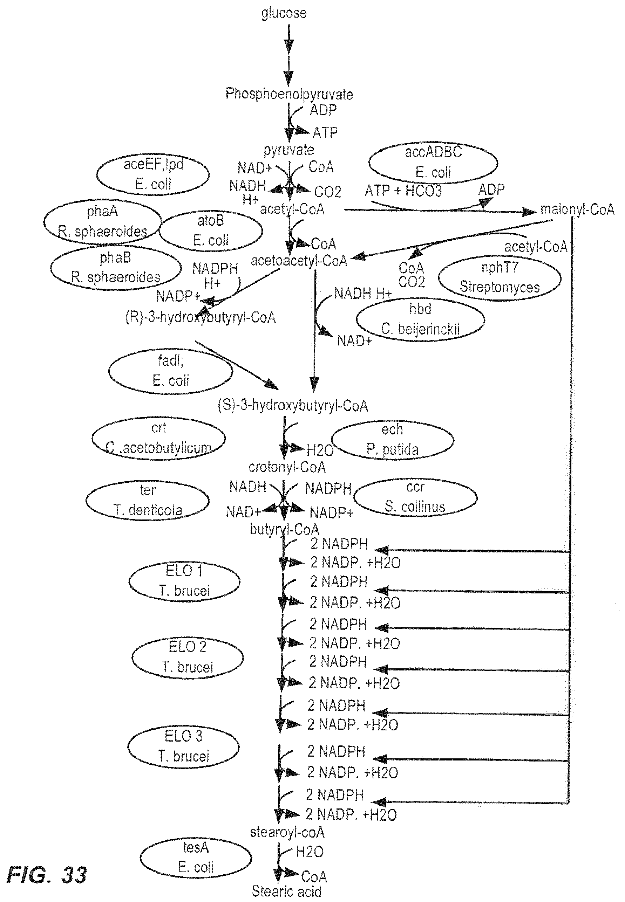

FIG. 33: elongase metabolic pathway
FIGs. 34A-C Process of converting biomass to a product.
FIGs. 35A-B Process of converting biomass to a product.

### DETAILED DESCRIPTION THE INVENTION

The present invention provides compositions and methods for production of malonyl-CoA derived products from genetically modified microorganisms. The microorganisms described herein have been engineered to increase yields and productivities to desired malonyl-CoA dependent chemical products by reducing carbon flow through native fatty acyl-ACP biosynthesis. More specifically, by reducing flux through native fatty acid synthesis a proportionally greater number of malonyl-CoA molecules are 1) produced and/or 2) converted via the metabolic pathway from malonyl-CoA to the selected chemical product. Disclosed and exemplified herewith are combinations of genetic modifications that are shown to provide unexpectedly elevated increases in productivity of a desired chemical product that is biosynthesized in a host cell with such genetic modifications. It was shown that certain combinations of genetic modifications would result in increased flux through malonyl-CoA, where any single modification alone may be enough to block the competing fatty acid synthesis pathway.

### I. DEFINITIONS

Definitions and abbreviations are as follows:
As used in the specification and the claims, the singular forms "a," "an," and "the" include plural referents unless the context clearly dictates otherwise. Thus, for example, reference to an "expression vector" includes a single expression vector as well as a plurality of expression vectors, either the same (e.g., the same operon) or different; reference to "microorganism" includes a single microorganism as well as a plurality of microorganisms; and the like.

"C" means Celsius or degrees Celsius, as is clear from its usage, DCW means dry cell weight, "s" means second(s), "min" means minute(s), "h," "hr," or "hrs" means hour(s), "psi" means pounds per square inch, "nm" means nanometers, "d" means day(s), "µL" or "µL" or "ul" means microliter(s), "mL" means milliliter(s), "L" means liter(s), "mm" means millimeter(s), "nm" means nanometers, "mM" means millimolar, "µM" or "uM" means micromolar, "M" means molar, "mmol" means millimole(s), "µmol" or "uMol" means micromole(s)", "g" means gram(s), "µg" or "ug" means microgram(s) and "ng" means nanogram(s), "PCR" means polymerase chain reaction, "OD" means optical density, "OD₆₀₀ means the optical density measured at a photon wavelength of 600 nm, "kDa" means kilodaltons, "g" means the gravitation constant, "bp" means base pair(s), "kbp" means kilobase pair(s), "% w/v" means weight/volume percent,"% v/v" means volume/volume percent, "IPTG" means isopropyl-µ-D-thiogalactopyranoiside, "RBS" means ribosome binding site, "rpm" means revolutions per minute, "HPLC" means high performance liquid chromatography, and "GC" means gas chromatography. Also, 10^5 and the like are taken to mean 10⁵ and the like.

As disclosed herein, "3-HP" and "3HP" means 3-hydroxypropionic acid.

As used herein, dry cell weight (DCW) for *E. coli* strains is calculated as 0.41 times the measured OD₆₀₀ value, based on baseline DCW to OD₆₀₀ determinations.

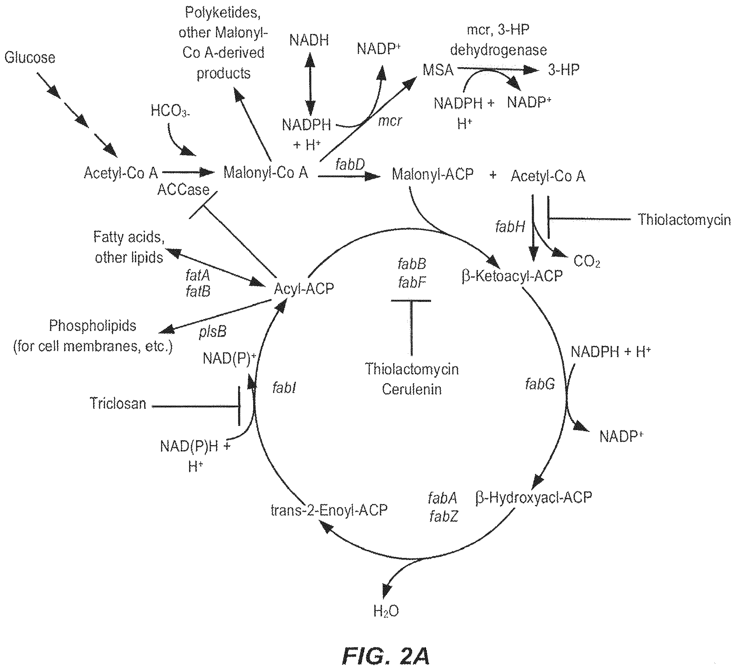

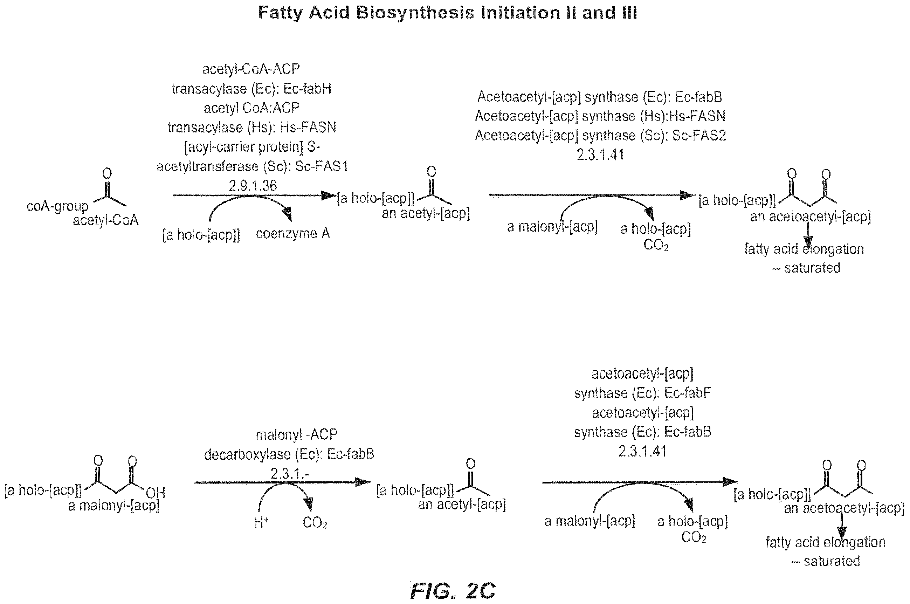

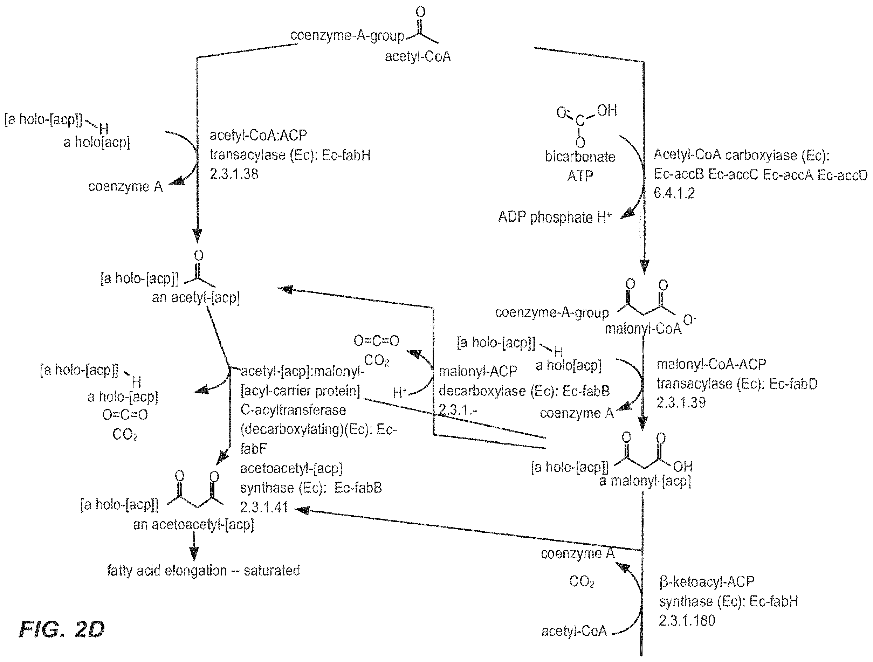

As used herein, "fatty acid synthase," whether followed by "pathway," "system," or "complex," is meant to refer to a metabolic pathway, often involving cyclic reactions to biosynthesize fatty acids in a host cell. For example, FIG. 2A depicts a fatty acid synthase pathway common to bacteria. It is noted that this may also be referred to as a "fatty acid synthesis," a "fatty acid biosynthesis," (or a "fatty acid synthetase") "pathway," "system," or "complex."

As used herein, "reduced enzymatic activity," "reducing enzymatic activity," and the like is meant to indicate that a microorganism cell's, or an isolated enzyme, exhibits a lower level of activity than that measured in a comparable cell of the same species or its native enzyme. That is, enzymatic conversion of the indicated substrate(s) to indicated product(s) under known standard conditions for that enzyme is at least 10, at least 20, at least 30, at least 40, at least 50, at least 60, at least 70, at least 80, or at least 90% less than the enzymatic activity for the same biochemical conversion by a native (non-modified) enzyme under a standard specified condition. This term also can include elimination of that enzymatic activity. A cell having reduced enzymatic activity of an enzyme can be identified using any method known in the art. For example, enzyme activity assays can be used to identify cells having reduced enzyme activity (see, for example, Enzyme Nomenclature, Academic Press, Inc., New York, NY 2007).

The term "reduction" or "to reduce" when used in such phrase and its grammatical equivalents are intended to encompass a complete elimination of such conversion(s).

The term "heterologous DNA," "heterologous nucleic acid sequence," and the like as used herein refers to a nucleic acid sequence wherein at least one of the following is true: (a) the sequence of nucleic acids is foreign to (i.e., not naturally found in) a given host microorganism; (b) the sequence may be naturally found in a given host microorganism, but in an unnatural (e.g., greater than expected) amount; or (c) the sequence of nucleic acids comprises two or more subsequences that are not found in the same relationship to each other in nature. For example, regarding instance (c), a heterologous nucleic acid sequence that is recombinantly produced will have two or more sequences from unrelated genes arranged to make a new functional nucleic acid.

The term "heterologous" is intended to include the term "exogenous" as the latter term is generally used in the art. With reference to the host microorganism's genome prior to the introduction of a heterologous nucleic acid sequence, the nucleic acid sequence that codes for the enzyme is heterologous (whether or not the heterologous nucleic acid sequence is introduced into that genome).

By "increase production," "increase the production," and like terms is meant to increase the quantity of one or more of enzymes, the enzymatic activity, the enzymatic specificity, and/or the overall flux through an enzymatic conversion step, biosynthetic pathway, or portion of a biosynthetic pathway. A discussion of non-limiting genetic modification techniques is discussed, infra, which may be used either for increasing or decreasing a particular enzyme's quantity, activity, specificity, flux, etc.

As used herein, the term "gene disruption," or grammatical equivalents thereof (and including "to disrupt enzymatic function," "disruption of enzymatic function," and the like), is intended to mean a genetic modification to a microorganism that renders the encoded gene product as having a reduced polypeptide activity compared with polypeptide activity in or from a microorganism cell not so modified. The genetic modification can be, for example, deletion of the entire gene, deletion or other modification of a regulatory sequence required for transcription or translation, deletion of a portion of the gene which results in a truncated gene product (e.g., enzyme) or by any of various mutation strategies that reduces activity (including to no detectable activity level) the encoded gene product. A disruption may broadly include a deletion of all or part of the nucleic acid sequence encoding the enzyme, and also includes, but is not limited to other types of genetic modifications, e.g., introduction of stop codons, frame shift mutations, introduction or removal of portions of the gene, and introduction of a degradation signal, those genetic modifications affecting mRNA transcription levels and/or stability, and altering the promoter or repressor upstream of the gene encoding the enzyme.

In various contexts, a gene disruption is taken to mean any genetic modification to the DNA, mRNA encoded from the DNA, and the corresponding amino acid sequence that results in reduced polypeptide activity. Many different methods can be used to make a cell having reduced polypeptide activity. For example, a cell can be engineered to have a disrupted regulatory sequence or polypeptide-encoding sequence using common mutagenesis or knock-out technology (see, e.g., Methods in Yeast Genetics (1997 edition), Adams et al., Cold Spring Harbor Press (1998)). One particularly useful method of gene disruption is complete gene deletion because it reduces or eliminates the occurrence of genetic reversions in the genetically modified microorganisms of the invention. Accordingly, a disruption of a gene whose product is an enzyme thereby disrupts enzymatic function. Alternatively, antisense technology can be used to reduce the activity of a particular polypeptide. For example, a cell can be engineered to contain an eDNA that encodes an antisense molecule that prevents a polypeptide from being translated. Further, gene silencing can be used to reduce the activity of a particular polypeptide.

The term "antisense molecule" as used herein encompasses any nucleic acid molecule or nucleic acid analog (e.g., peptide nucleic acids) that contains a sequence that corresponds to the coding strand of an endogenous polypeptide. An antisense molecule also can have flanking sequences (e.g., regulatory sequences). Thus, antisense molecules can be ribozymes or antisense oligonucleotides.

As used herein, a ribozyme can have any general structure including, without limitation, hairpin, hammerhead, or axhead structures, provided the molecule cleaves RNA.

Bio-production, as used herein, may be aerobic, microaerobic, or anaerobic.

As used herein, the language "sufficiently identical" refers to proteins or portions thereof that have amino acid sequences that include a minimum number of identical or equivalent amino acid residues when compared to an amino acid sequence of the amino acid sequences provided in this application (including the SEQ ID Nos./sequence listings) such that the protein or portion thereof is able to achieve the respective enzymatic reaction and/or other function. To determine whether a particular protein or portion thereof is sufficiently homologous may be determined by an assay of enzymatic activity, such as those commonly known in the art.

Descriptions and methods for sequence identity and homology are intended to be exemplary and it is recognized that these concepts are well-understood in the art. Further, it is appreciated that nucleic acid sequences may be varied and still encode an enzyme or other polypeptide exhibiting a desired functionality, and such variations are within the scope of the present invention. Also, it is intended that the phrase "equivalents thereof is mean to indicate functional equivalents of a referred to gene, enzyme or the like. Such an equivalent may be for the same species or another species, such as another microorganism species.

Further to nucleic acid sequences, a nucleic acid is "hybridizable" to another nucleic acid when a single stranded form of the nucleic acid can anneal to the other nucleic acid under appropriate conditions of temperature and solution ionic strength. Hybridization and washing conditions are well known and exemplified in Sambrook (1989), supra, (see in particular Chapters 9 and 11), incorporated by reference to such teachings. Low stringency hybridization conditions correspond to a Tm of 55°C (for example 5xSSC, 0.1% SDS, 0.25 milk and no formamide or 5xSSC, 0.5% SDS and 30% formamide). Moderate stringency hybridization conditions correspond for example, to Tm of 60°C (for example 6xSSC, 0.1% SDS, 0.05% milk with or without formamide, and stringent hybridization conditions correspond for example, to a Tm of 65°C. and 0.1xSSC and 0.1% SDS. For various embodiments of the invention a sequence of interest may be hybridizable under any such stringency condition - low, moderate or high.

The term "identified enzymatic functional variant" means a polypeptide that is determined to possess an enzymatic activity and specificity of an enzyme of interest but which has an amino acid sequence different from such enzyme of interest. A corresponding "variant nucleic acid sequence" may be constructed that is determined to encode such an identified enzymatic functional variant. These may be identified and/or developed from orthologs, paralogs, or nonorthologous gene displacements.

The use of the phrase "segment of interest" is meant to include both a gene and any other nucleic acid sequence segment of interest. One example of a method used to obtain a segment of interest is to acquire a culture of a microorganism, where that microorganism's genome includes the gene or nucleic acid sequence segment of interest.

When the genetic modification of a gene product, i.e., an enzyme, is referred to herein, including the claims, it is understood that the genetic modification is of a nucleic acid sequence, such as or including the gene, that normally encodes the stated gene product, i.e., the enzyme.

By "means for modulating" the conversion of malonyl-CoA to fatty acyl-ACP or fatty acyl-coA molecules, and to fatty acid molecules, is meant any one of the following: 1) providing in a microorganism cell at least one polynucleotide that encodes at least one polypeptide having activity of one of the fatty acid synthase system enzymes (such as recited herein), wherein the polypeptide so encoded has (such as by mutation and/or promoter substitution, etc., to lower enzymatic activity), or may be modulated to have (such as by temperature sensitivity, inducible promoter, etc.) a reduced enzymatic activity; 2) providing to a vessel comprising a microorganism cell or population an inhibitor that inhibits enzymatic activity of one or more of the fatty acid synthase system enzymes (such as recited herein), at a dosage effective to reduce enzymatic activity of one or more of these enzymes. These means may be provided in combination with one another. When a means for modulating involves a conversion, during a fermentation event, from a higher to a lower activity of the fatty acid synthetase system, such as by increasing temperature of a culture vessel comprising a population of genetically modified microorganism comprising a temperature-sensitive fatty acid synthetase system polypeptide (e.g., enoyl-ACP reductase), or by adding an inhibitor, there are conceived two modes - one during which there is higher activity, and a second during which there is lower activity, of such fatty acid synthetase system. During the lower activity mode, a shift to greater utilization of malonyl-CoA to a selected chemical product may proceed.

"CoA" means coenzyme-A, and "mcr" means malonyl-CoA reductase.

A "temperature-sensitive mutation" or "(ts)" refers to a modified gene product that can be induced to express the modified RNA at specific temperature.

### II. ORGANISMS

Features as described and claimed herein may be provided in a microorganism selected from the listing herein, or another suitable microorganism, that also comprises one or more natural, introduced, or enhanced chemical product biosynthesis pathway. Thus, in some embodiments the microorganism comprises an endogenous chemical product biosynthesis pathway (which may, in some such embodiments, be enhanced), whereas in other embodiments the microorganism does not comprise an endogenous biosynthesis pathway for the selected chemical product.

Varieties of these genetically modified microorganisms may comprise genetic modifications and/or other system alterations as may be described in other patent applications of one or more of the present inventor(s) and/or subject to assignment or license to the owner of the present patent application.

The examples describe specific modifications and evaluations to certain bacterial and yeast microorganisms. The scope of the invention is not meant to be limited to such species, but to be generally applicable to a wide range of suitable microorganisms. Generally, a microorganism used for the present invention may be selected from bacteria, cyanobacteria, filamentous fungi and yeasts.

For some embodiments, microbial hosts initially selected for a selected chemical product biosynthesis should also utilize sugars including glucose at a high rate. Most microbes are capable of utilizing carbohydrates. However, certain environmental microbes cannot utilize carbohydrates to high efficiency, and therefore would not be suitable hosts for such embodiments that are intended for glucose or other carbohydrates as the principal added carbon source.

As the genomes of various species become known, the present invention easily may be applied to an ever-increasing range of suitable microorganisms. Further, given the relatively low cost of genetic sequencing, the genetic sequence of a species of interest may readily be determined to make application of aspects of the present invention more readily obtainable (based on the ease of application of genetic modifications to an organism having a known genomic sequence). Public database sites, such as <<www.metacyc.org>>, <<www.ecocyc.org>>, <<www.biocyc.org>>, and <<www.ncbi.gov>>, << htpp://www.nchi.nlm.nih.gov/>> have various genetic and genomic information and associated tools to identify enzymes in various species that have desired function or that may be modified to achieve such desired function.

More particularly, based on the various criteria described herein, suitable microbial hosts for the biosynthesis of a chemical product generally may include, but are not limited to, any gram negative organisms, more particularly a member of the family Enterobacteriaceae, such as E. coli, or Oligotropha carboxidovorans, or Pseudomononas sp.; any gram positive microorganism, for example Bacillus subtilis, Lactobaccilus sp. or Lactococcus sp.; a yeast, for example Saccharomyces cerevisiae, Pichia pastoris or Pichia stipitis; and other groups or microbial species including those found in Actinomycetes (also referred to as Actinobacteria). More particularly, suitable microbial hosts for the biosynthesis of a chemical product generally include, but are not limited to, members of the genera Clostridium, Zymomonas, Escherichia, Salmonella, Rhodococcus, Pseudomonas, Bacillus, Lactobacillus, Enterococcus, Alcaligenes, Klebsiella, Paenibacillus,Arthrobacter, Corynebacterium, Brevibacterium, Pichia, Candida, Hansenula and Saccharomyces. Hosts that may be particularly of interest include: Oligotropha carboxidovorans (such as strain OMS), Escherichia coli, Alcaligenes eutrophus (Cupriavidus necator), Bacillus licheniformis, Paenibacillus macerans, Rhodococcus erythropolis, Pseudomonas putida, Lactobacillus plantarum, Enterococcus faecium, Enterococcus gallinarium, Enterococcus faecalis, Bacillus subtilis and Saccharomyces cerevisiae.

More particularly, suitable microbial hosts for the biosynthesis of selected chemical products generally include, but are not limited to, members of the genera Clostridium, Zymomonas, Escherichia, Salmonella, Rhodococcus, Pseudomonas, Bacillus, Lactobacillus, Enterococcus, Alcaligenes, Klebsiella, Paenibacillus, Arthrobacter, Corynebacterium, Brevibacterium, Pichia, Candida, Hansenula and Saccharomyces.

Hosts that may be particularly of interest include: Oligotropha carboxidovorans (such as strain 0M5T), Escherichia coli, Alcaligenes eutrophus (Cupriavidus necator), Bacillus licheniformis, Paenibacillus macerans, Rhodococcus erythropolis, Pseudomonas putida, Lactobacillus plantarum, Enterococcus faecium, Enterococcus gallinarium, Enterococcus faecalis, Bacillus subtilis and Saccharomyces cerevisiae. Also, any of the known strains of these species may be utilized as a starting microorganism, as may any of the following species including respective strains thereof- Cupriavidus basilensis, Cupriavidus campinensis, Cupriavidus gilardi, Cupriavidus laharsis, Cupriavidus metallidurans, Cupriavidus oxalaticus, Cupriavidus pauculus, Cupriavidus pinatubonensis, Cupriavidus respiraculi, and Cupriavidus taiwanensis.

In some embodiments, the recombinant microorganism is a gram-negative bacterium. In some embodiments, the recombinant microorganism is selected from the genera Zymomonas, Escherichia, Pseudomonas, Alcaligenes, and Klebsiella. In some embodiments, the recombinant microorganism is selected from the species Escherichia coli, Cupriavidus necator, Oligotropha carboxidovorans, and Pseudomonas putida. In some embodiments, the recombinant microorganism is an E. coli strain.

In some embodiments, the recombinant microorganism is a gram-positive bacterium. In some embodiments, the recombinant microorganism is selected from the genera Clostridium, Salmonella, Rhodococcus, Bacillus, Lactobacillus, Enterococcus, Paenibacillus, Arthrobacter, Corynebacterium, and Brevibacterium. In some embodiments, the recombinant microorganism is selected from the species Bacillus licheniformis, Paenibacillus macerans, Rhodococcus erythropolis, Lactobacillus plantarum, Enterococcus faecium, Enterococcus gallinarium, Enterococcus faecalis, and Bacillus subtilis. In particular embodiments, the recombinant microorganism is a B. subtilis strain.

In some embodiments, the recombinant microorganism is a yeast. In some embodiments, the recombinant microorganism is selected from the genera Pichia, Candida, Hansenula and Saccharomyces. In particular embodiments, the recombinant microorganism is Saccharomyces cerevisiae.

The ability to genetically modify the host is essential for the production of any recombinant microorganism. The mode of gene transfer technology may be by electroporation, conjugation, transduction or natural transformation. A broad range of host conjugative plasmids and drug resistance markers are available. The cloning vectors are tailored to the host organisms based on the nature of antibiotic resistance markers that can function in that host.

### III. CARBON SOURCES/GROWTH MEDIA/BIOREACTORS

### Carbon Sources

Bio-production media, which is used in the present invention with recombinant microorganisms having a biosynthetic pathway for 3-HP, must contain suitable carbon sources or substrates for the intended metabolic pathways. Suitable substrates may include, but are not limited to, monosaccharides such as glucose and fructose, oligosaccharides such as lactose or sucrose, polysaccharides such as starch or cellulose or mixtures thereof and unpurified mixtures from renewable feedstocks such as cheese whey permeate, cornsteep liquor, sugar beet molasses, and barley malt. Additionally the carbon substrate may also be one-carbon substrates such as carbon dioxide, carbon monoxide, or methanol for which metabolic conversion into key biochemical intermediates has been demonstrated. In addition to one and two carbon substrates methylotrophic organisms are also known to utilize a number of other carbon containing compounds such as methylamine, glucosamine and a variety of amino acids for metabolic activity.

Although it is contemplated that all of the above mentioned carbon substrates and mixtures thereof are suitable in the present invention as a carbon source, common carbon substrates used as carbon sources are glucose, fructose, and sucrose, as well as mixtures of any of these sugars. Other suitable substrates include xylose, arabinose, other cellulose-based C-5 sugars, high-fructose corn syrup, and various other sugars and sugar mixtures as are available commercially. Sucrose may be obtained from feedstocks such as sugar cane, sugar beets, cassava, bananas or other fruit, and sweet sorghum. Glucose and dextrose may be obtained through saccharification of starch based feedstocks including grains such as corn, wheat, rye, barley, and oats. Also, in some embodiments all or a portion of the carbon source may be glycerol. Alternatively, glycerol may be excluded as an added carbon source.

In one embodiment, the carbon source is selected from glucose, fructose, sucrose, dextrose, lactose, glycerol, and mixtures thereof. Variously, the amount of these components in the carbon source may be greater than about 50%, greater than about 60%, greater than about 70%, greater than about 80%, greater than about 90%, or more, up to 100% or essentially 100% of the carbon source.

In addition, methylotrophic organisms are known to utilize a number of other carbon containing compounds such as methylamine, glucosamine and a variety of amino acids for metabolic activity. For example, methylotrophic yeast are known to utilize the carbon from methylamine to form trehalose or glycerol (Bellion et al., Microb. Growth Cl Compd. (Int. Symp.), 7th (1993), 415-32. Editor(s): Murrell, J. Collin; Kelly, Don P. Publisher: Intercept, Andover, UK). Similarly, various species of Candida will metabolize alanine or oleic acid (Sulter et al., Arch. Microbiol. 153:485-489 (1990)). Hence it is contemplated that the source of carbon utilized in embodiments of the present invention may encompass a wide variety of carbon-containing substrates.

In addition, fermentable sugars may be obtained from cellulosic and lignocellulosic biomass through processes of pretreatment and saccharification, as described, for example, in U.S. Patent Publication No. 2007/0031918A1, which is herein incorporated by reference. Biomass refers to any cellulosic or lignocellulosic material and includes materials comprising cellulose, and optionally further comprising hemicellulose, lignin, starch, oligosaccharides and/or monosaccharides. Biomass may also comprise additional components, such as protein and/or lipid. Biomass may be derived from a single source, or biomass can comprise a mixture derived from more than one source; for example, biomass could comprise a mixture of corn cobs and corn stover, or a mixture of grass and leaves. Biomass includes, but is not limited to, bioenergy crops, agricultural residues, municipal solid waste, industrial solid waste, sludge from paper manufacture, yard waste, wood and forestry waste. Examples of biomass include, but are not limited to, corn grain, corn cobs, crop residues such as corn husks, corn stover, grasses, wheat, wheat straw, barley, barley straw, hay, rice straw, switchgrass, waste paper, sugar cane bagasse, sorghum, soy, components obtained from milling of grains, trees, branches, roots, leaves, wood chips, sawdust, shrubs and bushes, vegetables, fruits, flowers and animal manure. Any such biomass may be used in a bio-production method or system to provide a carbon source. Various approaches to breaking down cellulosic biomass to mixtures of more available and utilizable carbon molecules, including sugars, include: heating in the presence of concentrated or dilute acid (e.g., < 1% sulfuric acid); treating with ammonia; treatment with ionic salts; enzymatic degradation; and combinations of these. These methods normally follow mechanical separation and milling, and are followed by appropriate separation processes.

In various embodiments, any of a wide range of sugars, including, but not limited to sucrose, glucose, xylose, cellulose or hemicellulose, are provided to a microorganism, such as in an industrial system comprising a reactor vessel in which a defined media (such as a minimal salts media including but not limited to M9 minimal media, potassium sulfate minimal media, yeast synthetic minimal media and many others or variations of these), an inoculum of a microorganism providing one or more of the 3-HP biosynthetic pathway alternatives, and the a carbon source may be combined. The carbon source enters the cell and is cataboliized by well-known and common metabolic pathways to yield common metabolic intermediates, including phosphoenolpyruvate (PEP). (See Molecular Biology of the Cell, 3rd Ed., B. Alberts et al. Garland Publishing, New York, 1994, pp. 42-45, 66-74, incorporated by reference for the teachings of basic metabolic catabolic pathways for sugars; Principles of Biochemistry, 3rd Ed., D. L. Nelson & M. M. Cox, Worth Publishers, New York, 2000, pp 527-658, incorporated by reference for the teachings of major metabolic pathways; and Biochemistry, 4th Ed., L. Stryer, W. H. Freeman and Co., New York, 1995, pp. 463-650, also incorporated by reference for the teachings of major metabolic pathways.)

Bio-based carbon can be distinguished from petroleum-based carbon according to a variety of methods, including without limitation ASTM D6866, or various other techniques. For example, carbon-14 and carbon-12 ratios differ in bio-based carbon sources versus petroleum-based sources, where higher carbon-14 ratios are found in bio-based carbon sources. In various embodiments, the carbon source is not petroleum-based, or is not predominantly petroleum based. In various embodiments, the carbon source is greater than about 50% non-petroleum based, greater than about 60% non-petroleum based, greater than about 70% non-petroleum based, greater than about 80% non-petroleum based, greater than about 90% non-petroleum based, or more. In various embodiments, the carbon source has a carbon-14 to carbon-12 ratio of about 1.0 x 10-14 or greater.

Various components may be excluded from the carbon source. For example, in some embodiments, acrylic acid, 1,4-butanediol, and/or glycerol are excluded or essentially excluded from the carbon source. As such, the carbon source according to some embodiments of the invention may be less than about 50% glycerol, less than about 40% glycerol, less than about 30% glycerol, less than about 20% glycerol, less than about 10% glycerol, less than about 5% glycerol, less than about 1% glycerol, or less. For example, the carbon source may be essentially glycerol-free. By essentially glycerol-free is meant that any glycerol that may be present in a residual amount does not contribute substantially to the production of the target chemical compound.

### Growth Media

In addition to an appropriate carbon source, such as selected from one of the herein-disclosed types, bio-production media must contain suitable minerals, salts, cofactors, buffers and other components, known to those skilled in the art, suitable for the growth of the cultures and promotion of the enzymatic pathway necessary for 3-HP production, or other products made under the present invention.

Another aspect of the invention regards media and culture conditions that comprise genetically modified microorganisms of the invention and optionally supplements.

Typically cells are grown at a temperature in the range of about 25° C to about 40° C in an appropriate medium, as well as up to 70° C for thermophilic microorganisms. Suitable growth media in the present invention are common commercially prepared media such as Luria Bertani (LB) broth, M9 minimal media, Sabouraud Dextrose (SD) broth, Yeast medium (YM) broth, (Ymin) yeast synthetic minimal media, and minimal media as described herein, such as M9 minimal media. Other defined or synthetic growth media may also be used, and the appropriate medium for growth of the particular microorganism will be known by one skilled in the art of microbiology or bio-production science. In various embodiments a minimal media may be developed and used that does not comprise, or that has a low level of addition of various components, for example less than 10, 5, 2 or 1 g/L of a complex nitrogen source including but not limited to yeast extract, peptone, tryptone, soy flour, corn steep liquor, or casein. These minimal medias may also have limited supplementation of vitamin mixtures including biotin, vitamin B12 and derivatives of vitamin B12, thiamin, pantothenate and other vitamins. Minimal medias may also have limited simple inorganic nutrient sources containing less than 28, 17, or 2.5 mM phosphate, less than 25 or 4 mM sulfate, and less than 130 or 50mM total nitrogen.

Bio-production media, which is used in embodiments of the present invention with genetically modified microorganisms, must contain suitable carbon substrates for the intended metabolic pathways. As described hereinbefore, suitable carbon substrates include carbon monoxide, carbon dioxide, and various monomeric and oligomeric sugars.

Suitable pH ranges for the bio-production are between pH 3.0 to pH 10.0, where pH 6.0 to pH 8.0 is a typical pH range for the initial condition. However, the actual culture conditions for a particular embodiment are not meant to be limited by these pH ranges.

Bio-productions may be performed under aerobic, microaerobic, or anaerobic conditions, with or without agitation.

### Bioreactors

Fermentation systems utilizing methods and/or compositions according to the invention are also within the scope of the invention.

Any of the recombinant microorganisms as described and/or referred to herein may be introduced into an industrial bio-production system where the microorganisms convert a carbon source into a selected chemical product, such as 3-HP or a polyketide such as described herein (including in priority document(s)), in a commercially viable operation. The bio-production system includes the introduction of such a recombinant microorganism into a bioreactor vessel, with a carbon source substrate and bio-production media suitable for growing the recombinant microorganism, and maintaining the bio-production system within a suitable temperature range (and dissolved oxygen concentration range if the reaction is aerobic or microaerobic) for a suitable time to obtain a desired conversion of a portion of the substrate molecules to 3-HP. Industrial bio-production systems and their operation are well-known to those skilled in the arts of chemical engineering and bioprocess engineering.

Bio-productions may be performed under aerobic, microaerobic, or anaerobic conditions, with or without agitation. The operation of cultures and populations of microorganisms to achieve aerobic, microaerobic and anaerobic conditions are known in the art, and dissolved oxygen levels of a liquid culture comprising a nutrient media and such microorganism populations may be monitored to maintain or confirm a desired aerobic, microaerobic or anaerobic condition. When syngas is used as a feedstock, aerobic, microaerobic, or anaerobic conditions may be utilized. When sugars are used, anaerobic, aerobic or microaerobic conditions can be implemented in various embodiments.

Any of the recombinant microorganisms as described and/or referred to herein may be introduced into an industrial bio-production system where the microorganisms convert a carbon source into 3-HP, and optionally in various embodiments also to one or more downstream compounds of 3-HP in a commercially viable operation. The bio-production system includes the introduction of such a recombinant microorganism into a bioreactor vessel, with a carbon source substrate and bio-production media suitable for growing the recombinant microorganism, and maintaining the bio-production system within a suitable temperature range (and dissolved oxygen concentration range if the reaction is aerobic or microaerobic) for a suitable time to obtain a desired conversion of a portion of the substrate molecules to 3-HP.

In various embodiments, syngas components or sugars are provided to a microorganism, such as in an industrial system comprising a reactor vessel in which a defined media (such as a minimal salts media including but not limited to M9 minimal media, potassium sulfate minimal media, yeast synthetic minimal media and many others or variations of these), an inoculum of a microorganism providing an embodiment of the biosynthetic pathway(s) taught herein, and the carbon source may be combined. The carbon source enters the cell and is catabolized by well-known and common metabolic pathways to yield common metabolic intermediates, including phosphoenolpyruvate (PEP). (See Molecular Biology of the Cell, 3rd Ed., B. Alberts et al. Garland Publishing, New York, 1994, pp. 42-45, 66-74, incorporated by reference for the teachings of basic metabolic catabolic pathways for sugars; Principles of Biochemistry, 3'd Ed., D. L. Nelson & M. M. Cox, Worth Publishers, New York, 2000, pp. 527-658, incorporated by reference for the teachings of major metabolic pathways; and Biochemistry, 4th Ed., L. Stryer, W. H. Freeman and Co., New York, 1995, pp. 463-650, also incorporated by reference for the teachings of major metabolic pathways.).

Further to types of industrial bio-production, various embodiments of the present invention may employ a batch type of industrial bioreactor. A classical batch bioreactor system is considered "closed" meaning that the composition of the medium is established at the beginning of a respective bio-production event and not subject to artificial alterations and additions during the time period ending substantially with the end of the bio-production event. Thus, at the beginning of the bio-production event the medium is inoculated with the desired organism or organisms, and bio-production is permitted to occur without adding anything to the system. Typically, however, a "batch" type of bio-production event is batch with respect to the addition of carbon source and attempts are often made at controlling factors such as pH and oxygen concentration. In batch systems the metabolite and biomass compositions of the system change constantly up to the time the bio¬production event is stopped. Within batch cultures cells moderate through a static lag phase to a high growth log phase and finally to a stationary phase where growth rate is diminished or halted. If untreated, cells in the stationary phase will eventually die. Cells in log phase generally are responsible for the bulk of production of a desired end product or intermediate.

A variation on the standard batch system is the fed-batch system. Fed-batch bio-production processes are also suitable in the present invention and comprise a typical batch system with the exception that the nutrients, including the substrate, are added in increments as the bio-production progresses. Fed-Batch systems are useful when catabolite repression is apt to inhibit the metabolism of the cells and where it is desirable to have limited amounts of substrate in the media. Measurement of the actual nutrient concentration in Fed-Batch systems may be measured directly, such as by sample analysis at different times, or estimated on the basis of the changes of measurable factors such as pH, dissolved oxygen and the partial pressure of waste gases such as CO2. Batch and fed-batch approaches are common and well known in the art and examples may be found in Thomas D. Brock in Biotechnology: A Textbook of Industrial Microbiology, Second Edition (1989) Sinauer Associates, Inc., Sunderland, Mass., Deshpande, Mukund V., Appl. Biochem. Biotechnol., 36:227, (1992), and Biochemical Engineering Fundamentals, 2'd Ed. J. E. Bailey and D. F. 011is, McGraw Hill, New York, 1986, herein incorporated by reference for general instruction on bio-production.

Although embodiments of the present invention may be performed in batch mode, or in fed-batch mode, it is contemplated that the invention would be adaptable to continuous bio-production methods. Continuous bio-production is considered an "open" system where a defined bio-production medium is added continuously to a bioreactor and an equal amount of conditioned media is removed simultaneously for processing. Continuous bio-production generally maintains the cultures within a controlled density range where cells are primarily in log phase growth. Two types of continuous bioreactor operation include a chemostat, wherein fresh media is fed to the vessel while simultaneously removing an equal rate of the vessel contents. The limitation of this approach is that cells are lost and high cell density generally is not achievable. In fact, typically one can obtain much higher cell density with a fed-batch process. Another continuous bioreactor utilizes perfusion culture, which is similar to the chemostat approach except that the stream that is removed from the vessel is subjected to a separation technique which recycles viable cells back to the vessel. This type of continuous bioreactor operation has been shown to yield significantly higher cell densities than fed-batch and can be operated continuously. Continuous bio-production is particularly advantageous for industrial operations because it has less down time associated with draining, cleaning and preparing the equipment for the next bio-production event. Furthermore, it is typically more economical to continuously operate downstream unit operations, such as distillation, than to run them in batch mode.

Continuous bio-production allows for the modulation of one factor or any number of factors that affect cell growth or end product concentration. For example, one method will maintain a limiting nutrient such as the carbon source or nitrogen level at a fixed rate and allow all other parameters to moderate. In other systems a number of factors affecting growth can be altered continuously while the cell concentration, measured by media turbidity, is kept constant. Methods of modulating nutrients and growth factors for continuous bio-production processes as well as techniques for maximizing the rate of product formation are well known in the art of industrial microbiology and a variety of methods are detailed by Brock, supra.

It is contemplated that embodiments of the present invention may be practiced using either batch, fed-batch or continuous processes and that any known mode of bio-production would be suitable. It is contemplated that cells may be immobilized on an inert scaffold as whole cell catalysts and subjected to suitable bio¬production conditions for 3-HP production, or be cultured in liquid media in a vessel, such as a culture vessel. Thus, embodiments used in such processes, and in bio-production systems using these processes, include a population of genetically modified microorganisms of the present invention, a culture system comprising such population in a media comprising nutrients for the population, and methods of making 3-HP and thereafter, a downstream product of 3-HP.

Embodiments of the invention include methods of making 3-HP in a bio-production system, some of which methods may include obtaining 3-HP after such bio-production event. For example, a method of making 3-HP may comprise: providing to a culture vessel a media comprising suitable nutrients; providing to the culture vessel an inoculum of a genetically modified microorganism comprising genetic modifications described herein such that the microorganism produces 3-HP from syngas and/or a sugar molecule; and maintaining the culture vessel under suitable conditions for the genetically modified microorganism to produce 3-HP.

Also, it is within the scope of the present invention to produce, and to utilize in bio-production methods and systems, including industrial bio-production systems for production of a selected chemical product (chemical), a recombinant microorganism genetically engineered to modify one or more aspects effective to increase chemical product bio-production by at least 20 percent over control microorganism lacking the one or more modifications.

In various embodiments, the invention is directed to a system for bio-production of a chemical product as described herein, said system comprising: a fermentation tank suitable for microorganism cell culture; a line for discharging contents from the fermentation tank to an extraction and/or separation vessel; and an extraction and/or separation vessel suitable for removal of the chemical product from cell culture waste. In various embodiments, the system includes one or more pre-fermentation tanks, distillation columns, centrifuge vessels, back extraction columns, mixing vessels, or combinations thereof.

The following published resources are incorporated by reference herein for their respective teachings to indicate the level of skill in these relevant arts, and as needed to support a disclosure that teaches how to make and use methods of industrial bio-production of 3-HP, or other product(s) produced under the invention, from sugar sources, and also industrial systems that may be used to achieve such conversion with any of the recombinant microorganisms of the present invention (Biochemical Engineering Fundamentals, 2nd Ed. J. E. Bailey and D. F. 011is, McGraw Hill, New York, 1986, entire book for purposes indicated and Chapter 9, pages 533-657 in particular for biological reactor design; Unit Operations of Chemical Engineering, 5th Ed., W. L. McCabe et al., McGraw Hill, New York 1993, entire book for purposes indicated, and particularly for process and separation technologies analyses; Equilibrium Staged Separations, P. C. Wankat, Prentice Hall, Englewood Cliffs, NJ USA, 1988, entire book for separation technologies teachings). Generally, it is appreciated, in view of the disclosure, that any of the above methods and systems may be used for production of various chemical products such as those disclosed herein.

### IV. PRODUCTS

In various embodiments the compositions, methods and systems of the present invention involve inclusion of a metabolic production pathway that converts malonyl-CoA to a chemical product of interest.

One chemical product is 3-hydroxypropionic acid (CAS No. 503-66-2, "3-HP"). Chemical products further include tetracycline, erythromycin, avermectin, macrolides, vanomycin-group antibiotics, Type II polyketides, (5R)-carbapenem, 6-methoxymellein, acridone, actinorhodin, aloesone, apigenin, barbaloin, biochanin A, maackiain, medicarpin, cannabinoid, cohumulone, daidzein, flavonoid, formononetin, genistein, humulone, hyperforin, mycolate, olivetol, pelargonidin, pentaketide chromone, pinobanksin, pinosylvin, plumbagin, raspberry ketone, resveratrol, rifamycin B, salvianin, shisonin, sorgoleone, stearate, anthocyanin, ternatin, tetrahydroxyxanthone, usnate, and xanthohumol. Particular polyketide chemical products include 1,3,6,8- tetrahydroxynaphthalene (THN) or its derivative flaviolin (CAS No. 479-05-0). The production of 3-HP, or of THN or flaviolin, may be used herein to demonstrate the features of the invention as they may be applied to other chemical products. Alternatively, any of the above compounds may be excluded from a group of chemical products.

Numerous products can be made from malonyl-coA precursors alone, by expressing enzyme functions to convert malonyl-coA into products. Several examples of these non-limiting products are shown below in Table 1A. Any of the strains discussed in the specification that increase flux through malonyl-coA can be used to produce these products. Hexaketide pyrone can be made by expressing a hexaketide pyrone synthase from either Aloe arborescens or Plumbago indica. Octaketide 4b pyrone can be made by expressing an octaketide 4b pyrone synthase from Aloe arborescens. Octaketide can be made by expressing an octaketide synthase from Hypericum perforatum. Pentaketide chromone can be made by expressing a pentaketide chromone synthase from Aloe arborescens. 3-hydroxypropionic acid can be made by expressing a malonyl-coA reductase and 3-hydroxypropionic acid dehydrogenase from various sources.

**Table 1A: Products requiring malonyl-coA precursors alone**

| **Chemical product** | **Biosynthesis** |
|---|---|
| 3,5,7,9,11,13,15-hepta-oxo-hexadecanoyl-[PKS acp] | 8 malonyl-CoA+a polyketide synthase containing an [acp] domain-> a 3,5,7,9,11,13,15-hepta-oxo-hexadecanoyl-[PKS acp] + 8 CO2 + 8 coenzyme A |
| octoketide 4b | 8 malonyl-CoA+ 4 H2 = octoketide 4b + 8 CO2 + 8 coenzyme A+ H2O + H+ |
| octoketide | 8 malonyl-CoA+ 4 H2 = octoketide + 8 CO2 + 8 coenzyme A+ H2O + H+ |
| heptaketide pyrone | 7 malonyl-CoA+ 4 H2 = heptaketide pyrone+ 7 CO2 + 7 coenzyme A+ H2O + 2 H+ |
| hexaketide pyrone | 6 malonyl-CoA+ 3 H2 = hexaketide pyrone+ 6 CO2 + 6 coenzyme A+ H2O + H+ |
| pentaketide chromone | 5 malonyl-CoA+ 5 H+ = 5,7-dihydroxy-2-methylchromone + 5 CO2+ 5 coenzyme A+ H2O |
| 3-hydroxypropionate | malonyl-CoA+ NADPH + H+ =malonate semialdehyde + NADP+ +coenzyme A |

Furthermore, numerous polyketide products can be made from malonyl-coA precursors in combination with acetyl-coA precursors. As acetyl-coA is a precursor of malonyl-coA, no additional modifications to a malonyl-coA producing strain are needed. Products can be made by expressing enzyme functions to convert malonyl-coA in addition to acetyl-coA into products. Several examples of these non-limiting products are shown below in Table 1B. Any of the strains discussed in the specification that increase flux through malonyl-coA can be used to produce these products. 6-hydroxymellein can be made by expressing a 6-hydroxymellein synthase from either Aloe or Daucus carota. Aloesone can be made by expressing an aloesone synthase from either Aloe arborescens or Rheum palmatum. Olivetolic acid can be made by expressing an olivetolic acid synthase. Naphthylisoquinoline alkaloid precursor (a precursor to plumbagine) can be made by expressing a polyketide pyrone synthase such as PKS G-11468 from Plumbago indica. 6-methylsalicylate can be made by expressing a 6-methylsalicylate synthase from Penicillium species. Triacetic acid lactone can be made by expressing a triacetic acid lactone synthase.

**Table 1B: Products requiring malonyl-coA and acetyl-coA precursors**

| **Chemical product** | **Biosynthesis** |
|---|---|
| 6-hydroxymellein | acetyl-CoA + 4 malonyl-CoA+ NADPH + 5 H+ = 6-hydroxymellein + 4 CO2 + NADP+ + 5 coenzyme A+ H2O |
| aloesone | acetyl-CoA + 6 malonyl-CoA+ 6 H+ = aloesone + 7 CO2 + 7 coenzyme A+ H2O |
| olivetolic acid (cannabinoid) | acetyl-CoA + 5 malonyl-CoA+ 12 H+ = olivetolic acid+ 5 CO2 + 6 coenzyme A+ 2 H20 |
| plumbagin | acetyl-CoA + 5 malonyl-CoA+ 3 H2+ H+ = naphthylisoquinoline alkaloid precursor+ 6 CO2+ 6 coenzyme A + 2 H20, acetyl-CoA + 5 malonyl-CoA+ 2 H2 = hexaketide pyrone+ 5 CO2+ 6 coenzyme A+ H2 0 |
| 6-methylsalicylate | acetyl-CoA + 3 malonyl-CoA+ NADPH + 3 H+ = 6-methylsalicylate + 3 CO2 + NADP+ + 4 coenzyme A |
| triacetic acid lactone | acetyl-CoA + 2 malonyl-CoA + H⁺ → triacetic acid lactone + 2 CO₂ + 3 coenzyme A |

Furthermore, numerous polyketide products can be made from malonyl-coA precursors in combination with isobutyryl-coA precursors. It is described elsewhere how to construct a genetically modified organism to produce butyryl-coA or isobutyryl-coA from malonyl-coA. Products can be made by expressing enzyme functions to convert malonyl-coA in addition to isobutyryl-coA into products. Several examples of these non-limiting products are shown below in Table 1C. Any of the strains discussed in the specification that increase flux through malonyl-coA and also produce isobutyryl-coA can be used to produce these products. Phlorisobutyrophenone can be produced by expressing a phlorisobutyrophenone synthase from Humulus lupus.

**Table 1C: Products requiring malonyl-coA and isobutyryl-coA precursors**

| **Chemical product** | **Biosynthesis** |
|---|---|
| cohumulone | 3 malonyl-CoA+ isobutyryl-CoA + 3 H+ = phlorisobutyrophenone + 3 CO2 + 4 coenzyme A |
| hyperforin | 3 malonyl-CoA+ isobutyryl-CoA + 3 H+ = phlorisobutyrophenone + 3 CO2 + 4 coenzyme A |

In addition, numerous polyketide products can be made from malonyl-coA precursors in combination with coumaryl-coA or cinnamoyl-coA precursors. Coumaryl-coA or cinnamoyl-coA production in genetically modified organisms has been described, for example, in Yohei Katsuyama et al.("Production of curcuminoids by Escherichia coli carrying an artificial biosynthesis pathway", Microbiology (2008), 154, 2620-2628). These genetic modifications can be made in combination with modifications described herein to increase malonyl-coA production. Products can be made by expressing enzyme functions to convert malonyl-coA in addition to coumaryl-coA or cinnamoyl-coA into products. Several examples of these non-limiting products are shown below in Table 1D. p-coumaroyltriacetic acid lactone can be made by expressing a p-coumaroyltriacetic acid lactone synthase from humulus lupulus or rubeus idaeus. Naringenin chalcone can be made by expressing a naringenin chalcone synthase from either humulus lupulus or arabidopsis thaliana. Isoliquiritigenin or other flavonoids can be made by expressing a chalcone reductase from numerous sources. 4-hydroxybenzalacetone can be made by expressing a 4-hydroxybenzalacetone synthase from rubus idaeus. Pinocembrin chalcone can be made by expressing a pinocembrin chalcone synthase from pinus densiflora. Resvertrol can be made by expressing a resveratrol or stilbene synthase such as RS G-528 from arachis hypogaea or STS G-230 from rheum tatricum. Bis-noryangonin can be produced by expressing a styrylpyrone synthase such as from equisetum arvense. P-coumaroyltriacetate can be made by expressing a p-coumaroyltriacetic acid lactone synthase from hydrangea macrophylla. Pinosylvin can be made by expressing one of numeroues stilbene synthases such as stilbene synthase PDSTS2 from pinus denisflora.

**Table 1D: Products requiring malonyl-coA and coumaryl-coA OR cinnamoyl-coA precursors**

| **Chemical product** | **Biosynthesis** |
|---|---|
| p-coumaroyltriacetic acid lactone (aromatic polyketide) | 4-coumaroyl-CoA + 3 malonyl-CoA+ 2 H+ = p-coumaroyltriacetic acid lactone+ 3 CO2 + 4 coenzyme A |
| naringenin chalcone (flavonoid) | 4-coumaroyl-CoA + 3 malonyl-CoA+ 3 H+ = naringenin chalcone+ 3 CO2 + 4 coenzyme A |
| isoliquiritigenin (flavonoid) | 4-coumaroyl-CoA + 3 malonyl-CoA+ NADPH + 4 H+ = isoliquiritigenin + 3 CO2 + NADP+ + 4 coenzyme A+ H20, |
| pinocembrin chalcone (pinobanksin) | 3 malonyl-CoA+ (E)-cinnamoyl-CoA + 3 H+ = pinocembrin chalcone+ 3 CO2+ 4 coenzyme A |
| 4 hydroxybenzalacetone (raspberry ketone) | 4-coumaroyl-CoA + malonyl-CoA+ H 20 + H+ = 4 hydroxybenzalacetone + 2 CO2+ 2 coenzyme A |
| p-coumaroyltriacetate (resveratrol) | 4-coumaroyl-CoA + 3 malonyl-CoA+ H2O + 2 H+ = 3 CO2+ p-coumaroyltriacetate + 4 coenzyme A |
| bis-noryangonin (resveratrol) | 4-coumaroyl-CoA + 2 malonyl-CoA+ H+ = bis-noryangonin + 2 CO2+ 3 coenzyme A |
| resveratrol | 4-coumaroyl-CoA + 3 malonyl-CoA+ 3 H+ = resveratrol + 4 CO2+ 4 coenzyme A |
| xanthohumol | 4-coumaroyl-CoA + 3 malonyl-CoA+ 3 H+ = naringenin chalcone+ 3 CO2 + 4 coenzyme A |
| pinosylvin | (E)-cinnamoyl-CoA + 3 malonyl-CoA+ 3 H+ = 4 CO2+ pinosylvin + 4 coenzyme A |

Numerous polyketide products can also be made from malonyl-coA precursors in combination with isovaleryl-coA precursors. As isovaleryl-coA is a product of leucine degradation pathways, many metabolic pathways are known to produce this product. These genetic modifications can be made in combination with modifications described herein to increase malonyl-coA production. Products can be made by expressing enzyme functions to convert malonyl-coA in addition to isovaleryl-coA into products. Several examples of these non-limiting products are shown below in Table 1E. 6-isobutyl-4-hydroxy-2-pyrone can be made by expressing a 6-isobutyl-4-hydroxy-2-pyrone synthase from Humulus lupulus. 6-(4-methyl-2-oxopentyl)-4-hydroxy-2-pyrone can be made by expressing a 6-(4-methyl-2-oxopentyl)-4-hydroxy-2-pyrone synthase from Humulus lupulus.

**Table 1E: Products requiring Malonyl-coA and isovaleryl-coA precursors**

| **Chemical product** | **Biosynthesis** |
|---|---|
| 6-isobutyl-4-hydroxy-2-pyrone | isovaleryl-CoA + 2 malonyl-CoA+ H+ = 6-isobutyl-4-hydroxy-2-pyrone + 2 CO2 + 3 coenzyme A |
| 6-(4-methyl-2-oxopentyl)-4-hydroxy-2-pyrone | isovaleryl-CoA + 3 malonyl-CoA+ 2 H+ = 6-(4-methyl-2-oxopentyl)-4-hydroxy-2-pyrone + 3 CO2 + 4 coenzyme A |

In addition, numerous polyketide products can be made from malonyl-coA precursors in combination with benzoyl-coA or 3-hydroxybenzoyl-coA precursors. Benzoyl-coA or 3-hydroxybenzoyl-coA production in genetically modified organisms has been described. In particular, one route to produce benzoyl-coA is from benzoate by expressing a benzoate-coA ligase such as from Hypericum androsaemum. In addition, 3-hydroxybenzoyl-coA can be made from 3-hydroxybenzoate by expressing a 3-hydroxybenzoate-coA ligase such as from Hypericum androsaemum. Products can be made by expressing enzyme functions to convert malonyl-coA in addition to benzoyl-coA or 3-hydroxybenzoyl-coA into products. Several examples of these non-limiting products are shown below in Table 1F. 2,3',4,6-tetrahydroxybenzophenone can be made by expressing a benzophenone synthase from Centauraium erythraea. 2,4,6-trihydroxybenzophenone can be made by expressing a benzophenone synthase such as AF352395 from Hypericum androsaemum. 3,5-dihydroxybiphenyl can be made be expressing a 3,5-dihydroxybiphenyl synthase.

**Table IF: Products requiring Malonyl-coA and benzoyl-coA OR 3-hydroxy-benzoyl-coA precursors**

| **Chemical product** | **Biosynthesis** |
|---|---|
| 2,3',4,6-tetrahydroxybenzophenone (tetrahydroxyxanthone) | 3-hydroxybenzoyl-CoA + 3 malonyl-CoA+ 3 H+ = 3 CO2 + 2,3',4,6-tetrahydroxybenzophenone + 4 coenzyme A |
| 2,4,6-trihydroxybenzophenone (tetrahydroxyxanthone) | 3 malonyl-CoA+ benzoyl-CoA + 3 H+ = 2,4,6-trihydroxybenzophenone + 3 CO2 + 4 coenzyme A |
| 3,5-dihydroxybiphenyl | 3 malonyl-CoA+ benzoyl-CoA + 3 H+ = 3,5-dihydroxybiphenyl + 4 CO2 + 4 coenzyme A |

In addition to the above, numerous polyketide products can be made from malonyl-coA precursors in combination with other secondary metabolite precursors. For example, (2S,5S)-carboxymethylproline, the precursor to carbapenem can be made from malonyl-coA and (S)-1-pyrroline-5-carboxylate by expressing a carboxymethylproline synthase from Pectobacterium carotovorum. The (S)-1-pyrroline-5-carboxylate is a precursor to the amino acid proline, whose production is known. 1,3-dihydroxy-N-methylacridone can be made from malonyl-coA and the additional precursor N-methylanthraniloyl-CoA by expressing an acridone synthase such as ACSII from Ruta graveolens. N-methylanthraniloyl-CoA can be made metabolically from the aromatic amino acid precursor chorismate by expressing an anthranilate synthase such as Ruta graveolens to convert chorismate to anthranilate, additionally expressing an anthranilate N-methyltransferase from Ruta graveolens to produce N-methylanthranilate from anthranilate and finally expressing a N-methylanthranilate-coA ligase to produce N-methylanthraniloyl-coA from N-methylanthranilate. Several examples of non-limiting products are shown below in Table 1G.

**Table 1G: Products requiring Malonyl-coA and other secondary metabolites.**

| **Chemical product** | **Biosynthesis** |
|---|---|
| (2S,5S)-carboxymethylproline ((5R)-carbapenem) | (S)-1-pyrroline-5-carboxylate + malonyl-CoA+ H2O + H+ = (2S,5S)-carboxymethylproline + CO2 +coenzyme |
| 1,3-dihydroxy-N-methylacridone (acridone alkaloid) | N-methylanthraniloyl-CoA + 3 malonyl-CoA= 3 CO2 + 1,3-dihydroxy-N-methylacridone + 4 coenzyme A |

In addition to the above, several malonyl-coA conjugate products can be made by supplying a cell with an enzyme expressing a malonyl-transferase to add a malonyl group to a larger molecule. For example, an isoflavone-7-O-glucoside-6-O-malonyl-transferase can add malonyl group to biochanin glucosides to produce biochanin glucoside malonates, an isoflavone-7-O-glucoside-6-O-malonyl-transferase can add malonyl group to biochanin glucosides to produce biochanin glucoside malonates, or alternatively add a malonyl group to glucosyl-apegenins to produce apegenin malonyl-glucosides. Table 1H gives the reaction of several malonyltransferases and substrates and products. Enzymes that can be expressed to perform these conversions can be identified in numerous databases in the art such as <<www.metacyc.org>>.

**Table 1H: Products requiring Malonyl-coA and malonyltransferases**

| **Chemical product** | **Biosynthesis** |
|---|---|
| apigenin 7-0(6-malonyl-B-D-glucoside) (apigenin glycosides) | 7 0 B D glucosyl-apigenin+malonyl-CoA=apigenin 7-0(6-malonyl-B-D-glucoside)+coenzyme A |
| biochanin A 7 0 glucoside-6"-malonate (biochanin A conjugates interconversion) | biochanin A 7 0 glucoside + malonyl CoA + ATP + H2O = biochanin A 7 0 glucoside-6"-malonate +AMP+ diphosphate + coenzyme A + 2 H+ |
| maackiain 3 0 glucoside-6"-malonate (maackiain conjugates interconversion) | (-)-maackiain 3 0 glucoside + malonyl CoA + ATP + H2O = () maackiain 3 0 glucoside-6"-malonate +AMP + diphosphate + coenzyme A + 2 H+ |
| medicarpin 3 0 glucoside-6"-malonate (medicarpin conjugates | (-)-medicarpin 3 0 glucoside + malonyl CoA + ATP + H2O = () medicarpin 3 0 glucoside-6"-malonate + AMP + diphosphate + coenzyme A |
| interconversion) | + 2 H+ |
| Malonyldaidzin (daidzein conjugates interconversion) | daidzin + malonyl-CoA+ ATP + H2O = malonyldaidzin +AMP+ diphosphate+ coenzyme A+ 2 H+ |
| formononetin 7 0 glucoside-6"-malonate (formononetin conjugates interconversion) | ononin + malonyl-CoA+ ATP + H2O = formononetin 7 0 glucoside-6"-malonate +AMP+ diphosphate+ coenzyme A + 2 H+ |
| Malonylgenistin (genistein conjugates interconversion) | genistin + malonyl-CoA+ ATP + H2O = malonylgenistin +AMP+ diphosphate+ coenzyme A+ 2 H+ |
| pelargonidin 3 0 (6 0 malonyl B D glucoside) (pelargonidin conjugates) | pelargonidin 3 0 B D - glucoside + malonyl-CoA+ H+ = pelargonidin 3 0 (6 0 malonyl B D glucoside) + coenzyme A |
| salvianin | monodemalonylsalvianin + malonyl-CoA+ H+ = salvianin +coenzyme A, bisdemalonylsalvianin + malonyl-CoA+ H+ = monodemalonylsalvianin +coenzyme A |
| malonylshisonin | shisonin + malonyl-CoA+ H+ = malonylshisonin +coenzyme A |
| malonylshisonin | superpathway of anthocyanin biosynthesis (from cyanidin and cyanidin 3-0-glucoside) : shisonin + malonyl-CoA+ H+ = malonylshisonin +coenzyme A |
| delphinidin 3-0-(6"-0-malonyl)-B-glucoside (ternatin C5) | delphinidin 3 0 B D - glucoside + malonyl-CoA= delphinidin 3-0-(6"-0-malonyl)-B-glucoside +coenzyme A |
| N-Malonylanthranilate | anthranilate+ malonyl-CoA= N-Malonylanthranilate +coenzyme A |
| N-(3,4-dichlorophenyl)-malonamate | 3,4-dichloroaniline + malonyl-CoA= N-(3,4-dichlorophenyl)-malonamate +coenzyme A |
| anthocyanidin 3 0 (6 0 malonyl B D glucoside) | malonyl-CoA+an anthocyanidin 3 0 B D glucoside =an anthocyanidin 3 0 (6 0 malonyl B D glucoside) + coenzyme A |
| 7 hydroxyflavone 7 0 (6 malonyl-B-D-glucoside) | 7 0 B D glucosyl 7 hydroxyflavone + malonyl CoA = 7 hydroxyflavone 7 0 (6 malonyl-B-D-glucoside) + coenzyme A |

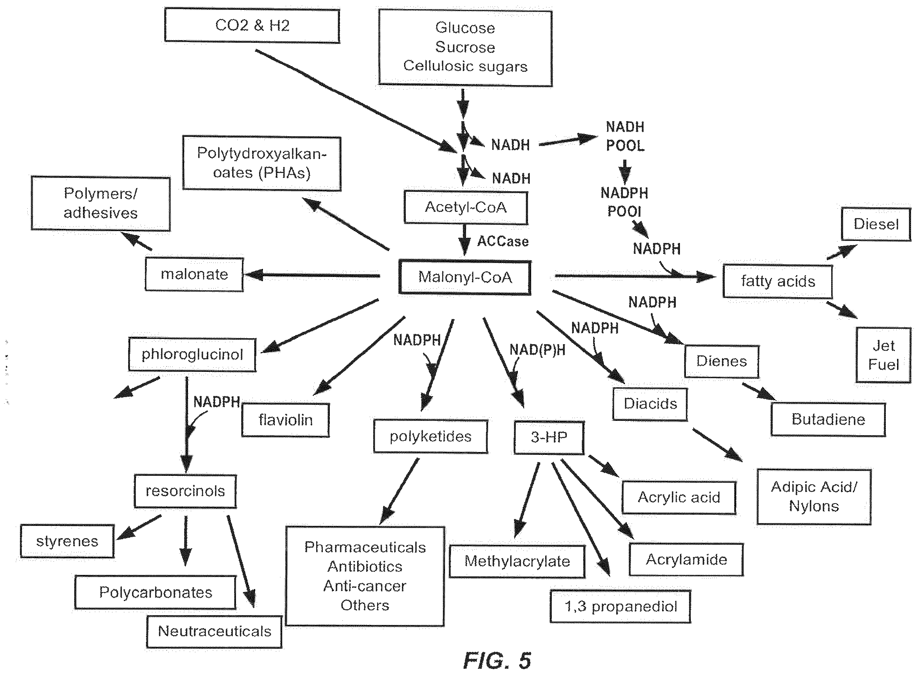

Also provided herein, in addition to 3-HP and chemcials and products made from it (including methylacrylate), and various polyketides provided in the tables, are teachings and other disclosures variously directed to production of phloroglucinol, resorcinol, malonic acid, diacids, dienes, flaviolin, malonate, chalcones, pyrones, type I, II and III polyketides more generally and other chemicals and other products made from these. FIG. 5 schematically depicts bioconversions from exemplary carbon sources to these various chemical products. It is noted that in various embodiments the invention may be applied to produce fatty acids and subsequently to obtain products made from these, such as jet fuel and diesel. In various embodiments, this is achieved by use of a pathway comprising an elongase as disclosed herein.

Any of the chemicals described or otherwise disclosed herein, including in the figures, may be a selected chemical product, or a chemical product of interest. Also, any grouping, including any sub-group, of the above listing may be considered what is referred to by "selected chemical product," "chemical product of interest," and the like. For any of these chemical products a microorganism may inherently comprise a biosynthesis pathway to such chemical product and/or may require addition of one or more heterologous nucleic acid sequences to provide or complete such a biosynthesis pathway, in order to achieve a desired production of such chemical product.

U.S. Patent Publication US2009/0111151 A1, incorporated by reference for its teachings of synthesis of various polyketides, describes illustrative polyketide synthase (PKS) genes and corresponding enzymes that can be utilized in the construction of genetically modified microorganisms and related methods and systems. Any of these may be employed in the embodiments of the present invention, such as in microorganisms that produce polyketides and also comprise modifications to reduce activity of fatty acid synthase enzymatic conversions.

U.S. Patent Publication US2011/0171702 A1, incorporated by reference for its teachings of synthesis of 2-hydroxybutyric acid (2-HIBA), describes production of 2-HIBA and corresponding enzymes that can be utilized in the construction of genetically modified microorganisms and related methods and systems. Any of these may be employed in the embodiments of the present invention, such as in microorganisms that produce 2-HIBA and also comprise modifications to reduce activity of fatty acid synthase enzymatic conversions.

U.S. Patent Publication US2010/0291644 A1, incorporated by reference for its teachings of preparing methacrylic acid or methacrylic esters from 3-hydroxyisobutyric acid (3-HIBA) or polyhydroxyalkanoates based on 3-HIBA, describes the production of 3-HIBA and corresponding enzymes that can be utilized in the construction of genetically modified microorganisms and related methods and systems and the dehydration to methacrylic. Any of these may be employed in the embodiments of the present invention, such as in microorganisms that produce 3-HIBA and/or polyhydroxyalkanoates and also comprise modifications to reduce activity of fatty acid synthase enzymatic conversions.

U.S. Patent Publication US2008/0274523 A1, incorporated by reference for its teachings of synthesis of various isoprenoids, describes illustrative isoprenoid synthesis genes and corresponding enzymes that can be utilized in the construction of genetically modified microorganisms and related methods and systems. Any of these may be employed in the embodiments of the present invention, such as in microorganisms that produce mevalonate or other isoprenoids and also comprise modifications to reduce activity of fatty acid synthase enzymatic conversions.

U.S. Patent number 6593116 incorporated by reference for its teachings of transgenic microbial production of polyhydroxyalkanoates, describes polyhydroxyalkanoate synthesis and corresponding enzymes that can be utilized in the construction of genetically modified microorganisms and related methods and systems. Any of these may be employed in the embodiments of the present invention, such as in microorganisms that produce polyhydroxybutyrate or other polyhydroxyalkanoates and also comprise modifications to reduce activity of fatty acid synthase enzymatic conversions.

Particular polyketide chemical products which are considered to be chemical product biosynthesis candidates under the present invention include the following: Amphotericin B; antimycin A; brefeldin A; candicidin; epothilones; erythromycin; azithromycin; clarithromycin; erythromycin estolate; erythromycin ethylsuccinate; roxithromycin; ivermectin; josamycin; ketolides; leucomycins; kitasamycin; spiramycin; lovastatin; lucensomycin; macrolides; maytansine; mepartricin; miocamycin; natamycin; nystatin; oleandomycin; troleandomycin; oligomycins; rutamycin; sirolimus; tacrolimus; tylosin; oleandomycin; deoxyoleandolide; narbonolide; narbomycin; and pikromycin.

### V. GENETIC MODIFICATIONS

Embodiments of the present invention may result from introduction of an expression vector into a host microorganism, wherein the expression vector contains a nucleic acid sequence coding for an enzyme that is, or is not, normally found in a host microorganism.

The ability to genetically modify a host cell is essential for the production of any genetically modified (recombinant) microorganism. The mode of gene transfer technology may be by electroporation, conjugation, transduction, or natural transformation. A broad range of host conjugative plasmids and drug resistance markers are available. The cloning vectors are tailored to the host organisms based on the nature of antibiotic resistance markers that can function in that host. Also, as disclosed herein, a genetically modified (recombinant) microorganism may comprise modifications other than via plasmid introduction, including modifications to its genomic DNA.

Those skilled in the art will understand that the genetic alterations, including metabolic modifications exemplified herein are described with reference to a suitable source organism such as E. coli, yeast, or other organisms disclosed herein and their corresponding metabolic enzymatic reactions or a suitable source organism for desired genetic material such as genes encoding enzymes for their corresponding metabolic enzymatic reactions. However, given the complete genome sequencing of a wide variety of organisms and the high level of skill in the area of genomics, those skilled in the art will readily be able to apply the teachings and guidance provided herein to essentially all other microorganisms. For example, the E. coli metabolic alterations exemplified herein can readily be applied to other species by incorporating the same or analogous encoding nucleic acid from species other than the referenced species. Such genetic alterations include, for example, genetic alterations of species homologs, in general, and in particular, orthologs, paralogs or nonorthologous gene displacements.

An ortholog is a gene or genes that are related by vertical descent and are responsible for substantially the same or identical functions in different organisms. Genes are related by vertical descent when, for example, they share sequence similarity of sufficient amount to indicate they are homologous, or related by evolution from a common ancestor. Genes can also be considered orthologs if they share three-dimensional structure but not necessarily sequence similarity, of a sufficient amount to indicate that they have evolved from a common ancestor to the extent that the primary sequence similarity is not identifiable. Genes that are orthologous can encode proteins with sequence similarity of about 25% to 100% amino acid sequence identity. Genes encoding proteins sharing an amino acid similarity less that 25% can also be considered to have arisen by vertical descent if their three-dimensional structure also shows similarities. Members of the serine protease family of enzymes, including tissue plasminogen activator and elastase, are considered to have arisen by vertical descent from a common ancestor.

Orthologs include genes or their encoded gene products that through, for example, evolution, have diverged in structure or overall activity. For example, where one species encodes a gene product exhibiting two functions and where such functions have been separated into distinct genes in a second species, the three genes and their corresponding products are considered to be orthologs. An example of orthologs exhibiting separable activities is where distinct activities have been separated into distinct gene products between two or more species or within a single species. A specific example is the separation of elastase proteolysis and plasminogen proteolysis, two types of serine protease activity, into distinct molecules as plasminogen activator and elastase.

In contrast, paralogs are homologues related by, for example, duplication followed by evolutionary divergence and have similar or common, but not identical functions. Paralogs can originate or derive from, for example, the same species or from a different species. For example, microsomal epoxide hydrolase (epoxide hydrolase I) and soluble epoxide hydrolase (epoxide hydrolase II) can be considered paralogs because they represent two distinct enzymes, co-evolved from a common ancestor, that catalyze distinct reactions and have distinct functions in the same species. Paralogs are proteins from the same species with significant sequence similarity to each other suggesting that they are homologous, or related through co-evolution from a common ancestor. Groups of paralogous protein families include HipA homologs, luciferase genes, peptidases, and others. A nonorthologous gene displacement is a nonorthologous gene from one species that can substitute for a referenced gene function in a different species. Substitution includes, for example, being able to perform substantially the same or a similar function in the species of origin compared to the referenced function in the different species. Although generally, a nonorthologous gene displacement will be identifiable as structurally related to a known gene encoding the referenced function, less structurally related but functionally similar genes and their corresponding gene products nevertheless will still fall within the meaning of the term as it is used herein. In some cases, functional similarity requires at least some structural similarity in the active site or binding region of a nonorthologous gene compared to a gene encoding the function sought to be substituted. Therefore, a nonorthologous gene includes, for example, a paralog or an unrelated gene.

Therefore, in identifying and designing a genetically modified microorganism of the present invention, those skilled in the art will understand with applying the teaching and guidance provided herein to a particular species that the identification of genetic modifications can include identification and inclusion or inactivation or other modification of orthologs. To the extent that paralogs and/or nonorthologous gene displacements are present in the referenced microorganism that encode an enzyme catalyzing a similar or substantially similar metabolic reaction, those skilled in the art also can utilize these evolutionarily related genes. Orthologs, paralogs and nonorthologous gene displacements can be determined by methods well known to those skilled in the art. For example, inspection of nucleic acid or amino acid sequences for two polypeptides will reveal sequence identity and similarities between the compared sequences. Based on such similarities, one skilled in the art can determine if the similarity is sufficiently high to indicate the proteins are related through evolution from a common ancestor. Algorithms well known to those skilled in the art, such as Align, BLAST, Clustal Wand others compare and determine a raw sequence similarity or identity, and also determine the presence or significance of gaps in the sequence which can be assigned a weight or score. Such algorithms also are known in the art and are similarly applicable for determining nucleotide sequence similarity or identity. Parameters for sufficient similarity to determine relatedness are computed based on well known methods for calculating statistical similarity, or the chance of finding a similar match in a random polypeptide, and the significance of the match determined. A computer comparison of two or more sequences can, if desired, also be optimized visually by those skilled in the art. Related gene products or proteins can be expected to have a high similarity, for example, 25% to 100% sequence identity. Proteins that are unrelated can have an identity which is essentially the same as would be expected to occur by chance, if a database of sufficient size is scanned(about 5%). Sequences between 5% and 24% sequence identity may or may not represent sufficient homology to conclude that the compared sequences are related. Additional statistical analysis to determine the significance of such matches given the size of the data set can be carried out to determine the relevance of these sequences.

Exemplary parameters for determining relatedness of two or more sequences using the BLAST algorithm, for example, can be as set forth below. Briefly, amino acid sequence alignments can be performed using BLASTP version 2.0.8 (Jan-05-1999) and the following parameters: Matrix: 0 BLOSUM62; gap open: 11; gap extension: 1; x_dropoff: 50; expect: 10.0; wordsize: 3; filter: on. Nucleic acid sequence alignments can be performed using BLASTN version 2.0.6 (Sept- 16- 1998) and the following parameters: Match: 1; mismatch: -2; gap open: 5; gap extension: 2; x_dropoff: 50; expect: 10.0; wordsize: 11; filter: off. Those skilled in the art will know what modifications can be made to the above parameters to either increase or decrease the stringency of the comparison, for example, and determine the relatedness of two or more sequences. Through such comparisons and analyses, one skilled in the art may be able to obtain a desired polypeptide in a particular species that functions similarly to a polypeptide (enzyme) disclosed herein, and/or a functional variant that possesses a desired enzymatic activity.

Also, in various embodiments polypeptides, such as enzymes, obtained by the expression of the any of the various polynucleotide molecules (i.e., nucleic acid sequences) of the present invention may have at least approximately 50%, 60%, 70%, 80%, 90%, 95%, 96%, 97%, 98%, 99% or 100% identity to one or more amino acid sequences encoded by the genes and/or nucleic acid sequences described herein.

Embodiments of the present invention may involve various nucleic acid sequences, such as heterologous nucleic acid sequences introduced into a cell's genome or may be episomal, and also encompasses antisense nucleic acid molecules, i.e., molecules which are complementary to a sense nucleic acid encoding a protein, e.g., complementary to the coding strand of a doublestranded eDNA molecule or complementary to an mRNA sequence.

For various embodiments of the invention the genetic manipulations may be described to include various genetic manipulations, including those directed to change regulation of, and therefore ultimate activity of, an enzyme or enzymatic activity of an enzyme identified in any of the respective pathways. Such genetic modifications may be directed to transcriptional, translational, and post-translational modifications that result in a change of enzyme activity and/or selectivity under selected and/or identified culture conditions and/or to provision of additional nucleic acid sequences such as to increase copy number and/or mutants of an enzyme related to 3-HP production. Specific methodologies and approaches to achieve such genetic modification are well known to one skilled in the art, and include, but are not limited to: increasing expression of an endogenous genetic element; decreasing functionality of a repressor gene; introducing a heterologous genetic element; increasing copy number of a nucleic acid sequence encoding a polypeptide catalyzing an enzymatic conversion step to produce 3-HP; mutating a genetic element to provide a mutated protein to increase specific enzymatic activity; over-expressing; under-expressing; over-expressing a chaperone; knocking out a protease; altering or modifying feedback inhibition; providing an enzyme variant comprising one or more of an impaired binding site for a repressor and/or competitive inhibitor; knocking out a repressor gene; evolution, selection and/or other approaches to improve mRNA stability as well as use of plasmids having an effective copy number and promoters to achieve an effective level of improvement. Random mutagenesis may be practiced to provide genetic modifications that may fall into any of these or other stated approaches. The genetic modifications further broadly fall into additions (including insertions), deletions (such as by a mutation) and substitutions of one or more nucleic acids in a nucleic acid of interest. In various embodiments a genetic modification results in improved enzymatic specific activity and/or turnover number of an enzyme. Without being limited, changes may be measured by one or more of the following: KM; Kam; and In various embodiments, to function more efficiently, a microorganism may comprise one or more gene deletions. For example, in E. coli, the genes encoding the lactate dehydrogenase (ldhA), phosphate acetyltransferase (pta), pyruvate oxidase (poxB), and pyruvate-formate lyase (pflB) may be disrupted, including deleted. Such gene disruptions, including deletions, are not meant to be limiting, and may be implemented in various combinations in various embodiments. Gene deletions may be accomplished by mutational gene deletion approaches, and/or starting with a mutant strain having reduced or no expression of one or more of these enzymes, and/or other methods known to those skilled in the art. Gene deletions may be effectuated by any of a number of known specific methodologies, including but not limited to the RED/ET methods using kits and other reagents sold by Gene Bridges (Gene Bridges GmbH, Dresden, Germany, <<www.genebridges.com>>). More particularly as to the latter method, use of Red/ET recombination, is known to those of ordinary skill in the art and described in U.S. Patent Nos. 6,355,412 and 6,509,156, issued to Stewart et al. and incorporated by reference herein for its teachings of this method. Material and kits for such method are available from Gene Bridges (Gene Bridges GmbH, Dresden, Germany, <<www.genebridges.com>>), and the method may proceed by following the manufacturer's instructions. The method involves replacement of the target gene by a selectable marker via homologous recombination performed by the recombinase from X-phage. The host organism expressing k-red recombinase is transformed with a linear DNA product coding for a selectable marker flanked by the terminal regions (generally -50 bp, and alternatively up to about -300 bp) homologous with the target gene. The marker could then be removed by another recombination step performed by a plasmid vector carrying the FLP-recombinase, or another recombinase, such as Cre. Targeted deletion of parts of microbial chromosomal DNA or the addition of foreign genetic material to microbial chromosomes may be practiced to alter a host cell's metabolism so as to reduce or eliminate production of undesired metabolic products. This may be used in combination with other genetic modifications such as described herein in this general example. In this detailed description, reference has been made to multiple embodiments and to the accompanying drawings in which is shown by way of illustration specific exemplary embodiments in which the invention may be practiced. These embodiments are described in sufficient detail to enable those skilled in the art to practice the invention, and it is to be understood that modifications to the various disclosed embodiments may be made by a skilled artisan. Further, for 3-HP production, such genetic modifications may be chosen and/or selected for to achieve a higher flux rate through certain enzymatic conversion steps within the respective 3-HP production pathway and so may affect general cellular metabolism in fundamental and/or major ways. It has long been recognized in the art that some amino acids in amino acid sequences can be varied without significant effect on the structure or function of proteins. Variants included can constitute deletions, insertions, inversions, repeats, and type substitutions so long as the indicated enzyme activity is not significantly adversely affected. Guidance concerning which amino acid changes are likely to be phenotypically silent can be found, inter alia, in Bowie, J. U., et al., "Deciphering the Message in Protein Sequences: Tolerance to Amino Acid Substitutions," Science 247:1306-1310 (1990). This reference is incorporated by reference for such teachings, which are, however, also generally known to those skilled in the art.

Further, it will be appreciated that amino acid "homology" includes conservative substitutions, i.e. those that substitute a given amino acid in a polypeptide by another amino acid of similar characteristics. Recognized conservative amino acid substitutions comprise (substitutable amino acids following each colon of a set): ala:ser; arg:lys; asn:gln or his; asp:glu; cys:ser; gln:asn; glu:asp; gly:pro; his:asn or gln; ile:leu or val; leu:ile or val; lys: arg or gln or glu; met:leu or ile; phe:met or leu or tyr; ser:thr; thr:ser; trp:tyr; tyr:trp or phe; val:ile or leu. Also generally recognized as conservative substitutions are the following replacements: replacements of an aliphatic amino acid such as Ala, Val, Leu and Ile with another aliphatic amino acid; replacement of a Ser with a Thr or vice versa; replacement of an acidic residue such as Asp or Glu with another acidic residue; replacement of a residue bearing an amide group, such as Asn or Gln, with another residue bearing an amide group; exchange of a basic residue such as Lys or Arg with another basic residue; and replacement of an aromatic residue such as Phe or Tyr with another aromatic residue.

For all polynucleotide (nucleic acid) and polypeptide (amino acid) sequences provided herein, it is appreciated that conservatively modified variants of these sequences are included, and are within the scope of the invention in its various embodiments. Conservatively modified variant include amino acid conservative substitutions such as those described in the previous paragraph as well as modified polynucleotide sequences such as based on codon degeneracy described in the following paragraph and table. Further, the following table also provides characteristics of amino acids that provide for additional conservative substitutions that may fall within the scope of conservatively modified variants, based on commonly shared properties of particular amino acids. Also, in various embodiments deletions and/or substitutions at either end, or in other regions, of a polynucleotide or polypeptide may be practiced for sequences, based on the present teachings and knowledge of those skilled in the art, and remain within the scope of conservatively modified variants.

Accordingly, functionally equivalent polynucleotides and polypeptides (functional variants), which may include conservatively modified variants as well as more extensively varied sequences, which are well within the skill of the person of ordinary skill in the art, and microorganisms comprising these, also are within the scope of various embodiments of the invention, as are methods and systems comprising such sequences and/or microorganisms. In various embodiments, nucleic acid sequences encoding sufficiently homologous proteins or portions thereof are within the scope of the invention. More generally, nucleic acids sequences that encode a particular amino acid sequence employed in the invention may vary due to the degeneracy of the genetic code, and nonetheless fall within the scope of the invention. The following table provides a summary of similarities among amino acids, upon which conservative substitutions may be based, and also various codon redundancies that reflect this degeneracy.

**TABLE 2**

| **Amino Acid** | **Relationships** | **DNA codons** |
|---|---|---|
| Alanine | - | GCT, GCC, GCA, GCG |
| Proline | N | CCT, CCC, CCA, CCG |
| Valine | N, Ali | GTT, GTC, GTA, GTG |
| Leucine | N, Ali | CTT, CTC, CTA, CTG, TTA , TTG |
| Isoleucine | N, Ali | ATT, ATC, ATA |
| Methionine | N | ATG |
| Phenylalanine | N, Aro | |
| Tryptophan | | TTT, TTC |
| Glycine | N | TGG |
| Serine | PU | GGT, GGC, GGA, GGG |
| Threonine | PU | TCT, TCC, TCA, TCG, AGT, AGC |
| Asparagine | PU | ACT, ACC, ACA, ACG |
| Glutamine | PU, Ami | AAT, AAC |
| Cysteine | PU, Ami | CAA, CAG |
| Aspartic acid | | TGT, TGC |
| Glutamic acid | | GAT, GAC |
| Arginine | NEG, A | GAA, GAG |
| Lysine | POS, B | CGT, CGC, CGA, CGG, AGA, AGG |
| Histidine | POS, B | AAA, AAG |
| Tyrosine | POS | CAT, CAC |
| *Stop Codons* | Aro | TAT, TAC |
| | | TAA, TAG, TGA |

| | | |
|---|---|---|
| Legend: side groups and other related properties: A=acidic; B=basic; Ali=aliphatic; Ami=amine; Aro= aromatic; N=nonpolar; PU=polar uncharged; NEG=negatively charged; POS=positively charged. | | |

It is noted that codon preferences and codon usage tables for a particular species can be used to engineer isolated nucleic acid molecules that take advantage of the codon usage preferences of that particular species. For example, the isolated nucleic acid provided herein can be designed to have codons that are preferentially used by a particular organism of interest. Numerous software and sequencing services are available for such codon-optimizing of sequences. It also is noted that less conservative substitutions may be made and still provide a functional variant.

In various embodiments polypeptides obtained by the expression of the polynucleotide molecules of the present invention may have at least approximately 50%, 60%, 70%, 80%, 90%, 95%, 96%, 97%, 98%, 99% or 100% identity to one or more amino acid sequences encoded by the genes and/or nucleic acid sequences described herein for chemical product biosynthesis pathways.

As a practical matter, whether any particular polypeptide is at least 50%, 60%, 70%, 80%, 85%, 90%, 92%, 95%, 96%, 97%, 98% or 99% identical to any reference amino acid sequence of any polypeptide described herein (which may correspond with a particular nucleic acid sequence described herein), such particular polypeptide sequence can be determined conventionally using known computer programs such the Bestfit program (Wisconsin Sequence Analysis Package, Version 8 for Unix, Genetics Computer Group, University Research Park, 575 Science Drive, Madison, Wis. 53711). When using Bestfit or any other sequence alignment program to determine whether a particular sequence is, for instance, 95% identical to a reference sequence according to the present invention, the parameters are set such that the percentage of identity is calculated over the full length of the reference amino acid sequence and that gaps in homology of up to 5% of the total number of amino acid residues in the reference sequence are allowed.

For example, in a specific embodiment the identity between a reference sequence (query sequence, i.e., a sequence of the present invention) and a subject sequence, also referred to as a global sequence alignment, may be determined using the FASTDB computer program based on the algorithm of Brutlag et al. (Comp. App. Biosci. 6:237-245 (1990)). Preferred parameters for a particular embodiment in which identity is narrowly construed, used in a FASTDB amino acid alignment, are: Scoring Scheme=PAM (Percent Accepted Mutations) 0, k-tuple=2, Mismatch Penalty=1, Joining Penalty=20, Randomization Group Length=0, Cutoff Score=1, Window Size=sequence length, Gap Penalty=5, Gap Size Penalty=0.05, Window Size=500 or the length of the subject amino acid sequence, whichever is shorter. According to this embodiment, if the subject sequence is shorter than the query sequence due to N- or C-terminal deletions, not because of internal deletions, a manual correction is made to the results to take into consideration the fact that the FASTDB program does not account for N- and C-terminal truncations of the subject sequence when calculating global percent identity. For subject sequences truncated at the N- and C-termini, relative to the query sequence, the percent identity is corrected by calculating the number of residues of the query sequence that are lateral to the N- and C-terminal of the subject sequence, which are not matched/aligned with a corresponding subject residue, as a percent of the total bases of the query sequence. A determination of whether a residue is matched/aligned is determined by results of the FASTDB sequence alignment. This percentage is then subtracted from the percent identity, calculated by the FASTDB program using the specified parameters, to arrive at a final percent identity score. This final percent identity score is what is used for the purposes of this embodiment. Only residues to the N- and C-termini of the subject sequence, which are not aligned with the query sequence, are considered for the purposes of manually adjusting the percent identity score. That is, only query residue positions outside the farthest N- and C-terminal residues of the subject sequence are considered for this manual correction. For example, a 90 amino acid residue subject sequence is aligned with a 100 residue query sequence to determine percent identity. The deletion occurs at the N-terminus of the subject sequence and therefore, the FASTDB alignment does not show a matching/alignment of the first 10 residues at the N-terminus. The 10 unpaired residues represent 10% of the sequence (number of residues at the N- and C-termini not matched/total number of residues in the query sequence) so 10% is subtracted from the percent identity score calculated by the FASTDB program. If the remaining 90 residues were perfectly matched the final percent identity would be 90%. In another example, a 90 residue subject sequence is compared with a 100 residue query sequence. This time the deletions are internal deletions so there are no residues at the N- or C-termini of the subject sequence which are not matched/aligned with the query. In this case the percent identity calculated by FASTDB is not manually corrected. Once again, only residue positions outside the N- and C-terminal ends of the subject sequence, as displayed in the FASTDB alignment, which are not aligned with the query sequence are manually corrected for.

More generally, nucleic acid constructs can be prepared comprising an isolated polynucleotide encoding a polypeptide having enzyme activity operably linked to one or more (several) control sequences that direct the expression of the coding sequence in a microorganism, such as E. coli, under conditions compatible with the control sequences. The isolated polynucleotide may be manipulated to provide for expression of the polypeptide. Manipulation of the polynucleotide's sequence prior to its insertion into a vector may be desirable or necessary depending on the expression vector. The techniques for modifying polynucleotide sequences utilizing recombinant DNA methods are well established in the art.

The control sequence may be an appropriate promoter sequence, a nucleotide sequence that is recognized by a host cell for expression of a polynucleotide encoding a polypeptide of the present invention. The promoter sequence contains transcriptional control sequences that mediate the expression of the polypeptide. The promoter may be any nucleotide sequence that shows transcriptional activity in the host cell of choice including mutant, truncated, and hybrid promoters, and may be obtained from genes encoding extracellular or intracellular polypeptides either homologous or heterologous to the host cell. Examples of suitable promoters for directing transcription of the nucleic acid constructs, especially in an E. coli host cell, are the lac promoter (Gronenborn, 1976, Mol. Gen. Genet. 148: 243-250), tac promoter (DeBoer et a/., 1983, Proceedings of the National Academy of Sciences USA 80: 21-25), trc promoter (Brosius et al, 1985, J. Biol. Chem. 260: 3539-3541), T7 RNA polymerase promoter (Studier and Moffatt, 1986, J. Mol. Biol. 189: 113¬130), phage promoter pL (Elvin et al., 1990, Gene 87: 123-126), tetA prmoter (Skerra, 1994, Gene 151 : 131¬135), araBAD promoter (Guzman et al., 1995, J. Bacteriol. 177: 4121-4130), and rhaPBAD promoter (Haldimann et al., 1998, J. Bacteriol. 180: 1277-1286). Other promoters are described in "Useful proteins from recombinant bacteria" in Scientific American, 1980, 242: 74-94; and in Sambrook and Russell, "Molecular Cloning: A Laboratory Manual," Third Edition 2001 (volumes 1-3), Cold Spring Harbor Laboratory Press, Cold Spring Harbor, N.Y.

The control sequence may also be a suitable transcription terminator sequence, a sequence recognized by a host cell to terminate transcription. The terminator sequence is operably linked to the 3' terminus of the nucleotide sequence encoding the polypeptide. Any terminator that is functional in an E. coli cell may be used in the present invention. It may also be desirable to add regulatory sequences that allow regulation of the expression of the polypeptide relative to the growth of the host cell. Examples of regulatory systems are those that cause the expression of the gene to be turned on or off in response to a chemical or physical stimulus, including the presence of a regulatory compound. Regulatory systems in prokaryotic systems include the lac, tac, and trp operator systems.

Also, variants and portions of particular nucleic acid sequences, and respective encoded amino acid sequences recited herein may be exhibit a desired functionality, e.g., enzymatic activity at a selected level, when such nucleic acid sequence variant and/or portion contains a 15 nucleotide sequence identical to any 15 nucleotide sequence set forth in the nucleic acid sequences recited herein including, without limitation, the sequence starting at nucleotide number 1 and ending at nucleotide number 15, the sequence starting at nucleotide number 2 and ending at nucleotide number 16, the sequence starting at nucleotide number 3 and ending at nucleotide number 17, and so forth. It will be appreciated that the invention also provides isolated nucleic acid that contains a nucleotide sequence that is greater than 15 nucleotides (e.g., 16, 17, 18, 19, 20, 21, 22, 23, 24, 25, 26, 27, 28, 29, 30 or more nucleotides) in length and identical to any portion of the sequence set forth in nucleic acid sequences recited herein. For example, the invention provides isolated nucleic acid that contains a 25 nucleotide sequence identical to any 25 nucleotide sequence set forth in any one or more (including any grouping of) nucleic acid sequences recited herein including, without limitation, the sequence starting at nucleotide number 1 and ending at nucleotide number 25, the sequence starting at nucleotide number 2 and ending at nucleotide number 26, the sequence starting at nucleotide number 3 and ending at nucleotide number 27, and so forth. Additional examples include, without limitation, isolated nucleic acids that contain a nucleotide sequence that is 50 or more nucleotides (e.g., 100, 150, 200, 250, 300, or more nucleotides) in length and identical to any portion of any of the sequences disclosed herein. Such isolated nucleic acids can include, without limitation, those isolated nucleic acids containing a nucleic acid sequence represented in any one section of discussion and/or examples, including nucleic acid sequences encoding enzymes of the fatty acid synthase system. For example, the invention provides an isolated nucleic acid containing a nucleic acid sequence listed herein that contains a single insertion, a single deletion, a single substitution, multiple insertions, multiple deletions, multiple substitutions, or any combination thereof (e. g., single deletion together with multiple insertions). Such isolated nucleic acid molecules can share at least 60, 65, 70, 75, 80, 85, 90, 95, 97, 98, or 99 percent sequence identity with a nucleic acid sequence listed herein (i.e., in the sequence listing).

Additional examples include, without limitation, isolated nucleic acids that contain a nucleic acid sequence that encodes an amino acid sequence that is 50 or more amino acid residues (e.g., 100, 150, 200, 250, 300, or more amino acid residues) in length and identical to any portion of an amino acid sequence listed or otherwise disclosed herein.

In addition, the invention provides isolated nucleic acid that contains a nucleic acid sequence that encodes an amino acid sequence having a variation of an amino acid sequence listed or otherwise disclosed herein. For example, the invention provides isolated nucleic acid containing a nucleic acid sequence encoding an amino acid sequence listed or otherwise disclosed herein that contains a single insertion, a single deletion, a single substitution, multiple insertions, multiple deletions, multiple substitutions, or any combination thereof (e.g., single deletion together with multiple insertions). Such isolated nucleic acid molecules can contain a nucleic acid sequence encoding an amino acid sequence that shares at least 60, 65, 70, 75, 80, 85, 90, 95, 97, 98, or 99 percent sequence identity with an amino acid sequence listed or otherwise disclosed herein.

The invention provides polypeptides that contain the entire amino acid sequence of an amino acid sequence listed or otherwise disclosed herein. In addition, the invention provides polypeptides that contain a portion of an amino acid sequence listed or otherwise disclosed herein. For example, the invention provides polypeptides that contain a 15 amino acid sequence identical to any 15 amino acid sequence of an amino acid sequence listed or otherwise disclosed herein including, without limitation, the sequence starting at amino acid residue number 1 and ending at amino acid residue number 15, the sequence starting at amino acid residue number 2 and ending at amino acid residue number 16, the sequence starting at amino acid residue number 3 and ending at amino acid residue number 17, and so forth. It will be appreciated that the invention also provides polypeptides that contain an amino acid sequence that is greater than 15 amino acid residues (e. g., 16, 17, 18, 19, 20, 21, 22, 23, 24, 25, 26, 27, 28, 29, 30 or more amino acid residues) in length and identical to any portion of an amino acid sequence listed or otherwise disclosed herein For example, the invention provides polypeptides that contain a 25 amino acid sequence identical to any 25 amino acid sequence of an amino acid sequence listed or otherwise disclosed herein including, without limitation, the sequence starting at amino acid residue number 1 and ending at amino acid residue number 25, the sequence starting at amino acid residue number 2 and ending at amino acid residue number 26, the sequence starting at amino acid residue number 3 and ending at amino acid residue number 27, and so forth. Additional examples include, without limitation, polypeptides that contain an amino acid sequence that is 50 or more amino acid residues (e.g., 100, 150, 200, 250, 300 or more amino acid residues) in length and identical to any portion of an amino acid sequence listed or otherwise disclosed herein. Further, it is appreciated that, per above, a 15 nucleotide sequence will provide a 5 amino acid sequence, so that the latter, and higher-length amino acid sequences, may be defined by the above-described nucleotide sequence lengths having identity with a sequence provided herein.

In addition, the invention provides polypeptides that an amino acid sequence having a variation of the amino acid sequence set forth in an amino acid sequence listed or otherwise disclosed herein. For example, the invention provides polypeptides containing an amino acid sequence listed or otherwise disclosed herein that contains a single insertion, a single deletion, a single substitution, multiple insertions, multiple deletions, multiple substitutions, or any combination thereof (e.g., single deletion together with multiple insertions). Such polypeptides can contain an amino acid sequence that shares at least 60, 65, 70, 75, 80, 85, 90, 95, 97, 98 or 99 percent sequence identity with an amino acid sequence listed or otherwise disclosed herein. A particular variant amino acid sequence may comprise any number of variations as well as any combination of types of variations.

As indicated herein, polypeptides having a variant amino acid sequence can retain enzymatic activity. Such polypeptides can be produced by manipulating the nucleotide sequence encoding a polypeptide using standard procedures such as site-directed mutagenesis or various PCR techniques. As noted herein, one type of modification includes the substitution of one or more amino acid residues for amino acid residues having a similar chemical and/or biochemical property. For example, a polypeptide can have an amino acid sequence set forth in an amino acid sequence listed or otherwise disclosed herein comprising one or more conservative substitutions.

More substantial changes can be obtained by selecting substitutions that are less conservative, and/or in areas of the sequence that may be more critical, for example selecting residues that differ more significantly in their effect on maintaining: (a) the structure of the polypeptide backbone in the area of the substitution, for example, as a sheet or helical conformation; (b) the charge or hydrophobicity of the polypeptide at the target site; or (c) the bulk of the side chain. The substitutions that in general are expected to produce the greatest changes in polypeptide function are those in which: (a) a hydrophilic residue, e.g., serine or threonine, is substituted for (or by) a hydrophobic residue, e.g., leucine, isoleucine, phenylalanine, valine or alanine; (b) a cysteine or proline is substituted for (or by) any other residue ; (c) a residue having an electropositive side chain, e.g., lysine, arginine, or histidine, is substituted for (or by) an electronegative residue, e.g., glutamic acid or aspartic acid; or (d) a residue having a bulky side chain, e.g., phenylalanine, is substituted for (or by) one not having a side chain, e.g., glycine. The effects of these amino acid substitutions (or other deletions or additions) can be assessed for polypeptides having enzymatic activity by analyzing the ability of the polypeptide to catalyze the conversion of the same substrate as the related native polypeptide to the same product as the related native polypeptide. Accordingly, polypeptides having 5, 10, 20, 30, 40, 50 or less conservative substitutions are provided by the invention.

Polypeptides and nucleic acids encoding polypeptides can be produced by standard DNA mutagenesis techniques, for example, M13 primer mutagenesis. Details of these techniques are provided in Sambrook and Russell, 2001. Nucleic acid molecules can contain changes of a coding region to fit the codon usage bias of the particular organism into which the molecule is to be introduced.

The invention also provides an isolated nucleic acid that is at least about 12 bases in length (e.g., at least about 13, 14, 15, 16, 17, 18, 19, 20, 25, 30, 40, 50, 60, 100, 250, 500, 750, 1000, 1500, 2000, 3000, 4000, or 5000 bases in length) and hybridizes, under hybridization conditions, to the sense or antisense strand of a nucleic acid having a sequence listed or otherwise disclosed herein. The hybridization conditions can be moderately or highly stringent hybridization conditions. Also, in some embodiments the microorganism comprises an endogenous chemical productproduction pathway (which may, in some such embodiments, be enhanced), whereas in other embodiments the microorganism does not comprise a chemical product production pathway, but is provided with one or more nucleic acid sequences encoding polypeptides having enzymatic activity or activities to complete a pathway, described herein, resulting in production of a selected chemical product. In some embodiments, the particular sequences disclosed herein, or conservatively modified variants thereof, are provided to a selected microorganism, such as selected from one or more of the species and groups of species or other taxonomic groups listed herein.

### VI. METABOLIC PATHWAYS

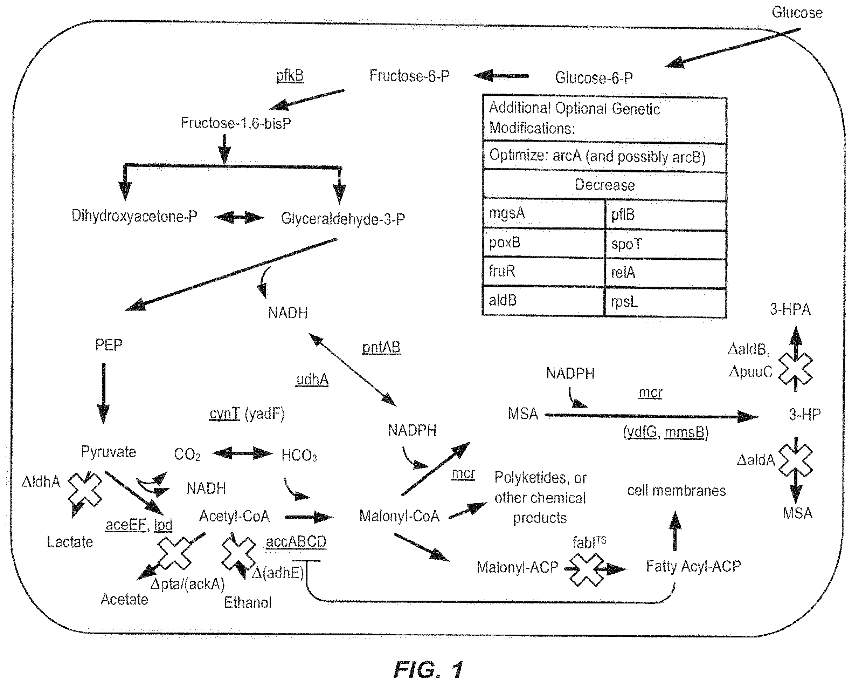

FIG. 1 diagrammatically depicts a cell showing key enzymatic conversion steps that include production of malonyl-CoA. In this figure enzyme functions are indicated by indicated enzymatic conversions and/or representative E. coli gene identifiers that encode proteins (polypeptides) having such enzyme functions (except that mcr indicates non-E. coli malonyl-CoA reductase), deletions are shown by the standard "A" before the respective gene identifier, and increased enzymatic activities are shown by underlining (noting that additional targets for modifications are as indicated in the embedded table of the figure). Genes in parentheses are possible substitutes for or supplements of an enzyme encoded by another gene also shown along the respective pathway step. Disrupted pathways also are shown by an "X" and the transient or partially disrupted step from malonyl-CoA to Fatty Acid-ACP is shown by a dashed "X." Also, the use of fabl(ts) represents a substitution for the native non-temperature-sensitive gene, thus the dashed X shape over the enzymatic conversion step is meant to indicate that a modification of this step may be made so that the enzymatic conversion is reduced, reduced under certain conditions, etc. This is not meant to be limiting; as described elsewhere there are a number of approaches to control and limit flux to fatty acyl-ACP and other intermediates or products of a fatty acid synthase pathway. Also, in this and various other figures, the use of "glucose" as a carbon source is exemplary and not meant to be limiting; pathways for other carbon sources to feed into the indicated pathways may be substituted.

The insert box of FIG. 1 provides a list, not to be limiting, of additional optional genetic modifications that may be made to the host cell to further improve chemical product biosynthesis under various culture conditions.

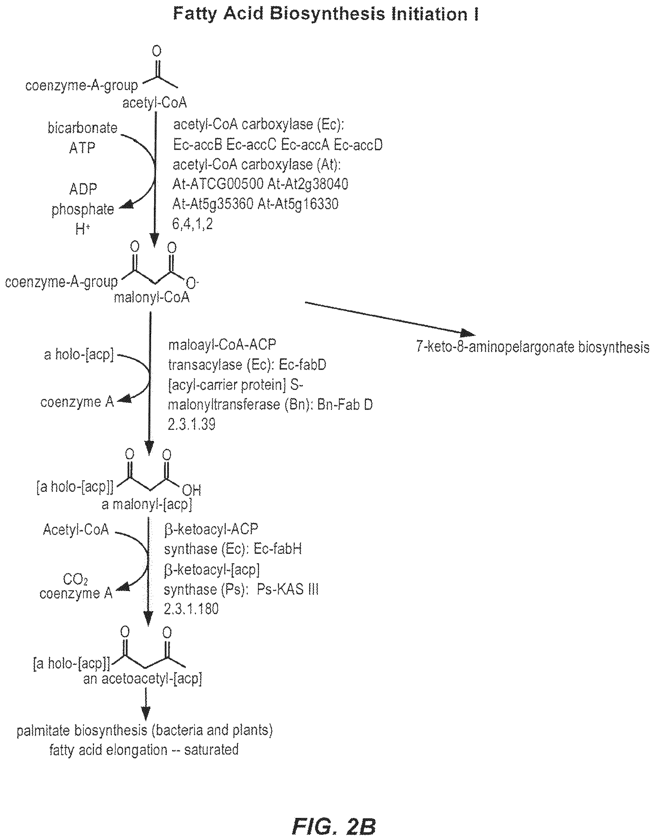

It is important to note that malonyl-CoA may be a substrate for a fatty acid synthase system such as that shown in FIG. 2A, which utilizes malonyl-CoA and produces fatty acids that are used for cell membranes and other cellular requirements. Also shown in FIG. 2A are E. coli gene names for genes that encode various steps of this representative fatty acid synthase (also referred to as fatty acid synthesis) pathway, the circular portion of which exemplifies fatty acid elongation. FIGs. 2B-D are provided to show fatty acid initiation reactions in greater detail (some of which are depicted in FIG. 2A). The gene names are not meant to be limiting, and are only exemplary. Table 3 provides enzyme function and corresponding EC numbers.

FIG. 2A also depicts a metabolic competition between utilization of malonyl-CoA for fatty acid synthesis (lower part of diagram) versus for production of polyketides and other malonyl-CoA-based chemical products, and also for production of 3-HP (which may be converted after biosynthesis to acrylic acid and other chemicals and products). Without being bound to a particular theory, when flux can be diverted from fatty acid synthesis that consumes malonyl-CoA, more malonyl-CoA may become available for biosynthesis of a desired chemical product of commercial interest (other than a fatty acid, a phospholipid, etc.). FIG. 2A also identifies certain inhibitors selected pathway steps, and certain feedback inhibition by metabolic intermediates (shown by a "Tee" shape extending from the inhibitor or metabolic intermediate to the inhibited step).

It is noteworthy that for purposes of the present invention, although traditionally fatty acid synthesis is often viewed to start with acetyl-CoA carboxylase, which produces malonyl-CoA, for the purposes of the present invention, as is clear from FIG. 2A and other teachings herein, modulation to decrease one or more, two or more, or three or more enzymatic functions of the fatty acid synthesis pathways downstream of malonyl-CoA are of interest to increase availability of malonyl-CoA for biosynthesis of the chemical products disclosed herein. Also, as taught herein, the genetic modification(s) and/or other system modifications (e.g., addition of an inhibitor) to modulate these enzymatic functions (i.e., enzymatic conversion steps) may be combined with various genetic modification(s) to increase production of and/or flux through malonyl-CoA, including increasing net activity (whether by increasing gene copy numbers, increasing activity, using a mutant form, etc.) of acetyl- coA carboxylase. The basis for this is observable in FIG. 2A, since malonyl-CoA is the product of this enzyme (ACCase being the abbreviation for acetyl¬CoA carboxylase in FIG. 2A).

CoA means coenzyme A; MSA means malonate semialdehyde; mcr means malonyl-CoA reductase (which may be monofunctional, reacting only malonyl¬CoA to MSA, or bifunctional, reacting this and also MSA to 3-HP (one bifunctional form being from Chloroflexus auriantiacus)); and ACP means acyl carrier protein. Also, representative (not to be limiting) gene names for E. coli genes that encode enzymes that carry out the indicated enzymatic conversion steps are provided. Table 3 provides additional information about these enzymatic conversion steps, which is incorporated into FIG. 2A.

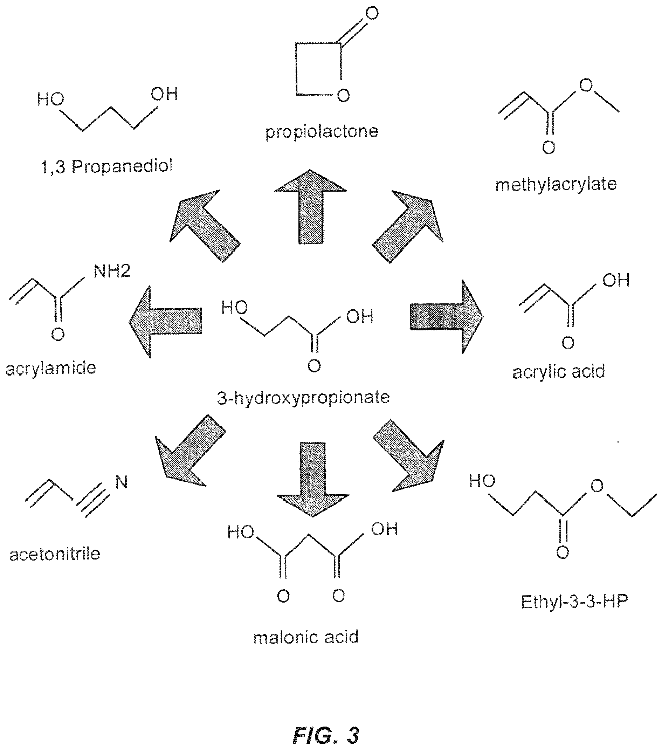

FIG. 3 shows various chemicals that may be derived from 3-HP. This is not meant to be limiting, and it is known that numerous products may be produced from each of these chemicals that may be derived from 3- HP.

Such products are referred to herein as 3-HP derivative products. Various reactions to obtain these and other chemical products from these are described in Ulmann's Encyclopedia of Industrial Chemistry, Acrylic Acid and Derivatives, Wiley VCH Verlag GmbH, Wienham (2005), incorporated by reference for its teachings of conversion reactions to these chemical products, and products derived from these, including for acrylic acid and its derivatives.

It is within the scope of the present invention to have a method of making any of the chemical compounds disclosed herein, including those in the preceding paragraph, that includes biosynthesis of malonyl- CoA as taught herein, wherein the malonyl-CoA is thereafter converted to a chemical product by the cell (e.g. 3-HP), and optionally thereafter converted to another chemical product such as those in or made from those in FIG. 3.

As indicated above, it was unexpected that particular combinations of modifications of fatty acid synthase pathway genes, such as modifications that transiently lower enzymatic function at elevated culture temperature or deletion of a gene encoding an enzyme of the pathway, would increase chemical product of mcA, 3-HP biosynthesis (such as measured by specific productivity). Particular combinations of such genetic modifications are described below, including in the examples, which are incorporated into this section.

Further, it has been found that combining genetic modifications directed to different metabolic effects in a host cell leads to greater production of a selected chemical product. In various embodiments, at least one genetic modification along a pathway to a selected chemical product is combined with one or more, two or more, or three or more, genetic modifications that are effective to reduce the overall activity and/or flux through one or more, two or more, or three or more, enzymatic conversion steps of a host cell's fatty acid synthase pathway. The enzymatic functions that catalyze the enzymatic conversions steps of the representative fatty acid synthase pathway, depicted in FIG. 2A, are summarized in Table 3, with reference to sequence listing numbers that correspond to representative DNA and amino acid sequences for these enzymatic functions in E. coli. These may be modified in accordance with embodiments of the invention.

**TABLE 3**

| **Enzyme Function(s) (with synonyms)** | **Enzyme Commission (EC) Number** | **Names of** | **SEQID NO. of** |
|---|---|---|---|
| malonyl-CoA-acyl carrier protein transacylase | 2.3.1.39 | fabD | 007 |
| B ketoacyl-acyl carrier protein synthase II, B-ketoacyl-ACP synthase II, 3-oxoacyl-ACP synthas II, KASII, acyl-[acyl carrier protein]:malonyl-[acyl carrier protein] C-acyl transferase (decarboxylating), | 2.3.41.41 | fabF | 008 |
| B-ketoacyl-ACP synthase, -ketoacyl-acyl carrier protein synthase I, 3-oxoacyl-ACP-synthase I, KASI | | fabB | 009 |
| B-ketoacyl-acyl carrier protein synthase III, B-ketoacyl-ACP synthase III, 3-oxoacyl-ACP synthase III, KASIII | | fabH | 010 |
| B-ketoacyl-[acyl-carrier protein] reductase; 3-oxoacyl-[acyl-carrier-protein] reductase | 1.1.1.100 | fabG | 011 |
| B-hydroxyacl-ACP dehydratase, 3-hydroxyacyl-[acp] dehydratase | 4.2.1.59 | fabZ | 012 |
| | | fabA | 013 |
| enoyl-ACP reductase (NADH), enoyl-acyl carrier protein (ACP) reductase | 1.3.1.9; 1.3.1.10 | fabI | 014 |

While not meant to be limiting one approach to modification is to use a temperature sensitive mutant of one or more of the above polypeptides. For example, a temperature-sensitive FabB is known, having the following mutation: A329V. Also, a temperature-sensitive FabI is known, having the following mutation: (S241F). Either or both of these may be combined with other modifications, such as a deletion of FabF. Non- limiting examples of such combinations are provided in the Examples section in strains having other indicated genetic modifications including introduction of specific plasmids.

It also is noted that any combination of modifications to reduce activity along the fatty acid synthesis pathway downstream of malonyl-CoA may include one or more modifications of the polypeptides that are responsible for enzymatic conversion steps shown in FIGs. 2B-D.

As noted herein, various aspects of the present invention are directed to a microorganism cell that comprises a metabolic pathway from malonyl-CoA to a chemical product of interest, such as those described above, and means for modulating conversion of malonyl-CoA to fatty acyl molecules (which thereafter may be converted to fatty acids) also are provided. Then, when the means for modulating modulate to decrease such conversion, a proportionally greater number of malonyl-CoA molecules are 1) produced and/or 2) converted via the metabolic pathway from malonyl-CoA to the chemical product. In various embodiments, additional genetic modifications may be made, such as to 1) increase intracellular bicarbonate levels, such as by increasing carbonic anhydrase, 2) increase enzymatic activity of acetyl-CoA carboxylase, and NADPH-dependent transhydrogenase, 3) increase production of coenzyme A.

In various embodiments the production of a selected chemical product is not linked to microorganism growth, that is to say, there are non-growth coupled embodiments in which production rate is not linked metabolically to cellular growth. For example, a microbial culture may be brought to a desired cell density, followed by a modulation (such as temperature shift of a temperature-sensitive protein) resulting in less malonyl-CoA being converted to fatty acids (generally needed for growth) and thus more to production of a selected chemical product.

Other additional genetic modifications are disclosed herein for various embodiments.

Included among additional genetic modifications are genetic modifications directed to reduce (including eliminate) flux through and/or activity of enzymes in the glyoxylate bypass of the microorganism, for example one or more of malate synthase, isocitrate lyase, and isocitrate dehydrogenase kinase-phosphatase. Such modification has been demonstrated to substantially reduce the flux of carbon through oxaloacetate generated via the glyoxylate bypass shunt. For example, in E. coli a deletion of the operon identified as aceBAK may be made.

More generally embodiments of the invention comprise a modification to reduce production of undesired metabolic products, which may be selected from various amino acids and other metabolic products, said modification resulting in a reduction (including elimination) of enzymatic activity of or an enzyme of or controlling flux through the glyoxylate bypass, optionally further selected from one or more of malate synthase A, isocitrate lyase, and kinase-phosphatase that controls activity of an isocitrate dehydrogenase.

As one example, not to be limiting, a deletion may be made to aceBAK in an E. coli strain that comprises other genetic modifications taught herein. One example of an E. coli strain comprising such deletion is:

**TABLE 4**

| **Strain** | **Parent** | **Genotype** | **Plasmid(s)** |
|---|---|---|---|
| BX3_547 | BX_0775.0 | *F-, Δ(araD-araB)567, ΔlacZ4787(::rrnB-3), LAM-, rph-1, Δ(rhaD-rhaB)568, hsdR514,* ΔldhA::frt, ApflB::frt, ΔmgsA::frt, ΔpoxB::frt, Δpta-ack::frt, fabI(ts) (S241F)-zeoR, fabB(ts), ΔfabF::frt, coaA*, fabD(ts), ΔaceBAK::frt | pTRC-kan-PyibD-mcr, pACYC-CAT-accADBC/pntAB |

Genotype traits denoted in bold italic font are present in the BW25113 host strain (available from the Coli Genetic Stock Center, Yale University, New Haven, CT USA). Additional genotype traits denoted in normal font were engineered by the inventors using standard methods as described and/or referenced herein. Parent BX_0775.0 is E. coli BW25113 (modifications shown in bold italic font) to which the other genetic modifications in the above genotype were made by methods described elsewhere herein.

Strain BX3_547 also comprises the plasmids identified in the above table. In pTRC-kan-PyibD-mcr, a promoter for malonyl-CoA reductase that is operative under low phosphate conditions. Phosphate starvation inducible promoters located upstream of native E. coli yibD and ytfK genes have been identified (Yoshida, et al., J Microbiol, 49(2), pp285-289, 2010). The target promoter sequence was ordered (Integrated DNA Technologies, Coralville, Iowa USA) including with modifications to the native ribosome binding site to be compatible with existing expression vectors and to accommodate expression of key downstream gene(s) within the vector(s) (see examples).

Strain BX3_547 was evaluated under various fermentation conditions that modulated or otherwise controlled temperature, pH, oxygen concentration, glucose feed rate and concentration, and other media conditions. Among the parameters used to determine other process steps, low ambient phosphate concentration was used as a control point that lead to temperature shift from approximately 30°C to approximately 37°C. A promoter sensitive to low ambient phosphate was utilized to control expression of the gene encoding malonyl-CoA reductase in the plasmid identified as pTrc-PyibD-mcr (SEQ ID NO:170). Also, during one or more evaluations, any one or more of dissolved oxygen, redox potential, aeration rate, agitation rate, oxygen transfer rate, and oxygen utilization rate was/were used to control the system and/or measured.

Strain BX3_547 was evaluated over 36 fermentation events that were conducted over an 8 week period using FM11 medium (described in the Common Methods Section). Duration of the fermentation events were all less than 80 hours, of which a portion was after temperature increase to effectuate reduced enzymatic activity of fabl(ts), fabB(ts), and fabD(ts). While not meant to be particularly limiting in view of other genetic modifications and culture conditions that may be employed, these results demonstrated microbial performance over a range of reduced oxygen conditions, with final 3-HP titers ranging between 50 and 62 grams of 3-HP/liter of final culture media volume.

It is appreciated that the PyibD promoter, or a similar low-phosphate induction promoter, could be utilized in a genetic construct to induce any one or more of the sequences described and/or taught herein, so as to enable production of any other of the chemical products disclosed herein, including in the examples provided herein.

In various embodiments, expression of desired genes is induced when the environmental phosphate concentration is maintained or adjusted to a low, more particularly, an effectively low concentration. This may be achieved, for example, by introduction of a promoter induced by low phosphate concentration for promotion of one or more nucleic acid sequences of interest. For example, increased production of a desired chemical product may result when environmental phosphate concentration is at or less than a concentration effective for the increased production, including but not limited to when phosphate concentration is not detectable by standard analytical techniques. One example of a low phosphate promoter that may be used with such embodiments is the PyibD promoter, exemplified such as by SEQ ID NO:169.

Further, various embodiments of the invention accordingly are directed to methods, compositions and systems that regard culturing modified microorganisms, such as those comprising a low-phosphate inducible enzyme such as described above, in a two-phase approach, the first phase substantially to increase microorganism biomass and the second phase substantially to produce a desired product (such as but not limited to 3-HP).

To constructe pTRC-kan-PyibD-mcr, the promoter from *E. coli yibD,* with nearby native sequences and selected restriction sites, was synthesized (Integrated DNA Technologies). Also, changes to the native ribosome binding site were made to accommodate appropriate expression of MCR:

CACGTGCGTAATTGTGCTGATCTCTTATATAGCTGCTCTCATTATCTCTCTAC
CCTGAAGTGACTCTCTCACCTGTAAAAATAATATCTCACAGGCTTAATAGTTTCTTAA
TACAAAGCCTGTAAAACGTCAGGATAACTTCTGTGTAGGAGGATAATCCATGGAATT
CCGCACGTG (SEQ ID NO:211)

This sequence as provided in pTrc-PyibD-mcr to induce mcr is as follows:

GTGCGTAATTGTGCTGATCTCTTATATAGCTGCTCTCATTATCTCTCTACCCT
GAAGTGACTCTCTCACCTGTAAAAATAATATCTCACAGGCTTAATAGTTTCTTAATAC
AAAGCCTGTAAAACGTCAGGATAACTTCTGTGTAGGAGGATAATC (SEQ ID NO:210).

The Examples further describes the construction of the plasmid pTrc-PyibD-mcr (SEQ ID NO:170).

Strain BX3_547 also comprises coaA*, a pantothenate kinase which is refractory to feedback inhibition. An exemplary sequence of such coaA* is SEQ ID NO:173. CoaA* R106A Mutant Sequence (SEQ ID NO:173) is provided below. This coaA* mutant also is provided in other strains listed in Table 6.

ATGAGTATAAAAGAGCAAACGTTAATGACGCCTTACCTACAGTTTGACCGCA
ACCAGTGGGCAGCTCTGCGTGATTCCGTACCTATGACGTTATCGGAAGATGAGATCG
CCCGTCTCAAAGGTATTAATGAAGATCTCTCGTTAGAAGAAGTTGCCGAGATCTATT
TACCTTTGTCACGTTTGCTGAACTTCTATATAAGCTCGAATCTGCGCCGTCAGGCAGT
TCTGGAACAGTTTCTTGGTACCAACGGGCAACGCATTCCTTACATTATCAGTATTGCT
GGCAGTGTCGCGGTGGGAAAAGTACAACGGCGGCTGTGCTCCAGGCGCTATTAAG
CCGTTGGCCGGAACATCGTCGTGTTGAACTGATCACTACAGATGGCTTCCTTCACCCT
AATCAGGTTCTGAAAGAACGTGGTCTGATGAAGAAGAAAGGCTTCCCGGAATCGTA
TGATATGCATCGCCTGGTGAAGTTTGTTTCCGATCTCAAATCCGGCGTGCCAAACGTT
ACAGCACCTGTTTACTCACATCTTATTTATGATGTGATCCCGGATGGAGATAAAACG
GTTGTTCAGCCTGATATTTTAATTCTTGAAGGGTTAAATGTCTTACAGAGCGGGATG
GATTATCCACACGATCCACATCATGTATTTGTTTCTGATTTTGTCGATTTTTCGATATA
TGTTGATGCACCGGAAGACTTACTTCAGACATGGTATATCAACCGTTTTCTGAAATTC
CGCGAAGGGGCTTTTACCGACCCGGATTCCTATTTTCATAACTACGCGAAATTAACT
AAAGAAGAAGCGATTAAGACTGCCATGACATTGTGGAAAGAGATCAACTGGCTGAA
CTTAAAGCAAAATATTCTACCTACTCGTGAGCGCGCCAGTTTAATCCTGACGAAAAG
TGCTAATCATGCGGTAGAAGAGGTCAGACTACGCAAATAA (SEQ ID NO:173)

It is appreciated that the particular constructs in these examples, and the product obtained (3-HP), are not meant to be limiting. In various embodiments genetic constructs, microorganisms, methods and systems are produced and/or employed that comprise and utilize induction under low environmental phosphate conditions. Any of the products described herein, including those in Tables 1A-1H may be produced using a microorganism that comprises a modification that leads to induction of one or more enzymes under low phosphate conditions. Increased production of a selected chemical product may be obtained when phosphate concentration is maintained or adjusted to a low, more particularly, an effectively low concentration. This may be achieved, for example, by introduction of a promoter induced by low phosphate concentration for promotion of one or more nucleic acid sequences of interest. For example, increased production of a desired chemical product may result when environmental phosphate concentration is at or less than a concentration effective for the increased production, including but not limited to when phosphate concentration is not detectable by standard analytical techniques. One example of a low phosphate promoter is the PyibD promoter, exemplified such as by SEQ ID NO:210. This aspect of the invention may be combined, in any combination, with any of the other aspects of the invention taught herein.

Unexpected increases in specific productivity by a population of a genetically modified microorganism may be achieved in methods and systems in which that microorganism has a microbial production pathway from malonyl-CoA to a selected chemical product as well as a reduction in the enzymatic activity of a selected enzyme of the microorganism's fatty acid synthase system (more particularly, its fatty acid elongation enzymes). In various embodiments, specific supplements to a bioreactor vessel comprising such microorganism population may also be provided to further improve the methods and systems.

In various embodiments one or more, two or more, or three or more, enzymatic conversion steps of a host cell's pathway(s) to biosynthesize coenzyme-A ("CoA", "coA", "Co-A" or "co-A") are modified to increase cellular production of CoA. This further increases chemical product biosynthesis when combined with either or both of the two types of genetic modifications just described- regarding increasing production of a chemical product and toward reducing fatty acid synthase pathway activities/flux.

Accordingly, in various embodiments of the invention, to more effectively redirect malonyl-CoA from fatty acid synthesis and toward the biosynthesis of such a desired chemical product, genetic modifications are made to:
(a) increase production along the biosynthetic pathway that includes malonyl-CoA and leads to a desired chemical product;
(b) reduce the activity of one or more, or of two or more, or of three or more of enoyl- acyl carrier protein (ACP) reductase, B-ketoacyl-acyl carrier protein synthase I (such as fabB), B-ketoacyl-acyl carrier protein synthase II, and malonyl-CoA-acyl carrier protein transacylase; and optionally to c. increase the production of coenzyme A in the microorganism cell.

In various microorganisms conversion of the metabolic intermediate malonyl-CoA to fatty acids via a fatty acid synthase (also referred to by "synthesis") system (also referred to as "pathway" or "complex") is the only or the major use of malonyl-CoA. A representative fatty acid synthase pathway known to function in microorganisms is depicted in FIG. 2A. This has a cyclic component by which fatty acid molecules are elongated to a final length; these may thereafter be further modified to phospholipids, etc., which are used in cell membranes and other cellular functions.

It has been determined that when a production pathway to an alternative chemical product exists in a microorganism, reducing such conversion of malonyl-CoA to fatty acids can improve metrics for production of that alternative chemical product (e.g., a polyketide or 3-HP). For example, as depicted in FIG. 2A and listed in Table 3, in many microorganism cells the fatty acid synthase system comprises polypeptides that have the following enzymatic activities: malonyl-CoA-acyl carrier protein (ACP) transacylase; B-ketoacyl-ACP synthase; B-ketoacyl-ACP reductase; B-hydroxyacyl-ACP dehydratase; 3- hydroxyacyl-(acp) dehydratase; and enoyl-acyl carrier protein reductase (enoyl-ACP reductase). In various embodiments nucleic acid sequences that encode temperature-sensitive forms of these polypeptides may be introduced in place of the native enzymes, and when such genetically modified microorganisms are cultured at elevated temperatures (at which these thermolabile polypeptides become inactivated, partially or completely, due to alterations in protein structure or complete denaturation), there is observed an increase in a product such as 3-HP, THN, or flaviolin. In other embodiments other types of genetic modifications may be made to otherwise modulate, such as lower, enzymatic activities of one or more of these polypeptides. In various embodiments a result of such genetic modifications is to shift malonyl-CoA utilization so that there is a reduced conversion of malonyl-CoA to fatty acids, overall biomass, and proportionally greater conversion of carbon source to a chemical product such as 3-HP. In various embodiments, the specific productivity for the microbially produced chemical product is unexpectedly high. Also, additional genetic modifications, such as to increase malonyl-CoA production, may be made for certain embodiments.

In various embodiments genetic modifications are made to reduce the overall function, whether measureable as enzymatic activity, enzyme concentration, and/or flux, of two or more, or of three or more, of the enzymatic functions of a host cell's fatty acid synthase pathway. These may be combined with other types of genetic modifications described herein, such as to a chemical product pathway and/or to genetic modifications that result in greater production of coenzyme A.

Accordingly, in some embodiments the present invention comprises a genetically modified microorganism that comprises at least one genetic modification that provides, completes, or enhances a chemical production pathway effective to convert malonyl-CoA to a chemical product, and further comprises at least two, or at least three genetic modifications of at least two, or at least three enzymes of the fatty acid synthase system of a host cell, such as selected from enoyl-acyl carrier protein reductase (enoyl-ACP reductase) or enoyl-coenzyme A reductase (enoyl-CoA reductase), B-ketoacyl-ACP synthase or B-ketoacyl-CoA synthase, malonyl-CoA-ACP, where such latter genetic modifications have a cumulative effect to reduce conversion of malonyl-CoA to fatty acids. Other genetic modifications may be provided to such host cell as described elsewhere herein. The latter include those depicted in FIG. 1, such as but not limited to a genetic modification of carbonic anhydrase to increase bicarbonate levels in the microorganism cell (and/or a supplementation of its culture medium with bicarbonate and/or carbonate), and one or more genetic modifications to increase enzymatic activity of one or more of acetyl-CoA carboxylase and NADPH-dependent transhydrogenase. Related methods and systems utilize such any of such genetically modified microorganisms.

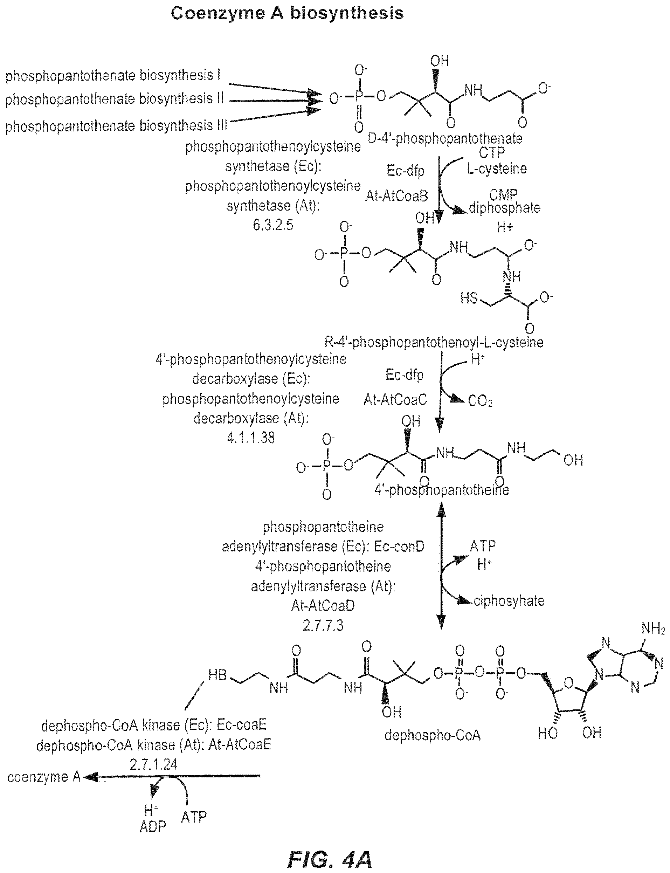

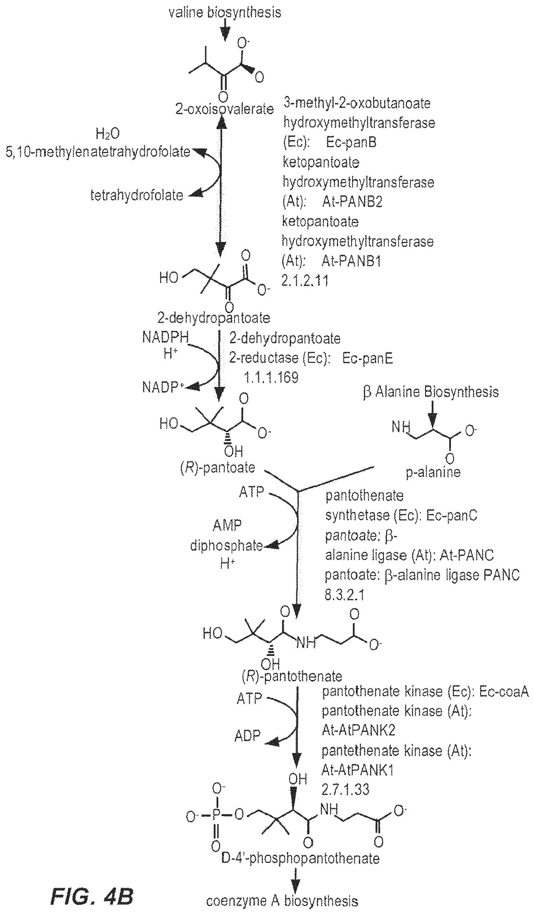

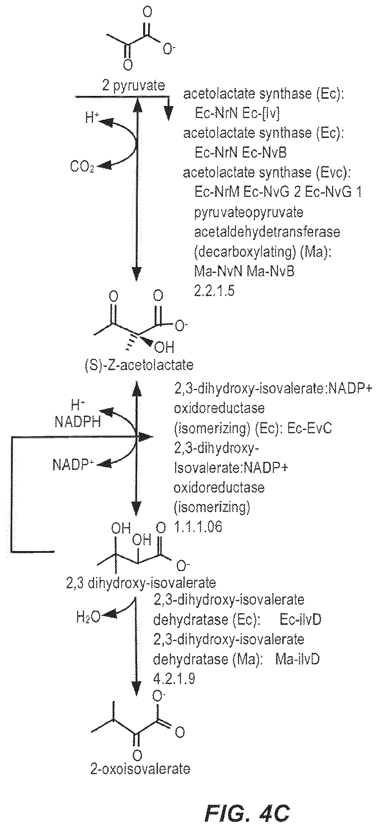

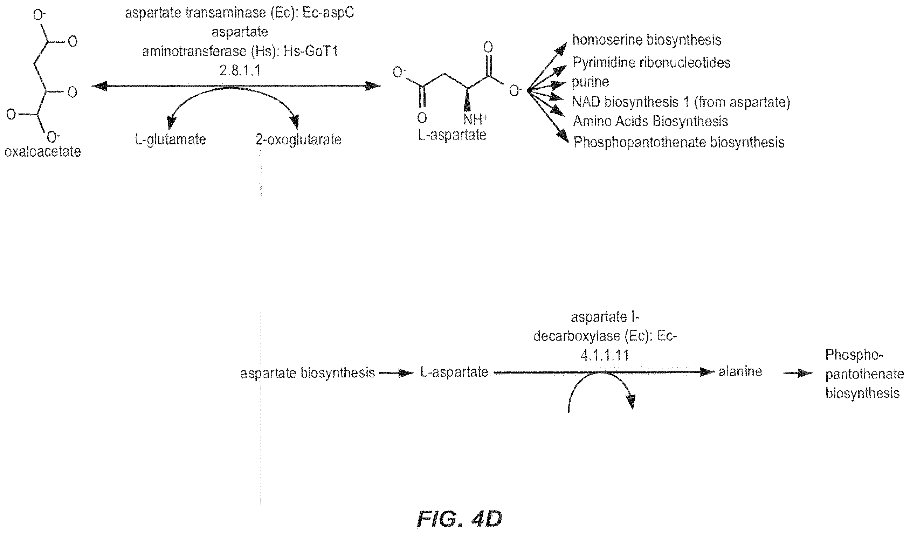

Also, as noted, the invention may comprise, in various embodiments, one or more genetic modifications that result in greater production of coenzyme A. Such genetic modification may be to any of the genes encoding enzymes along the pathways leading to production of coenzyme A. For example, FIGs. 4A-D depict representative biosynthetic pathways (and portions thereof) that lead to coenzyme A. Any of the genes encoding these enzymes may be modified to increase respective enzymatic conversion step activity and/or flux in order to increase coenzyme A production in a host cell. In a particular embodiment, one or more, two or more, or three or more genetic modifications may be made to achieve this end. Table 5 summarizes the enzymatic conversions shown in FIGs. 4A-D.

**TABLE 5**

| **Enzyme Function** | **E.C. Classification** | **Gene Name in E. coli** |
|---|---|---|
| aspartate transaminase | 2.6.1.1 | aspC |
| aspartate 1-decarboxylase | 4.1.1.11 | panD |
| acetolactate synthase | 2.2.1.6 | i1vH, ilvi |
| 2,3-dihydroxy-isovalerate:NADP+ oxidoreductase (isomerizing) | 1.1.1.86 | ilvC |
| 2,3-dihydroxy-isovalerate dehydratase | 4.2.1.9 | ilvD |
| 3-methyl-2-oxobutanoate | 2.1.2.11 | panB |
| 2-dehydropantoate | 1.1.1.169 | panE |
| pantothenate synthetase | 6.3.2.1 | pane |
| pantothenate kinase | 2.7.1.33 | coaA (panK) |
| phosphopantothenoylcysteine synthetase | 6.3.2.5 | dfp |
| 4'-phosphopantothenoylcysteine decarboxylase | 4.1.1.36 | dfp |
| phosphopantetheine adenylytransferase | 2.7.7.3 | coaD |
| Dephospho-CoA kinase | 2.7.1.24 | coaE |

A plurality of strains are described in the Examples section, hereby incorporated into this section. In addition, the following table lists additional strains of value in production of chemical products such as 3-HP and related chemicals. For construction of these strains, generally all plasmids were introduced at the same time via electroporation using standard methods. Transformed cells were grown on the appropriate media with antibiotic supplementation and colonies were selected based on their appropriate growth on the selective media. The base strains were derived from E. coli BW25113 (F-, Δ(araD-araB)567, ΔlacZ4787(::rrnB-3), lamba-, rph-1, Δ(rhaD-rhaB)568, hsdR514), these strains comprising additional chromosomal modifications generally introduced using Gene Bridges technology as described herein, such as in the Common Methods Section. Temperature-sensitive mutant forms are designated herein by "ts" or "(ts)," either of which may be as a superscript.

**TABLE 6**

| **Strain** | **Background** | **Genotype** | **Plasmids** |
|---|---|---|---|
| BX3_0451.0 | BX_0701.0 | F-, Δ(araD-araB)567, ΔlacZ4787(::rrnB-3), LAM-, rph-1, Δ(rhaD-rhaB)568, hsdR514, , ΔldhA:frt, ΔpflB:frt, ΔmgsA:frt,ΔpoxB:frt, Δpta-ack:frt, fabI^{ts} (S241F)-zeoR, Δalda::CSC | pTRC-KAN-mcr, pACYC-CAT-accADBC/pntAB |
| BX3_0467 | BX 00704.0 | F-, Δ(araD-araB)567, ΔlacZ4787(::rrnB-3), LAM-, rph-1, Δ(rhaD-rhaB)568, hsdR514,, ΔldhA::frt, ΔpflB::frt, ΔmgsA::frt, ΔpoxB::frt, Δpta-ack::frt, fabIts (S241F)-zeoR, ΔfabF::frt | pTRC-KAN-mcr, pACYC-CAT-accADBC/pntAB |
| BX3_0472 | BX_00706.0 | F-, Δ(araD-araB)567, ΔlacZ4787(::rrnB-3), LAM-, rph-1, Δ(rhaD-rhaB)568, hsdR514, , ΔldhA::frt, ΔpflB::frt, ΔmgsA::frt, ΔpoxB::frt, Δpta-ack::frt, fabIts (S241F)-zeoR, fabBts, ΔfabF::frt | pTRC-KAN-mcr, pACYC-CAT-accADBC/pntAB |
| BX3_0478 | BX_00725.0 | F-, Δ(araD-araB)567, ΔlacZ4787(::rrnB-3), LAM-, rph-1, Δ(rhaD-rhaB)568, hsdR514,, ΔldhA:frt, ΔpflB:frt, ΔmgsA:frt,ΔpoxB:frt, Δpta-ack:frt, fabI^{ts} (S241F)-zeoR, coaA* | pTRC-KAN-mcr, pACYC-CAT-accADBC/pntAB |
| BX3_0491.0 | BX_0726.0 | F-, Δ(araD-araB)567, ΔlacZ4787(::rrnB-3), LAM-, rph-1, Δ(rhaD-rhaB)568, hsdR514,, ΔldhA::frt, ΔpflB::frt, ΔmgsA::frt,ΔpoxB::frt, Δpta-ack::frt, fablts (S241F)-zeoR, fabDts | pTRC-KAN-mcr, pACYC-CAT-accADBC/pntAB |
| BX3_0492.0 | BX_0735.0 | F-, Δ(araD-araB)567, ΔlacZ4787(::rrnB-3), LAM-, rph-1, Δ(rhaD-rhaB)568, hsdR514, , ΔldhA::frt, ΔpflB::frt, ΔmgsA::frt, ΔpoxB::frt, Δpta-ack::frt, fabIts (S241F)-zeoR, fabBts, ΔfabF::frt, coaA* | pTRC-KAN-mcr, pACYC-CAT-accADBC/pntAB |
| BX3_0495.0 | BX0746.0 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts), ΔfabF::frt, fabD(ts) | pTRC-KAN-mcr, pACYC-CAT-accADBC/pntAB |
| BX3_0494.0 | BX_0738.0 | F-, Δ(araD-araB)567, ΔlacZ4787(::rrnB-3), LAM-, rph-1, Δ(rhaD-rhaB)568, hsdR514, , ΔldhA::frt, ΔpflB::frt, ΔmgsA::frt, ΔpoxB::frt, Δpta-ack::frt, fabIts (S241F)-zeoR, fabBts, ΔfabF::frt, coaA*, fabDts | pTRC-KAN-mcr, pACYC-CAT-accADBC/pntAB |
| BX3_0501.0 | BX_0728.0 | F-, Δ(araD-araB)567, ΔlacZ4787(::rrnB-3), LAM-, rph-1, Δ(rhaD-rhaB)568, hsdR514, , ΔldhA::frt, ΔpflB::frt, ΔmgsA::frt,ΔpoxB::frt, Δpta-ack::frt, fabIts (S241F)-zeoR, ΔgapA::frt | pTRC-KAN-ptrc-mcr-gapA, pACYC-CAT-accADBC/pntAB-ccdAB |
| BX3_0537.0 | BX_0775.0 | F-, Δ(araD-araB)567, ΔlacZ4787(::rrnB-3), LAM-, rph-1, Δ(rhaD-rhaB)568, hsdR514, , ΔldhA::frt, ΔpflB::frt, ΔmgsA::frt, Δpta-ack::frt, fabIts (S241F)-zeoR, fabBts, ΔfabF::frt, coaA*, fabDts, ΔaceBAK::frt | pTRC-KAN-ptrc-mcr, pACYC-CAT-accADBC/pntAB |
| BX3_0538.0 | BX_0775.0 | F-, Δ(araD-araB)567, ΔlacZ4787(::rrnB-3), LAM-, rph-1, Δ(rhaD-rhaB)568, hsdR514, , ΔldhA::frt, ΔpflB::frt, ΔmgsA::frt, Δpta-ack::frt, fabIts (S241F)-zeoR, fabBts, ΔfabF::frt, coaA*, fabDts, ΔaceBAK::frt | pTRC-KAN-ptrc-mcr-gapA, pACYC-CAT-accADBC/pntAB-ccdAB |
| BX3_0547.0 | BX_0775.0 | F-, Δ(araD-araB)567, ΔlacZ4787(::rrnB-3), LAM-, rph-1, Δ(rhaD-rhaB)568, hsdR514, , ΔldhA::frt, ΔpflB::frt, ΔmgsA::frt, Δpta-ack::frt, fabIts (S241F)-zeoR, fabBts, ΔfabF::frt, coaA*, fabDts, ΔaceBAK::frt | pTRC-KAN-PyibD--mcr, pACYC-CAT-accADBC/pntAB |

The genetic modifications described and exemplified herein may be provided in various combinations in a microorganism strain so as to achieve a desired improvement in production rate, yield, and final titer of a selected chemical product. Various embodiments of the invention additionally may comprise a genetic modification to increase the availability of the cofactor NADPH, which can increase the NADPH/NADP+ ratio as may be desired. Non-limiting examples for such genetic modification are pgi (E.C. 5.3.1.9, in a mutated form), pntAB (E.C. 1.6.1.2), overexpressed, gapA (E.C. 1.2.1.12):gapN (E.C. 1.2.1.9, from Streptococcus mutans) substitution/replacement (including replacement in a plasmid), and disrupting or modifying a soluble transhydrogenase such as sthA (E.C. 1.6.1.2), and/or genetic modifications of one or more of zwf (E.C. 1.1.1.49), gnd (E.C. 1.1.1.44), and edd (E.C. 4.2.1.12). Sequences of these genes are available at <<www.metacyc.org>>, and also are available at <<www.ncbi.gov>> as well as <<www.ecocyc.org>>.

Polypeptides, such as encoded by the various specified genes, may be NADH- or NADPH¬dependent, and methods known in the art may be used to convert a particular enzyme to be either form. More particularly, as noted in WO 2002/042418, "any method can be used to convert a polypeptide that uses NADPH as a cofactor into a polypeptide that uses NADH as a cofactor such as those described by others (Eppink et al., J Mol. Biol., 292 (1): 87-96 (1999), Hall and Tomsett, Microbiology, 146 (Pt 6): 1399-406 (2000), and Dohr et al., Proc. Natl. Acad. Sci., 98 (1) : 81-86 (2001))."

### Redirecting Malonyl-CoA from Fatty Acid Synthesis to a Chemical Product

Compositions of the present invention, such as genetically modified microorganisms, comprise a production pathway for a chemical product in which malonyl-CoA is a substrate, and may also comprise one or more genetic modifications to reduce the activity of enzymes encoded by one or more of the fatty acid synthetase system genes. The compositions may be used in the methods and systems of the present invention.

Regarding microbial fermentation of a number of chemical products in many microorganisms of commercial fermentation interest, malonyl-CoA is a metabolic intermediate that, under normal growth conditions, is converted to fatty acids and derivatives thereof, such as phospholipids, that are then used in cell membranes and for other key cellular functions. For example, in Escherichia coli, the fatty acid synthase system is a type II or dissociated fatty acid synthase system. In this system the enzymes of fatty acid production pathway are encoded by distinct genes, and, common for many critical metabolic pathways, is well-regulated, including by downstream products inhibiting upstream enzymes.

In various microorganisms conversion of the metabolic intermediate malonyl-CoA to fatty acids via a fatty acid synthesis system (i.e., pathway or complex) is the only or the major use of malonyl-CoA. It has been determined that when a production pathway to an alternative chemical product exists in a microorganism, reducing such conversion of malonyl-CoA to fatty acids can improve metrics for production of that alternative chemical product (e.g., a polyketide or 3-HP). For example, in many microorganism cells the fatty acid synthase system comprises polypeptides that have the following enzymatic activities: malonyl-CoA-acyl carrier protein (ACP) transacylase; β-ketoacyl-ACP synthase; β-ketoacyl-ACP reductase; β-hydroxyacyl-ACP dehydratase; 3-hydroxyacyl-(acp) dehydratase; and enoyl-acyl carrier protein reductase (enoyl-ACP reductase). In various embodiments nucleic acid sequences that encode temperature-sensitive forms of these polypeptides may be introduced in place of the native enzymes, and when such genetically modified microorganisms are cultured at elevated temperatures (at which these thermolabile polypeptides become inactivated, partially or completely, due to alterations in protein structure or complete denaturation), there is observed an increase in a product such as 3-HP THN or flaviolin. In other embodiments other types of genetic modifications may be made to otherwise modulate, such as lower, enzymatic activities of one or more of these polypeptides. In various embodiments a result of such genetic modifications is to shift malonyl-CoA utilization so that there is a reduced conversion of malonyl-CoA to fatty acids, overall biomass, and proportionally greater conversion of carbon source to a chemical product such as 3-HP. In various embodiments, the specific productivity for the microbially produced chemical product is unexpectedly high. Also, additional genetic modifications, such as to increase malonyl-CoA production, may be made for certain embodiments.

One enzyme, enoyl(acyl carrier protein) reductase (EC No. 1.3.1.9, also referred to as enoyl-ACP reductase) is a key enzyme for fatty acid biosynthesis from malonyl-CoA. In Escherichia coli this enzyme, FabI, is encoded by the gene fabl (See "Enoyl-Acyl Carrier Protein (fab1) Plays a Determinant Role in Completing Cycles of Fatty Acid Elongation in Escherichia coli," Richard J. Heath and Charles 0. Rock, J. Biol. Chem. 270:44, pp. 26538-26543 (1995), incorporated by reference for its discussion of fabl and the fatty acid synthase system).

The present invention may utilize a microorganism that is provided with a nucleic acid sequence (polynucleotide) that encodes a polypeptide having enoyl-ACP reductase enzymatic activity that may be modulated during a fermentation event. For example, a nucleic acid sequence encoding a temperature-sensitive enoyl-ACP reductase may be provided in place of the native enoyl-ACP reductase, so that an elevated culture temperature results in reduced enzymatic activity, which then results in a shifting utilization of malonyl-CoA to production of a desired chemical product. At such elevated temperature the enzyme is considered non-permissive, as is the temperature. One such sequence is a mutant temperature-sensitive fabl (fabI(TS)) of E. coli, SEQ ID NO:28 for DNA, SEQ ID NO:29 for protein.

It is appreciated that nucleic acid and amino acid sequences for enoyl-ACP reductase in species other than E. coli are readily obtained by conducting homology searches in known genomics databases, such as BLASTN and BLASTP. Approaches to obtaining homologues in other species and functional equivalent sequences are described herein. Accordingly, it is appreciated that the present invention may be practiced by one skilled in the art for many microorganism species of commercial interest.

Other approaches than a temperature-sensitive enoyl-ACP reductase may be employed as known to those skilled in the art, such as, but not limited to, replacing a native enoyl-ACP or enoyl-CoA reductase with a nucleic acid sequence that includes an inducible promoter for this enzyme, so that an initial induction may be followed by no induction, thereby decreasing enoyl-ACP or enoyl-CoA reductase enzymatic activity after a selected cell density is attained.

In some aspects, compositions, methods and systems of the present invention shift utilization of malonyl-CoA in a genetic modified microorganism, which comprises at least one enzyme of the fatty acid synthase system, such as enoyl-acyl carrier protein reductase (enoyl-ACP reductase) or enoyl-coenzyme A reductase (enoyl-CoA reductase), β-ketoacyl-ACP synthase or β-ketoacyl-CoA synthase malonyl-CoA-ACP, and may further comprise at least one genetic modification of nucleic acid sequence encoding carbonic anhydrase to increase bicarbonate levels in the microorganism cell and/or a supplementation of its culture medium with bicarbonate and/or carbonate, and may further comprise one or more genetic modifications to increase enzymatic activity of one or more of acetyl-CoA carboxylase and NADPH-dependent transhydrogenase. More generally, addition of carbonate and/or bicarbonate may be used to increase bicarbonate levels in a fermentation broth.

In some aspects, the present invention comprises a genetically modified microorganism that comprises at least one genetic modification that provides, completes, or enhances a 3-HP production pathway effective to convert malonyl-CoA to 3-HP, and further comprises a genetic modification of carbonic anhydrase to increase bicarbonate levels in the microorganism cell and/or a supplementation of its culture medium with bicarbonate and/or carbonate, and may further comprise one or more genetic modifications to increase enzymatic activity of one or more of acetyl-CoA carboxylase and NADPH-dependent transhydrogenase. Related methods and systems utilize such genetically modified microorganism.

In some aspects, the present invention comprises a genetically modified microorganism that comprises at least one genetic modification that provides, completes, or enhances a 3-HP production pathway effective to convert malonyl-CoA to 3-HP, and further comprises a genetic modification of at least one enzyme of the fatty acid synthase system, such as enoyl-acyl carrier protein reductase (enoyl-ACP reductase) or enoyl-coenzyme A reductase (enoyl-CoA reductase), β-ketoacyl-ACP synthase or β-ketoacyl-CoA synthase, malonyl-CoA-ACP, and may further comprise a genetic modification of carbonic anhydrase to increase bicarbonate levels in the microorganism cell and/or a supplementation of its culture medium with bicarbonate and/or carbonate, and may further comprise one or more genetic modifications to increase enzymatic activity of one or more of acetyl-CoA carboxylase and NADPH-dependent transhydrogenase. Related methods and systems utilize such genetically modified microorganism.

In some aspects, the present invention comprises a genetically modified microorganism that comprises at least one genetic modification that provides, completes, or enhances a 3-HP production pathway effective to convert malonyl-CoA to 3-HP, and further comprises a genetic modification of carbonic anhydrase to increase bicarbonate levels in the microorganism cell and/or a supplementation of its culture medium with bicarbonate and/or carbonate, and may further comprise one or more genetic modifications to increase enzymatic activity of one or more of acetyl-CoA carboxylase and NADPH-dependent transhydrogenase.

In some aspects, the present invention comprises a genetically modified microorganism that comprises at least one genetic modification that provides, completes, or enhances a 3-HP production pathway effective to convert malonyl-CoA to 3-HP, and further comprises a genetic modification of at least one enzyme of the fatty acid synthase system, such as enoyl-acyl carrier protein reductase (enoyl-ACP reductase) or enoyl-coenzyme A reductase (enoyl-CoA reductase), β-ketoacyl-ACP synthase or β-ketoacyl-CoA synthase , malonyl-CoA-ACP, and may further comprise a genetic modification of carbonic anhydrase to increase bicarbonate levels in the microorganism cell and/or a supplementation of its culture medium with bicarbonate and/or carbonate, and may

In various embodiments the present invention is directed to a method of making a chemical product comprising: providing a selected cell density of a genetically modified microorganism population in a vessel, wherein the genetically modified microorganism comprises a production pathway for production of a chemical product from malonyl-CoA; and reducing enzymatic activity of at least one enzyme of the genetically modified microorganism's fatty acid synthase pathway.

In various embodiments, reducing the enzymatic activity of an enoyl-ACP reductase in a microorganism host cell results in production of 3-HP at elevated specific and volumetric productivity. In still other embodiments, reducing the enzymatic activity of an enoyl-CoA reductase in a microorganism host cell results in production of 3-HP at elevated specific and volumetric productivity.

Another approach to genetic modification to reduce enzymatic activity of these enzymes is to provide an inducible promoter that promotes one such enzyme, such as the enoyl-ACP reductase gene (e.g., fabl in E. coli). In such example this promoter may be induced (such as with isopropyl-u-D-thiogalactopyranoiside (IPTG)) during a first phase of a method herein, and after the IPTG is exhausted, removed or diluted out the second step, of reducing enoyl-ACP reductase enzymatic activity, may begin. Other approaches may be applied to control enzyme expression and activity such as are described herein and/or known to those skilled in the art.

While enoyl-CoA reductase is considered an important enzyme of the fatty acid synthase system, genetic modifications may be made to any combination of the polynucleotides (nucleic acid sequences) encoding the polypeptides exhibiting the enzymatic activities of this system, such as are listed herein. For example, FabB, β-ketoacyl-acyl carrier protein synthase I, is an enzyme in E. coli that is essential for growth and the biosynthesis of both saturated and unsaturated fatty acids. Inactivation of FabB results in the inhibition of fatty acid elongation and diminished cell growth as well as eliminating a futile cycle that recycles the malonate moiety of malonyl-ACP back to acetyl-CoA. FabF, β-ketoacyl-acyl carrier protein synthase II, is required for the synthesis of saturated fatty acids and the control membrane fluidity in cells. Both enzymes are inhibited by cerulenin.

It is reported that overexpression of FabF results in diminished fatty acid biosynthesis. It is proposed that FabF outcompetes FabB for association with FabD, malonyl-CoA:ACP transacylase. The association of FabB with FabD is required for the condensation reaction that initiates fatty acid elongation. (See Microbiological Reviews, Sept. 1993, p. 522-542 Vol. 57, No. 3; K. Magnuson et al., "Regulation of Fatty Acid Biosynthesis in Escherichia coli," American Society for Microbiology; W. Zha et al., "Improving cellular malonyl-CoA level in Escherichia coli via metabolic engineering," Metabolic Engineering 11 (2009) 192-198). An alternative to genetic modification to reduce such fatty acid synthase enzymes is to provide into a culture system a suitable inhibitor of one or more such enzymes. This approach may be practiced independently or in combination with the genetic modification approach. Inhibitors, such as cerulenin, thiolactomycin, and triclosan (this list not limiting) or genetic modifications directed to reduce activity of enzymes encoded by one or more of the fatty acid synthetase system genes may be employed, singly or in combination.

Without being bound to a particular theory, it is believed that reducing the enzymatic activity of enoyl-ACP reductase (and/or of other enzymes of the fatty acid synthase system) in a microorganism leads to an accumulation and/or shunting of malonyl-CoA, a metabolic intermediate upstream of the enzyme, and such malonyl-CoA may then be converted to a chemical product for which the microorganism cell comprises a metabolic pathway that utilizes malonyl-CoA. In certain compositions, methods and systems of the present invention the reduction of enzymatic activity of enoyl-ACP reductase (or, more generally, of the fatty acid synthase system) is made to occur after a sufficient cell density of a genetically modified microorganism is attained. This bi-phasic culture approach balances a desired quantity of catalyst, in the cell biomass which supports a particular production rate, with yield, which may be partly attributed to having less carbon be directed to cell mass after the enoyl-ACP reductase activity (and/or activity of other enzymes of the fatty acid synthase system) is/are reduced. This results in a shifting net utilization of malonyl-CoA, thus providing for greater carbon flux to a desired chemical product.

In various embodiments of the present invention the specific productivity is elevated and this results in overall rapid and efficient microbial fermentation methods and systems. In various embodiments the volumetric productivity also is substantially elevated.

In various embodiments a genetically modified microorganism comprises a metabolic pathway that includes conversion of malonyl-CoA to a desired chemical product, 3-hydroxypropionic acid (3-HP). This is viewed as quite advantageous for commercial 3-HP production economics and is viewed as an advance having clear economic benefit.

In various embodiments a genetically modified microorganism comprises a metabolic pathway that includes conversion of malonyl-CoA to a selected chemical product, selected from various polyketides such as those described herein. This is viewed as quite advantageous for commercial production economics for such polyketide chemical products and is viewed as an advance having clear economic benefit. Other chemical products also are disclosed herein.

The improvements in both specific and volumetric productivity parameters are unexpected and advance the art.

The reduction of enoyl-ACP reductase activity and/or of other enzymes of the fatty acid synthase system may be achieved in a number of ways, as is discussed herein.

By "means for modulating" the conversion of malonyl-CoA to fatty acyl-ACP or fatty acyl-CoA molecules, and to fatty acid molecules, is meant any one of the following: 1) providing in a microorganism cell at least one polynucleotide that encodes at least one polypeptide having activity of one of the fatty acid synthase system enzymes (such as recited herein), wherein the polypeptide so encoded has (such as by mutation and/or promoter substitution, etc., to lower enzymatic activity), or may be modulated to have (such as by temperature sensitivity, inducible promoter, etc.) a reduced enzymatic activity; 2) providing to a vessel comprising a microorganism cell or population an inhibitor that inhibits enzymatic activity of one or more of the fatty acid synthase system enzymes (such as recited herein), at a dosage effective to reduce enzymatic activity of one or more of these enzymes. These means may be provided in combination with one another. When a means for modulating involves a conversion, during a fermentation event, from a higher to a lower activity of the fatty acid synthetase system, such as by increasing temperature of a culture vessel comprising a population of genetically modified microorganism comprising a temperature-sensitive fatty acid synthetase system polypeptide (e.g., enoyl-ACP reductase), or by adding an inhibitor, there are conceived two modes - one during which there is higher activity, and a second during which there is lower activity, of such fatty acid synthetase system. During the lower activity mode, a shift to greater utilization of malonyl-CoA to a selected chemical product may proceed.

Once the modulation is in effect to decrease the noted enzymatic activity(ies), each respective enzymatic activity so modulated may be reduced by at least 10, at least 20, at least 30, at least 40, at least 50, at least 60, at least 70, at least 80, or at least 90 percent compared with the activity of the native, non-modulated enzymatic activity (such as in a cell or isolated). Similarly, the conversion of malonyl-CoA to fatty acyl-ACP or fatty acyl-CoA molecules may be reduced by at least 10, at least 20, at least 30, at least 40, at least 50, at least 60, at least 70, at least 80, or at least 90 percent compared with such conversion in a non-modulated cell or other system. Likewise, the conversion of malonyl-CoA to fatty acid molecules may be reduced by at least 10, at least 20, at least 30, at least 40, at least 50, at least 60, at least 70, at least 80, or at least 90 percent compared with such conversion in a non-modulated cell or other system.

Also Table 7 below provides additional information regarding genes and sequences that may be used in various embodiments of the invention. Information in this table is incorporated into the respective examples below.

**TABLE 7**

| **Enzyme Function** | **E.C. Classification** | **Gene Name(s)** |
|---|---|---|
| Phloroglucinol reductase | 1.3.1.57 | phloroglucinol reductase Eubacterium oxidoreducens 041). |
| 6-methylsalicylic-acid synthase | 2.3.1.165 | 6-MSAS |
| crotonyl-CoA reductase | 1.3.1.86 | ccr |
| acyl-coA thioesterase I | 3.1.1.5 | tesA |
| butanol dehydrogenase/ butanal dehydrogenase | 1.2.1.- | adhE2 |
| isobutanol dehydrogenase/isobutanal | 1.2.1.- | adhE |
| Butyryl-CoA dehydrogenase | 1.3.8.1 | bcd |
| Electron transfer flavoprotein | 1.3.8.1 | etfAB |
| phosphotransbutyrylase | 2.3.1.19 | ptb |
| butyrate kinase | 2.7.2.7 | bukl |
| (S)-3-hydroxybutyryl-CoA dehydrogenase | 1.1.1.35 | hbd |
| 3-hydroxybutyryl-CoA dehydratase | 4.2.1.55 | crt |
| (R)-3-hydroxybutyryl-CoA dehydrogenase | 1.1.1.100 | phaB |
| acetyl-coA acetyltransferase | 2.3.1.9 | phaA |
| Polyhydroxybutyrate polymerase | 2.3.1.- | phaC |
| 3-ketoacyl-CoA thiolase | 2.3.1.16 | fall |
| *Enoyl-CoA hydratase*/*3-hydroxybutyryl-CoA epimerase* | 4.2.1.17/5.1.2.3 | fadJ |
| trans-2-enoyl-CoA reductase (NAD+) | 1.3.1.44 | ter |
| butanoyl-CoA/ | | |
| 2-methylpropanoyl-CoA mutase | 5.4.99.13 | icmA, icmB |
| phloroisovalerophenon e synthase | 2.3.1.156 | VPS |
| Acyl-coA dehydrogenase | 1.3.99 | acdH |
| Hydroxymethylglutaryl -CoA synthase | 2.3.3.10 | hmgS |
| HMG-CoA reductase 1 | 1.1.1.34 | HMG1 |
| HMG-CoA reductase 1 | 1.1.1.34 | HMG2 |
| short chain enoyl-CoA hydratase | 4.2.1.17 | ECHS 1 |
| 3-hydroxyisobutyryl-CoA hydrolase | 3.1.2.4 | Hibch |
| enoyl-CoA hydratase | 4.2.1.17 | ech |
| Phosphoenolpyruvate carboxylase | 4.1.1.31 | ppc |
| citrate synthase | 2.3.3.1 | gltA |
| phosphoenolpyruvate carboxykinase | 4.1.1.49 | pck/pckA |

As to ph1D, the sequence listing is provided below:

phld> MSTLCLPHVM FPQHKITQQQ MVDHLENLHA DHPRMALAKR
MIANTEVNER HLVLPIDELA VHTGFTHRSI VYEREARQMS SAAARQAIEN
AGLQISDIRM VIVTSCTGFM MPSLTAHLIN DLALPTSTVQ LPIAQLGCVA
GAAAINRAND FARLDARNHV LIVSLEFSSL CYQPDDTKLH AFISAALFGD
AVSACVLRAD DQAGGFKIKK TESYFLPKSE HYIKYDVKDT GFHFTLDKAV
MNSIKDVAPV MERLNYESFE QNCAHNDFFI FHTGGRKILD ELVMHLDLAS
NRVSQSRSSL SEAGNIASW VFDVLKRQFD SNLNRGDIGL LAAFGPGFTA
EMAVGEWTA (SEQ ID NO:161)

### Production Pathway from Malonyl-CoA to 3-HP

In various embodiments the compositions, methods and systems of the present invention involve inclusion of a metabolic production pathway that converts malonyl-CoA to a chemical product of interest.

As one example, 3-HP is selected as the chemical product of interest.

Further as to specific sequences for 3-HP production pathway, malonyl-CoA reductase (mcr) from C. aurantiacus was gene synthesized and codon optimized by the services of DNA 2.0. The FASTA sequence is shown in SEQ ID NO:15 (gi142561982IgbIAAS20429.11malonyl-CoA reductase (Chloroflexus aurantiacus)).

Mcr has very few sequence homologs in the NCBI data base. Blast searches finds 8 different sequences when searching over the entire protein. Hence development of a pile-up sequences comparison is expected to yield limited information. However, embodiments of the present invention nonetheless may comprise any of these eight sequences, shown herein and identified as SEQ ID NOs:42 to 49, which are expected to be but are not yet confirmed to be bi-functional as to this enzymatic activity. Other embodiments may comprise mutated and other variant forms of any of SEQ ID NOs:42 to 49, as well as polynucleotides (including variant forms with conservative and other substitutions), such as those introduced into a selected microorganism to provide or increase 3-HP production therein.

The portion of a CLUSTAL 2.0.11 multiple sequence alignment identifies these eight sequences with respective SEQ ID NOs: 15, 42-49, as shown in the following table.

**TABLE 8**

| **Reference Nos.** | **Seq ID No** | **Genus Species** |
|---|---|---|
| gi142561982IgbIAAS20429.1 | 15 | *Chloroflexus aurantiacus* |
| gi11638481651reflYP_001636209 | 42 | *Chloroflexus aurantiacus* J-10-fl |
| giI2198481671reflYP_002462600 | 43 | *Chloroflexus aggregans* DSM 9485 |
| gi11567428801reflYP_001433009 | 44 | *Roseiflexus castenholzii* DSM 13941 |
| gi11486573071reflYP_ 001277512 | 45 | *Roseiflexus* sp. RS-1 |
| gi185708113IreflZP_01039179.1 | 46 | *Erythrobacter* sp. NAP1 |
| gi1254282228IreflZP_04957196.1 | 47 | gamma proteobacterium NOR51 -B |
| gi1254513883IreflZP_05125944.1 | 48 | gamma proteobacterium NOR5-3 |
| gi11195043131reflZP_01626393.1 | 49 | 3marine gamma proteobacterium HTCC208 |

Malonyl-CoA may be converted to 3-HP in a microorganism that comprises one or more of the following:

A bi-functional malonyl-CoA reductase, such as may be obtained from Chloroflexus aurantiacus and other microorganism species. By bi-functional in this regard is meant that the malonyl-CoA reductase catalyzes both the conversion of malonyl-CoA to malonate semialdehyde, and of malonate semialdehyde to 3-HP.

A mono-functional malonyl-CoA reductase in combination with a 3-HP dehydrogenase. By mono-functional is meant that the malonyl-CoA reductase catalyzes the conversion of malonyl-CoA to malonate semialdehyde.

Any of the above polypeptides may be NADH- or NADPH-dependent, and methods known in the art may be used to convert a particular enzyme to be either form. More particularly, as noted in WO 2002/042418, "any method can be used to convert a polypeptide that uses NADPH as a cofactor into a polypeptide that uses NADH as a cofactor such as those described by others (Eppink et al., J Mol. Biol., 292 (1) : 87-96 (1999), Hall and Tomsett, Microbiology, 146 (Pt 6): 1399-406 (2000), and Dohr et al., Proc. Natl. Acad. Sci., 98 (1) : 81-86 (2001))."

Without being limiting, a bi-functional malonyl-CoA reductase may be selected from the malonyl-CoA reductase of Chloroflexus aurantiacus (such as from ATCC 29365) and other sequences. Also without being limiting, a mono-functional malonyl-CoA reductase may be selected from the malonyl-CoA reductase of Sulfolobus tokodaii (SEQ ID NO:83). As to the malonyl-CoA reductase of C. aurantiacus, that sequence and other species' sequences may also be bi-functional as to this enzymatic activity.

When a mono-functional malonyl-CoA reductase is provided in a microorganism cell, 3-HP dehydrogenase enzymatic activity also may be provided to convert malonate semialdehyde to 3-HP. As shown in the examples, a mono-functional malonyl-CoA reductase may be obtained by truncation of a bi-functional mono-functional malonyl-CoA, and combined in a strain with an enzyme that converts malonate semialdehyde to 3-HP.

Also, it is noted that another malonyl-CoA reductase is known in Metallosphaera sedula (Msed_709, identified as malonyl-CoA reductase/succinyl-CoA reductase).

By providing nucleic acid sequences that encode polypeptides having the above enzymatic activities, a genetically modified microorganism may comprise an effective 3-HP pathway to convert malonyl-CoA to 3-HP in accordance with the embodiments of the present invention.

Other 3-HP pathways, such as those comprising an aminotransferase (see, e.g., WO 2010/011874, published January 28, 2010), may also be provided in embodiments of a genetically modified microorganism of the present invention.

Incorporated into this section, the present invention provides for elevated specific and volumetric productivity metrics as to production of a selected chemical product, such as 3-hydroxypropionic acid (3-HP). In various embodiments, production of a chemical product, such as 3-HP, is not linked to growth.

In various embodiments, production of 3-HP, or alternatively one of its downstream products such as described herein, may reach at least 1, at least 2, at least 5, at least 10, at least 20, at least 30, at least 40, and at least 50 g/liter titer, such as by using one of the methods disclosed herein.

As may be realized by appreciation of the advances disclosed herein as they relate to commercial fermentations of selected chemical products, embodiments of the present invention may be combined with other genetic modifications and/or method or system modulations so as to obtain a microorganism (and corresponding method) effective to produce at least 10, at least 20, at least 30, at least 40, at least 45, at least 50, at least 80, at least 100, or at least 120 grams of a chemical product, such as 3-HP, per liter of final (e.g., spent) fermentation broth while achieving this with specific and/or volumetric productivity rates as disclosed herein.

In some embodiments a microbial chemical production event (i.e., a fermentation event using a cultured population of a microorganism) proceeds using a genetically modified microorganism as described herein, wherein the specific productivity is between 0.01 and 0.60 grams of 3-HP produced per gram of microorganism cell on a dry weight basis per hour (g 3-HP/g DCW-hr). In various embodiments the specific productivity is greater than 0.01, greater than 0.05, greater than 0.10, greater than 0.15, greater than 0.20, greater than 0.25, greater than 0.30, greater than 0.35, greater than 0.40, greater than 0.45, or greater than 0.50 g 3-HP/g DCW-hr. Specific productivity may be assessed over a 2, 4, 6, 8, 12 or 24 hour period in a particular microbial chemical production event. More particularly, the specific productivity for 3-HP or other chemical product is between 0.05 and 0.10, 0.10 and 0.15, 0.15 and 0.20, 0.20 and 0.25, 0.25 and 0.30, 0.30 and 0.35, 0.35 and 0.40, 0.40 and 0.45, or 0.45 and 0.50 g 3-HP/g DCW-hr., 0.50 and 0.55, or 0.55 and 0.60 g 3-HP/g DCW-hr. Various embodiments comprise culture systems demonstrating such productivity.

Also, in various embodiments of the present invention the volumetric productivity achieved may be 0.25 g 3-HP (or other chemical product) per liter per hour (g (chemical product)/L-hr), may be greater than 0.25 g 3-HP (or other chemical product)/L-hr, may be greater than 0.50 g 3-HP (or other chemical product)/L-hr, may be greater than 1.0 g 3-HP (or other chemical product)/L-hr, may be greater than 1.50 g 3-HP (or other chemical product)/L-hr, may be greater than 2.0 g 3-HP (or other chemical product)/L-hr, may be greater than 2.50 g 3-HP (or other chemical product)/L-hr, may be greater than 3.0 g 3-HP (or other chemical product)/L-hr, may be greater than 3.50 g 3-HP (or other chemical product)/L-hr, may be greater than 4.0 g 3-HP (or other chemical product)/L-hr, may be greater than 4.50 g 3-HP (or other chemical product)/L-hr, may be greater than 5.0 g 3-HP (or other chemical product)/L-hr, may be greater than 5.50 g 3-HP (or other chemical product)/L-hr, may be greater than 6.0 g 3-HP (or other chemical product)/L-hr, may be greater than 6.50 g 3-HP (or other chemical product)/L-hr, may be greater than 7.0 g 3-HP (or other chemical product)/L-hr, maybe greater than 7.50 g 3-HP (or other chemical product)/L-hr, may be greater than 8.0 g 3-HP (or other chemical product)/L-hr, may be greater than 8.50 g 3-HP (or other chemical product)/L-hr, may be greater than 9.0 g 3-HP (or other chemical product)/L-hr, maybe greater than 9.50 g 3-HP (or other chemical product)/L-hr, or may be greater than 10.0 g 3-HP (or other chemical product)/L-hr.

In some embodiments, specific productivity as measured over a 24-hour fermentation (culture) period may be greater than 0.01, 0.05, 0.10, 0.20, 0.5, 1.0, 2.0, 3.0, 4.0, 5.0, 6.0, 7.0, 8.0, 9.0, 10.0, 11.0 or 12.0 grams of chemical product per gram DCW of microorganisms (based on the final DCW at the end of the 24-hour period).

In various aspects and embodiments of the present invention, there is a resulting substantial increase in microorganism specific productivity that advances the fermentation art and commercial economic feasibility of microbial chemical production, such as of 3-HP (but not limited thereto).

Stated in another manner, in various embodiments the specific productivity exceeds (is at least) 0.01 g chemical product/g DCW-hr, exceeds (is at least) 0.05 g chemical product/g DCW-hr, exceeds (is at least) 0.10 g chemical product/g DCW-hr, exceeds (is at least) 0.15 g chemical product/g DCW-hr, exceeds (is at least) 0.20 g chemical product/g DCW-hr, exceeds (is at least) 0.25 g chemical product/g DCW-hr, exceeds (is at least) 0.30 g chemical product/g DCW-hr, exceeds (is at least) 0.35 g chemical product/g DCW-hr, exceeds (is at least) 0.40 g chemical product/g DCW-hr, exceeds (is at least) 0.45 g chemical product/g DCW-hr, exceeds (is at least) 0.50 g chemical product/g DCW-hr, exceeds (is at least) 0.60 g chemical product/g DCW-hr.

More generally, based on various combinations of the genetic modifications described herein, optionally in combination with supplementations described herein, specific productivity values for 3-HP, and for other chemical products described herein, may exceed 0.01 g chemical product/g DCW-hr, may exceed 0.05 g chemical product/g DCW-hr, may exceed 0.10 g chemical product/g DCW-hr, may exceed 0.15 g chemical product/g DCW-hr, may exceed 0.20 g chemical product/g DCW-hr, may exceed 0.25 g chemical product/g DCW-hr, may exceed 0.30 g chemical product/g DCW-hr, may exceed 0.35 g chemical product/g DCW-hr, may exceed 0.40 g chemical product/g DCW-hr, may exceed 0.45 g chemical product/g DCW-hr, and may exceed 0.50 g or 0.60 chemical product/g DCW-hr. Such specific productivity may be assessed over a 2, 4, 6, 8, 12 or 24 hour period in a particular microbial chemical production event.

The improvements achieved by embodiments of the present invention may be determined by percentage increase in specific productivity, or by percentage increase in volumetric productivity, compared with an appropriate control microorganism lacking the particular genetic modification combinations taught herein (with or without the supplements taught herein, added to a vessel comprising the microorganism population). For particular embodiments and groups thereof, such specific productivity and/or volumetric productivity improvements is/are at least 10, at least 20, at least 30, at least 40, at least 50, at least 100, at least 200, at least 300, at least 400, and at least 500 percent over the respective specific productivity and/or volumetric productivity of such appropriate control microorganism.

The specific methods and teachings of the specification, and/or cited references that are incorporated by reference, may be incorporated into the examples. Also, production of 3-HP, or one of its downstream products such as described herein, may reach at least 1, at least 2, at least 5, at least 10, at least 20, at least 30, at least 40, and at least 50 g/liter titer in various embodiments.

The metrics may be applicable to any of the compositions, e.g., genetically modified microorganisms, methods, e.g., of producing 3-HP or other chemical products, and systems, e.g., fermentation systems utilizing the genetically modified microorganisms and/or methods disclosed herein.

It is appreciated that iterative improvements using the strategies and methods provided herein, and based on the discoveries of the interrelationships of the pathways and pathway portions, may lead to even greater production of a selected chemical product.

Any number of strategies may lead to development of a suitable modified enzyme suitable for use in a chemical production pathway. With regard to malonyl-CoA-reductase, one may utilize or modify an enzyme such as encoded by the sequences in the table immediately above, to achieve a suitable level of chemical production capability in a microorganism strain.

### Production Pathway from Malonyl-CoA to 3-HP - Additional Aspects

In various embodiments the compositions, methods and systems of the present invention involve inclusion of a metabolic production pathway that converts malonyl-CoA to a chemical product of interest. As one example, 3-HP is selected as the chemical product of interest.

Further as to specific sequences for 3-HP production pathway, malonyl-CoA reductase (mcr) from C. aurantiacus was gene synthesized and codon optimized by the services of DNA 2.0. The FASTA sequence is shown in SEQ ID NO:015 (gi1425619821gbIAAS20429.1I malonyl-CoA reductase (Chloroflexus aurantiacus)).

Mcr has very few sequence homologs in the NCBI data base. Blast searches finds 8 different sequences when searching over the entire protein. Hence development of a pile-up sequences comparison is expected to yield limited information. However, embodiments of the present invention nonetheless may comprise any of these eight sequences, which are expected to be but are not yet confirmed to be bi-functional as to this enzymatic activity. Other embodiments may comprise mutated and other variant forms of any of these sequences, as well as polynucleotides (including variant forms with conservative and other substitutions), such as those introduced into a selected microorganism to provide or increase 3-HP production therein.

The portion of a CLUSTAL 2.0.11 multiple sequence alignment identifies these eight sequences as shown in the following table.

| **Reference Nos.** | **Genus Species** |
|---|---|
| gii425619821gb I AAS20429.1 | *Chloroflexus aurantiacus* |
| 811163848165Iref I | *Chloroflexus aurantiacus* J-10-fl |
| YP_001636209 | |
| 8112198481671ref I | *Chloroflexus aggregans* DSM 9485 |
| YP_002462600 | |
| 8111567428801ref I | *Roseiflexus castenholzii* DSM 13941 |
| YP_001433009 | |
| 8111486573071ref I | *Roseiflexus* sp. RS-1 |
| YP_001277512 | |
| 811857081131ref I ZP_01039179.1 | *Erythrobacter* sp. NAP1 |
| 8112542822281ref I | NOR51-B |
| ZP_ 04957196.1 | gamma proteobacterium |
| 811254513883Iref I | NOR5-3 |
| ZP_05125944.1 | gamma proteobacterium |
| 8¹1119504313 I ref I | 3marine gamma proteobacterium |
| ZP_01626393.1 | HTCC208 |

Malonyl-CoA may be converted to 3-HP in a microorganism that comprises one or more of the following:
A bi-functional malonyl-CoA reductase, such as may be obtained from Chloroflexus aurantiacus and other microorganism species. By bi-functional in this regard is meant that the malonyl-CoA reductase catalyzes both the conversion of malonyl-CoA to malonate semialdehyde, and of malonate semialdehyde to 3-HP.

A mono-functional malonyl-CoA reductase may be used in combination with a 3-HP dehydrogenase. By mono-functional is meant that the malonyl-CoA reductase catalyzes the conversion of malonyl-CoA to malonate semialdehyde.

Any of the above polypeptides may be NADH- or NADPH-dependent, and methods known in the art may be used to convert a particular enzyme to be either form. More particularly, as noted in WO 2002/042418, "any method can be used to convert a polypeptide that uses NADPH as a cofactor into a polypeptide that uses NADH as a cofactor such as those described by others (Eppink et al., J Mol. Biol., 292 (1) : 87-96 (1999), Hall and Tomsett, Microbiology, 146 (Pt 6): 1399-406 (2000), and Dohr et al., Proc. Natl. Acad. Sci., 98 (1) : 81-86 (2001))."

Without being limiting, a bi-functional malonyl-CoA reductase may be selected from the malonyl-CoA reductase of Chloroflexus aurantiacus (such as from ATCC 29365) and other sequences. Also without being limiting, a mono-functional malonyl-CoA reductase may be selected from the malonyl-CoA reductase of Sulfolobus tokodaii. As to the malonyl-CoA reductase of C. aurantiacus, that sequence and other species' sequences may also be bi-functional as to this enzymatic activity.

When a mono-functional malonyl-CoA reductase is provided in a microorganism cell, 3-HP dehydrogenase enzymatic activity also may be provided to convert malonate semialdehyde to 3-HP. A mono-functional malonyl-CoA reductase may be obtained by truncation of a bi-functional mono-functional malonyl-CoA, and combined in a strain with an enzyme that converts malonate semialdehyde to 3-HP.

Also, it is noted that another malonyl-CoA reductase is known in Metallosphaera sedula (Msed_709, identified as malonyl-CoA reductase/succinyl-CoA reductase).

By providing nucleic acid sequences that encode polypeptides having the above enzymatic activities, a genetically modified microorganism may comprise an effective 3-HP pathway to convert malonyl-CoA to 3-HP in accordance with the embodiments of the present invention.

Other 3-HP pathways, such as those comprising an aminotransferase (see, e.g., WO 2010/011874, published January 28, 2010), incorporated by reference herein for such 3-HP pathway teachings, may also be provided in embodiments of a genetically modified microorganism of the present invention.

Any number of strategies may lead to development of a suitable modified enzyme suitable for use in a 3-HP production pathway. With regard to malonyl-CoA-reductase, one may utilize or modify an enzyme such as encoded by the sequences in the table immediately above, to achieve a suitable level of 3-HP production capability in a microorganism strain.

### Combinations of Genetic Modifications

In some embodiments, the genetically modified microorganism additionally comprises at least one genetic modification to increase, in the genetically modified microorganism, a protein function selected from the protein functions of Table 9 (Glucose transporter function (such as by galP), pyruvate dehydrogenase Elp, dihydrolipoamide acetyltransferase, and pyruvate dehydrogenase E3). In certain embodiments, the genetically modified microorganism comprises at least one genetic modification to increase two, three, or four protein functions selected from the protein functions of Table 9.

In some embodiments, such genetically modified microorganism additionally comprises at least one genetic modification to decrease protein functions selected from the protein functions of Table 10, lactate dehydrogenase, pyruvate formate lyase, pyruvate oxidase, phosphate acetyltransferase, histidyl phosphorylatable protein (of PTS), phosphoryl transfer protein (of PTS), and the polypeptide chain (of PTS).

In various embodiments, such genetically modified microorganism comprises at least one genetic modification to decrease enzymatic activity of two, three, four, five, six, or seven protein functions selected from the protein functions of Table 10. Also, in various embodiments at least one, or more than one, genetic modification is made to modify the protein functions of Table 11 in accordance with the Comments therein.including in Table 11.

It will be appreciated that, in various embodiments, there can be many possible combinations of increases in one or more protein functions of Table 9, with reductions in one or more protein functions of Table 9 in the genetically modified microorganism comprising at least one genetic modification to provide or increase malonyl-CoA-reductase protein function (i.e, enzymatic activity). Protein functions can be independently varied, and any combination (i.e., a full factorial) of genetic modifications of protein functions in Tables 9, 10, and 11 herein can be adjusted by the methods taught and provided into said genetically modified microorganism.

In some embodiments, at least one genetic modification to decrease enzymatic activity is a gene disruption. In some embodiments, at least one genetic modification to decrease enzymatic activity is a gene deletion.

Certain embodiments of the invention additionally comprise a genetic modification to increase the availability of the cofactor NADPH, which can increase the NADPH/NADP+ ratio as may be desired. Non-limiting examples for such genetic modification are pgi (E.C. 5.3.1.9, in a mutated form), pntAB (E.C. 1.6.1.2), overexpressed, gapA (E.C. 1.2.1.12):gapN (E.C. 1.2.1.9, from Streptococcus mutans) substitution/replacement, and disrupting or modifying a soluble transhydrogenase such as sthA (E.C. 1.6.1.2), and/or genetic modifications of one or more of zwf (E.C. 1.1.1.49), gnd (E.C. 1.1.1.44), and edd (E.C. 4.2.1.12). Sequences of these genes are available at <<www.metacyc.org>>. Also, the sequences for the genes and encoded proteins for the E. coli gene names shown in Tables 9, 10, and 11 are provided in U.S. Provisional Patent Application No.: 61/246,141, incorporated herein in its entirety and for such sequences, and also are available at <<www.ncbi.gov>> as well as <<www.metacyc.org>> or <<www.ecocyc.org>>.

In some embodiments, the genetic modification increases microbial synthesis of a selected chemical product above a rate or titer of a control microorganism lacking said at least one genetic modification to produce the selected chemical product. In some embodiments, the genetic modification is effective to increase enzymatic conversions to the selected chemical product by at least about 5 percent, at least about 10 percent, at least about 20 percent, at least about 30 percent, or at least about 50 percent above the enzymatic conversion of a control microorganism lacking the genetic modification.

**TABLE 9**

| **Enzyme Function** | **E.C. Classification** | **Gene Name in E. coli** |
|---|---|---|
| Glucose transporter | N/A | galP |
| Pyruvate dehydrogenase Elp | 1.2.4.1 | aceE |
| lipoate acetyltransferase / dihydrolipoamide acetyltransferase | 2.3.1.12 | aceF |
| Pyruvate dehydrogenase E3 (lipoamide dehydrogenase) | 1.8.1.4 | 1pd |

**TABLE 10**

| **Enzyme Function** | **E.C. Classification** | **Gene Name in E. coli** |
|---|---|---|
| Lactate dehydrogenase | 1.1.1.28 | ldhA |
| Pyruvate formate lyase (B "inactive") | 2.3.1.- | pflB |
| Pyruvate oxidase | 1.2.2.2 | poxB |
| Phosphate acetyltransferase | 2.3.1.8 | Pta |
| Heat stable, histidyl phosphorylatable protein (of PTS) | N/A | ptsH (HPr) |
| Phosphoryl transfer protein (of PTS) | N/A | ptsl |
| Polypeptide chain (of PTS) | N/A | Crr |

**TABLE 11**

| **Enzyme Function** | **E.C. Classification** | **Gene Name in E. coli** | **Comments** |
|---|---|---|---|
| 0 ketoacyl-acyl carrier protein synthase 13-0X0ACYL-ACP-SYNTHASE II MONOMER | 2.3.1.179 | fabF | Decrease function, including by mutation |
| | 2.3.1.41 | | |
| β-ketoacyl-ACP synthase I, 3-oxoacyl-ACP-synthase I | 2.3.1.41 | fabB | Decrease function, including by mutation |
| | 2.3.1.- | | |
| Malonyl-CoA-ACP transacylase | 2.3.1.39 | fabD | Decrease function, including by mutation |
| enoyl acyl carrier protein reductase | 1.3.1.9, | fabl | Decrease function, including by mutation |
| | 1.3.1.10 | | |
| β-ketoacyl-acyl carrier protein synthase III | 2.3.1.180 | fabH | Decrease function, including by mutation |
| Carboxyl transferase subunit a subunit | 6.4.1.2 | accA | Increase function |
| Biotin carboxyl carrier protein | 6.4.1.2 | accB | Increase function |
| Biotin carboxylase subunit | 6.3.4.14 | accC | Increase function |
| Carboxyl transferase subunit β subunit | 6.4.1.2 | accD | Increase function |
| long chain fatty acyl thioesterase I | 3.1.2.2, | tesA | Increase function |
| | 3.1.1.5 | | |
| GDP pyrophosphokinase / GTP pyrophosphokinase | 2.7.6.5 | relA | Decrease function, including by mutation |
| GDP | 2.7.6.5, | Spot | Decrease function, |
| diphosphokinase / guanosine-3',5'-bis(diphosphate) 3'-diphosphatase | 3.1.7.2 | | including by mutation |

Further with regard to descrasing enzyme function based on Table 11's teachings, any one or a combination of enzyme functions of the following may be decreased in a particular embodiment combined with other genetic modifications described herein: β-ketoacyl-ACP synthase I, 3-oxoacyl-ACP-synthase I; Malonyl-CoA-ACP transacylase; enoyl acyl carrier protein reductase; and β-ketoacyl-acyl carrier protein synthase III.

Moreover, in particular embodiments to more effectively redirect malonyl-CoA from fatty acid synthesis and toward the biosynthesis of such a desired chemical product, genetic modifications are made to:
(a) increase production along the biosynthetic pathway that includes malonyl-CoA and leads to a desired chemical product;
(b) reduce the activity of one or more, or of two or more, or of three or more of enoyl acyl carrier protein (ACP) reductase, β-ketoacyl-acyl carrier protein synthase I (such as fabB), B-ketoacyl-acyl carrier protein synthase II, and malonyl-CoA-acyl carrier protein transacylase; and optionally
(c) increase the production of coenzyme A in the microorganism cell.

Accordingly, as described in various sections above, some compositions, methods and systems of the present invention comprise providing a genetically modified microorganism that comprises both a production pathway to a selected chemical product, and a modified polynucleotide that encodes an enzyme of the fatty acid synthase system that exhibits reduced activity, so that utilization of malonyl-CoA shifts toward the production pathway compared with a comparable (control) microorganism lacking such modifications. The methods involve producing the chemical product using a population of such genetically modified microorganism in a vessel, provided with a nutrient media. Other genetic modifications described herein, to other enzymes, such as acetyl-CoA carboxylase and/or NADPH-dependent transhydrogenase, may be present in some such embodiments. Providing additional copies of polynucleotides that encode polypeptides exhibiting these enzymatic activities is shown to increase production of a selected chemical. Other ways to increase these respective enzymatic activities is known in the art and may be applied to various embodiments of the present invention. SEQ ID NOs for these polynucleotides and polypeptides of E. coli are: acetyl-CoA carboxylase (accABCD, SEQ ID NOs:30-37); and NADPH-dependent transhydrogenase (SEQ ID NOs:38-41), also referred to as pyridine nucleotide transhydrogenase, pntAB in E. coli).

Also, without being limiting, a first step in some multi-phase method embodiments of making a chemical product may be exemplified by providing into a vessel, such as a culture or bioreactor vessel, a nutrient media, such as a minimal media as known to those skilled in the art, and an inoculum of a genetically modified microorganism so as to provide a population of such microorganism, such as a bacterium, and more particularly a member of the family Enterobacteriaceae, such as E. coli, where the genetically modified microorganism comprises a metabolic pathway that converts malonyl-CoA to molecules of a selected chemical. Also, in various embodiments an inoculum of a microorganism of the present invention is cultured in a vessel so that the cell density increases to a cell density suitable for reaching a production level of a selected chemical that meets overall productivity metrics taking into consideration the next step of the method. In various alternative embodiments, a population of these genetically modified microorganisms may be cultured to a first cell density in a first, preparatory vessel, and then transferred to the noted vessel so as to provide the selected cell density. Numerous multi-vessel culturing strategies are known to those skilled in the art. Any such embodiments provide the selected cell density according to the first noted step of the method.

Also without being limiting, a subsequent step may be exemplified by two approaches, which also may be practiced in combination in various embodiments. A first approach provides a genetic modification to the genetically modified microorganism such that its enoyl-ACP reductase enzymatic activity may be controlled. As one example, a genetic modification may be made to substitute for the native enoyl-ACP reductase a temperature-sensitive mutant enoyl-ACP reductase (e.g., fabEs in E. coli). The latter may exhibit reduced enzymatic activity at temperatures above 30 C but normal enzymatic activity at 30 C, so that elevating the culture temperature to, for example to 34C, 35C, 36C, 37C or even 42C, reduces enzymatic activity of enoyl-ACP reductase. In such case, more malonyl-CoA is converted to 3-HP or another chemical product than at 30 C, where conversion of malonyl-CoA to fatty acids is not impeded by a less effective enoyl-ACP reductase.

For the second approach, an inhibitor of enoyl-ACP reductase, or another of the fatty acid synthase enzyme, is added to reduce conversion of malonyl-CoA to fatty acids. For example, the inhibitor cerulenin is added at a concentration that inhibits one or more enzymes of the fatty acid synthase system. FIG. 2A depicts relevant pathways and shows three inhibitors - thiolactomycin, triclosan, and cerulenin, next to the enzymes that they inhibit. Encircled E. coli gene names indicate a temperature-sensitive mutant is available for the polypeptide encoded by the gene. FIG. 2B provides a more detailed depiction of representative enzymatic conversions and exemplary E. coli genes of the fatty acid synthetase system that was more generally depicted in FIG. 2A. This listing of inhibitors of microorganism fatty acid synthetase enzymes is not meant to be limiting. Other inhibitors, some of which are used as antibiotics, are known in the art and include, but are not limited to, diazaborines such as thienodiazaborine, and, isoniazid.

In some embodiments, the genetic modification increases microbial synthesis of a selected chemical above a rate or titer of a control microorganism lacking said at least one genetic modification to produce a selected chemical. In some embodiments, the genetic modification is effective to increase enzymatic conversions to a selected chemical by at least about 5 percent, at least about 10 percent, at least about 20 percent, at least about 30 percent, or at least about 50 percent above the enzymatic conversion of a control microorganism lacking the genetic modification.

Genetic modifications as described herein may include modifications to reduce enzymatic activity of any one or more of: β-ketoacyl-ACP synthase I, 3-oxoacyl-ACP-synthase I; Malonyl-CoA-ACP transacylase; enoyl acyl carrier protein reductase; and β-ketoacyl-acyl carrier protein synthase III.

Accordingly, as described in various sections above, some compositions, methods and systems of the present invention comprise providing a genetically modified microorganism that comprises both a production pathway to a selected chemical product, such as a selected chemical, and a modified polynucleotide that encodes an enzyme of the fatty acid synthase system that exhibits reduced activity, so that utilization of malonyl-CoA shifts toward the production pathway compared with a comparable (control) microorganism lacking such modifications. The methods involve producing the chemical product using a population of such genetically modified microorganism in a vessel, provided with a nutrient media. Other genetic modifications described herein, to other enzymes, such as acetyl-CoA carboxylase and/or NADPH-dependent transhydrogenase, may be present in some such embodiments. Providing additional copies of polynucleotides that encode polypeptides exhibiting these enzymatic activities is shown to increase a selected chemical production. Other ways to increase these respective enzymatic activities is known in the art and may be applied to various embodiments of the present invention. SEQ ID NOs for these polynucleotides and polypeptides of E. coli are: acetyl-CoA carboxylase (accABCD, SEQ ID NOs:30-37); and NADPH-dependent transhydrogenase (SEQ ID NOs:38-41), also referred to as pyridine nucleotide transhydrogenase, pntAB in E. coli).

Also, without being limiting, a first step in some multi-phase method embodiments of making a chemical product may be exemplified by providing into a vessel, such as a culture or bioreactor vessel, a nutrient media, such as a minimal media as known to those skilled in the art, and an inoculum of a genetically modified microorganism so as to provide a population of such microorganism, such as a bacterium, and more particularly a member of the family Enterobacteriaceae, such as E. coli, where the genetically modified microorganism comprises a metabolic pathway that converts malonyl-CoA to a selected chemical molecules. For example, genetic modifications may include the provision of at least one nucleic acid sequence that encodes a gene encoding the enzyme malonyl-CoA reductase in one of its bi-functional forms, or that encodes genes encoding a mono-functional malonyl-CoA reductase and an NADH- or NADPH-dependent 3- hydroxypropionate dehydrogenase (e.g., ydfG or mmsB from E. coli, or mmsB from Pseudomonas aeruginosa). In either case, when provided into an E. coli host cell, these genetic modifications complete a metabolic pathway that converts malonyl-CoA to a selected chemical. This inoculum is cultured in the vessel so that the cell density increases to a cell density suitable for reaching a production level of a selected chemical that meets overall productivity metrics taking into consideration the next step of the method. In various alternative embodiments, a population of these genetically modified microorganisms may be cultured to a first cell density in a first, preparatory vessel, and then transferred to the noted vessel so as to provide the selected cell density. Numerous multi-vessel culturing strategies are known to those skilled in the art. Any such embodiments provide the selected cell density according to the first noted step of the method.

Thus, for various embodiments of the invention the genetic manipulations to any pathways described herein also may include various genetic manipulations, including those directed to change regulation of, and therefore ultimate activity of, an enzyme or enzymatic activity of an enzyme identified in any of the respective pathways. Such genetic modifications may be directed to transcriptional, translational, and post-translational modifications that result in a change of enzyme activity and/or selectivity under selected and/or identified culture conditions. Thus, in various embodiments, to function more efficiently, a microorganism may comprise one or more gene deletions. For example, for a particular embodiment in E. coli, the genes encoding lactate dehydrogenase (ldhA), phosphate acetyltransferase (pta), pyruvate oxidase (poxB) and pyruvate-formate lyase (pflB) may be deleted. Additionally, a further deletion or other modification to reduce enzymatic activity, of multifunctional 2-keto-3-deoxygluconate 6-phosphate aldolase and 2-keto-4-hydroxyglutarate aldolase and oxaloacetate decarboxylase (eda in E. coli), may be provided to various strains. Further to the latter, in various embodiments combined with such reduction of enzymatic activity of multifunctional 2-keto-3-deoxygluconate 6-phosphate aldolase and 2-keto-4-hydroxyglutarate aldolase and oxaloacetate decarboxylase (eda in E. coli), further genetic modifications may be made to increase a glucose transporter (e.g. galP in E. coli) and/or to decrease activity of one or more of heat stable, histidyl phosphorylatable protein (of PTS) (ptsH (HPr) in E. coli), phosphoryl transfer protein (of PTS) (ptsl in E. coli), and the polypeptide chain of PTS (Crr in E. coli).

Gene deletions may be accomplished by mutational gene deletion approaches, and/or starting with a mutant strain having reduced or no expression of one or more of these enzymes, and/or other methods known to those skilled in the art.

Aspects of the invention also regard provision of multiple genetic modifications to improve microorganism overall effectiveness in converting a selected carbon source into a chemical product such as 3-HP. Particular combinations are shown, such as in the Examples, to increase specific productivity, volumetric productivity, titer and yield substantially over more basic combinations of genetic modifications.

Further to FIG. 1 genetic modifications, appropriate additional genetic modifications can provide further improved production metrics. Various strains may comprise genetic modifications for a selected chemical, and additional genetic modifications as disclosed herein (including a particular genetic modification regarding the fatty acid synthase system, not to be limiting, such modifications more generally disclosed elsewhere herein). The embodiment of FIG. 1 depicts a number of genetic modifications in combination, however in various embodiments of the present invention other combinations of the genetic modifications of these enzymatic functions may be provided to achieve a desired level of increased rate, titer and yield as to bio-production of a chemical product.

Additional genetic modifications may be provided in a microorganism strain of the present invention. As one example, a deletion, of multifunctional 2-keto-3-deoxygluconate 6-phosphate aldolase and 2- keto-4-hydroxyglutarate aldolase and oxaloacetate decarboxylase (eda in E. coli), may be provided to various strains.

For example, the ability to utilize sucrose may be provided, and this would expand the range of feed stocks that can be utilized to produce a selected chemical or other chemical products. Common laboratory and industrial strains of E. coli, such as the strains described herein, are not capable of utilizing sucrose as the sole carbon source. Since sucrose, and sucrose-containing feed stocks such as molasses, are abundant and often used as feed stocks for the production by microbial fermentation, adding appropriate genetic modifications to permit uptake and use of sucrose may be practiced in strains having other features as provided herein. Various sucrose uptake and metabolism systems are known in the art (for example, U.S. Pat. No. 6,960,455), incorporated by reference for such teachings. These and other approaches may be provided in strains of the present invention. The examples provide at least two approaches.

Also, genetic modifications may be provided to add functionality for breakdown of more complex carbon sources, such as cellulosic biomass or products thereof, for uptake, and/or for utilization of such carbon sources. For example, numerous cellulases and cellulase-based cellulose degradation systems have been studied and characterized (see, for example, and incorporated by reference herein for such teachings, Beguin, P and Aubert, J-P (1994) FEMS Microbial. Rev. 13: 25-58; Ohima, K. et al. (1997) Biotechnol. Genet. Eng. Rev. 14: 365414).

In addition to the above-described genetic modifications, in various embodiments genetic modifications also are provided to increase the pool and availability of the cofactor NADPH, and/or, consequently, the NADPH/NADP+ ratio. For example, in various embodiments for E. coli, this may be done by increasing activity, such as by genetic modification, of one or more of the following genes- pgi (in a mutated form), pntAB, overexpressed, gapA:gapN substitution/replacement, and disrupting or modifying a soluble transhydrogenase such as sthA, and/or genetic modifications of one or more of zwf, gnd, and edd.

Any such genetic modifications may be provided to species not having such functionality, or having a less than desired level of such functionality.

More generally, and depending on the particular metabolic pathways of a microorganism selected for genetic modification, any subgroup of genetic modifications may be made to decrease cellular production of fermentation product(s) selected from the group consisting of acetate, acetoin, acetone, acrylic, malate, fatty acid ethyl esters, isoprenoids, glycerol, ethylene glycol, ethylene, propylene, butylene, isobutylene, ethyl acetate, vinyl acetate, other acetates, 1,4-butanediol, 2,3-butanediol, butanol, isobutanol, sec-butanol, butyrate, isobutyrate, 2-OH-isobutryate, 3-OH-butyrate, ethanol, isopropanol, D-lactate, L-lactate, pyruvate, itaconate, levulinate, glucarate, glutarate, caprolactam, adipic acid, propanol, isopropanol, fusel alcohols, and 1,2- propanediol, 1,3-propanediol, formate, fumaric acid, propionic acid, succinic acid, valeric acid, and maleic acid. Gene deletions may be made as disclosed generally herein, and other approaches may also be used to achieve a desired decreased cellular production of selected fermentation products.

### Elongase Pathway

In various embodiments an elongase is used in a metabolic pathway to produce a selected chemical product. The following paragraphs are provided to describe elongases and their utilization.

Trypanosoma brucei, a eukaryotic human parasite, is known to evade the host immune response by synthesizing a specific surface coating comprised of glycoproteins tethered to fatty acids anchored into the membrane. To accommodate the surface coating, T brucei produces large quantities of the anchor molecule, which is composed exclusively of C14 saturated fatty acid (myristic acid). Myristic acid synthesis in T. brucei is initiated by the formation of butyryl-CoA and utilizes membrane-bound elongation enzymes, similar to those typically used to extend fatty acids, to condense malonyl-CoA with the growing acyl chain . This particular ELO system esterifies the growing fatty acid chain to CoA rather than ACP like the bacterial and other microbial counterparts (see Lee et al, Cell 126, 691-699, 2006 and Cronan, Cell, 126, 2006). This is in contrast to typical bacterial fatty acid elongation which is initiated following the formation of acetoacetyl acyl-ACP from malonyl-ACP. Microbial fatty acid synthesis is then catalysed in the cytosol by multiple soluble proteins, each responsible for a single reaction where the growing fatty acid chain is esterified to an acyl carrier protein (ACP). The carbon-donor molecule is a malonyl-ACP which extends the growing carbon chain length by 2 carbons at a time.

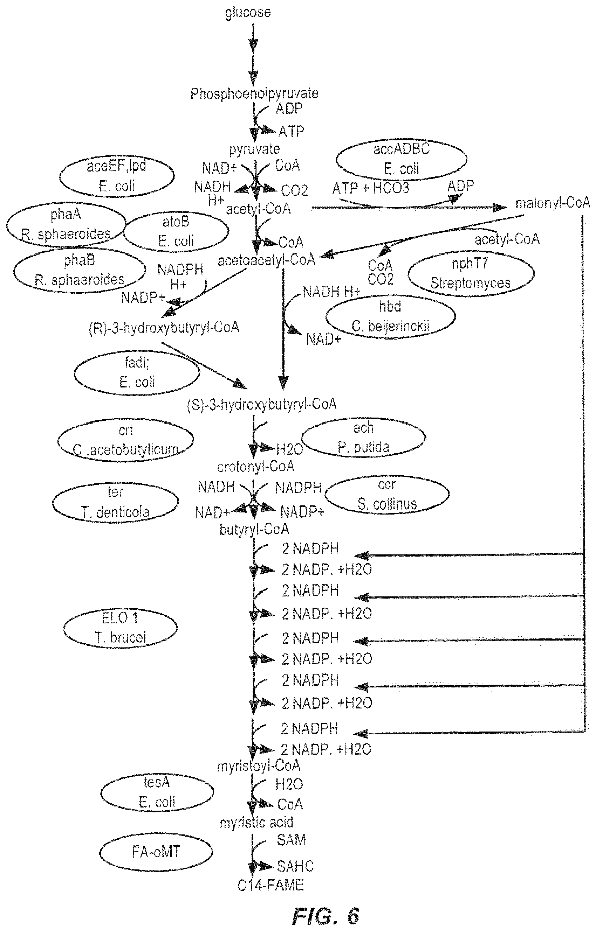

A proposed elongase-utilizing pathway (see FIG. 6) builds from PHB and n-butanol production pathways. The first committed step requires the formation of acetoacetyl-CoA, which is traditionally catalysed by either the b-ketothiolase (phaA) or acetyl-CoA acetyltransferase (atoB) using 2 acetyl-CoA molecules as a substrate. A recently reported malonyl-CoA dependent route for synthesis of acetoacetyl-CoA in Streptomyces sp. has been cloned and expressed in E. coli (Okamura, et al., PNAS, 107, 25, 2010). When considered with other aspects of the invention, including those increasing malonyl-CoA pools and fluxes, this reaction appears to provide an attractive alternative to the common route to acetoacetyl-CoA formation, which is an unfavorable reaction, preferring the hydrolysis of acetoacetyl-coA The novel enzyme, nphT7, catalyzes the irreversible reaction to form acetoacetyl-CoA from malonyl-CoA and acetyl-CoA and would allow for two driving forces towards acetoacetyl-CoA production (irreversibility and release of CO2). The protein and oligonucleotide sequences for NphT7 (AB540131.1 GI:299758081) are provided below (SEQ ID NOs:159; 160):
**SEQ ID NO:159** 
MTDVRFRIIGTGAYVPERIVSNDEVGAPAGVDDDWITRKTGIRQ
RRWAADDQATSDLATAAGRAALKAAGITPEQLTVIAVATSTPDRPQPPTAAYVQHHLG
ATGTAAFDVNAVCSGTVFALSSVAGTLVYAGGYALVIGADLYSRILNPADRKTVVLFG
DGAGAMVLGPTSTGTGPIVRAVALHTEGGLTDLIRVPAGGSRQPLDTDGLDAGLQYFA
MDGREVRREVTEHLPQLIKGFLHEAGVDAADISHFVPHQANGVMLDEVEGELHLPRAT
MHRTVETYGNTGAASIPITMDAAVRAGSFRPGELVLLAGFGGGMAASFALIEW
 **SEQ ID NO:160**

| | | | | | | |
|---|---|---|---|---|---|---|
| 1 | cctgcaggcc | gtcgagggcg | cctggaagga | ctacgcggag | caggacggcc | ggtcgctgga |
| 61 | ggagttcgcg | gcgttcgtct | accaccagcc | gttcacgaag | atggcctaca | aggcgcaccg |
| 121 | ccacctgctg | aacttcaacg | gctacgacac | cgacaaggac | gccatcgagg | gcgccctcgg |
| 181 | ccagacgacg | gcgtacaaca | acgtcatcgg | caacagctac | accgcgtcgg | tgtacctggg |
| 241 | cctggccgcc | ctgctcgacc | aggcggacga | cctgacgggc | cgttccatcg | gcttcctgag |
| 301 | ctacggctcg | ggcagcgtcg | ccgagttctt | ctcgggcacc | gtcgtcgccg | ggtaccgcga |
| 361 | gcgtctgcgc | accgaggcga | accaggaggc | gatcgcccgg | cgcaagagcg | tcgactacgc |
| 421 | cacctaccgc | gagctgcacg | agtacacgct | cccgtccgac | ggcggcgacc | acgccacccc |
| 481 | ggtgcagacc | accggcccct | tccggctggc | cgggatcaac | gaccacaagc | gcatctacga |
| 541 | ggcgcgctag | cgacacccct | cggcaacggg | gtgcgccact | gttcggcgca | ccccgtgccg |
| 601 | ggctttcgca | cagctattca | cgaccatttg | aggggcgggc | agccgcatga | ccgacgtccg |
| 661 | attccgcatt | atcggtacgg | gtgcctacgt | accggaacgg | atcgtctcca | acgatgaagt |
| 721 | cggcgcgccg | gccggggtgg | acgacgactg | gatcacccgc | aagaccggta | tccggcagcg |
| 781 | tcgctgggcc | gccgacgacc | aggccacctc | ggacctggcc | acggccgcgg | ggcgggcagc |
| 841 | gctgaaagcg | gcgggcatca | cgcccgagca | gctgaccgtg | atcgcggtcg | ccacctccac |
| 901 | gccggaccgg | ccgcagccgc | ccacggcggc | ctatgtccag | caccacctcg | gtgcgaccgg |
| 961 | cactgcggcg | ttcgacgtca | acgcggtctg | ctccggcacc | gtgttcgcgc | tgtcctcggt |
| 1021 | ggcgggcacc | ctcgtgtacc | ggggcggtta | cgcgctggtc | atcggcgcgg | acctgtactc |
| 1081 | gcgcatcctc | aacccggccg | accgcaagac | ggtcgtgctg | ttcggggacg | gcgccggcgc |
| 1141 | aatggtcctc | gggccgacct | cgaccggcac | gggccccatc | gtccggcgcg | tcgccctgca |
| 1201 | caccttcggc | ggcctcaccg | acctgatccg | tgtgcccgcg | ggcggcagcc | gccagccgct |
| 1261 | ggacacggat | ggcctcgacg | cgggactgca | gtacttcgcg | atggacgggc | gtgaggtgcg |
| 1321 | ccgcttcgtc | acggagcacc | tgccgcagct | gatcaagggc | ttcctgcacg | aggccggggt |
| 1381 | cgacgccgcc | gacatcagcc | acttcgtgcc | gcatcaggcc | aacggtgtca | tgctcgacga |
| 1441 | ggtcttcggc | gagctgcatc | tgccgcgggc | gaccatgcac | cggacggtcg | agacctacgg |
| 1501 | caacacggga | gcggcctcca | tcccgatcac | catggacgcg | gccgtgcgcg | ccggttcctt |
| 1561 | ccggccgggc | gagctggtcc | tgctggccgg | gttcggcggc | ggcatggccg | cgagcttcgc |
| 1621 | cctgatcgag | tggtagtcgc | ccgtaccacc | acagcggtcc | ggcgccacct | gttccctgcg |
| 1681 | ccgggccgcc | ctcggggcct | ttaggcccca | caccgcccca | gccgacggat | tcagtcgcgg |
| 1741 | cagtacctca | gatgtccgct | gcgacggcgt | cccggagagc | ccgggcgaga | tcgcgggccc |
| 1801 | ccttctgctc | gtccccggcc | cctcccgcga | gcaccacccg | cggcggacgg | ccgccgtcct |
| 1861 | ccgcgatacg | ccgggcgagg | tcgcaggcga | gcacgccgga | cccggagaag | ccccccagca |
| 1921 | ccagcgaccg | gccgactccg | tgcgcggcca | gggcaggctg | cgcgccgtcg | acgtcggtga |
| 1981 | gcagcaccag | gagctcctgc | ggcccggcgt | agaggtcggc | cagccggtcg | tagcaggtcg |
| 2041 | cgggcgcgcc | cggcggcggg | atcagacaga | tcgtgcccgc | ccgctcgtgc | ctcgccgccc |
| 2101 | gcagcgtgac | cagcggaatg | tcccgcccag | ctccgga | | |

The immediate next step in the pathway involves the reduction of acetoacetyl-CoA to 3-HB-CoA. There are two primary routes characterized to convert acetoacetyl-CoA to (S)3-HB-CoA. The first is catalysed by an NADH-dependent (S)-3HB-CoA dehydrogenase, which has been well characterized in C. beijerinckii.(Tseng, et al., AEM, 75, 10, 2009). The second is an NADPH-dependent (R)-3HB-CoA dehydrogenase followed by a non-specific epimerase reaction in E. coli to convert (R) to (S)-3HB-CoA. The NADH-dependent dehydrogenase is the more desirable activity to pursue due to higher reported specific activities, direct conversion to (S)-3HB-CoA, and more thermodynamically favourable reaction.

The crotonase reaction (3HB-CoA -> crotonyl-CoA) is immediately followed by a reduction to form butyryl-CoA, which is the substrate for the ELO1 elongase . The trans-2-enoyl-CoA reductase, ter, from T denticola has been characeterized as an NADH-dependent reductase specific for the conversion of crotonyl¬CoA to butyryl-CoA without flavoproteins (Tucci, et al. PEBS Letters, 581, 2007). Ter has been cloned and expressed in E. coli and in vitro enzyme characterization have suggested irreversible activity in the desired direction of butyryl-CoA production (Shen, et al., AEM, 77, 9, 2011). Ccr from S. collinus, which has been reported to catalyze the same flavoprotein-independent reaction, has also been expressed in E. coli. However, ccr requires NADPH as a cofactor.

### VII. COMPOSITIONS OF MATTER

In various embodiments, a method is provided for producing a C4-C18 fatty acid, the method comprising combining a carbon source and a microorganism cell culture to produce the C4-C18 fatty acid. For example, the method includes combing a caron source and a microorganism cell culture, where the cell culture comprises an inhibitor of fatty acid synthase and/or the microorganism of said cell culture is genetically modified for reduced enzymatic activity in at least one of an organism's native fatty acid synthase pathway enzymes, providing for reduced conversion of malonyl-CoA to fatty acyl-ACPs; and the microorganism of said cell culture additionally has one or more genetic modifications conferring fatty acid production. In various embodiments, the C4-C18 fatty acid is selected from the group consisting of C4, C6, C8, C10, C12, C14, C16 and C18 fatty acids. Preferably, the fatty acid production occurs through the synthesis of fatty acyl-coAs of chain length 4-18. Alternatively, the fatty acid production occurs through the synthesis of fatty acyl-coAs via the expression of at least one elongase enzyme.

The fatty acids produced according to the method exist as a mixture. In various embodiments, the mixture is processed to remove cellular debris. However, residual components may be present in the mixture. For example, the mixture may further contain E. coli DNA. Preferably, the mixture consists essentially of C4-C18 compounds. For example, preferably the mixture is essentially free of one or more additional components selected from the group consisting of opacifying agents, pearlescent aids, astringents anti-acne agents, anti-caking agents, antimicrobial agents, antioxidants, biocides, pH modifiers, skin treating agents, vitamins, and humectants. In various embodiments, the mixture is essentially free of one or more essential oils selected from the group consisting of almond, anise, basil, bay, bergamot, bitter almond, camphor, cassia, cedarwood, chamomile, cinnamon, citronella, clove, clove bud, coriander, cypress, eucalyptus, fennel, fir, frankincense, geranium, ginger, grapefruit, jasmine, lavender, lemon, lemongrass, lime, litsea, marigold, marjoram, myrtle, neroli, nutmeg, oakmoss, orange, palmorosa, patchouli, pennyroyal, peppermint, pine, rose, rosemary, sage, sandalwood, spearmint, spruce, sweet birch, tangerine, tea tree, vanilla, vetivert, white thyme, and wintergreen.

In various embodiments, the mixture is isolated from fermentation media, and processed as a component in a consumer product. For example, the consumer product is selected from the group consisting of a detergent, soap, resin, emulsifier, lubricant, grease, and wax. The mixture may be incorporated into the consumer product as the same chemical entity as produced by the genetically modified organism, or the mixture may be further processed as a mixture of fatty acids, fatty alcohols, fatty acid esters, fatty aldehydes, fatty amines, fatty amides, or fatty acid salts.

### VIII. SEPARATION AND PURIFICATION FROM FERMENTATION

A selected chemical product may be separated and purified by the approaches described in the following paragraphs, taking into account that many methods of separation and purification are known in the art and the following disclosure is not meant to be limiting. Osmotic shock, sonication, homogenization, and/or a repeated freeze-thaw cycle followed by filtration and/or centrifugation, among other methods, such as pH adjustment and heat treatment, may be used to produce a cell-free extract from intact cells. Any one or more of these methods also may be employed to release the selected chemical producxt from cells as an extraction step. Further as to general processing of a bio-production broth comprising a selected chemical product, various methods may be practiced to remove biomass and/or separate the chemical product from the culture broth and its components, including centrifugation, filtration, extraction, chemical conversion such as esterification, distillation, crystallization, chromatography, and ion-exchange, in various forms. Additionally, cell rupture may be conducted as needed to release a selected chemical from the cell mass, such as by sonication, homogenization, pH adjustment or heating. a selected chemical may be further separated and/or purified by methods known in the art, including any combination of one or more of centrifugation, liquid-liquid separations, including extractions such as solvent extraction, reactive extraction, two-phase aqueous extraction and two-phase solvent extraction, membrane separation technologies, distillation, evaporation, ion-exchange chromatography, adsorption chromatography, reverse phase chromatography and crystallization. Any of the above methods may be applied to a portion of a bio-production broth (i.e., a fermentation broth, whether made under aerobic, anaerobic, or microaerobic conditions), such as may be removed from a bio-production event gradually or periodically, or to the broth at termination of a bio-production event.

Polypeptides, such as encoded by the various specified genes, may be NADH- or NADPH-dependent, and methods known in the art may be used to convert a particular enzyme to be either form. More particularly, as noted in WO 2002/042418, "any method can be used to convert a polypeptide that uses NADPH as a cofactor into a polypeptide that uses NADH as a cofactor such as those described by others (Eppink et al., J Mol. Biol., 292 (1) : 87-96 (1999), Hall and Tomsett, Microbiology, 146 (Pt 6): 1399-406 (2000), and Dohr et al., Proc. Natl. Acad. Sci., 98 (1) : 81-86 (2001))."

In various embodiments, bio-production of a selected chemical product may reach at least 1, at least 2, at least 5, at least 10, at least 20, at least 30, at least 40, and at least 50 g/liter titer, such as by using one of the methods disclosed herein.

As may be realized by appreciation of the advances disclosed herein as they relate to commercial fermentations of selected chemical products, embodiments of the present invention may be combined with other genetic modifications and/or method or system modulations so as to obtain a microorganism (and corresponding method) effective to produce at least 10, at least 20, at least 30, at least 40, at least 45, at least 50, at least 80, at least 100, or at least 120 grams of a chemical product per liter of final (e.g., spent) fermentation broth while achieving this with specific and/or volumetric productivity rates as disclosed herein.

In some embodiments a microbial chemical bio-production event (i.e., a fermentation event using a cultured population of a microorganism) proceeds using a genetically modified microorganism as described herein, wherein the specific productivity is between 0.01 and 0.60 grams of selected chemical product produced per gram of microorganism cell on a dry weight basis per hour (g chemical product/g DCW-hr). In various embodiments the specific productivity is greater than 0.01, greater than 0.05, greater than 0.10, greater than 0.15, greater than 0.20, greater than 0.25, greater than 0.30, greater than 0.35, greater than 0.40, greater than 0.45, or greater than 0.50 g chemical product/g DCW-hr. Specific productivity may be assessed over a 2, 4, 6, 8, 12 or 24 hour period in a particular microbial chemical production event. More particularly, the specific productivity for a chemical product is between 0.05 and 0.10, 0.10 and 0.15, 0.15 and 0.20, 0.20 and 0.25, 0.25 and 0.30, 0.30 and 0.35, 0.35 and 0.40, 0.40 and 0.45, or 0.45 and 0.50 g chemical product/g DCW-hr., 0.50 and 0.55, or 0.55 and 0.60 g chemical product/g DCW-hr. Various embodiments comprise culture systems demonstrating such productivity.

In some embodiments, specific productivity as measured over a 24-hour fermentation (culture) period may be greater than 0.01, 0.05, 0.10, 0.20, 0.5, 1.0, 2.0, 3.0, 4.0, 5.0, 6.0, 7.0, 8.0, 9.0, 10.0, 11.0 or 12.0 grams of chemical product per gram DCW of microorganisms (based on the final DCW at the end of the 24-hour period).

In various aspects and embodiments of the present invention, there is a resulting substantial increase in microorganism specific productivity that advances the fermentation art and commercial economic feasibility of microbial chemical production, such as of phloroglucinol or resorcinol.

Stated in another manner, in various embodiments the specific productivity exceeds (is at least) 0.01 g chemical product/g DCW-hr, exceeds (is at least) 0.05 g chemical product/g DCW-hr, exceeds (is at least) 0.10 g chemical product/g DCW-hr, exceeds (is at least) 0.15 g chemical product/g DCW-hr, exceeds (is at least) 0.20 g chemical product/g DCW-hr, exceeds (is at least) 0.25 g chemical product/g DCW-hr, exceeds (is at least) 0.30 g chemical product/g DCW-hr, exceeds (is at least) 0.35 g chemical product/g DCW-hr, exceeds (is at least) 0.40 g chemical product/g DCW-hr, exceeds (is at least) 0.45 g chemical product/g DCW-hr, exceeds (is at least) 0.50 g chemical product/g DCW-hr, exceeds (is at least) 0.60 g chemical product/g DCW-hr.

More generally, based on various combinations of the genetic modifications described herein, optionally in combination with supplementations described herein, specific productivity values for 3-HP, and for other chemical products described herein, may exceed 0.01 g chemical product/g DCW-hr, may exceed 0.05 g chemical product/g DCW-hr, may exceed 0.10 g chemical product/g DCW-hr, may exceed 0.15 g chemical product/g DCW-hr, may exceed 0.20 g chemical product/g DCW-hr, may exceed 0.25 g chemical product/g DCW-hr, may exceed 0.30 g chemical product/g DCW-hr, may exceed 0.35 g chemical product/g DCW-hr, may exceed 0.40 g chemical product/g DCW-hr, may exceed 0.45 g chemical product/g DCW-hr, and may exceed 0.50 g or 0.60 chemical product/g DCW-hr. Such specific productivity may be assessed over a 2, 4, 6, 8, 12 or 24 hour period in a particular microbial chemical production event.

In some embodiments a microbial chemical biosynthesis event (i.e., a fermentation event using a cultured population of a microorganism) proceeds using a genetically modified microorganism as described herein, wherein the specific productivity is between 0.01 and 0.60 grams of selected chemical product produced per gram of microorganism cell on a dry weight basis per hour (g chemical product/g DCW-hr). In various embodiments the specific productivity is greater than 0.01, greater than 0.05, greater than 0.10, greater than 0.15, greater than 0.20, greater than 0.25, greater than 0.30, greater than 0.35, greater than 0.40, greater than 0.45, or greater than 0.50 g chemical product/g DCW-hr. Specific productivity may be assessed over a 2, 4, 6, 8, 12 or 24 hour period in a particular microbial chemical production event. More particularly, the specific productivity for a chemical product is between 0.05 and 0.10, 0.10 and 0.15, 0.15 and 0.20, 0.20 and 0.25, 0.25 and 0.30, 0.30 and 0.35, 0.35 and 0.40, 0.40 and 0.45, or 0.45 and 0.50 g chemical product/g DCW-hr., 0.50 and 0.55, or 0.55 and 0.60 g chemical product/g DCW-hr. Various embodiments comprise culture systems demonstrating such productivity.

Also, in various embodiments of the present invention the volumetric productivity achieved may be 0.25 g polyketide (or other chemical product) per liter per hour (g (chemical product)IL-hr), may be greater than 0.25 g polyketide (or other chemical product)/L-hr, may be greater than 0.50 g polyketide (or other chemical product)/L-hr, may be greater than 1.0 g polyketide (or other chemical product)IL-hr, may be greater than 1.50 g polyketide (or other chemical product)IL-hr, may be greater than 2.0 g polyketide (or other chemical product)IL- hr, may be greater than 2.50 g polyketide (or other chemical product)IL¬hr, may be greater than 3.0 g polyketide (or other chemical product)IL-hr, may be greater than 3.50 g polyketide (or other chemical product)IL-hr, may be greater than 4.0 g polyketide (or other chemical product)IL-hr, may be greater than 4.50 g polyketide (or other chemical product)/L-hr, may be greater than 5.0 g polyketide (or other chemical product)/L-hr, may be greater than 5.50 g polyketide (or other chemical product)IL-hr, may be greater than 6.0 g polyketide (or other chemical product)IL-hr, may be greater than 6.50 g polyketide (or other chemical product)/L-hr, may be greater than 7.0 g polyketide (or other chemical product)IL-hr, may be greater than 7.50 g polyketide (or other chemical product)/L-hr, may be greater than 8.0 g polyketide (or other chemical product)IL-hr, may be greater than 8.50 g polyketide (or other chemical product)IL-hr, may be greater than 9.0 g polyketide (or other chemical product)IL- hr, may be greater than 9.50 g polyketide (or other chemical product)IL-hr, or may be greater than 10.0 g polyketide (or other chemical product)IL-hr.

In some embodiments, specific productivity as measured over a 24-hour fermentation (culture) period may be greater than 0.01, 0.05, 0.10, 0.20, 0.5, 1.0, 2.0, 3.0, 4.0, 5.0, 6.0, 7.0, 8.0, 9.0, 10.0, 11.0 or 12.0 grams of chemical product per gram DCW of microorganisms (based on the final DCW at the end of the 24-hour period).

The improvements achieved by embodiments of the present invention may be determined by percentage increase in specific productivity, or by percentage increase in volumetric productivity, compared with an appropriate control microorganism lacking the particular genetic modification combinations taught herein (with or without the supplements taught herein, added to a vessel comprising the microorganism population). For particular embodiments and groups thereof, such specific productivity and/or volumetric productivity improvements is/are at least 10, at least 20, at least 30, at least 40, at least 50, at least 100, at least 200, at least 300, at least 400, and at least 500 percent over the respective specific productivity and/or volumetric productivity of such appropriate control microorganism.

The specific methods and teachings of the specification, and/or cited references that are incorporated by reference, may be incorporated into the examples. Also, production of a chemical product may reach at least 1, at least 2, at least 5, at least 10, at least 20, at least 30, at least 40, and at least 50 g/liter titer in various embodiments.

The metrics may be applicable to any of the compositions, e.g., genetically modified microorganisms, methods, e.g., of producing chemical products, and systems, e.g., fermentation systems utilizing the genetically modified microorganisms and/or methods disclosed herein.

The amount of 3-HP or other product(s), including a polyketide, produced in a bio-production media generally can be determined using a number of methods known in the art, for example, high performance liquid chromatography (HPLC), gas chromatography (GC), GC/Mass Spectroscopy (MS), or spectrometry.

When 3-HP is the chemical product, the 3-HP may be separated and purified by the approaches described in the following paragraphs, taking into account that many methods of separation and purification are known in the art and the following disclosure is not meant to be limiting. Osmotic shock, sonication, homogenization, and/or a repeated freeze-thaw cycle followed by filtration and/or centrifugation, among other methods, such as pH adjustment and heat treatment, may be used to produce a cell-free extract from intact cells. Any one or more of these methods also may be employed to release 3-HP from cells as an extraction step.

Further as to general processing of a bio-production broth comprising 3-HP, various methods may be practiced to remove biomass and/or separate 3-HP from the culture broth and its components. Methods to separate and/or concentrate the 3-HP include centrifugation, filtration, extraction, chemical conversion such as esterification, distillation (which may result in chemical conversion, such as dehydration to acrylic acid, under some reactive-distillation conditions), crystallization, chromatography, and ion-exchange, in various forms. Additionally, cell rupture may be conducted as needed to release 3-HP from the cell mass, such as by sonication, homogenization, pH adjustment or heating. 3-HP may be further separated and/or purified by methods known in the art, including any combination of one or more of centrifugation, liquid-liquid separations, including extractions such as solvent extraction, reactive extraction, two-phase aqueous extraction and two-phase solvent extraction, membrane separation technologies, distillation, evaporation, ion-exchange chromatography, adsorption chromatography, reverse phase chromatography and crystallization. Any of the above methods may be applied to a portion of a bio-production broth (i.e., a fermentation broth, whether made under aerobic, anaerobic, or microaerobic conditions), such as may be removed from a bio-production event gradually or periodically, or to the broth at termination of a bio-production event. Conversion of 3-HP to downstream products, such as described herein, may proceed after separation and purification, or, such as with distillation, thin-film evaporation, or wiped-film evaporation optionally also in part as a separation means.

For various of these approaches, one may apply a counter-current strategy, or a sequential or iterative strategy, such as multi-pass extractions. For example, a given aqueous solution comprising 3-HP may be repeatedly extracted with a non-polar phase comprising an amine to achieve multiple reactive extractions.

When a culture event (fermentation event) is at a point of completion, the spent broth may transferred to a separate tank, or remain in the culture vessel, and in either case the temperature may be elevated to at least 60°C for a minimum of one hour in order to kill the microorganisms. (Alternatively, as noted above other approaches to killing the microorganisms may be practiced, or centrifugation may occur prior to heating.) By spent broth is meant the final liquid volume comprising the initial nutrient media, cells grown from the microorganism inoculum (and possibly including some original cells of the inoculum), 3-HP, and optionally liquid additions made after providing the initial nutrient media, such as periodic additions to provide additional carbon source, etc. It is noted that the spent broth may comprise organic acids other than 3-HP, such as for example acetic acid and/or lactic acid.

A centrifugation step may then be practiced to filter out the biomass solids (e.g., microorganism cells). This may be achieved in a continuous or batch centrifuge, and solids removal may be at least about 80%, 85%, 90%, or 95% in a single pass, or cumulatively after two or more serial centrifugations.

An optional step is to polish the centrifuged liquid through a filter, such as microfiltration or ultrafiltration, or may comprise a filter press or other filter device to which is added a filter aid such as diatomaceous earth. Alternative or supplemental approaches to this and the centrifugation may include removal of cells by a flocculent, where the cells floc and are allowed to settle, and the liquid is drawn off or otherwise removed. A flocculent may be added to a fermentation broth after which settling of material is allowed for a time, and then separations may be applied, including but not limited to centrifugation.

After such steps, a spent broth comprising 3-HP and substantially free of solids is obtained for further processing. By "substantially free of solids" is meant that greater than 98%, 99%, or 99.5% of the solids have been removed.

In various embodiments this spent broth comprises various ions of salts, such as Na, Cl, SO4, and PO4. In some embodiments these ions may be removed by passing this spent broth through ion exchange columns, or otherwise contacting the spent broth with appropriate ion exchange material. Here and elsewhere in this document, "contacting" is taken to mean a contacting for the stated purpose by any way known to persons skilled in the art, such as, for example, in a column, under appropriate conditions that are well within the ability of persons of ordinary skill in the relevant art to determine. As but one example, these may comprise sequential contacting with anion and cation exchange materials (in any order), or with a mixed anion/cation material. This demineralization step should remove most such inorganic ions without removing the 3-HP. This may be achieved, for example, by lowering the pH sufficiently to protonate 3-HP and similar organic acids so that these acids are not bound to the anion exchange material, whereas anions, such as Cl and SO4, that remain charged at such pH are removed from the solution by binding to the resin. Likewise, positively charged ions are removed by contacting with cation exchange material. Such removal of ions may be assessed by a decrease in conductivity of the solution. Such ion exchange materials may be regenerated by methods known to those skilled in the art.

In some embodiments, the spent broth (such as but not necessarily after the previous demineralization step) is subjected to a pH elevation, after which it is passed through an ion exchange column, or otherwise contacted with an ion exchange resin, that comprises anionic groups, such as amines, to which organic acids, ionic at this pH, associate. Other organics that do not so associate with amines at this pH (which may be over 6.5, over 7.5, over 8.5, over 9.5, over 10.5, or higher pH) may be separated from the organic acids at this stage, such as by flushing with an elevated pH rinse. Thereafter elution with a lower pH and/or elevated salt content rinse may remove the organic acids. Eluting with a gradient of decreasing pH and/or increasing salt content rinses may allow more distinct separation of 3-HP from other organic acids, thereafter simplifying further processing.

This latter step of anion-exchange resin retention of organic acids may be practiced before or after the demineralization step. However, the following two approaches are alternatives to the anion-exchange resin step.

A first alternative approach comprises reactive extraction (a form of liquid-liquid extraction) as exemplified in this and the following paragraphs. The spent broth, which may be at a stage before or after the demineralization step above, is combined with a quantity of a tertiary amine such as Alamine336® (Cognis Corp., Cincinnati, OH USA) at low pH. Co-solvents for the Alamine336 or other tertiary amine may be added and include, but are not limited to benzene, carbon tetrachloride, chloroform, cyclohexane, disobutyl ketone, ethanol, #2 fuel oil, isopropanol, kerosene, n-butanol, isobutanol, octanol, and n-decanol that increase the partition coefficient when combined with the amine. After appropriate mixing a period of time for phase separation transpires, after which the non-polar phase, which comprises 3-HP associated with the Alamine336 or other tertiary amine, is separated from the aqueous phase.

When a co-solvent is used that has a lower boiling point than the 3-HP/tertiary amine, a distilling step may be used to remove the co-solvent, thereby leaving the 3-HP-tertiary amine complex in the non-polar phase.

Whether or not there is such a distillation step, a stripping or recovery step may be used to separate the 3-HP from the tertiary amine. An inorganic salt, such as ammonium sulfate, sodium chloride, or sodium carbonate, or a base such as sodium hydroxide or ammonium hydroxide, is added to the 3-HP/tertiary amine to reverse the amine protonation reaction, and a second phase is provided by addition of an aqueous solution (which may be the vehicle for provision of the inorganic salt). After suitable mixing, two phases result and this allows for tertiary amine regeneration and re-use, and provides the 3-HP in an aqueous solution. Alternatively, hot water may also be used without a salt or base to recover the 3HP from the amine.

In the above approach the phase separation and extraction of 3-HP to the aqueous phase can serve to concentrate the 3-HP. It is noted that chromatographic separation of respective organic acids also can serve to concentrate such acids, such as 3-HP. In similar approaches other suitable, non-polar amines, which may include primary, secondary and quaternary amines, may be used instead of and/or in combination with a tertiary amine.

A second alternative approach is crystallization. For example, the spent broth (such as free of biomass solids) may be contacted with a strong base such as ammonium hydroxide, which results in formation of an ammonium salt of 3-HP. This may be concentrated, and then ammonium-3-HP crystals are formed and may be separated, such as by filtration, from the aqueous phase. Once collected, ammonium-3-HP crystals may be treated with an acid, such as sulfuric acid, so that ammonium sulfate is regenerated, so that 3-HP and ammonium sulfate result.

Also, various aqueous two-phase extraction methods may be utilized to separate and/or concentrate a desired chemical product from a fermentation broth or later-obtained solution. It is known that the addition of polymers, such as dextran and glycol polymers, such as polyethylene glycol (PEG) and polypropylene glycol (PPG) to an aqueous solution may result in formation of two aqueous phases. In such systems a desired chemical product may segregate to one phase while cells and other chemicals partition to the other phase, thus providing for a separation without use of organic solvents. This approach has been demonstrated for some chemical products, but challenges associated with chemical product recovery from a polymer solution and low selectivities are recognized (See "Extractive Recovery of Products from Fermentation Broths," Joong Kyun Kim et al., Biotechnol. Bioprocess Eng., 1999(4)1-11, incorporated by reference for all of its teachings of extractive recovery methods).

Various substitutions and combinations of the above steps and processes may be made to obtain a relatively purified 3-HP solution. Also, methods of separation and purification disclosed in US 6,534,679, issued March 18, 2003, and incorporated by reference herein for such methods disclosures, may be considered based on a particular processing scheme. Also, in some culture events periodic removal of a portion of the liquid volume may be made, and processing of such portion(s) may be made to recover the 3-HP, including by any combination of the approaches disclosed above.

As noted, solvent extraction is another alternative. This may use any of a number of and/or combinations of solvents, including alcohols, esters, ketones, and various organic solvents. Without being limiting, after phase separation a distillation step or a secondary extraction may be employed to separate 3-HP from the organic phase.

The following published resources are incorporated by reference herein for their respective teachings to indicate the level of skill in these relevant arts, and as needed to support a disclosure that teaches how to make and use methods of industrial bio-production of 3-HP, and also industrial systems that may be used to achieve such conversion with any of the recombinant microorganisms of the present invention (Biochemical Engineering Fundamentals, 2'd Ed. J. E. Bailey and D. F. 011is, McGraw Hill, New York, 1986, entire book for purposes indicated and Chapter 9, pp. 533-657 in particular for biological reactor design; Unit Operations of Chemical Engineering, 5th E a W. L. McCabe et al., McGraw Hill, New York 1993, entire book for purposes indicated, and particularly for process and separation technologies analyses; Equilibrium Staged Separations, P. C. Wankat, Prentice Hall, Englewood Cliffs, NJ USA, 1988, entire book for separation technologies teachings).

### IX. CONVERSION OF FERMENTATION PRODUCT TO DOWNSTREAM PRODUCT(S)

### Conversion of 3-HP to Acrylic Acid and Downstream Products

As discussed herein, various embodiments described herein are related to production of a particular chemical product, 3-hydroxypropionic acid (3-HP). This organic acid, 3-HP, may be converted to various other products having industrial uses, such as but not limited to acrylic acid, esters of acrylic acid, and other chemicals obtained from 3-HP, referred to as "downstream products." Under some approaches the 3-HP may be converted to acrylic acid, acrylamide, and/or other downstream chemical products, in some instances the conversion being associated with the separation and/or purification steps. Many conversions to such downstream products are described herein. The methods of the invention include steps to produce downstream products of 3-HP.

As a C3 building block, 3-HP offers much potential in a variety of chemical conversions to commercially important intermediates, industrial end products, and consumer products. For example, 3-HP may be converted to acrylic acid, acrylates (e.g., acrylic acid salts and esters), 1,3-propanediol, malonic acid, ethyl-3- hydroxypropionate, ethyl ethoxy propionate, propiolactone, acrylamide, or acrylonitrile.

For example, methyl acrylate may be made from 3-HP via dehydration and esterification, the latter to add a methyl group (such as using methanol); acrylamide may be made from 3-HP via dehydration and amidation reactions; acrylonitrile may be made via a dehydration reaction and forming a nitrile moiety; propriolactone may be made from 3-HP via a ring-forming internal esterification reaction (eliminating a water molecule); ethyl-3-HP may be made from 3-HP via esterification with ethanol; malonic acid may be made from 3-HP via an oxidation reaction; and 1,3-propanediol may be made from 3-HP via a reduction reaction. Also, acrylic acid, first converted from 3-HP by dehydration, may be esterified with appropriate compounds to form a number of commercially important acrylate-based esters, including but not limited to methyl acrylate, ethyl acrylate, methyl acrylate, 2-ethylhexyl acrylate, butyl acrylate, and lauryl acrylate. Alternatively, 3HP may be esterified to form an ester of 3HP and then dehydrated to form the acrylate ester.

Additionally, 3-HP may be oligomerized or polymerized to form poly(3-hydroxypropionate) homopolymers, or co-polymerized with one or more other monomers to form various co-polymers. Because 3-HP has only a single stereoisomer, polymerization of 3-HP is not complicated by the stereo-specificity of monomers during chain growth. This is in contrast to (S)-2-Hydroxypropanoic acid (also known as lactic acid), which has two (D, L) stereoisomers that must be considered during its polymerizations.

As will be further described, 3-HP can be converted into derivatives starting (i) substantially as the protonated form of 3-hydroxypropionic acid; (ii) substantially as the deprotonated form, 3- hydroxypropionate; or (iii) as mixtures of the protonated and deprotonated forms. Generally, the fraction of 3-HP present as the acid versus the salt will depend on the pH, the presence of other ionic species in solution, temperature (which changes the equilibrium constant relating the acid and salt forms), and to some extent pressure. Many chemical conversions may be carried out from either of the 3-HP forms, and overall process economics will typically dictate the form of 3-HP for downstream conversion.

Also, as an example of a conversion during separation, 3-HP in an amine salt form, such as in the extraction step herein disclosed using Alamine 336 as the amine, may be converted to acrylic acid by contacting a solution comprising the 3-HP amine salt with a dehydration catalyst, such as aluminum oxide, at an elevated temperature, such as 170 to 180 C, or 180 to 190 C, or 190 to 200 C, and passing the collected vapor phase over a low temperature condenser. Operating conditions, including 3-HP concentration, organic amine, co-solvent (if any), temperature, flow rates, dehydration catalyst, and condenser temperature, are evaluated and improved for commercial purposes. Conversion of 3-HP to acrylic acid is expected to exceed at least 80 percent, or at least 90 percent, in a single conversion event. The amine may be re-used, optionally after clean-up. Other dehydration catalysts, as provided herein, may be evaluated. It is noted that U.S. Patent No.7,186,856 discloses data regarding this conversion approach, albeit as part of an extractive salt-splitting conversion that differs from the teachings herein. However, U.S. Patent No. 7,186,856 is incorporated by reference for its methods, including extractive salt-splitting, the latter to further indicate the various ways 3-HP may be extracted from a microbial fermentation broth.

Further as to embodiments in which the chemical product being synthesized by the microorganism host cell is 3-HP, made as provided herein and optionally purified to a selected purity prior to conversion, the methods of the present invention can also be used to produce "downstream" compounds derived from 3-HP, such as polymerized-3-HP (poly-3-HP), acrylic acid, polyacrylic acid (polymerized acrylic acid, in various forms), methyl acrylate, acrylamide, acrylonitrile, propiolactone, ethyl 3-HP, malonic acid, and 1,3- propanediol. Numerous approaches may be employed for such downstream conversions, generally falling into enzymatic, catalytic (chemical conversion process using a catalyst), thermal, and combinations thereof (including some wherein a desired pressure is applied to accelerate a reaction).

As noted, an important industrial chemical product that may be produced from 3-HP is acrylic acid. Chemically, one of the carbon-carbon single bonds in 3-HP must undergo a dehydration reaction, converting to a carbon-carbon double bond and rejecting a water molecule. Dehydration of 3-HP in principle can be carried out in the liquid phase or in the gas phase. In some embodiments, the dehydration takes place in the presence of a suitable homogeneous or heterogeneous catalyst. Suitable dehydration catalysts are both acid and alkaline catalysts. Following dehydration, an acrylic acid-containing phase is obtained and can be purified where appropriate by further purification steps, such as by distillation methods, extraction methods, or crystallization methods, or combinations thereof.

Making acrylic acid from 3-HP via a dehydration reaction may be achieved by a number of commercial methodologies including via a distillation process, which may be part of the separation regime and which may include an acid and/or a metal ion as catalyst. More broadly, incorporated herein for its teachings of conversion of 3-HP, and other β-hydroxy carbonyl compounds, to acrylic acid and other related downstream compounds, is U.S. Patent Publication No. 2007/0219390 A1, published September 20, 2007, now abandoned. This publication lists numerous catalysts and provides examples of conversions, which are specifically incorporated herein. Also among the various specific methods to dehydrate 3-HP to produce acrylic acid is an older method, described in U.S. Patent No. 2,469,701 (Redmon). This reference teaches a method for the preparation of acrylic acid by heating 3-HP to a temperature between 130 and 190°C, in the presence of a dehydration catalyst, such as sulfuric acid or phosphoric acid, under reduced pressure. U.S. Patent Publication No. 2005/0222458 A1(Craciun et al.) also provides a process for the preparation of acrylic acid by heating 3-HP or its derivatives. Vapor-phase dehydration of 3-HP occurs in the presence of dehydration catalysts, such as packed beds of silica, alumina, or titania. These patent publications are incorporated by reference for their methods relating to converting 3-HP to acrylic acid.

The dehydration catalyst may comprise one or more metal oxides, such as A1203, Si02, or Ti02. In some embodiments, the dehydration catalyst is a high surface area A1203 or a high surface area silica wherein the silica is substantially Si02. High surface area for the purposes of the invention means a surface area of at least about 50, 75, 100 m2/g, or more. In some embodiments, the dehydration catalyst may comprise an aluminosilicate, such as a zeolite.

For example, including as exemplified from such incorporated references, 3-HP may be dehydrated to acrylic acid via various specific methods, each often involving one or more dehydration catalysts. One catalyst of particular apparent value is titanium, such as in the form of titanium oxide, TiO(2). A titanium dioxide catalyst may be provided in a dehydration system that distills an aqueous solution comprising 3-HP, wherein the 3-HP dehydrates, such as upon volatilization, converting to acrylic acid, and the acrylic acid is collected by condensation from the vapor phase.

As but one specific method, an aqueous solution of 3-HP is passed through a reactor column packed with a titanium oxide catalyst maintained at a temperature between 170 and 190 C and at ambient atmospheric pressure. Vapors leaving the reactor column are passed over a low temperature condenser, where acrylic acid is collected. The low temperature condenser may be cooled to 30 C or less, 2 C or less, or at any suitable temperature for efficient condensation based on the flow rate and design of the system. Also, the reactor column temperatures may be lower, for instance when operating at a pressure lower than ambient atmospheric pressure. It is noted that Example 1 of U.S. Patent Publication No. 2007/0219390, published September 20, 2007, now abandoned, provides specific parameters that employs the approach of this method. As noted, this publication is incorporated by reference for this teaching and also for its listing of catalysts that may be used in a 3-HP to acrylic acid dehydration reaction.

Further as to dehydration catalysts, the following table summarizes a number of catalysts (including chemical classes) that may be used in a dehydration reaction from 3-HP (or its esters) to acrylic acid (or acrylate esters). Such catalysts, some of which may be used in any of solid, liquid or gaseous forms, may be used individually or in any combination. This listing of catalysts is not intended to be limiting, and many specific catalysts not listed may be used for specific dehydration reactions. Further without being limiting, catalyst selection may depend on the solution pH and/or the form of 3-HP in a particular conversion, so that an acidic catalyst may be used when 3-HP is in acidic form, and a basic catalyst may be used when the ammonium salt of 3-HP is being converted to acrylic acid. Also, some catalysts may be in the form of ion exchange resins.

**Table 12: Dehydration Catalysts**

| Catalyst by Chemical Class | Non-limiting Examples |
|---|---|
| Acids (including weak and strong) | H2SO4,HCI, titanic acids, metal oxide hydrates, metal sulfates (MSO4,.where M=Zn, Sn, Ca, Ba, Ni, Co, or other transition metals), metal oxide sulfates, metal phosphates (e.g., M3,(PO4) 2, where M=Ca, Ba), metal phosphates, metal oxide phosphates, carbon (e.g., transition metals on a carbon support), mineral acids, carboxylic acids, salts thereof, acidic resins, acidic zeolites, clays, Si02/H3PO4, fluorinated A1203, Nb203/P05 3, N b203/SO4 2, Nb2O5H2O, phosphotungstic acids, phosphomolybdic acids, silicomolybdic acids, silicotungstic acids, carbon dioxide |
| Bases (including weak and strong) | NaOH, ammonia, polyvinylpyridine, metal hydroxides, Zr(OH)4, and substituted amines |
| Oxides (generally metal oxides) | TiO₂, Zr02, Al203, SiO2, Zn02, Sn02, W03, Mn02, Fe2O3, V205 |

As to another specific method using one of these catalysts, concentrated sulfuric acid and an aqueous solution comprising 3-HP are separately flowed into a reactor maintained at 150 to 165°C at a reduced pressure of 100 mm Hg. Flowing from the reactor is a solution comprising acrylic acid. A specific embodiment of this method, disclosed in Example 1 of US2009/0076297, incorporated by reference herein, indicates a yield of acrylic acid exceeding 95 percent.

Based on the wide range of possible catalysts and knowledge in the art of dehydration reactions of this type, numerous other specific dehydration methods may be evaluated and implemented for commercial production.

The dehydration of 3-HP may also take place in the absence of a dehydration catalyst. For example, the reaction may be run in the vapor phase in the presence of a nominally inert packing such as glass, ceramic, a resin, porcelain, plastic, metallic or brick dust packing and still form acrylic acid in reasonable yields and purity. The catalyst particles can be sized and configured such that the chemistry is, in some embodiments, mass-transfer-limited or kinetically limited. The catalyst can take the form of powder, pellets, granules, beads, extrudates, and so on. When a catalyst support is optionally employed, the support may assume any physical form such as pellets, spheres, monolithic channels, etc. The supports may be co-precipitated with active metal species; or the support may be treated with the catalytic metal species and then used as is or formed into the aforementioned shapes; or the support may be formed into the aforementioned shapes and then treated with the catalytic species.

A reactor for dehydration of 3-HP may be engineered and operated in a wide variety of ways. The reactor operation can be continuous, semi-continuous, or batch. It is perceived that an operation that is substantially continuous and at steady state is advantageous from operations and economics perspectives. The flow pattern can be substantially plug flow, substantially well-mixed, or a flow pattern between these extremes. A "reactor" can actually be a series or network of several reactors in various arrangements.

For example, without being limiting, acrylic acid may be made from 3-HP via a dehydration reaction, which may be achieved by a number of commercial methodologies including via a distillation process, which may be part of the separation regime and which may include an acid and/or a metal ion as catalyst. More broadly, incorporated herein for its teachings of conversion of 3-HP, and other β-hydroxy carbonyl compounds, to acrylic acid and other related downstream compounds, is U.S. Patent Publication No. 2007/0219390 A1, published September 20, 2007, now abandoned. This publication lists numerous catalysts and provides examples of conversions, which are specifically incorporated herein.

For example, including as exemplified from such incorporated references, 3-HP may be dehydrated to acrylic acid via various specific methods, each often involving one or more dehydration catalysts.

One catalyst of particular apparent value is titanium, such as in the form of titanium oxide, TiO2. A titanium dioxide catalyst may be provided in a dehydration system that distills an aqueous solution comprising 3-HP, wherein the 3-HP dehydrates, such as upon volatilization, converting to acrylic acid, and the acrylic acid is collected by condensation from the vapor phase.

As but one specific method, an aqueous solution of 3-HP is passed through a reactor column packed with a titanium oxide catalyst maintained at a temperature between 170 and 190°C and at ambient atmospheric pressure. Vapors leaving the reactor column are passed over a low temperature condenser, where acrylic acid is collected. The low temperature condenser may be cooled to 30°C or less, 20°C or less, 2°C or less, or at any suitable temperature for efficient condensation based on the flow rate and design of the system. Also, the reactor column temperatures may be lower, for instance when operating at a pressure lower than ambient atmospheric pressure. It is noted that Example 1 of U.S. Patent Publication No. 2007/0219390, published September 20, 2007, now abandoned, provides specific parameters that employs the approach of this method. As noted, this publication is incorporated by reference for this teaching and also for its listing of catalysts that may be used in a 3-HP to acrylic acid dehydration reaction.

Crystallization of the acrylic acid obtained by dehydration of 3-HP may be used as one of the final separation/purification steps. Various approaches to crystallization are known in the art, including crystallization of esters.

As noted above, in some embodiments, a salt of 3-HP is converted to acrylic acid or an ester or salt thereof. For example, U.S. Patent No. 7,186,856 (Meng et al.) teaches a process for producing acrylic acid from the ammonium salt of 3-HP, which involves a first step of heating the ammonium salt of 3-HP in the presence of an organic amine or solvent that is immiscible with water, to form a two-phase solution and split the 3-HP salt into its respective ionic constituents under conditions which transfer 3-HP from the aqueous phase to the organic phase of the solution, leaving ammonia and ammonium cations in the aqueous phase. The organic phase is then back-extracted to separate the 3-HP, followed by a second step of heating the 3-HP-containing solution in the presence of a dehydration catalyst to produce acrylic acid. U.S. Patent No. 7,186,856 is incorporated by reference for its methods for producing acrylic acid from salts of 3-HP. Various alternatives to the particular approach disclosed in this patent may be developed for suitable extraction and conversion processes.

Methyl acrylate may be made from 3-HP via dehydration and esterification, the latter to add a methyl group (such as using methanol), acrylamide may be made from 3-HP via dehydration and amidation reactions, acrylonitrile may be made via a dehydration reaction and forming a nitrile moiety, propriolactone may be made from 3-HP via a ring-forming internal esterification reaction (eliminating a water molecule), ethyl-3¬HP may be made from 3-HP via esterification with ethanol, malonic acid may be made from 3-HP via an oxidation reaction, and 1,3-propanediol may be made from 3-HP via a reduction reaction.

Malonic acid may be produced from oxidation of 3-HP as produced herein. U.S. Patent No. 5,817,870 (Haas et al.) discloses catalytic oxidation of 3-HP by a precious metal selected from Ru, Rh, Pd, Os, Ir or Pt. These can be pure metal catalysts or supported catalysts. The catalytic oxidation can be carried out using a suspension catalyst in a suspension reactor or using a fixed-bed catalyst in a fixed-bed reactor. If the catalyst, such as a supported catalyst, is disposed in a fixed-bed reactor, the latter can be operated in a trickle-bed procedure as well as also in a liquid-phase procedure. In the trickle-bed procedure the aqueous phase comprising the 3-HP starting material, as well as the oxidation products of the same and means for the adjustment of pH, and oxygen or an oxygen-containing gas can be conducted in parallel flow or counter-flow. In the liquid-phase procedure the liquid phase and the gas phase are conveniently conducted in parallel flow.

In order to achieve a sufficiently short reaction time, the conversion is carried out at a pH equal or greater than 6, such as at least 7, and in particular between 7.5 and 9. According to a particular embodiment, during the oxidation reaction the pH is kept constant, such as at a pH in the range between 7.5 and 9, by adding a base, such as an alkaline or alkaline earth hydroxide solution. The oxidation is usefully carried out at a temperature of at least 10°C and maximally 70°C. The flow of oxygen is not limited. In the suspension method it is important that the liquid and the gaseous phase are brought into contact by stirring vigorously. Malonic acid can be obtained in nearly quantitative yields. U.S. Patent No. 5,817,870 is incorporated by reference herein for its methods to oxidize 3-HP to malonic acid.

1,3-Propanediol may be produced from hydrogenation of 3-HP as produced herein. U.S. Patent Publication No. 2005/0283029 (Meng et al.) is incorporated by reference herein for its methods to hydrogenation of 3-HP, or esters of the acid or mixtures, in the presence of a specific catalyst, in a liquid phase, to prepare 1,3-propanediol. Possible catalysts include ruthenium metal, or compounds of ruthenium, supported or unsupported, alone or in combination with at least one or more additional metal(s) selected from molybdenum, tungsten, titanium, zirconium, niobium, vanadium or chromium. The ruthenium metal or compound thereof, and/or the additional metal(s), or compound thereof, may be utilized in supported or unsupported form. If utilized in supported form, the method of preparing the supported catalyst is not critical and can be any technique such as impregnation of the support or deposition on the support. Any suitable support may be utilized. Supports that may be used include, but are not limited to, alumina, titania, silica, zirconia, carbons, carbon blacks, graphites, silicates, zeolites, aluminosilicate zeolites, aluminosilicate clays, and the like.

The hydrogenation process may be carried out in liquid phase. The liquid phase includes water, organic solvents that are not hydrogenatable, such as any aliphatic or aromatic hydrocarbon, alcohols, ethers, toluene, decalin, dioxane, diglyme, n-heptane, hexane, xylene, benzene, tetrahydrofuran, cyclohexane, methylcyclohexane, and the like, and mixtures of water and organic solvent(s). The hydrogenation process may be carried out batch wise, semi-continuously, or continuously. The hydrogenation process may be carried out in any suitable apparatus. Exemplary of such apparatus are stirred tank reactors, trickle-bed reactors, high pressure hydrogenation reactors, and the like.

The hydrogenation process is generally carried out at a temperature ranging from about 20 to about 250°C, more particularly from about 100 to about 200°C. Further, the hydrogenation process is generally carried out in a pressure range of from about 20 psi to about 4000 psi. The hydrogen containing gas utilized in the hydrogenation process is, optionally, commercially pure hydrogen. The hydrogen containing gas is usable if nitrogen, gaseous hydrocarbons, or oxides of carbon, and similar materials, are present in the hydrogen containing gas. For example, hydrogen from synthesis gas (hydrogen and carbon monoxide) may be employed, such synthesis gas potentially further including carbon dioxide, water, and various impurities.

As is known in the art, it is also possible to convert 3-HP to 1,3-propanediol using biological methods. For example, 1,3-propanediol can be created from either 3-HP-CoA or 3-HP via the use of polypeptides having enzymatic activity. These polypeptides can be used either in vitro or in vivo. When converting 3-HP-CoA to 1,3-propanediol, polypeptides having oxidoreductase activity or reductase activity (e.g., enzymes from the 1.1.1.-class of enzymes) can be used. Alternatively, when creating 1,3-propanediol from 3-HP, a combination of a polypeptide having aldyhyde dehydrogenase activity (e.g., an enzyme from the 1.1.1.34 class) and a polypeptide having alcohol dehydrogenase activity (e.g., an enzyme from the 1.1.1.32 class) can be used.

Another downstream production of 3-HP, acrylonitrile, may be converted from acrylic acid by various organic syntheses, including by not limited to the Sohio acrylonitrile process, a single-step method of production known in the chemical manufacturing industry

Also, addition reactions may yield acrylic acid or acrylate derivatives having alkyl or aryl groups at the carbonyl hydroxyl group. Such additions may be catalyzed chemically, such as by hydrogen, hydrogen halides, hydrogen cyanide, or Michael additions under alkaline conditions optionally in the presence of basic catalysts. Alcohols, phenols, hydrogen sulfide, and thiols are known to add under basic conditions. Aromatic amines or amides, and aromatic hydrocarbons, may be added under acidic conditions. These and other reactions are described in Ulmann's Encyclopedia of Industrial Chemistry, Acrylic Acid and Derivatives, WileyVCH Verlag GmbH, Wienham (2005), incorporated by reference for its teachings of conversion reactions for acrylic acid and its derivatives.

Acrylic acid obtained from 3-HP made by the present invention may be further converted to various chemicals, including polymers, which are also considered downstream products in some embodiments. Acrylic acid esters may be formed from acrylic acid (or directly from 3-HP) such as by condensation esterification reactions with an alcohol, releasing water. This chemistry described in Monomeric Acrylic Esters, E. H. Riddle, Reinhold, NY (1954), incorporated by reference for its esterification teachings. Among esters that are formed are methyl acrylate, ethyl acrylate, n-butyl acrylate, hydroxypropyl acrylate, hydroxyethyl acrylate, isobutyl acrylate, and 2-ethylhexyl acrylate, and these and/or other acrylic acid and/or other acrylate esters may be combined, including with other compounds, to form various known acrylic acid-based polymers. Although acrylamide is produced in chemical syntheses by hydration of acrylonitrile, herein a conversion may convert acrylic acid to acrylamide by amidation.

Acrylic acid obtained from 3-HP made by the present invention may be further converted to various chemicals, including polymers, which are also considered downstream products in some embodiments. Acrylic acid esters may be formed from acrylic acid (or directly from 3-HP) such as by condensation esterification reactions with an alcohol, releasing water. This chemistry is described in Monomeric Acrylic Esters, E. H. Riddle, Reinhold, NY (1954), incorporated by reference for its esterification teachings. Among esters that are formed are methyl acrylate, ethyl acrylate, n-butyl acrylate, hydroxypropyl acrylate, hydroxyethyl acrylate, isobutyl acrylate, and 2-ethylhexyl acrylate, and these and/or other acrylic acid and/or other acrylate esters may be combined, including with other compounds, to form various known acrylic acid-based polymers. Although acrylamide is produced in chemical syntheses by hydration of acrylonitrile, herein a conversion may convert acrylic acid to acrylamide by amidation.

Direct esterification of acrylic acid can take place by esterification methods known to the person skilled in the art, by contacting the acrylic acid obtained from 3-HP dehydration with one or more alcohols, such as methanol, ethanol, 1-propanol, 2-propanol, n-butanol, tert-butanol or isobutanol, and heating to a temperature of at least 50, 75, 100, 125, or 150°C. The water formed during esterification may be removed from the reaction mixture, such as by azeotropic distillation through the addition of suitable separation aids, or by another means of separation. Conversions up to 95%, or more, may be realized, as is known in the art.

Several suitable esterification catalysts are commercially available, such as from Dow Chemical (Midland, Michigan US). For example, AmberlystTM 131 Wet Monodisperse gel catalyst confers enhanced hydraulic and reactivity properties and is suitable for fixed bed reactors. AmberlystTM 39Wet is a macroreticular catalyst suitable particularly for stirred and slurry loop reactors. AmberlystTM 46 is a macroporous catalyst producing less ether byproducts than conventional catalyst (as described in U.S. Patent No. 5,426,199 to Rohm and Haas, which patent is incorporated by reference for its teachings of esterification catalyst compositions and selection considerations).

Acrylic acid, and any of its esters, may be further converted into various polymers. Polymerization may proceed by any of heat, light, other radiation of sufficient energy, and free radical generating compounds, such as azo compounds or peroxides, to produce a desired polymer of acrylic acid or acrylic acid esters. As one example, an aqueous acrylic acid solution's temperature raised to a temperature known to start polymerization (in part based on the initial acrylic acid concentration), and the reaction proceeds, the process frequently involving heat removal given the high exothermicity of the reaction. Many other methods of polymerization are known in the art. Some are described in Ulmann's Encyclopedia of Industrial Chemistry, Polyacrylamides and Poly(Acrylic Acids), WileyVCH Verlag GmbH, Wienham (2005), incorporated by reference for its teachings of polymerization reactions.

For example, the free-radical polymerization of acrylic acid takes place by polymerization methods known to the skilled worker and can be carried out either in an emulsion or suspension in aqueous solution or another solvent. Initiators, such as but not limited to organic peroxides, often are added to aid in the polymerization. Among the classes of organic peroxides that may be used as initiators are diacyls, peroxydicarbonates, monoperoxycarbonates, peroxyketals, peroxyesters, dialkyls, and hydroperoxides. Another class of initiators is azo initiators, which may be used for acrylate polyermization as well as co-polymerization with other monomers. U.S. Patent Nos. 5,470,928; 5,510,307; 6,709,919; and 7,678,869 teach various approaches to polymerization using a number of initiators, including organic peroxides, azo compounds, and other chemical types, and are incorporated by reference for such teachings as applicable to the polymers described herein.

Accordingly, it is further possible for co-monomers, such as crosslinkers, to be present during the polymerization. The free-radical polymerization of the acrylic acid obtained from dehydration of 3-HP, as produced herein, in at least partly neutralized form and in the presence of crosslinkers is practiced in certain embodiments. This polymerization may result in hydrogels which can then be comminuted, ground and, where appropriate, surface-modified, by known techniques.

An important commercial use of polyacrylic acid is for superabsorbent polymers. This specification hereby incorporates by reference Modern Superabsorbent Polymer Technology, Buchholz and Graham (Editors), Wiley-VCH, 1997, in its entirety for its teachings regarding superabsorbent polymers components, manufacture, properties and uses. Superabsorbent polymers are primarily used as absorbents for water and aqueous solutions for diapers, adult incontinence products, feminine hygiene products, and similar consumer products. In such consumer products, superabsorbent materials can replace traditional absorbent materials such as cloth, cotton, paper wadding, and cellulose fiber. Superabsorbent polymers absorb, and retain under a slight mechanical pressure, up to 25 times or their weight in liquid. The swollen gel holds the liquid in a solid, rubbery state and prevents the liquid from leaking. Superabsorbent polymer particles can be surface-modified to produce a shell structure with the shell being more highly crosslinked. This technique improves the balance of absorption, absorption under load, and resistance to gel-blocking. It is recognized that superabsorbent polymers have uses in fields other than consumer products, including agriculture, horticulture, and medicine.

Superabsorbent polymers are prepared from acrylic acid (such as acrylic acid derived from 3 HP provided herein) and a crosslinker, by solution or suspension polymerization. Exemplary methods include U.S. Patent Nos. 5,145,906; 5,350,799; 5,342,899; 4,857,610; 4,985,518; 4,708, 997; 5,180,798; 4,666,983; 4,734,478; and 5,331,059, each incorporated by reference for their teachings relating to superabsorbent polymers.

Among consumer products, a diaper, a feminine hygiene product, and an adult incontinence product are made with superabsorbent polymer that itself is made substantially from acrylic acid converted from 3-HP made in accordance with the present invention.

Diapers and other personal hygiene products may be produced that incorporate superabsorbent polymer made from acrylic acid made from 3-HP which is bio-produced by the teachings of the present application. The following provides general guidance for making a diaper that incorporates such superabsorbent polymer. The superabsorbent polymer first is prepared into an absorbent pad that may be vacuum formed, and in which other materials, such as a fibrous material (e.g., wood pulp) are added. The absorbent pad then is assembled with sheet(s) of fabric, generally a nonwoven fabric (e.g., made from one or more of nylon, polyester, polyethylene, and polypropylene plastics) to form diapers.

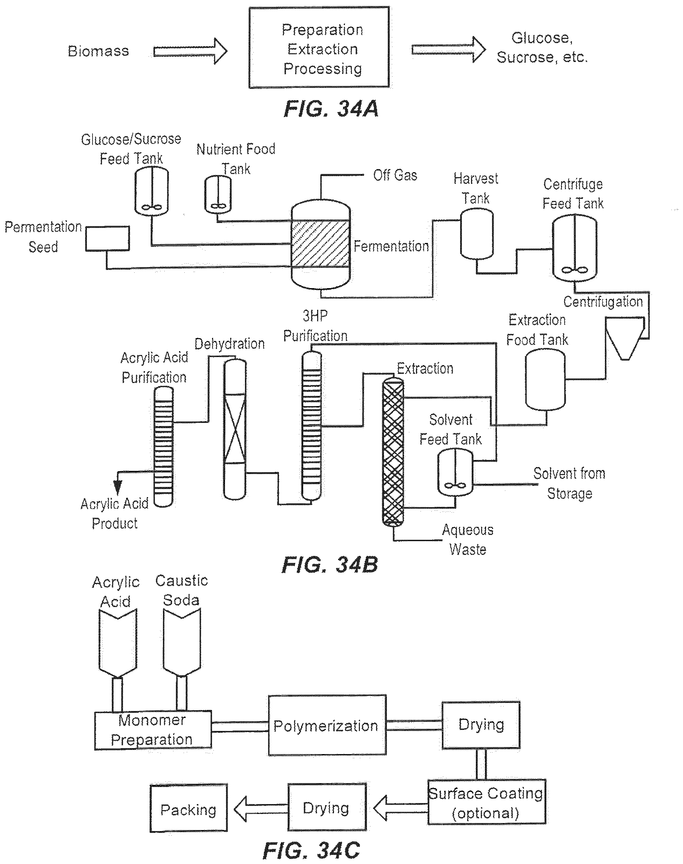

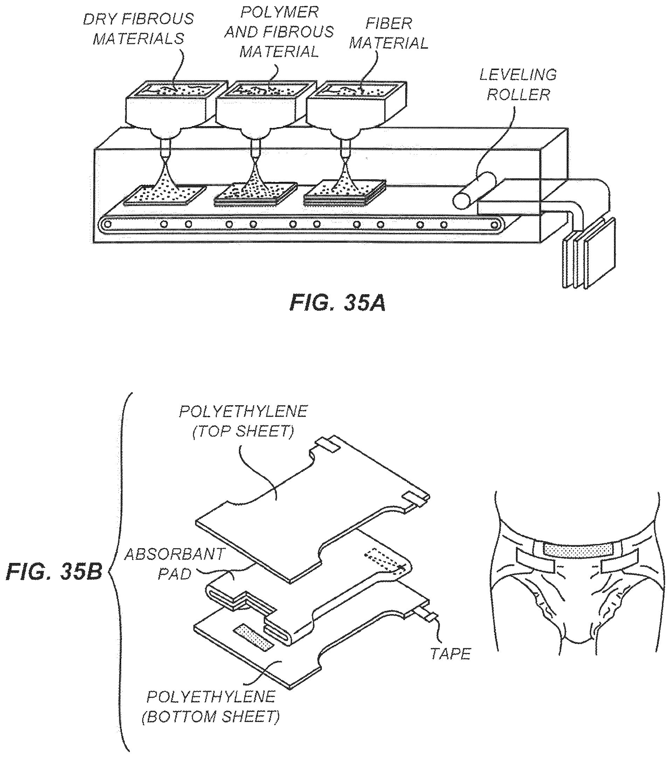

More particularly, in one non-limiting process, above a conveyer belt multiple pressurized nozzles spray superabsorbent polymer particles (such as about 400 micron size or larger), fibrous material, and/or a combination of these onto the conveyer belt at designated spaces/intervals. The conveyor belt is perforated and under vacuum from below, so that the sprayed on materials are pulled toward the belt surface to form a flat pad. In various embodiments, fibrous material is applied first on the belt, followed by a mixture of fibrous material and the superabsorbent polymer particles, followed by fibrous material, so that the superabsorbent polymer is concentrated in the middle of the pad. A leveling roller may be used toward the end of the belt path to yield pads of uniform thickness. Each pad thereafter may be further processed, such as to cut it to a proper shape for the diaper, or the pad may be in the form of a long roll sufficient for multiple diapers. Thereafter, the pad is sandwiched between a top sheet and a bottom sheet of fabric (one generally being liquid pervious, the other liquid impervious), such as on a conveyor belt, and these are attached together such as by gluing, heating or ultrasonic welding, and cut into diaper-sized units (if not previously so cut). Additional features may be provided, such as elastic components, strips of tape, etc., for fit and ease of wearing by a person. FIG. 34A, B, and C and FIG. 35A and B show a schematic of an entire process of converting biomass to a finished product such as a diaper. These are meant to be exemplary and not limiting.

The ratio of the fibrous material to polymer particles is known to effect performance characteristics. In some embodiments, this ratio is between 75:25 and 90:10 (see U.S. Patent No. 4,685,915, incorporated by reference for its teachings of diaper manufacture). Other disposable absorbent articles may be constructed in a similar fashion, such as for adult incontinence, feminine hygiene (sanitary napkins), tampons, etc. (see, for example, U.S. Patent Nos. 5,009,653, 5,558,656, and 5,827,255 incorporated by reference for their teachings of sanitary napkin manufacture).

Low molecular-weight polyacrylic acid has uses for water treatment, flocculants, and thickeners for various applications including cosmetics and pharmaceutical preparations. For these applications, the polymer may be uncrosslinked or lightly crosslinked, depending on the specific application. The molecular weights are typically from about 200 to about 1,000,000 g/mol. Preparation of these low molecular-weight polyacrylic acid polymers is described in U.S. Patent Nos. 3,904,685; 4,301,266; 2,798,053; and 5,093,472, each of which is incorporated by reference for its teachings relating to methods to produce these polymers.

Acrylic acid may be co-polymerized with one or more other monomers selected from acrylamide, 2-acrylamido-2-methylpropanesulfonic acid, N,N-dimethylacrylamide, N-isopropylacrylamide, methacrylic acid, and methacrylamide, to name a few. The relative reactivities of the monomers affect the microstructure and thus the physical properties of the polymer. Co-monomers may be derived from 3-HP, or otherwise provided, to produce co-polymers. Ulmann's Encyclopedia of Industrial Chemistry, Polyacrylamides and Poly(Acrylic Acids), WileyVCH Verlag GmbH, Wienham (2005), is incorporated by reference herein for its teachings of polymer and co-polymer processing.

Acrylic acid can in principle be copolymerized with almost any free-radically polymerizable monomers including styrene, butadiene, acrylonitrile, acrylic esters, maleic acid, maleic anhydride, vinyl chloride, acrylamide, itaconic acid, and so on. End-use applications typically dictate the co-polymer composition, which influences properties. Acrylic acid also may have a number of optional substitutions on it, and after such substitutions be used as a monomer for polymerization, or co-polymerization reactions. As a general rule, acrylic acid (or one of its co-polymerization monomers) may be substituted by any substituent that does not interfere with the polymerization process, such as alkyl, alkoxy, aryl, heteroaryl, benzyl, vinyl, allyl, hydroxy, epoxy, amide, ethers, esters, ketones, maleimides, succinimides, sulfoxides, glycidyl and silyl (see U.S. Patent No. 7,678,869, incorporated by reference above, for further discussion). The following paragraphs provide a few non-limiting examples of copolymerization applications.

Paints that comprise polymers and copolymers of acrylic acid and its esters are in wide use as industrial and consumer products. Aspects of the technology for making such paints can be found in U.S. Patent Nos. 3,687,885 and 3,891,591, incorporated by reference for its teachings of such paint manufacture. Generally, acrylic acid and its esters may form homopolymers or copolymers among themselves or with other monomers, such as amides, methacrylates, acrylonitrile, vinyl, styrene and butadiene. A desired mixture of homopolymers and/or copolymers, referred to in the paint industry as 'vehicle' (or 'binder') are added to an aqueous solution and agitated sufficiently to form an aqueous dispersion that includes sub-micrometer sized polymer particles. The paint cures by coalescence of these 'vehicle' particles as the water and any other solvent evaporate. Other additives to the aqueous dispersion may include pigment, filler (e.g., calcium carbonate, aluminum silicate), solvent (e.g., acetone, benzol, alcohols, etc., although these are not found in certain no VOC paints), thickener, and additional additives depending on the conditions, applications, intended surfaces, etc. In many paints, the weight percent of the vehicle portion may range from about nine to about 26 percent, but for other paints the weight percent may vary beyond this range.

Acrylic-based polymers are used for many coatings in addition to aints. For example, for paper coating latexes, acrylic acid is used from 0.1-5.0%, along with styrene and butadiene, to enhance binding to the paper and modify rheology, freeze-thaw stability and shear stability. In this context, U.S. Patent Nos. 3,875,101 and 3,872,037 are incorporated by reference for their teachings regarding such latexes. Acrylate-based polymers also are used in many inks, particularly UV curable printing inks. For water treatment, acrylamide and/or hydroxy ethyl acrylate are commonly co-polymerized with acrylic acid to produce low molecular-weight linear polymers. In this context, U.S. Patent Nos. 4,431,547 and 4,029,577 are incorporated by reference for their teachings of such polymers. Co-polymers of acrylic acid with maleic acid or itaconic acid are also produced for water-treatment applications, as described in U.S. Patent No. 5,135,677, incorporated by reference for that teaching. Sodium acrylate (the sodium salt of glacial acrylic acid) can be co-polymerized with acrylamide (which may be derived from acrylic acid via amidation chemistry) to make an anionic co-polymer that is used as a flocculant in water treatment.

For thickening agents, a variety of co-monomers can be used, such as described in U.S. Patent Nos. 4,268,641 and 3,915,921, incorporated by reference for description of these co-monomers. U.S. Patent No. 5,135,677 describes a number of co-monomers that can be used with acrylic acid to produce water-soluble polymers, and is incorporated by reference for such description.

Also as noted, some conversions to downstream products may be made enzymatically. For example, 3-HP may be converted to 3-HP-CoA, which then may be converted into polymerized 3-HP with an enzyme having polyhydroxyacid synthase activity (EC 2.3.1.-). Also, 1,3-propanediol can be made using polypeptides having oxidoreductase activity or reductase activity (e.g. , enzymes in the EC 1.1.1.- class of enzymes). Alternatively, when creating 1,3-propanediol from 3HP, a combination of (1) a polypeptide having aldehyde dehydrogenase activity (e.g., an enzyme from the 1.1.1.34 class) and (2) a polypeptide having alcohol dehydrogenase activity (e.g., an enzyme from the 1.1.1.32 class) can be used. Polypeptides having lipase activity may be used to form esters. Enzymatic reactions such as these may be conducted in vitro, such as using cell-free extracts, or in vivo. Thus, various embodiments of the present invention, such as methods of making a chemical, include conversion steps to any such noted downstream products of microbially produced 3-HP, including but not limited to those chemicals described herein and in the incorporated references (the latter for jurisdictions allowing this). For example, one embodiment is making 3-HP molecules by the teachings herein and further converting the 3-HP molecules to polymerized-3-HP (poly-3-HP) or acrylic acid, and such as from acrylic acid then producing from the 3-HP molecules any one of polyacrylic acid (polymerized acrylic acid, in various forms), methyl acrylate, acrylamide, acrylonitrile, propiolactone, ethyl 3-HP, malonic acid, 1,3-propanediol, ethyl acrylate, n-butyl acrylate, hydroxypropyl acrylate, hydroxyethyl acrylate, isobutyl acrylate, 2-ethylhexyl acrylate, and acrylic acid or an acrylic acid ester to which an alkyl or aryl addition is made, and/or to which halogens, aromatic amines or amides, and aromatic hydrocarbons are added.

Also as noted, some conversions to downstream products may be made enzymatically. For example, 3-HP may be converted to 3-HP-CoA, which then may be converted into polymerized 3-HP with an enzyme having polyhydroxyacid synthase activity (EC 2.3.1.-). Also, 1,3-propanediol can be made using polypeptides having oxidoreductase activity or reductase activity (e.g., enzymes in the EC 1.1.1.- class of enzymes). Alternatively, when creating 1,3-propanediol from 3HP, a combination of (1) a polypeptide having aldehyde dehydrogenase activity (e.g., an enzyme from the 1.1.1.34 class) and (2) a polypeptide having alcohol dehydrogenase activity (e.g., an enzyme from the 1.1.1.32 class) can be used. Polypeptides having lipase activity may be used to form esters. Enzymatic reactions such as these may be conducted in vitro, such as using cell-free extracts, or in vivo.

Thus, various embodiments of the present invention, such as methods of making a chemical, include conversion steps to any such noted downstream products of microbially produced 3-HP, including but not limited to those chemicals described herein and in the incorporated references (the latter for jurisdictions allowing this). For example, one embodiment is making 3-HP molecules by the teachings herein and further converting the 3-HP molecules to polymerized-3-HP (poly-3-HP) or acrylic acid, and such as from acrylic acid then producing from the 3-HP molecules any one of polyacrylic acid (polymerized acrylic acid, in various forms), methyl acrylate, acrylamide, acrylonitrile, propiolactone, ethyl 3-HP, malonic acid, 1,3-propanediol, ethyl acrylate, n-butyl acrylate, hydroxypropyl acrylate, hydroxyethyl acrylate, isobutyl acrylate, 2-ethylhexyl acrylate, and acrylic acid or an acrylic acid ester to which an alkyl or aryl addition is made, and/or to which halogens, aromatic amines or amides, and aromatic hydrocarbons are added.

Reactions that form downstream compounds such as acrylates or acrylamides can be conducted in conjunction with use of suitable stabilizing agents or inhibiting agents reducing likelihood of polymer formation. See, for example, U.S. Patent Publication No. 2007/0219390 Al. Stabilizing agents and/or inhibiting agents include, but are not limited to, e.g., phenolic compounds (e.g., dimethoxyphenol (DMP) or alkylated phenolic compounds such as di-tert-butyl phenol), quinones (e.g., t-butyl hydroquinone or the monomethyl ether of hydroquinone (MEHQ)), and/or metallic copper or copper salts (e.g., copper sulfate, copper chloride, or copper acetate). Inhibitors and/or stabilizers can be used individually or in combinations as will be known by those of skill in the art. Also, in various embodiments, the one or more downstream compounds is/are recovered at a molar yield of up to about 100 percent, or a molar yield in the range from about 70 percent to about 90 percent, or a molar yield in the range from about 80 percent to about 100 percent, or a molar yield in the range from about 90 percent to about 100 percent. Such yields may be the result of single-pass (batch or continuous) or iterative separation and purification steps in a particular process.

Acrylic acid and other downstream products are useful as commodities in manufacturing, such as in the manufacture of consumer goods, including diapers, textiles, carpets, paint, adhesives, and acrylic glass.

### EXAMPLES

The following examples are given for the purpose of illustrating various embodiments of the invention and are not meant to limit the present invention in any fashion. Changes therein and other uses which are encompassed within the spirit of the invention as defined by the scope of the claims will occur to those skilled in the art.

### COMMON METHODS

### Common Method Example 1: Microorganism Species and Strains

Bacterial species, that may be utilized as needed, are as follows:
*Acinetobacter calcoaceticus* (DSMZ # 1139) is obtained from the German Collection of Microorganisms and Cell Cultures (Braunschweig, Germany) as a vacuum dried culture. Cultures are then resuspended in Brain Heart Infusion (BHI) Broth (RPI Corp., Mt. Prospect, IL, USA). Serial dilutions of the resuspended *A. calcoaceticus* culture are made into BHI and are allowed to grow for aerobically for 48 hours at 37°C at 250 rpm until saturated.
*Bacillus subtilis* is a gift from the Gill lab (University of Colorado at Boulder, Boulder CO USA) and is obtained as an actively growing culture. Serial dilutions of the actively growing *B. subtilis* culture are made into Luria Broth (RPI Corp., Mt. Prospect, IL, USA) and are allowed to grow for aerobically for 24 hours at 37°C at 250 rpm until saturated.
*Chlorobium limicola* (DSMZ# 245) is obtained from the German Collection of Microorganisms and Cell Cultures (Braunschweig, Germany) as a vacuum dried culture. Cultures are then resuspended using Pfennig's Medium I and II (#28 and 29) as described per DSMZ instructions. *C. limicola* is grown at 25°C under constant vortexing.
*Citrobacter braakii* (DSMZ # 30040) is obtained from the German Collection of Microorganisms and Cell Cultures (Braunschweig, Germany) as a vacuum dried culture. Cultures are then resuspended in Brain Heart Infusion (BHI) Broth (RPI Corp., Mt. Prospect, IL USA). Serial dilutions of the resuspended *C. braakii* culture are made into BHI and are allowed to grow for aerobically for 48 hours at 30°C at 250 rpm until saturated.
*Clostridium acetobutylicum* (DSMZ # 792) is obtained from the German Collection of Microorganisms and Cell Cultures (Braunschweig, Germany) as a vacuum dried culture. Cultures are then resuspended in *Clostridium acetobutylicum* medium (#411) as described per DSMZ instructions. *C*. *acetobutylicum* is grown anaerobically at 37°C at 250 rpm until saturated.
*Clostridium aminobutyricum* (DSMZ # 2634) is obtained from the German Collection of Microorganisms and Cell Cultures (Braunschweig, Germany) as a vacuum dried culture. Cultures are then resuspended in *Clostridium aminobutyricum* medium (#286) as described per DSMZ instructions. *C*. *aminobutyricum* is grown anaerobically at 37°C at 250 rpm until saturated.
*Clostridium kluyveri* (DSMZ #555) is obtained from the German Collection of Microorganisms and Cell Cultures (Braunschweig, Germany) as an actively growing culture. Serial dilutions of *C*. *kluyveri* culture are made into *Clostridium kluyveri* medium (#286) as described per DSMZ instructions. *C*. *kluyveri* is grown anaerobically at 37°C at 250 rpm until saturated.
*Cupriavidus metallidurans* (DMSZ # 2839) is obtained from the German Collection of Microorganisms and Cell Cultures (Braunschweig, Germany) as a vacuum dried culture. Cultures are then resuspended in Brain Heart Infusion (BHI) Broth (RPI Corp., Mt. Prospect, IL USA). Serial dilutions of the resuspended *C*. *metallidurans* culture are made into BHI and are allowed to grow for aerobically for 48 hours at 30°C at 250 rpm until saturated.
*Cupriavidus necator* (DSMZ # 428) is obtained from the German Collection of Microorganisms and Cell Cultures (Braunschweig, Germany) as a vacuum dried culture. Cultures are then resuspended in Brain Heart Infusion (BHI) Broth (RPI Corp., Mt. Prospect, IL USA). Serial dilutions of the resuspended *C*. *necator* culture are made into BHI and are allowed to grow for aerobically for 48 hours at 30°C at 250 rpm until saturated. As noted elsewhere, previous names for this species are Alcaligenes eutrophus and Ralstonia eutrophus.
*Desulfovibrio fructosovorans* (DSMZ # 3604) is obtained from the German Collection of Microorganisms and Cell Cultures (Braunschweig, Germany) as a vacuum dried culture. Cultures are then resuspended in *Desulfovibrio fructosovorans* medium (#63) as described per DSMZ instructions. *D. fructosovorans* is grown anaerobically at 37°C at 250 rpm until saturated.
*Escherichia coli Crooks* (DSMZ#1576) is obtained from the German Collection of Microorganisms and Cell Cultures (Braunschweig, Germany) as a vacuum dried culture. Cultures are then resuspended in Brain Heart Infusion (BHI) Broth (RPI Corp., Mt. Prospect, IL USA). Serial dilutions of the resuspended *E. coli Crooks* culture are made into BHI and are allowed to grow for aerobically for 48 hours at 37°C at 250 rpm until saturated.
*Escherichia coli 12* a gift from the Gill lab (University of Colorado at Boulder, Boulder, CO USA) and is obtained as an actively growing culture. Serial dilutions of the actively growing *E. coli K12* culture are made into Luria Broth (RPI Corp., Mt. Prospect, IL USA) and are allowed to grow for aerobically for 24 hours at 37°C at 250 rpm until saturated.
*Halobacterium salinarum* (DSMZ# 1576) is obtained from the German Collection of Microorganisms and Cell Cultures (Braunschweig, Germany) as a vacuum dried culture. Cultures are then resuspended in *Halobacterium* medium (#97) as described per DSMZ instructions. *H. salinarum* is grown aerobically at 37°C at 250 rpm until saturated.
*Lactobacillus delbruecki* (#4335) is obtained from WYEAST USA (Odell, OR USA) as an actively growing culture. Serial dilutions of the actively growing *L. delbruecki* culture are made into Brain Heart Infusion (BHI) broth (RPI Corp., Mt. Prospect, IL USA) and are allowed to grow for aerobically for 24 hours at 30°C at 250 rpm until saturated.
*Metallosphaera sedula* (DSMZ #5348) is obtained from the German Collection of Microorganisms and Cell Cultures (Braunschweig, Germany) as an actively growing culture. Serial dilutions of *M*. *sedula* culture are made into *Metallosphaera* medium (#485) as described per DSMZ instructions. *M. sedula* is grown aerobically at 65°C at 250 rpm until saturated.
*Propionibacterium freudenreichii* subsp. *shermanii* (DSMZ# 4902) is obtained from the German Collection of Microorganisms and Cell Cultures (Braunschweig, Germany) as a vacuum dried culture. Cultures are then resuspended in PYG-medium (#104) as described per DSMZ instructions. *P. freudenreichii* subsp. *shermanii* is grown anaerobically at 30°C at 250 rpm until saturated.
*Pseudomonas putida* is a gift from the Gill Lab (University of Colorado at Boulder, Boulder, CO USA) and is obtained as an actively growing culture. Serial dilutions of the actively growing *P. putida* culture are made into Luria Broth (RPI Corp., Mt. Prospect, IL USA) and are allowed to grow for aerobically for 24 hours at 37°C at 250 rpm until saturated.
*Streptococcus mutans* (DSMZ# 6178) is obtained from the German Collection of Microorganisms and Cell Cultures (Braunschweig, Germany) as a vacuum dried culture. Cultures are then resuspended in Luria Broth (RPI Corp., Mt. Prospect, IL USA). *S. mutans* is grown aerobically at 37°C at 250 rpm until saturated.

The following non-limiting strains may also be used as starting strains in the Examples: DF40 Hfr(P02A), garB10, fhuA22, ompF627(T2R), fadL701(T2R), relA1, pitA10, spoTl, rrnB-2, pgi-2, mcrB1, creC510, BW25113 F-, Δ(araD-araB)567, ΔlacZ4787(::rmB-3), &lambda·, rph-1, Δ(rhaD-rhaB)568, hsdR514, JP111 Hfr(POl), *galE45*(Ga1S), *&lambda*⁻ *,fab*/*392*(ts), *relAl*, *spoTl, thi-1.* These strains possess recognized genetic modifications, and are available from public culture sources such as the Yale Coli Genetic Stock Collection (New Haven, CT USA). Strains developed from these strains are described in the Examples.

### Common Method Example 2: Cultures and Growth Media

Bacterial growth culture media and associated materials and conditions, are as follows:
Fed-batch medium contained (per liter): 10 g tryptone, 5 g yeast extract, 1.5 g NaCl, 2 g Na2HPO4.7 H2O, 1 g KH2PO4, and glucose as indicated.

AM2 medium contained (per liter): 2.87 g K₂HPO₄, 1.50g KH₂PI₄, 3.13g (NH4)₂SO₄, 0.15g KCl, 1.5 mM MgSO₄, 0.1M K⁺ MOPS pH 7.2, 30 g glucose, and 1 ml trace Mineral Stock prepared as described in Martinez et al. Biotechnol Lett 29:397-404 (2007). Concentration of glucose in glucose feed for AM2 vessels: 200 g/L glucose.

AM2 Medium used in Fermenters for Initial Batch Medium

| | |
|---|---|
| K2HP04 | 2.87 g/L |
| KH2P04 | 1.50 g/L |
| (NH4)2S04 | 3.13 g/L |
| KCl | 0.15 g/L |
| Glucose | 6.0 g/L |
| MgS04 | 0.18 g/L |
| AM2 Trace Metals Stock Solution | 1.0 ml/L |
| Calcium | 0.005 g/L |
| Ampicillin | 0.1 g/L |
| Kanamycin | 0.02 g/L |
| Chloramphenicol | 0.02 g/L |

Trace Metals Stock Solution for AM2 medium used in Fermenters

| | |
|---|---|
| Concentrated HCl | 10.0 ml/L |
| FeCl₃•6H₂O | 2.4 g/L |
| COC₁₂•6 H₂O | 0.17 g/L |
| CuCb•2 H₂O | 0.15g/L |
| ZnCb | 0.3 g/L |
| Na₂MoO₄•2H₂O | 0.3 g/L |
| H₃BO₃ | 0.07 g/L |
| MnCb•4H₂O | 0.5 g/L |

Rich Medium used in Fermenters Initial Batch Medium

| | |
|---|---|
| Tryptone | 10 g/L |
| Yeast Extract | 5 g/L |
| Glucose | 4 g/L |
| Na₂HPO₄•7H₂O | 2 g/L |
| KH₂PO₄ | 1 g/L |
| MgSO₄ | 2 g/L |
| Ampicillin | 0.1 g/L |
| Kanamycin | 0.02 g/L |
| Chloramphenicol | 0.02 g/L |

Feed Formulation for additional glucose feed for rich media

| | |
|---|---|
| Glucose | 200 g/L |
| (NH₄)₂SO₄ | 30 g/L |
| KH₂PO₄ | 7.5 g/L |
| Citric Acid | 3 g/L |
| MgSO₄ | 2.93 g/L |
| FeSO₄•7H₂O | 0.05 g/L |

SM3 minimal medium for *E. coli* (Final phosphate concentration = 27.5 mM; Final N concentration = 47.4 mM NH₄⁺).

Components per liter: 700 mL DI water, 100 mL 10X SM3 Salts, 2 ml 1M MgSO₄, 1 mL 100X Trace Mineral Stock, 60 mL 500 g/L glucose, 100 mL 0.1 M MOPS (pH 7.4), 0.1 mL of 1 M CaCl₂, Q.S. with DI water to 1000 mL, and 0.2 µm filter sterilize.

### To Make SM8 Minimal Media

SM8 minimal medium for *E. coli* (Final phosphate concentration= 3.2 mM; Final N concentration= 45 mM NH₄)+. Components per liter: 600 mL DI water, 100 mL 10X FM8 Salts, 2.26 mL 1M MgSO₄, 2 mL FM10 Trace Mineral Stock, 10 mL 100 g/L yeast extract, 60 mL 500 g/L glucose, 200 mL 1M MOPS (pH 7.4), Q.S. with DI water to 1000 mL, and 0.2 µm filter sterilize.

### Preparation of Stock Solutions:

To make 10X SM3 Salts (1 L): 800 mL DI water, 28.7 g K₂HPO₄, 15 g KH2PO4, 31.3 g (NH4)₂SO₄, 1.5 g KCl, 0.5 g Citric Acid (anhydrous), and Q.S. with DI water to 1000 mL.

To make 100X Trace Mineral Stock (1L): save in 50-mL portions at room temp: Per liter in 0.12M HCl (dilute 10 mL cone HCl into 1 liter water):2.4 g FeCL₃ 6H₂O, 0.17 g CoCh₆H₂O, 0.15 g CuCl₂, 2H₂O, 0.3 g ZnCl₂, 0.3 g NaMoO₄.2H₂O (Molybdic acid, disodium salt, dihydrate), 0.07 g H₃BO₃, and 0.5 g MnCh₄H₂O.

To make 1M MOPS:209.3 g MOPS, dissolve in 700 mL water. Take 70-mL portions and adjust to desired pH with 50% KOH, adjust to 100 mL final volume, and 0.2 µm filter sterilize.

To make 1M MgSO₄:120.37 g dissolved in 1000 mL water.

To make 500 g/L (50%) glucose stock solution: 900 mL DI water, 500 g glucose, and Q.S. to 1000 mL.

To make 10X FM8 Salts (1 L): 800 mL DI water, 3.29 g K₂HPO₄, 1.73 g KH₂PO₄, 30 g (NH4)₂SO₄, 1.5 g Citric Acid (anhydrous), and Q.S. with DI water to 1000 mL.

To make FM10 Trace Mineral Stock (100 mL): 50 mL DI water, 1 mL 13M HCl, 4.9 g CaCl₂*2H20, 0.97 g FeCL₃•6H₂O, 0.04 g COCl₂•6H₂O, 0.27 g CuCl₂•H₂O, 0.02 g ZnCl₂, 0.024 g NaMoO₄•2H₂O, 0.007 g H₃BO₃, and 0.036 g MnCl₂.4H₂O, Q.S. with DI water to 100 mL.

To make 1M MOPS: 209.3 g MOPS, dissolve in 700 mL water, take 70-mL portions and adjust to desired pH with 50% KOH, adjust to 100 mL final volume, and 0.2 µm filter sterilize.

To make 1M MgSO₄: 120.37 g dissolved in 1000 mL water.

To make 500 g/L (50%) glucose stock solution: 900 mL DI water, 500 g glucose, and Q.S. to 1000 mL.

To make 100 g/L yeast extract: 900 mL water: 100 g yeast extract, and Q.S. to 1000 mL.

Additional Growth Media Formulation(s) is/are summarized as:

| | Ingredient | Concentration in FM11 |
|---|---|---|
| 1 | K₂HPO₄ | 0.329 g/L |
| 2 | K₂HPO₄ | 0.173 g/L |
| 3 | (NH₄)₂SO₄ | 3 g/L |
| 4 | NaCl | -------- |
| 5 | Citric Acid•H₂O | 0.15 g/L |
| 6 | Yeast Extract | 1 g/L |
| 7 | Antifoam 204 | 0.1 mL/L |
| 8 | Glucose | 30 g/L |
| 9 | MgSO₄ •7H₂O | 0.82 g/L |
| 10 | FM10 Trace Metals Stock | 2 mL/L |
| | Solution | |
| 11 | Kanamycin | 35 mg/L |
| 12 | Chloramphenicol | 20 mg/L |
| 13 | 1000x Vitamin Mixture | 1.25 mL/L |

FM10: Trace Metals Stock Solution formulation:

| Ingredient | Concentration |
|---|---|
| Concentrated HCl | 10.0 ml/L |
| CaCl₂.2H₂O | 49 g/L |
| FeCl₃•6H₂O | 9.7 g/L |
| COCl₂•6H₂O | 0.4 g/L |
| CuCl₂•2H₂O | 2.7g/L |
| ZnCl₂ | 0.2 g/L |
| Na₂MoO₄•2H₂O | 0.24 g/L |
| H₃BO₃ | 0.07 g/L |
| MnCl₂•4H₂O | 0.36 g/L |

1000x Vitamin Mixture:

| Component | Amount (g/L) |
|---|---|
| Thiamine Hydrochloride | 5.0 |
| D-Pantothenic Acid | 5.4 |
| Nicotinic Acid | 6.0 |
| Biotin | 0.06 |
| q.s. with DI water to final volume | |

### To make 1 L M9 minimal media:

*M9* minimal media was made by combining 5X M9 salts, 1M MgS0₄, 20% glucose, 1M CaCl₂ and sterile deionized water. The 5X M9 salts are made by dissolving the following salts in deionized water to a final volume of 1L: 64g Na₂HPO₄ • 7H₂O, 15g KH₂PO₄,2.5g NaCl, 5.0g NH₄Cl. The salt solution was divided into 200 mL aliquots and sterilized by autoclaving for 15 minutes at 15 psi on the liquid cycle. A 1M solution of MgSO₄ and 1M CaCl₂ were made separately, then sterilized by autoclaving. The glucose was filter sterilized by passing it thought a 0.22µm filter. All of the components are combined as follows to make 1L of M9: 750mL sterile water, 200 mL 5X M9 salts, 2mL of 1M MgSO₄, 20 mL 20% glucose, 0.1 mL CaCl₂, Q.S. to a final volume of 1L.

### To make EZ rich media:

All media components were obtained from TEKnova (Hollister, CA USA) and combined in the following volumes. 100 mL 10X MOPS mixture, 10 mL 0.132M K₂ HPO₄, 100 mL 10X ACGU, 200 mL 5X Supplement EX, 10 mL 20% glucose, 580 mL sterile water.

### To make FGN30 Medium:

FGN30 medium is made of the following components in the concentrations listed:

| Chemical | FGN Medium Final concentration | |
|---|---|---|
| K2HPO4 | 3.746 | g/L |
| K2HPO4 | 1.156 | g/L |
| NH4Cl | 0.962 | g/L |
| NaCl | 0.702 | g/L |
| Citric Acid | 66 | mg/L |
| FeSO4.7H2O | 16.68 | mg/L |
| ZnCl2 | 0.1 | mg/L |
| MnCl2.4H2O | 0.03 | mg/L |
| CoCl2.6H2O | 0.05 | mg/L |
| CuCl2.2H2O | 0.07 | mg/L |
| NiCl2.6H2O | 0.12 | mg/L |
| Na2MoO4.2H2O | 0.03 | mg/L |
| CrCl3.6H2O | 0.05 | mg/L |
| H3BO3 | 0.3 | mg/L |
| CaCl2 | 11 | mg/L |
| MgSO4 | 240 | mg/L |
| Fructose | 2 | g/L |
| Glycerol | 2 | g/L |

FGN30 medium can be made according to the following protocol:
Measure each stock solution in the order as listed below. Mix well after adding each stock solution before adding the next stock solution. Filter and sterilize using a 0.2 µm bottle top vacuum filter.

| Chemical Name | Quantity | |
|---|---|---|
| Water (DI) | 800 | mL |
| 10x Mineral Salts Solution | 100 | mL |
| 1000x Micronutrient stock solution | 1 | mL |
| 1 M MgSO₄ stock solution | 2 | mL |
| Fructose | 15 | g |
| Glycerol | 15 | g |
| 1 M CaCl₂ stock solution | 100 | µL |
| Q.S. using DI water to: | 1000 | mL |

### Preparation of FGN30 Stock solutions:

10x Mineral Salts Solution: Measure each chemical in the order as listed below. Allow each chemical to dissolve completely before adding the next chemical.

| Chemical Name | Quantity | |
|---|---|---|
| Water (DI) | 800 | mL |
| K2HPO4 | 37.46 | g |
| K2HPO4 | 11.56 | g |
| NH4Cl | 9.62 | g |
| NaCl | 7.02 | g |
| Citric Acid, anhydrous | 0.5 | g |
| Q.S. using DI water to: | 1000 | mL |

1000x Micronutrient Stock Solution: Measure each chemical in the order as listed below. Allow each chemical to dissolve completely before adding the next chemical.

| Chemical Name | Quantity | |
|---|---|---|
| Water (DI) | 800 | mL |
| Citric Acid anhydrous | 16 | g |
| FeSO4.7H2O | 16.68 | g |
| ZnCl2 | 0.1 | g |
| MnCl2.4H2O | 0.03 | g |
| CoCl2.6H2O | 0.05 | g |
| CuCl2.2H2O | 0.07 | g |
| NiCl2.6H2O | 0.12 | g |
| Na2MoO4.2H2O | 0.03 | g |
| CrCl3.6H2O | 0.05 | g |
| H3BO3 | 0.3 | g |
| Q.S. using DI water to: | 1000 | mL |

### To make FGN30HN Medium:

FGN30HN medium can be made according to the following protocol: Measure each stock solution in the order as listed below. Mix well after adding each stock solution before adding the next stock solution. Filter sterilize using a 0.2 µm bottle top vacuum filter.

| Chemical Name | Quantity | |
|---|---|---|
| Water (DI) | 800 | mL |
| 10x Mineral Salts Solution | 100 | mL |
| 1000x Micronutrient stock solution | 1 | mL |
| 1 M MgSO₄ stock solution | 2 | mL |
| Fructose | 15 | g |
| Glycerol | 15 | g |
| 1 M CaCl₂ stock solution | 100 | µL |
| Q.S. using DI water to: | 1000 | mL |

### Preparation of FGN30HN Stock solutions:

10x High nitrogen Mineral Salts Solution: Measure each chemical in the order as listed below. Allow each chemical to dissolve completely before adding the next chemical.

| Chemical Name | Quantity | |
|---|---|---|
| Water (DI) | 800 | mL |
| K2HPO4 | 7.492 | g |
| KH2PO4 | 2.312 | g |
| NH4Cl | 28.86 | g |
| NaCl | 7.02 | g |
| Citric Acid, anhydrous | 0.5 | g |
| Q.S. using DI water to: | 1000 | mL |

1000x Micronutrient Stock Solution: Measure each chemical in the order as listed below. Allow each chemical to dissolve completely before adding the next chemical.

| Chemical Name | Quantity | |
|---|---|---|
| Water (DI) | 800 | mL |
| Citric Acid anhydrous | 16 | g |
| FeSO4.7H2O | 16.68 | g |
| ZnCl2 | 0.1 | g |
| MnCl2.4H2O | 0.03 | g |
| CoCl2.6H2O | 0.05 | g |
| CuCl2.2H2O | 0.07 | g |
| NiCl2.6H2O | 0.12 | g |
| Na2MoO4.2H2O | 0.03 | g |
| CrCl3.6H2O | 0.05 | g |
| H3BO3 | 0.3 | g |
| Q.S. using DI water to: | 1000 | mL |

To make MSM Medium: MSM medium is made of the following components in the concentrations listed:

| Chemical | MSM Medium Final concentration | |
|---|---|---|
| K2HPO4 | 3.746 | g/L |
| K2HPO4 | 1.156 | g/L |
| NH4Cl | 0.962 | g/L |
| NaCl | 0.702 | g/L |
| Citric Acid | 66 | mg/L |
| FeSO4.7H2O | 16.68 | mg/L |
| ZnCl2 | 0.1 | mg/L |
| MnCl2.4H2O | 0.03 | mg/L |
| CoCl2.6H2O | 0.05 | mg/L |
| CuCl2.2H2O | 0.07 | mg/L |
| NiCl2.6H2O | 0.12 | mg/L |
| Na2MoO4.2H2O | 0.03 | mg/L |
| CrCl3.6H2O | 0.05 | mg/L |
| H3BO3 | 0.3 | mg/L |
| CaCl2 | 11 | mg/L |
| MgSO4 | 240 | mg/L |
| Fructose | NA | |
| Glycerol | NA | |

MSM Medium (for chemolithotropic growth of *C. necator*): Measure each stock solution in the order as listed below. Mix well after adding each stock solution before adding the next stock solution. Filter and sterilize using a 0.2 µm bottle top vacuum filter.

| Chemical Name | Quantity | |
|---|---|---|
| Water (DI) | 650 | mL |
| 1M MOPS pH 7.4 | | 150mL |
| 10x Mineral Salts Solution with 3x NH₄Cl and 0.2x PO₄ (HN mineral salts) | 100 | mL |
| 1000x Micronutrient stock solution | 1 | mL |
| 1 M MgSO₄ stock solution | 2 | mL |
| 1 M CaCl₂ stock solution | 100 | µL |
| Q.S. using DI water to: | 1000 | mL |

### Common Method Example 3: Gel Preparation, DNA Separation, Extraction, Ligation, and Transformation

Molecular biology grade agarose (RPI Corp., Mt. Prospect, IL USA) is added to 1x TAE to make a 1% Agarose in TAE. To obtain 50x TAE add the following to 900 mL distilled H₂O : 242g Tris base (RPI Corp., Mt. Prospect, IL USA), 57.1 mL Glacial Acetic Acid (Sigma-Aldrich, St. Louis, MO USA), 18.6 g EDTA (Fisher Scientific, Pittsburgh, PA USA), and adjust volume to 1L with additional distilled water. To obtain 1x TAE, add 20 mL of 50x TAE to 980 mL of distilled water. The agarose-TAE solution is then heated until boiling occurred and the agarose is fully dissolved. The solution is allowed to cool to 50°C before 10 mg/mL ethidium bromide (Acros Organics, Morris Plains, NJ USA) is added at a concentration of 5 µL per 100 mL of 1% agarose solution. Once the ethidium bromide is added, the solution is briefly mixed and poured into a gel casting tray with the appropriate number of combs (Idea Scientific Co., Minneapolis, MN USA) per sample analysis. DNA samples are then mixed accordingly with 5X TAE loading buffer. 5X TAE loading buffer consists of 5X TAE(diluted from 50X TAE as described herein), 20% glycerol (Acros Organics, Morris Plains, NJ USA), 0.125% Bromophenol Blue (Alfa Aesar, Ward Hill, MA USA), and adjust volume to 50 mL with distilled water. Loaded gels are then run in gel rigs (Idea Scientific Co., Minneapolis, MN USA) filled with 1X TAE at a constant voltage of 125 volts for 25-30 minutes. At this point, the gels are removed from the gel boxes with voltage and visualized under a UV transilluminator (FOTODYNE Inc., Hartland, WI USA).

The DNA isolated through gel extraction is then extracted using the QIAquick Gel Extraction Kit following manufacturer's instructions (Qiagen, Valencia, CA USA). Similar methods are known to those skilled in the art.

The thus-extracted DNA then may be ligated into pSMART (Lucigen Corp., Middleton, WI USA), StrataClone (Stratagene, La Jolla, CA USA) or pCR2.1-TOPO TA (Invitrogen Corp., Carlsbad, CA USA) according to manufacturer's instructions.

### Ligation Methods

### For ligations into pSMART vectors:

Gel extracted DNA is blunted using PCRTerminator (Lucigen Corp., Middleton, WI USA) according to manufacturer's instructions. Then 500 ng of DNA is added to 2.5 µL 4x CloneSmart vector premix, 1 µL CloneSmart DNA ligase (Lucigen Corp., Middleton, WI USA) and distilled water is added for a total volume of 10 µL. The reaction is then allowed to sit at room temperature for 30 minutes and then heat inactivated at 70°C for 15 minutes and then placed on ice. *E. coli* 10G Chemically Competent cells (Lucigen Corp., Middleton, WI USA) are thawed for 20 minutes on ice. 40 µL of chemically competent cells are placed into a microcentrifuge tube and 1 µL of heat inactivated CloneSmart Ligation is added to the tube. The whole reaction is stirred briefly with a pipette tip. The ligation and cells are incubated on ice for 30 minutes and then the cells are heat shocked for 45 seconds at 42°C and then put back onto ice for 2 minutes. 960 µL of room temperature Recovery media (Lucigen Corp., Middleton, WI USA) and places into microcentrifuge tubes. Shake tubes at 250 rpm for 1 hour at 37°C. Plate 100 µL of transformed cells on Luria Broth plates (RPI Corp., Mt. Prospect, IL USA) plus appropriate antibiotics depending on the pSMART vector used. Incubate plates overnight at 37°C.

### For Litigations into StrataClone:

Gel extracted DNA is blunted using PCRTerminator (Lucigen Corp., Middleton, WI USA) according to manufacturer's instructions. Then 2 µL of DNA is added to 3 µL StrataClone Blunt Cloning buffer and 1 µL StrataClone Blunt vector mix amplkan (Stratagene, La Jolla, CA USA) for a total of 6 µL. Mix the reaction by gently pipeting up and down and incubate the reaction at room temperature for 30 minutes then place onto ice. Thaw a tube of StrataClone chemically competent cells (Stratagene, La Jolla, CA USA) on ice for 20 minutes. Add 1 µL of the cloning reaction to the tube of chemically competent cells and gently mix with a pipette tip and incubate on ice for 20 minutes. Heat shock the transformation at 42°C for 45 seconds then put on ice for 2 minutes. Add 250 µL pre-warmed Luria Broth (RPI Corp., Mt. Prospect, IL USA) and shake at 250 rpm for 37°C for 2 hours. Plate 100 µL of the transformation mixture onto Luria Broth plates (RPI Corp., Mt. Prospect, IL USA) plus appropriate antibiotics. Incubate plates overnight at 37°C.

### For Ligations into pCR2.1-TOPO TA:

Add 1 µL TOPO vector, 1 µL Salt Solution (Invitrogen Corp., Carlsbad, CA USA) and 3 µL gel extracted DNA into a microcentrifuge tube. Allow the tube to incubate at room temperature for 30 minutes then place the reaction on ice. Thaw one tube of TOP10F chemically competent cells (Invitrogen Corp., Carlsbad, CA USA) per reaction. Add 1 µL of reaction mixture into the thawed TOP10F cells and mix gently by swirling the cells with a pipette tip and incubate on ice for 20 minutes. Heat shock the transformation at 42°C for 45 seconds then put on ice for 2 minutes. Add 250 µL pre-warmed SOC media (Invitrogen Corp., Carlsbad, CA USA) and shake at 250 rpm for 37°C for 1 hour. Plate 100 µL of the transformation mixture onto Luria Broth plates (RPI Corp., Mt. Prospect, IL USA) plus appropriate antibiotics. Incubate plates overnight at 37°C.

### General Transformation and Related Culture Methodologies:

Chemically competent transformation protocols are carried out according to the manufacturer's instructions or according to the literature contained in Molecular Cloning (Sambrook and Russell, 2001). Generally, plasmid DNA or ligation products are chilled on ice for 5 to 30 minutes in solution with chemically competent cells. Chemically competent cells are a widely used product in the field of biotechnology and are available from multiple vendors, such as those indicated in this Subsection. Following the chilling period cells generally are heat-shocked for 30 seconds at 42°C without shaking, re-chilled and combined with 250 microliters of rich media, such as SOC. Cells are then incubated at 37°C while shaking at 250 rpm for 1 hour. Finally, the cells are screened for successful transformations by plating on media containing the appropriate antibiotics.

Alternatively, selected cells may be transformed by electroporation methods such as are known to those skilled in the art.

The choice of an *E. coli* host strain for plasmid transformation is determined by considering factors such as plasmid stability, plasmid compatibility, plasmid screening methods and protein expression. Strain backgrounds can be changed by simply purifying plasmid DNA as described herein and transforming the plasmid into a desired or otherwise appropriate *E. coli* host strain such as determined by experimental necessities, such as any commonly used cloning strain (e.g., DH5α, Top10F', *E. coli* 10G, etc.).

Plasmid DNA was prepared using the commercial miniprep kit from Qiagen (Valencia, CA USA) according to manufacturer's instructions.

### Common Method Example 4: 3-HP Preparation and Analysis

A 3-HP stock solution was prepared as follows. A vial of β-propiolactone (Sigma-Aldrich, St. Louis, MO USA) was opened under a fume hood and the entire bottle contents was transferred to a new container sequentially using a 25-mL glass pipette. The vial was rinsed with 50 mL of HPLC grade water and this rinse was poured into the new container. Two additional rinses were performed and added to the new container. Additional HPLC grade water was added to the new container to reach a ratio of 50 mL water per 5 mL β-propiolactone. The new container was capped tightly and allowed to remain in the fume hood at room temperature for 72 hours. After 72 hours the contents were transferred to centrifuge tubes and centrifuged for 10 minutes at 4,000 rpm. Then the solution was filtered to remove particulates and, as needed, concentrated by use of a rotary evaporator at room temperature. Assay for concentration was conducted, and dilution to make a standard concentration stock solution was made as needed.

### Analytical Methods for 3-HP Detection

Analysis of Cultures for 3-HP Production: For HPLC analysis of 3-HP, the Waters Chromatography System (Milford, MA USA) consisted of the following: 600S Controller, 616 Pump, 717 Plus Autosampler, 486 Tunable UV Detector, and an in-line mobile phase Degasser. In addition, an Eppendorf (Hamburg, Germany) external column heater is used and the data are collected using an SRI (Torrance, CA USA) analog-to-digital converter linked to a standard desk top computer. Data are analyzed using the SRI Peak Simple software. A Coregel 64H ion exclusion column (Transgenomic, Inc., San Jose, CA USA) is employed. The column resin is a sulfonated polystyrene divinyl benzene with a particle size of 10µm and column dimensions are 300 x 7.8 mm. The mobile phase consisted of sulfuric acid (Fisher Scientific, Pittsburgh, PA USA) diluted with deionized (18 MΩcm) water to a concentration of 0.02 N and vacuum filtered through a 0.2 µm nylon filter. The flow rate of the mobile phase is 0.6 mL/min. The UV detector is operated at a wavelength of 210 nm and the column is heated to 60°C. The same equipment and method as described herein is used for 3-HP analyses for relevant examples.

The following method is used for GC-MS analysis of 3-HP. Soluble monomeric 3-HP is quantified using GC-MS after a single extraction of the fermentation media with ethyl acetate. Once the 3-HP has been extracted into the ethyl acetate, the active hydrogens on the 3-HP are replaced with trimethylsilyl groups using N,O-Bis-(*Trimethylsilyl)* trifluoroacetamide to make the compound volatile for GC analysis. A standard curve of known 3-HP concentrations is prepared at the beginning of the run and a known quantity of ketohexanoic acid (1 g/L) is added to both the standards and the samples to act as an internal standard for Quantitation, with tropic acid as an additional internal standard. The 3-HP content of individual samples is then assayed by examining the ratio of the ketohexanoic acid ion (m/z = 247) to the 3-HP ion (219) and compared to the standard curve. 3-HP is quantified using a 3HP standard curve at the beginning of the run and the data are analyzed using HP Chemstation. The GC-MS system consists of a Hewlett Packard model 5890 GC and Hewlett Packard model5972 MS. The column is Supelco SPB-1 (60m X 0.32mm X 0.25 flm film thickness). The capillary coating is a non-polar methylsilicone. The carrier gas is helium at a flow rate of 1 mL/min. The 3-HP as derivatized is separated from other components in the ethyl acetate extract using either of two similar temperature regimes. In a first temperature gradient regime, the column temperature starts with 40°C for 1 minute, then is raised at a rate of 10°C/minute to 235°C, and then is raised at a rate of 50°C/minute to 300°C. In a second temperature regime, which was demonstrated to process samples more quickly, the column temperature starts with 70°C which is held for 1 minute, followed by a ramp-up of 10°C/minute to 235°C which is followed by a ramp-up of 50°C/minute to 300°C.

A bioassay for detection of 3-HP also was used in various examples. This determination of 3-HP concentration was carried out based on the activity of the *E. coli* 3-HP dehydrogenase encoded by the *ydfG* gene (the YDFG protein). Reactions of 200-µl were carried out in 96-well microtiter plates, and contained 100 mM Tris-HCl, pH 8.8, 2.5 mM MgCl₂, 2.625 mM NADP⁺, 3 µg g purified YDFG and 20 µL culture supernatant. Culture supernatants were prepared by centrifugation in a microfuge (14,000 rpm, 5 min) to remove cells. A standard curve of 3-HP (containing from 0.025 to 2 g/L) was used in parallel reactions to quantitate the amount of 3-HP in culture supernatants. Uninoculated medium was used as the reagent blank. Where necessary, the culture supernatant was diluted in medium to obtain a solution with 3-HP concentrations within that of the standard curve.

The reactions were incubated at 37°C for 1 hr, and 20 µL of color developer containing 1.43 mM nitroblue tetrazolium, 0.143 phenazine methosulfate, and 2.4% bovine serum albumin were added to each reaction. Color development was allowed to proceed at 37°C for an additional hour, and the absorbance at 580 nm was measured. 3-HP concentration in the culture supernatants was quantitated by comparison with the values obtained from the standard curve generated on the same microtiter plate. The results obtained with the enzymatic assay were verified to match those obtained by one of the analytical methods described above.

### Common Method Example 5: Enzyme assay methods for the quantification of enzyme activities

### Pyruvate dehydrogenase assay

Strains to be evaluated were started in 5ml TB overnights, and 1 ml were diluted into 100 ml SM8 medium and grown at 30°C for ∼10 hr. The SM8 cultures was harvested in 2 x 50-ml aliquots by centrifugation and the cell pellet washed into Eppendorf tubes with 1 ml Butterfield's diluent. After recentrifugation, the diluent was removed and the cell pellets stored at -80°C until lysis. Cell pellets were resuspended with 1 ml 50 mM Tris-HCl, pH 8.0, 25 mM NaCl, 2 mM EDTA, 1 mM DTT, 250 U/ml Benzonase and transferred to a 2-ml screw cap Eppendorf tube half-filled with glass beads. A lysate was prepared by disruption of the cells in the BeadBeater for 90s, and clarified by centrifugation (13.2 krpm, 5 min, 4°C). PDH assays were carried out according to the SOP using the supernatant, and protein concentrations in the lysates determined using the Pierce660 reagent. Lysate protein was varied between 0.01 and 0.04 mg total protein per 200 µL reaction, and the specific activity calculated from the slope of the linear curve fitted to the data. The effect of NADH was measured by adding varying amounts of NADH to an assay containing 1 mM NAD+, and monitoring the increase in A340.

### St-Mcr

Cell line carrying plasmids able to over express malonyl CoA reductases were grown with antibiotic selection in LB media overnight as starter cultures. These overnight starter cultures were used to inoculate either 50 mL to 100 mL expression cultures grown with antibiotic selection in LB media supplemented with 1mM Isopropyl β-D-1-thiogalactopyranoside (IPTG) to induce protein production. Cultures were grown 24 hr, after which the cells were collected by centrifugation. Cell pellets were lysed using a mixture of Bugbuster, benzonase nuclease, and rLysozyme (all from Novagen). Once lysed, the lysate mixture was centrifuged at 14000 RPM in a standard table top centrifuge. The resulting supernatant was removed to another tube. The clarified supernatant was measure for protein concentration using a Biorad Total Protein determination kit (BioRad). For each measurement, 20 uL of lysate was added to a reaction buffer filled well of the 96-well plate used to perform the assay. All samples were performed in duplicates. The assay was initiated by addition of malonyl CoA to a final concentration of 0.3 mM or ImM, which is well above the reported Km binding constant for these enzymes. Once the reaction time course was read and the slopes of each well were calculated, the specific activities were compared to a negative control to determine a background rate. All values reported are the average specific activities measured in triplicate.

### MmsB

The coupled assay uses lysates overexpressing various dehydrogenases (YdfG, MmsB, and the dehydrogenase domain of *Chloroflexus aurantiacus* MCR) able to convert malonate semialdehyde formed by the *Sulfolobus tokodaii* MCR to 3-hydroxypropionate. The formation of 3-hydroxypropionate was assessed using gas chromatography - mass spectrometry (GC-MS). The reactions for these assays were performed as 750 uL reactions containing 20 uL of clarified whole cell lysates from *Sulfolobus tokodaii* MCR over expressing cultures and 20 uL of clarified whole cell lysates from cells expressing one of three dehydrogenases (ydfG, mmsB, the *Chloroflexus aurantiacus)*. The buffer conditions consisted of 1 mM malonyl CoA, 2 mM NADH or 2mM NADH, 5 mM dithiothreitol, 3 mM magnesium chloride, 100 mM Trizma-HCl pH7.6 buffer. Lysates for these assays were prepared as follows. Cell line carrying plasmids able to over express ydfG, mmsB, the *Chloroflexus aurantiacus* dehydrogenase domain, or the *Sulfolobus tokodaii* malonyl CoA reductases were grown with antibiotic selection in LB media overnight as starter cultures. These overnight starter cultures were used to inoculate either 50 mL to 100 mL expression cultures grown with antibiotic selection in LB media supplemented with 1mM Isopropyl β-D-1-thiogalactopyranoside (IPTG) to induce protein production. Cultures were grown 24 hr, after which the cells were collected by centrifugation. Cell pellets were lysed using a mixture of Bugbuster, benzonase nuclease, and rLysozyme (all from Novagen). Once lysed, the lysate mixtures were centrifuged at 14000 RPM in a standard table top centrifuge. The resulting supernatants were removed to another tube. Various combination dehydrogenases with and without the *Sulfolobus tokodaii* malonyl CoA reductases were evaluated. Reactions were incubated at 37 degrees Celsius for 12 to 15 hours. With each assay set, negative control samples for of the proteins overexpressed were included to make sure no lysate had the ability to form 3-hydroxypropionate with a combination of a CoA reductase domain and a dehydrogenase domain. After incubation, all samples were submitted for GS-MS analysis as described elsewhere herein.

### Acetyl-CoA carboxylase assay

Acetyl-CoA carboxylase assay (AccADBC) activities were determined by a coupled enzymatic assay with mcr. Cells were resuspended in ∼1 mL of Lysis/Assay Buffer (50 mM Tris, pH 8.0, 25 mM NaCl, 2 mM EDTA, 2% PEG and 1 mM DTT) containing 250 U/mL Benzonase (EMD Chemicals). Cells were lysed using Bead Beater at ∼75% intensity for ∼1.5 minutes and spin to remove cellular debris. The assay reaction mixture was made as follows: 25 mM Trizma, pH 8.0, 2.5 mM MgCl₂, 1 mM DTT, 15 mM Ammonium Sulfate, 7.5 mM NaHCO₃, 1 mM NADPH, 1 mM ATP, 0.005 mM Biotin, 2% PEG and titrated the cell lysate to a final volume of 200 ul. Reactions were initiated with 1 mM Acetyl CoA and kinetic reads were performed at Abs 340nm. (Note: for strains which are not overexpressing mcr, the addition of purified mcr is required for the assay to work).

### Glutamate dehydrogenase assay

The reaction was performed in a 96 well plate format with samples for each lysate performed in duplicate. Each reaction was carried out in a 200 uL volume, and the buffer conditions for the assay were 50 mM Trizma buffer pH7.5, 15mM ammonium chloride, 1 mM NADPH. For lysates, samples of each culture were pelleted and then lysed using a mixture of Bugbuster, benzonase nuclease, and rLysozyme (all from Novagen). To each tech well, 10 microliters of lysate was added. A baseline activity for each well was measured for 10 minutes using a Molecular Dynamics SpectraMax 384 microplate reader with SoftmaxPro software (Molecular Dynamics, Sunnyvale CA) to quantitate the rate of change in the 340 nm absorbance. All assays were conducted at 30°C, and the progress of each reaction was monitored for 30 minutes during which measurements were made every 20 seconds. To initiate the glutamate dehydrogenase specific activity, 2-ketoglutarate was added to a 15 mM concentration and again the progress of each reaction was monitored for 30 minutes during which measurements were made every 20 seconds. From these two sets of reading, a rate was calculated by subtracting the baseline rate from the 2-ketoglutarate rate. The specific glutamate dehydrogenase activity was calculated by adjusting the observed rates by the amount of total lysate protein added to each well. The total lysate protein of each prepared lysate was determined using the Pierce 660nm Protein Assay (Rockville, Illinois).

### Common Method Example 6: Strain evaluation methods for evaluating 3-HP production

### Low Phosphate Shake Flask Method

3-HP production using production strains was demonstrated at 100-mL scale in SM11 (minimal salts) media without phosphate. Cultures were started from freezer stocks by standard practice (Sambrook and Russell, 2001) into 50 mL of SM11 (minimal salts) media containing 30 mM phosphate plus 35 µg/mL kanamycin and 20 µg/mL chloramphenicol and grown to stationary phase overnight at 30°C with rotation at 250 rpm. Three mL of this culture were transferred to 100 ml of SM11 No Phosphate media plus 30 g/L glucose, 35 µg/ml kanamycin, and 20 µg/mL chloramphenicol in triplicate 250-ml baffled flasks and incubated at 30°C, 250 rpm. To monitor cell growth by these cultures, samples (2 ml) were withdrawn at designated time points for optical density measurements at 600nm (OD₆₀₀, 1 cm pathlength). Cultures were shifted to production by transferring the cultures to 37°C at 6 hours post-inoculation. A sample was collected at this time for analysis of 3HP and enzyme activities. Samples were also collected at 10 and 22 hours post-inoculation for monitoring 3HP production and enzyme activity. To monitor 3HP production by these cultures, samples (10 mL) were withdrawn at the designated time points and pelleted by centrifugation at 12,000rpm for 10 min and the supernatant collected for analysis of 3-HP production as described elsewhere herein. The pellet was frozen at -80°C for analysis of enzyme activity as described under elsewhere herein. Dry cell weight (DCW) is calculated as 0.40 times the measured OD₆₀₀ value, based on baseline DCW to OD₆₀₀ determinations. All data are the average of triplicate cultures. For comparison purposes, the specific productivity is calculated from the averaged data at the 24-h time point and expressed as g 3-HP produced per gDCW.

### Syngas Fermentation Method

3HP production using syngas feed stocks is demonstrated at 0.6 L scale in SM11 (minimal salts) media. Cultures are started from freezer stocks by standard practice (Sambrook and Russell, 2001) into 49 mL of FGN30 medium supplemented with appropriate antibiotics and incubated at 30°C for 24 hours with rotation at 250 rpm. 5 mL of this culture is transferred to 45 ml of FGN30HN with appropriate antibiotics and is incubated at 30°C for 24 hours with rotation at 250 rpm. Columns are setup with appropriate gas flow rates (132ml/min for 600ml columns and 20 ml/min for 20 ml columns - 70% H₂, 20% O₂, 10% CO₂). Water baths are warmed to 30C and columns are filled with MSM-HN media with appropriate antibiotics. FGN30HN overnight cultures are diluted to OD600 = 10-20 and are inoculated into gas-fed columns (60 mL inoculumn into 540 mL of MSM-HN medium for 600 mL cultures and 2 mL into 18 mL MSM-HN for 20 mL cultures). Columns are operated for approximately 72 hours at 30C and samples are removed for 3-HP quantification.

### Common Method Example 7: Minimum Inhibitory Concentration Evaluation (MIC) Protocols

For MIC evaluations, the final results are expressed in chemical agent concentrations determined by analysis of the stock solution by HPLC.

### E. coli aerobic MIC.

The (MIC) was determined aerobically in a 96 well-plate format. Plates were setup such that each individual well, when brought to a final volume of 100 uL following inoculation, had the following component levels (corresponding to standard M9 media): 47.7 mM Na2HPO4, 22 mM KH2PO4, 8.6 mM NaCl, 18.7 mM NH4C1, 2 mM MgS04, 0.1 mM CaC12, and 0.4% glucose. Overnight cultures of strains were grown in triplicate in 5 mL LB (with antibiotic where appropriate). A 1% (v/v) inoculum was introduced into a 5 ml culture of M9 minimal media. After the cells reached mid-exponential phase, the culture was diluted to an OD600 of about 0.200 (i.e., 0.195 - 0.205. The cells were further diluted 1:50 and a 10 µL aliquot was used to inoculate each well of a 96 well plate (-104 cells per well) to total volume of 100 uL. The plate was arranged to measure the growth of variable strains or growth conditions in increasing 3-HP concentrations, 0 to 60 g/L, in 5 g/L increments. Plates were incubated for 24 hours at 37C. The minimum inhibitory 3-HP concentration and maximum 3-HP concentration corresponding to visible cell growth (OD-0.1) was recorded after 24 hours. For cases when MIC > 60 g/L, assessments were performed in plates with extended 3-HP concentrations (0-100 g/L, in 5 g/L increments). (OD-0.1) was recorded after 24 hours. For cases when MIC > 60 g/L, assessments were performed in plates with extended 3-HP concentrations (0-100 g/L, in 5 g/L increments) E. *coli* anaerobic. The minimum inhibitory concentration (MIC) was determined anaerobically in a 96 well-plate format. Plates were setup such that each individual well, when brought to a final volume of 100 uL following inoculation, had the following component levels (corresponding to standard M9 media): 47.7 mM Na2HPO4, 22 mM KH2PO4, 8.6 mM NaCl, 18.7 mM NH4C1, 2 mM MgS04, 0.1 mM CaC12, and 0.4% glucose. Overnight cultures of strains were grown in triplicate in 5 mL LB (with antibiotic where appropriate). A 1% (v/v) inoculum was introduced into a 5 ml culture of M9 minimal media. After the cells reached mid-exponential phase, the culture was diluted to an OD600 of about 0.200 (i.e., 0.195 - 0.205. The cells were further diluted 1:50 and a 10 µL aliquot was used to inoculate each well of a 96 well plate (-104 cells per well) to total volume of 100 uL. The plate was arranged to measure the growth of variable strains or growth conditions in increasing 3-HP concentrations, 0 to 60 g/L, in 5 g/L increments. Plates were sealed in biobag anaerobic chambers that contained gas generators for anaerobic conditions and incubated for 24 hours at 37C. The minimum inhibitory 3-HP concentration and maximum 3-HP concentration corresponding to visible cell growth (OD-0.1) was recorded after 24 hours. For cases when MIC > 60 g/L, assessments were performed in plates with extended 3-HP concentrations (0-100 g/L, in 5 g/L increments).

### B. subtilis aerobic MIC

The minimum inhibitory concentration (MIC) was determined aerobically in a 96 well-plate format. Plates were setup such that each individual well, when brought to a final volume of 100 uL following inoculation, had the following component levels (corresponding to standard M9 media + supplemental glutamate): 47.7 mM Na2HPO4, 22 mM KH2PO4, 8.6 mM NaCl, 18.7 mM NH4Cl, 2 mM MgS04, 0.1 mM CaC12, 10 mM glutamate and 0.4% glucose. Overnight cultures of strains were grown in triplicate in 5 mL LB (with antibiotic where appropriate). A 1% (v/v) inoculum was introduced into a 5 ml culture of M9 minimal media + glutamate. After the cells reached mid-exponential phase, the culture was diluted to an OD₆₀₀ of about 0.200 (i.e., 0.195 - 0.205. The cells were further diluted 1:50 and a 10 µL aliquot was used to inoculate each well of a 96 well plate (-104 cells per well) to total volume of 100 uL. The plate was arranged to measure the growth of variable strains or growth conditions in increasing 3-HP concentrations, 0 to 60 g/L, in 5 g/L increments. Plates were incubated for 24 hours at 37C. The minimum inhibitory 3-HP concentration and maximum 3-HP concentration corresponding to visible cell growth (0D-0.1) was recorded after 24 hours. For cases when MIC > 60 g/L, assessments were performed in plates with extended 3-HP concentrations (0-100 g/L, in 5 g/L increment).

### C. necator (R. eutropha) aerobic MIC

The minimum inhibitory concentration (MIC) was determined aerobically in a 96 well-plate format. Plates were setup such that each individual well, when brought to a final volume of 100 uL following inoculation, had the following component levels (corresponding to FGN media): 21.5 mM K2HPO4, 8.5 mM KH2PO4, 18 mM NH4C1, 12 mM NaCl, 7.3 uM ZnCl, 0.15 uM MnC12, 4.85 uM H3B03, 0.21 uM CoC12, 0.41 uM CuC12, 0.50 uM NiC12, 0.12 uM Na2MoO4, 0.19 uM CrC13, 0.06 mM CaC12, 0.5 mM MgS04, 0.06 mM FeS04, 0.2% glycerol, 0.2% fructose. Overnight cultures of strains were grown in triplicate in 5 mL LB (with antibiotic where appropriate). A 1% (v/v) inoculum was introduced into a 5 ml culture of FGN media. After the cells reached mid-exponential phase, the culture was diluted to an OD600 of about 0.200 (i.e., 0.195 - 0.205. The cells were further diluted 1:50 and a 10 µL aliquot was used to inoculate each well of a 96 well plate (-104 cells per well) to total volume of 100 uL. The plate was arranged to measure the growth of variable strains or growth conditions in increasing 3-HP concentrations, 0 to 60 g/L, in 5 g/L increments. Plates were incubated for 24 hours at 30C. The minimum inhibitory 3-HP concentration and maximum 3-HP concentration corresponding to visible cell growth (0D-0.1) was recorded after 24 hours. For cases when MIC > 60 g/L, assessments were performed in plates with extended 3-HP concentrations (0-100 g/L, in 5 g/L increments).

### Example 1: Construction of plasmids expressing malonyl-CoA reductase (mcr)

The nucleotide sequence for the malonyl-CoA reductase gene from Chloroflexus aurantiacus was codon-optimized for E. coli according to a service from DNA2.0 (Menlo Park, CA USA), a commercial DNA gene synthesis provider. This gene sequence (SEQ ID NO:61) incorporated an EcoRI restriction site before the start codon and was followed by a HindIII restriction site. In addition, a ribosomal binding site was placed in front of the start codon. This gene construct was synthesized by DNA2.0 and provided in a pJ206 vector backbone (SEQ ID NO:62). Plasmid DNA pJ206 containing the synthesized mcr gene was subjected to enzymatic restriction digestion with the enzymes EcoRI and HindIII obtained from New England BioLabs (Ipswich, MA USA) according to manufacturer's instructions. The digestion mixture was separated by agarose gel electrophoresis and the appropriate DNA fragment recovered as described in the Common Methods Section. An E. coli cloning strain bearing pKK223-aroH was obtained as a kind gift from the laboratory of Prof. Ryan T. Gill from the University of Colorado at Boulder. Cultures of this strain bearing the plasmid were grown and plasmid DNA prepared as described in the Common Methods Section. Plasmid DNA was digested with the restriction endonucleases EcoRI and HindIII obtained from New England Biolabs (Ipswich, MA USA) according to manufacturer's instructions. This digestion served to separate the aroH reading frame from the pKK223 backbone. The digestion mixture was separated by agarose gel electrophoresis, and he agarose gel slice containing the DNA piece corresponding to the backbone of the pKK223 plasmid was recovered as described in the Common Methods Section.

Purified DNA fragments corresponding to the mcr gene and pK223 vector backbone were ligated and the ligation product was transformed and electroporated according to manufacturer's instructions. The sequence of the resulting vector termed pKK223-mcr was confirmed by routine sequencing performed by a commercial provider (SEQ ID NO:212). pKK223-mcr confers resistance to ampicillin and contains the mcr gene of C. aurantiacus under control of a Piac promoter inducible in E. coli hosts by IPTG.

To express the mcr gene under the regulation of other promoters besides the Piac on pKK223, the synthetic mcr gene was transferred to other plasmids. Plasmid pTrc-Pfrc-mcr was based on pTrcHisA (Invitrogen, Carlsbad, CA; Catalog Number V360-20) and the expression of mcr is directed by the Pfrc IPTG-inducible promoter. The inducer-independent Ptau promoter is based on sequences upstream of the E. coli talA gene. The nucleotide sequence of this promoter, placed immediately upstream of the initiator ATG codon of the synthetic mcr gene, is listed as SEQ ID NO:63.

The P,AA:mcr construct was incorporated by PCR into a pSC-B vector (Stratagene Corporation ,La Jolla, CA, USA), which was propagated in an E. coli stock, the plasmid DNA purified according to methods described elsewhere herein. The P,AA:mcr region in pSC-B-P,AA:mcr was transferred to a plasmid vector, pSMART-HCamp (Lucigen Corporation, Middleton, WI, catalog number 40041-2, GenBank AF399742) by PCR using vector primers, M13F and M13R. The fragment generated by PCR was cloned into pSMART-HCamp according to the manufacturer's protocol resulting in plasmid pSMART(HC)Amp-PwA-mcr (SEQ ID NO:64) in which mcr expression does not require induction with IPTG.

### Example 2: Construction of a plasmid expressing transhydrogenase (pntAB)

A fusion of the inducer-independent E. coli promoter derived from the tpiA gene (P,p,A) and the pyridine nucleotide transhydrogenase genes, pntAB, (SEQ ID NO:38 and SEQ ID NO:40) was created by amplifying the tpiA promoter region and pntAB region from genomic E. coli K12 DNA by polymerase chain reactions. For the pntAB genes, the region was amplified using the pntAB forward primer GGGAACCATGGCAATTGGCATACCAAG (SEQ ID NO:65), noting that all primers disclosed herein are artificial sequences) containing a Ncol site that incorporates the initiator Met for the protein sequence of pntA and the pntAB reverse primer GGGTTACAGAGCTTTCAGGATTGCATCC (SEQ ID NO:66). Likewise, the PtpiA region was amplified using the forward primer GGGAACGGCGGGGAAAAACAAACGTT (SEQ ID NO:67) and the reverse primer GGTCCATGGTAATTCTCCACGCTTATAAGC (SEQ ID NO:68) containing a Ncol restriction site. Polymerase chain reaction products were purified using a PCR purification kit from Qiagen Corporation (Valencia, CA, USA) using the manufacturer's instructions. Following purification, the products were subjected to enzymatic restriction digestion with the enzyme Ncol. Restriction enzymes were obtained from New England BioLabs (Ipswich, MA USA), and used according to manufacturer's instructions. The digestion mixtures were separated by agarose gel electrophoresis, and visualized under UV transillumination as described in the Common Methods Section. Agarose gel slices containing the DNA fragment corresponding to the amplified pntAB gene product and the P""A product were excised from the gel and the DNA recovered with a gel extraction kit from Qiagen used according to manufacturer's instructions. The recovered products were ligated together with T4 DNA ligase (New England BioLabs, Ipswich, MA USA) according to manufacturer's instructions.

Because the ligation reaction can result in several different products, the desired product corresponding to the P,,,,A fragment ligated to the pntAB genes was amplified by polymerase chain reaction and isolated by a second gel purification. For this polymerase chain reaction, the forward primer was GGGAACGGCGGGGAAAAACAAACGTT (SEQ ID NO:67), and the reverse primer was GGGTTACAGAGCTTTCAGGATTGCATCC (SEQ ID NO:66), and the ligation mixture was used as template. The digestion mixtures were separated by agarose gel electrophoresis, and visualized under UV transillumination as described the Common Methods Section. Agarose gel slices containing the DNA piece corresponding to the amplified PJ,iA - pntAB fusion was cut from the gel and the DNA recovered with a standard gel extraction protocol and components from Qiagen according to manufacturer's instructions. This extracted DNA was inserted into a pSC-B vector using the Blunt PCR Cloning kit obtained from Stratagene Corporation (La Jolla, CA, USA) using the manufacturer's instructions. Colonies were screened by colony polymerase chain reactions. Plasmid DNA from colonies showing inserts of correct size were cultured and miniprepped using a standard miniprep protocol and components from Qiagen according to the manufacturer's instruction. Isolated plasmids were checked by restriction digests and confirmed by sequencing. The sequenced-verified isolated plasmids produced with this procedure were designated pSC-B-P,p,A:pntAB.

The Pti,,A:pntAB region in pSC-B-P,i,,A:pntAB was transferred to a pBT-3 vector (SEQ ID NO:69) which provides a broad host range origin of replication and a chloramphenicol selection marker. To achieve this construct, a fragment from pBT-3 vector was produced by polymerase chain amplification using the forward primer AACGAATTCAAGCTTGATATC (SEQ ID NO:70), and the reverse primer GAATTCGTTGACGAATTCTCT (SEQ ID NO:71), using pBT-3 as template. The amplified product was subjected to treatment with DpnI to restrict the methylated template DNA, and the mixture was separated by agarose gel electrophoresis, and visualized under UV transillumination as described in the Common Methods Section. The agarose gel slice containing the DNA fragment corresponding to amplified pBT-3 vector product was cut from the gel and the DNA recovered with a standard gel extraction protocol and components from Qiagen according to manufacturer's instructions. The P,p,A:pntAB insert in pSC-B-P,p,A:pntAB was amplified using a polymerase chain reaction with the forward primer GGAAACAGCTATGACCATGATTAC (SEQ ID NO:72) and the reverse primer TTGTAAAACGACGGCCAGTGAGCGCG (SEQ ID NO:73. Both primers were 5' phosphorylated.

The PCR product was separated by agarose gel electrophoresis, and visualized under UV transillumination as described in the Common Methods Section. Agarose gel slices containing the DNA fragment corresponding to the amplified Pti,,A:pntAB insert was excised from the gel and the DNA recovered with a standard gel extraction protocol and components from Qiagen according to manufacturer's instructions. This insert DNA was ligated into the pBT-3 vector prepared as described herein with T4 DNA ligase obtained from New England Biolabs (Bedford, MA, USA), following the manufacturer's instructions. Ligation mixtures were transformed into E. coli 100 cells obtained from Lucigen Corp according to the manufacturer's instructions. Colonies were screened by colony polymerase chain reactions. Plasmid DNA from colonies showing inserts of correct size were cultured and purified using a standard miniprep protocol and components from Qiagen according to the manufacturer's instruction. Isolated plasmids were checked by restriction digests and confirmed by sequencing. The sequenced-verified isolated plasmid produced with this procedure was designated pBT-3-P,i,,A:pntAB (SEQ ID NO:6).

### Example 3A: Construction of a plasmid expressing acetyl-CoA carboxylase (accABCD).

A plasmid carrying two operons able to express the components the acetyl-CoA carboxyltransferase complex from E. coli was constructed by DNA2.0 (Menlo Park, CA USA), a commercial DNA gene synthesis provider. This construct incorporated the DNA sequences of the accA and accD genes under control of an inducer-independent promoter derived from the E. coli tpiA gene, and the DNA sequences of the accB and accC genes under control of an inducer-independent promoter derived from the E. coli rpiA genes. Each coding sequence was preceded by a ribosome-binding sequence. The designed operons were provided in a pJ251 vector backbone and was designated pJ251:26385 (SEQ ID NO:74).

The tpiA promoter of the pJ251:26385 plasmid was altered to provide better expression. This modification was incorporated by amplifying the pJ251:26385 plasmid with the forward primer GCGGGGCAGGAGGAAAAACATG (SEQ ID NO:75) and the reverse primer GCTTATAAGCGAATAAAGGAAGATGGCCGCCCCGCAGGGCAG (SEQ ID NO:76). Each of these primers were synthesized with a 5' phosphorylation modification. The resulting PCR product was separated by agarose gel electrophoresis, and the appropriate DNA fragment recovered as described in the Common Methods Section. The recovered product was self-ligated with T4 DNA ligase obtained from New England BioLabs (Ipswich, MA USA) and digested with Dpnl according to manufacturer's instructions. Plasmid DNA from colonies showing inserts of correct size were cultured and purified using a standard miniprep protocol and components from Qiagen according to the manufacturer's instruction. Isolated plasmids were checked by restrictions digests and confirmed by sequencing. The sequenced-verified isolated plasmids produced with this procedure were designated pJ251(26385)-P,,,A:accAD-P,,A:accBC (SEQ ID NO:77).

### Example 3B: Construction of pTrc-PyibD-mcr.

Plasmid maps are shown below. Plasmid 1 was digested with NcoI/Bst1107. A fragment size of 7059 bases was excised from a gel and purified (SEQ ID NO:168). The target promoter sequence was ordered (Integrated DNA Technologies, Coralville, Iowa USA) including with modifications to the native ribosome binding site and subsequently changed to be compatible with existing expression vectors and to accommodate expression of key downstream gene(s) within the vector(s), in this example malonyl CoA reductase (MCR, mcr). Plasmid 2, synthesized by (Integrated DNA Technologies, Coralville, IA USA) to comprise this low-phosphate promoter (see discussion above regarding SEQ ID NOs: 210 and 211), was digested with NcoI/PmlI. A fragment size of 156 bases was excised from a gel and purified, this fragment is the pYibD promoter (SEQ ID NO:210). Fragments were ligated overnight using T4 DNA ligase to create Plasmid 3, identified as pTrc-PyibD-mcr (SEQ ID NO:170).
Plasmid Map 1: Original pTrc-ptrc-MCR
Plasmid Map 2: pIDTSMART-pYibD promoter. Synthesized by Integrated DNA Technologies.
Plasmid Map 3: New MCR construct. pTrc-PyibD-mcr (low phosphate induction) (SEQ ID NO:170).

### Example 4: Construction of specific strains that produce 3-hydroxypropionic acid

According to the respective combinations indicated in the following table, the plasmids described herein were introduced into the respective base strains. All plasmids were introduced at the same time via electroporation using standard methods. Transformed cells were grown on the appropriate media with antibiotic supplementation and colonies were selected based on their appropriate growth on the selective media. The mcr expression plasmid pKK223-mcr was transformed into E. coli DF40 (Hfr, garB10, fhuA22, ompF627, fadL701, relAl, pitA10, spoTl, rrnB-2, pgi-2, mcrB1, creC527) or E. coli JP1111 (Hfr, galE45(GalS), LAM-, fabI392(ts, temperature-sensitive), relAl, spoTl, thi-1) as described in the Common Methods Section. As is known in the art, the strains DF40 and JP1111 are generally available E. coli strains, available from sources including the Yale Coli Genetic Stock Collection (New Haven, CT USA). Strains carrying multiple compatible plasmids were constructed from these mcr transformants by preparing cells competent for transformation by electroporation as described in the Common Methods Section and transforming with the additional plasmids. Transformants were subsequently selected for on media containing the appropriate combination of antibiotics.

**Table: Strain names and characteristics**

| Strain name | Host | Plasmids |
|---|---|---|
| KX3_0001 | DF40 | pKK223-mcr |
| JX3_0077 | JP1111 | pKK223-mcr |
| JX3_0087 | JP1111 | pkk223-mcr + pBT-3-PtpiA:pntAB |
| JX3_0097 | JP1111 | pkk223-mcr +pJ25 1 (26385)PtpiA:accAD-PrpiA:accBC |
| JX3_0098 | JP1111 | pKK223-mcr + pJ251 (26385)PtpiA:accAD-PrpiA:accBC + pBT-3-PtpiA:pntAB |

### Example 5: Production of 3-hydroxypropionic acid

3-HP production by KX3_0001 was demonstrated at 100-mL scale in fed-batch (rich) or AM2 (minimal salts) media. Cultures were started from freezer stocks by standard practice (Sambrook and Russell, 2001) into 50 mL of LB media plus 100 µg/mL ampicillin and grown to stationary phase overnight at 37°C with rotation at 225 rpm. Five ml of this culture were transferred to 100 ml of fed-batch or AM2 media plus 40 g/L glucose, 100 kg/ml ampicillin, 1 mM IPTG in triplicate 250-ml baffled flasks, and incubated at 37°C, 225 rpm. To monitor cell growth and 3-HP production by these cultures, samples (2 ml) were withdrawn at designated time points for optical density measurements at 600nm (0D600, 1 cm pathlength) and pelleted by centrifugation at 12,000rpm for 5 min and the supernatant collected for analysis of 3-HP production as described under "Analysis of cultures for 3-HP production" in the Common Methods section. Dry cell weight (DCW) is calculated as 0.33 times the measured OD600 value, based on baseline DCW to OD600 determinations. All data are the average of triplicate cultures. For comparison purposes, the specific productivity is calculated from the averaged data at the 24-h time point and expressed as g 3-HP produced per gDCW. Production of 3-HP by strain KX3_0001 in fed-batch medium is shown in the following table. Under these conditions, the specific productivity after 24 h is 0.0041 g 3-HP per gDCW.

**Table: Production of 3-HP by KX3_0001 in fed-batch medium**

| Time (hr) | 3HP (g/L) | OD600 |
|---|---|---|
| 0 | 0.002 | 0.118 |
| 3 | 0.002 | 0.665 |
| 4 | 0.005 | 1.44 |
| 6 | 0.008 | 2.75 |
| 8 | 0.009 | 3.35 |
| 24 | 0.008 | 5.87 |

### Example 6: Effect on 3-HP production of increased malonyl-CoA precursor pools by inhibition of fatty acid synthesis

As described herein, certain chemicals are known to inhibit various enzymes of the fatty acid synthase system, some of which are used as antibiotics given the role of fatty acid synthesis in membrane maintenance and growth, and microorganism growth. Among these inhibitors is cerulenin, which inhibits the KASI ketoacyl-ACP synthase (e.g., fabB in E. coli). To further evaluate approaches to modulate and shift malonyl-CoA utilization in microorganisms that comprise production pathways to a selected chemical product, here 3-HP, wherein malonyl-CoA is a substrate in that pathway, addition of cerulenin during a culture was evaluated.

Pathways downstream of malonyl-CoA are limited to fatty acid biosynthesis and 3HP production (when a pathway to the latter via malonyl-CoA exists or is provided in a cell). This experiment is designed to determine how to control the use of malonyl-CoA pools in 3HP production strains and further improve the rate of 3HP production. It is hypothesized that by inhibiting fatty acid biosynthesis and regulating malonyl-CoA pools, flux through the pathway will be shifted toward 3HP production. A representative inhibitor has been selected to both interrupt fatty acid elongation and disrupt a futile cycle that recaptures the malonate moiety back to the acetyl-CoA pool. Production by strain KX3_0001 in fed-batch medium in the presence of 10 kg/ml cerulenin is shown in the following table.

In the presence of the inhibitor, internal pools of the malonyl-CoA precursor are proposed to increase thus leading to increased production of 3-HP. As may be seen by comparison to the results without cerulenin (Example 5), substantially more 3-HP is produced at every time point, and the specific productivity at 24 h is 0.128 g 3-HP per gDCW, a 31-fold increase relative to the results without cerulenin.

**Table: Production of 3-HP by KX3_0001 in fed-batch medium and the presence of 10 kg/ml cerulenin**

| 3HP (g/L) | OD600 |
|---|---|
| 0.002 | 0.118 |
| 0.002 | 0.724 |
| 0.020 | 1.59 |
| 0.060 | 2.80 |
| 0.090 | 3.45 |
| 0.200 | 4.73 |

### Example 7: Effect on 3-HP production of increased malonyl-CoA precursor pools using temperature-sensitive fatty acid synthesis mutant.

An alternative approach to increasing internal malonyl-CoA pools is to use genetic mutations rather than chemical inhibitors. While inactivating mutations in the genes encoding fatty acid synthesis functions are usually lethal and thus not obtainable, conditional mutants, such as temperature-sensitive mutants, have been described (de Mendoza, D., and Cronan, J. E., Jr. (1983) Trends Biochem. Sci., 8, 49-52). For example, a temperature-sensitive mutation in the fabl gene, encoding enoyl-ACP reductase, of strain JP1111 (genotype fab1392(ts)) has relatively normal activity at reduced temperature, such as 30C, and becomes non-permissive, likely through denaturation and inactivation, at elevated temperature, such that when cultured at 37 to 42C a microorganism only comprising this temperature-sensitive mutant as its enoyl-ACP reductase will produce substantially less fatty acids and phospholipids. This leads to decreased or no growth. However, it was hypothesized that when such mutant is provided in a genetically modified microorganism that also comprises a production pathway, such as to 3-HP, from malonyl-CoA, effective culture methods involving elevating culture temperature can result in increased 3-HP specific productivity.

Production of 3-HP by strain JX3_0077 in fed-batch medium at a constant temperature of 30°C and by a culture subjected to a temperature shift from 30°C to 42°C is shown in the following Table. The temperature shift is designed to inactivate the enoyl-ACP reductase, hence eliminating the accumulation of fatty acid which in turn increases the internal malonyl-CoA pool. Substantially more 3-HP is produced at every time point, and the specific productivity at 24 h by the temperature-shifted culture is 1.15 g 3-HP per gDCW, a greater than 100-fold increase over the specific productivity of 0.011 g 3-HP per gDCW by the culture maintained constantly at 30°C. This increased productivity of 3-HP by the culture in which the enoyl-ACP reductase is inactivated by elevated temperature supports the view that shifting of malonyl-CoA utilization leads to increased 3-HP production.

**Table: Production of 3-HP by JX3_0077 in fed-batch medium**

| | Constant 30°C | | Shifted to 42°C | |
|---|---|---|---|---|
| Time (hr) | 3HP (g/L) | OD600 | 3HP (g/L) | OD600 |
| 0 | 0 | 0.065 | 0.0007 | 0.068 |
| 3 | 0.003 | 0.273 | 0.004 | 0.25 |
| 4 | 0.010 | 0.409 | 0.037 | 0.79 |
| 6 | 0.030 | 1.09 | 0.096 | 0.91 |
| 8 | 0.016 | 1.81 | 0.193 | 0.81 |
| 24 | 0.014 | 3.8 | 0.331 | 0.87 |

The following Table shows the 3-HP production by strain JX3_0087 which carried a plasmid overexpressing the transhydrogenase gene in addition to a plasmid carrying the mcr gene. In the culture maintained at a constant temperature of 30°C, a specific productivity of 0.085 g 3-HP per gDCW in 24 h was attained. This is significantly higher than the specific productivity of JX3_0077 which does not carry the overexpressed transhydrogenase gene (above table). The specific productivity of the temperature-shifted culture of JX3_0087 was 1.68 g 3-HP per gDCW, a 20-fold increase over the specific productivity of the culture maintained constantly at 30°C in which the enoyl-ACP reductase was not inactivated.

**Table: Production of 3-HP by JX3_0087 in fed-batch medium**

| | Constant 30°C | | Shifted to 42°C | |
|---|---|---|---|---|
| Time (hr) | 3HP (g/L) | OD600 | 3HP (g/L) | OD600 |
| 0 | 0 | 0.008 | 0 | 0.004 |
| 3 | 0.0007 | 0.008 | 0.0007 | 0.011 |
| 4 | 0 | 0.04 | 0.002 | 0.063 |
| 6 | 0.0007 | 0.05 | 0.009 | 0.193 |
| 8 | 0.003 | 0.157 | 0.050 | 0.257 |
| 24 | 0.003 | 0.107 | 0.455 | 0.820 |

The following Table shows the 3-HP production by strain JX3_0097 which carried a plasmid overexpressing genes encoding the acetyl-CoA carboxylase complex in addition to a plasmid carrying the mcr gene. In the culture maintained at a constant temperature of 30°C, a specific productivity of 0.0068 g 3-HP per gDCW in 24 h was attained. This specific productivity is similar to that attained by strain JX3_0077 in which acetyl-CoA carboxylase is not overexpressed. The specific productivity of the temperature-shifted culture of JX3_0097 was 0.29 g 3-HP per gDCW, a 42-fold increase over the specific productivity of the culture maintained constantly at 30°C in which the enoyl-ACP reductase was not inactivated.

**Table 13: Production of 3-HP by JX3_0097 in fed-batch medium**

| | Constant 30°C* | | Shifted to 42°C* | |
|---|---|---|---|---|
| Time (hr) | 3HP (g/L) | OD600 | 3HP (g/L) | OD600 |
| 0 | | 0.016 | 0 | 0.014 |
| 4 | 0.004 | 0.3 | 0.004 | 0.31 |
| 5 | | 0.36 | 0.006 | 0.59 |
| 6 | | 0.65 | 0.062 | 1.51 |
| 8 | 0.006 | 1.46 | 0.178 | 1.91 |
| 24 | 0.006 | 2.66 | 0.176 | 1.87 |

Fed-batch medium, a rich medium, may contain components that serve as fatty acid precursors and thus may reduce the demand for malonyl-CoA. Thus the production of 3-HP by the strains derived from JP1111 in AM2, a minimal medium was verified. As shown in Table 14, 3-HP was produced by JX3_0077 in AM2 medium. A specific productivity of 0.024 g 3-HP per gDCW in 24 h was obtained by the culture maintained constantly at 30°C, approximately twice the value obtained in fed-batch medium. The temperature-shifted culture attained a specific productivity of 1.04 g 3-HP per gDCW over 24 h, a 44-fold increase compared to the specific productivity of the culture maintained constantly at 30°C, again indicating that conditional inactivation of the enoyl-ACP reductase increased the internal malonyl-CoA pool and hence increased the 3-HP production, as envisioned by the inventors.

**Table 14. Production of 3-HP by JX3_0077 in AM2 medium**

| | Constant 30°C | | Shifted to 42°C | |
|---|---|---|---|---|
| Time (hr) | 3HP (g/L) | OD600 | 3HP (g/L) | OD600 |
| 0 | 0 | 0.066 | 0 | 0.063 |
| 4 | 0.002 | 0.360 | 0.002 | 0.40 |
| 5 | 0.004 | 0.253 | 0.015 | 0.39 |
| 6 | 0.004 | 0.413 | 0.1 | 0.68 |
| 8 | 0.005 | 0.476 | 0.2 | 0.71 |
| 24 | 0.008 | 1.03 | 0.25 | 0.73 |

Production of 3-HP in AM2 medium by strain JX3_0087, which carried a plasmid overexpressing the transhydrogenase gene in addition to a plasmid carrying the mcr gene, is shown in Table 15. In the JX3_0087 culture maintained at a constant temperature of 30°C, a specific productivity of 0.018 g 3-HP per gDCW in 24 h was attained. In contrast to results obtained in fed-batch medium, this value is not higher than the specific productivity obtained in AM2 with strain JX3_0077 which does not carry the overexpressed transhydrogenase gene (Table 14). The specific productivity of the temperature-shifted culture of JX3_0087 was 0.50 g 3-HP per gDCW, a 27-fold increase over the specific productivity of the culture maintained constantly at 30°C in which the enoyl-ACP reductase was not inactivated.

**Table 15. Production of 3-HP by JX3_0087 in AM2**

| | Constant 30°C | | Shifted to 42°C | |
|---|---|---|---|---|
| Time (hr) | 3HP (g/L) | OD600 | 3HP (g/L) | OD600 |
| 0 | 0 | 0.08 | 0 | 0.086 |
| 4 | 0.002 | 0.363 | 0.002 | 0.380 |
| 5 | 0.002 | 0.273 | 0.011 | 0.360 |
| 6 | 0.003 | 0.297 | 0.050 | 0.520 |
| 8 | 0.005 | 0.467 | 0.100 | 0.607 |
| 24 | 0.006 | 1.0 | 0.112 | 0.683 |

Table 16 shows the 3-HP production in AM2 medium by strain JX3_0097 which carried a plasmid overexpressing genes encoding the acetyl-CoA carboxylase complex in addition to a plasmid carrying the mcr gene. In the culture maintained at a constant temperature of 30°C, a specific productivity of 0.021 g 3-HP per gDCW in 24 h was attained. This specific productivity is similar to that attained by strain JX3_0077 in which acetyl-CoA carboxylase is not overexpressed. The specific productivity of the temperature-shifted culture of JX3_0097 was 0.94 g 3-HP per gDCW in 24 h, a 45-fold increase over the specific productivity of the culture maintained constantly at 30°C in which the enoyl-ACP reductase was not inactivated.

**Table 16. Production of 3-HP by JX3_0097.0 in AM2**

| | Constant 30°C | | Shifted to 42°C | |
|---|---|---|---|---|
| Time (hr) | 3HP (g/L) | OD600 | 3HP (g/L) | OD600 |
| 0 | 0 | 0.085 | 0.001 | 0.085 |
| 4 | 0.002 | 0.500 | 0.003 | 0.483 |
| 5 | 0.003 | 0.287 | 0.015 | 0.473 |
| 6 | 0.005 | 0.417 | 0.073 | 0.510 |
| 8 | 0.005 | 0.520 | 0.198 | 0.590 |
| 24 | 0.013 | 1.91 | 0.192 | 0.620 |

The effect of combining the plasmids expressing mcr (malonyl-CoA reductase), pntAB (transhydrogenase), and accABCD (acetyl-CoA carboxylase complex) in the same organism was tested by constructing strain JX3_0098. The Table below shows the production of 3-HP by this strain in AM2 medium. A specific productivity of 0.54 g 3-HP per gDCW in 24 h was obtained in the culture maintained constantly at 30°C, representing a >20-fold increase over strains carrying mcr alone or mcr with either pntAB or accABCD, but not both. Shifting the temperature to inactivate enoyl-ACP reductase resulted in a specific productivity of 2.01 g 3-HP per gDCW in 24 h, a further 3.8-fold increase. Thus the combination of overexpression of pntAB and of accABCD, plus the inactivation of enoyl-ACP reductase via the temperature-sensitive fabr' allele, resulted in an approximately 500-fold increase in specific productivity of 3-HP by mcr-bearing cells (specific productivity of 2.01 vs. 0.0041 g 3-HP per gDCW in 24 h).

**Table 17. Production of 3-HP by JX3_0098.0 in AM2 medium**

| | Constant 30°C | | Shifted to 42°C | |
|---|---|---|---|---|
| Time (hr) | 3HP (g/L) | OD600 | 3HP (g/L) | OD600 |
| 0 | 0.007 | 0.117 | 0 | 0.13 |
| 4 | 0.013 | 0.303 | 0.017 | 0.47 |
| 5 | 0.017 | 0.600 | 0.060 | 0.75 |
| 6 | 0.033 | 0.730 | 0.107 | 0.87 |
| 8 | 0.053 | 0.9107 | 0.263 | 0.81 |
| 24 | 0.670 | 3.790 | 0.577 | 0.81 |

### Example 8: Sequence of the fabits mutation

The nature of the exact sequence change in the fabr' allele carried by strains JP1111 was reconfirmed. Confirmation of this change allows targeted mutagenesis to generate alternative strains with different temperature sensitivities and mutants with stabilities intermediate between wild type and the fabI392 temperature-sensitive allele, allowing growth at a constant temperature higher than 30°C while providing the benefit of increased internal malonyl-CoA pools. To confirm the DNA sequence of this segment of the chromosome of a wild type (BW25113) and the JP1111 mutant E. coli, chromosomal DNA was prepared from these strains. These DNA were used as templates in a PCR reaction with primers:

| | | |
|---|---|---|
| FW043 | ATGGGTTTTCTTTCCGG | SEQ ID NO:78 |
| FW047 | TTATTTCAGTTCGAGTTCG | SEQ ID NO:79 |

Thermocyler conditions for the PCR were: 95°C, 10 min; 30 cycles of 95°C, 10s; 47°C increasing to 58°C, 30s; 72°C, 1 min; followed by a final incubation at 72°C for 5 min. The PCR product was separated on an agarose gel and the appropriate sized fragment recovered as described in the Common Methods Section, and sequenced using primers:

| | | |
|---|---|---|
| FW044 | CTATCCATCGCCTACGGTATC | SEQ ID NO:80 |
| FW045 | CGTTGCAATGGCAAAAGC | SEQ ID NO:81 |
| FW046 | CGGCGGTTTCAGCATTGC | SEQ ID NO:82 |

A comparison of the DNA sequence obtained from the fabI392 (SEQ ID NO:28) and wild type strains reveals a single difference between the alleles of C at position 722 of the wild type gene to T, leading to a protein change of Ser at codon 241 to Phe.

The identification of the affected residue at codon 241 indicates that targeted mutagenesis at this codon, for example to amino acid residues such as Trp, Tyr, His, Ile, or other amino acids other than Ser or Phe, may result in fah./ alleles with different properties than the fabI392 originally isolated in JP1111. Targeted mutagenesis at codons near to codon 241 may also be contemplated to obtain the desired fah./ mutants with altered properties.

### Example 9: Effect on volumetric 3-HP production in 1L fermentations, of increased malonyl-CoA precursor pools using temperature sensitive fatty acid synthesis mutants.

Four 1 L fed batch fermentation experiments were carried out using the strain JX3_0098. Briefly, seed cultures were started and grown overnight in LB media (Luria Broth) and used to inoculate four 1 L New Brunswick fermentation vessels. The first vessel contained defined AM2 medium at 30°C, IPTG induction was added at 2mM at an OD600nm of 2, additional glucose feed was initiated when glucose was depleted to between 1-2 g/L. The temperature was shifted 37°C over 1 hr at target OD of 10. A high glucose feed rate was maintained at > 3g/L/hr until glucose began to accumulate at concentrations greater than 1g/L at which time feed rate was varied to maintain residual glucose between 1 and 10g/L. The second vessel contained defined AM2 medium at 30°C, IPTG induction was added at 2mM at an OD600nm of 2, additional glucose feed was initiated when glucose was depleted to 0 g/L. The temperature was shifted 37°C over 1 hr at target OD of 10. The glucose feed rate was maintained less than or equal to 3g/L/hr. The third vessel contained rich medium at 30°C, IPTG induction was added at 2mM at an OD600nm of 2, additional glucose feed was initiated when glucose was depleted to 1-2 g/L. The temperature was shifted 37°C over 1 hr at target OD of 10. A high glucose feed rate was maintained at > 3g/L/hr until glucose began to accumulate at concentrations greater than 1g/L at which time feed rate was varied to maintain residual glucose between 1 and 10g/L. The fourth vessel contained rich medium at 30°C, IPTG induction was added at 2mM at an OD600nm of 2, additional glucose feed was initiated when glucose was depleted to 0 g/L. The temperature was shifted 37°C over 1 hr at target OD of 10. The glucose feed rate was maintained less than or equal to 3g/L/hr.

All fermentation vessels were maintained at pH = 7.4 by the controlled addition of 50% v/v ammonium hydroxide (Fisher Scientific). All vessels were maintained at least 20% dissolved oxygen by aeration with sparged filtered air. Samples were taken for optical density measurements as well as HPLC analysis for 3-HP concentration. (Refer to common methods). Maximum volumetric productivities reached 2.99 g/L/hr. In addition, the figures demonstrate the correlation between the 3-4 hour average biomass concentration and 3-4 hr average volumetric productivity rates in these 4 vessels.

### Example 10A: Production of 3-HP in 250 liter fermentations

Examples of two fed batch fermentations in a 250 liter volume stainless steel fermentor were carried out using the strain BX3_0240, the genotype of which is described elsewhere herein. A two stage seed process was used to generate inoculum for the 250 L fermentor. In the first stage, one ml of glycerol stock of the strain was inoculated into 100 ml of TB medium (Terrific Broth) in a shake flask and incubated at 30°C until the OD600 was between 3 and 4. In the second stage, 85 ml of the shake flask culture was aseptically transferred to a 14 L New Brunswick fermentor containing 8 L of TB medium and grown at 30°C and 500 rpm agitation until the OD600 was between 5 and 6. The culture from the 14 L fermentor was used to aseptically inoculate the 250 L volume bioreactor containing defined FM5 medium (see Common Methods Section) at 30°C so that the post-inoculation volume was 155 L.

In the first fermentation, induction was effected by adding IPTG to a final concentration of 2 mM at an OD600 of 20. Glucose feed (consisting of a 700 g/L glucose solution) was initiated when the residual glucose in the fermentor was 10-15 g/L. The feed rate was adjusted to maintain the residual glucose between 10 and 15 g/L until about the last 6 hours of the fermentation when the feed rate was reduced so that the residual glucose at harvest was <1 g/L to facilitate 3-HP recovery. Three hours after induction, the temperature was shifted to 37°C over 1 hour. At the time the temperature shift was initiated, the dissolved oxygen (DO) set point was changed from 20% of air saturation to a point where the DO was maintained between 2-4% of air saturation. The fermentation broth was harvested 48 hours after inoculation. The final broth volume was 169.5 liters.

The second fermentation was run identically to the first example fermentation described above except for the following differences: induction with IPTG was effected at an OD₆₀₀ of 15, the residual glucose (after the glucose feed was started) ranged between 3-30 g/L, and the fermentation broth was harvested at 38.5 hours after inoculation so that the final residual glucose concentration was 25 g/L. The final broth volume was 167 liters.

Each fermentation broth was maintained at a pH of approximately 7.4 by the controlled addition of anhydrous ammonia gas. Dissolved oxygen was maintained at the desired levels by aeration with sparged, sterile-filtered air. Samples were taken for optical density measurements as well as HPLC analysis for 3-HP concentration. In the first fermentation, the maximum biomass concentration was 12.0 g dry cell weight/L and the biomass concentration at harvest was 11.4 g dry cell weight/L. The maximum 3-HP titer in this fermentation was 20.7 g/L. In the second fermentation, the maximum biomass concentration was 10.2 g dry cell weight/L and the biomass concentration at harvest was 9.5 g dry cell weight/L. The maximum 3-HP titer in this fermentation was 20.7 g/L.

### Example 10B: Effect of growth medium on 3-HP production in 1 L fermentations

Eight 1 L fed batch fermentation experiments were carried out using the strain BX3_0240. Seed culture was started from 1 ml of glycerol stock of the strain inoculated into 400 ml of TB medium (Terrific Broth) in a shake flask and incubated at 30°C until the OD600 was between 5 and 6. The shake flask culture was used to aseptically inoculate each 1 L volume bioreactor so that the post-inoculation volume was 653 ml in each vessel.

Fermentors 1 and 2 contained defined FM3 medium. Fermentors 3-5 contained defined FM4 medium. Fermentors 6-8 contained defined FM5 medium. All media formulations are listed in the Common Methods Section. In each fermentor, the initial temperature was 30°C.

Induction was effected by adding IPTG to a final concentration of 2 mM at OD600 values of 15-16. Glucose feed (consisting of a 500 g/L glucose solution for FM3 and FM5 media and 500 g/L glucose plus 75 mM MgS04 for FM4) was initiated when the residual glucose in the fermentor was about 10 g/L. The feed rate was adjusted to maintain the residual glucose >3 g/L (the exception was fermentor 8 in which the residual glucose temporarily reached 0.1 g/L before the feed rate was increased). Three hours after induction, the temperature was shifted to 37°C over 1 hour. At the time the temperature shift was initiated, the dissolved oxygen (DO) set point was changed from 20% of air saturation to 1% of air saturation. The fermentations were stopped 48 hours after inoculation.

The broth of each fermentor was maintained at a pH of approximately 7.4 by the controlled addition of a pH titrant. The pH titrant for FM3 medium was 5 M NaOH and for FM4 and FM5 it was a 50:50 mixture of concentrated ammonium hydroxide and water. Dissolved oxygen was maintained at the desired levels by sparging with sterile-filtered air. Samples were taken for optical density measurements as well as HPLC analysis for 3-HP concentration. The maximum biomass concentration and the biomass concentration at harvest as well as the maximum 3-HP titer in each fermentor are summarized in the Table 18 below.

**Table 18**

| **Fermentor No.** | **Growth Medium** | **Maximum Biomass Conc. (g DCW/L)** | **Biomass Conc. at Harvest (g DCW/L)** | **Maximum 3HP Titer (g/L)** |
|---|---|---|---|---|
| 1 | FM3 | 8.7 | 8.7 | 12.3 |
| 2 | FM3 | 9.6 | 9.5 | 16.7 |
| 3 | FM4 | 10.9 | 10.9 | 20.7 |
| 4 | FM4 | 11.5 | 11.5 | 18.3 |
| 5 | FM4 | 11.3 | 11.3 | 22.1 |
| 6 | FM5 | 11.3 | 11.3 | 35.2 |
| 7 | FM5 | 11.2 | 11.0 | 34.0 |
| 8 | FM5 | 11.6 | 10.6 | 31.2 |

### Example 10C: Effect of batch phosphate concentration on 3-HP production in 1 L fermentations.

Four 1 L fed batch fermentation experiments were carried out using the strain BX3_0240. Seed culture was started from 1 ml of glycerol stock of the strain inoculated into 400 ml of TB medium (Terrific Broth) in a shake flask and incubated at 30°C until the OD600 was between 5 and 7. The shake flask culture was used to aseptically inoculate each 1 L volume bioreactor so that the post-inoculation volume was 653 ml in each vessel.

All fermentors contained defined FM5 growth medium, but each had different initial concentrations of monobasic and dibasic potassium phosphate. The phosphate concentrations in the batch medium in each fermentor are summarized in the Table 19.

**Table 19**

| **Fermentor No.** | **K2HPO4 conc. in batch medium (g/L)** | **KH2PO4 conc. in batch medium (g/L)** |
|---|---|---|
| 1 | 6.1 | 1.92 |
| 2 | 2.63 | 1.38 |
| 3 | 0.87 | 0.14 |
| 4 | 0.043 | 0.070 |

In each fermentor, the initial temperature was 30°C. Induction was effected by adding IPTG to a final concentration of 2 mM when the OD600 values were at the following values: fermentor 1, 15.3; fermentor 2, 16.0; fermentor 3, 18.1; fermentor 4, 18.4. Glucose feed (consisting of a 500 g/L glucose solution for FM3 and FM5 media and 500 g/L glucose plus 75 mM MgS04 for FM4) was initiated when the residual glucose in the fermentor was about 10 g/L. The feed rate was adjusted to maintain the residual glucose >6.5 g/L. Three hours after induction, the temperature was shifted to 37°C over 1 hour. At the time the temperature shift was initiated, the dissolved oxygen (DO) set point was changed from 20% of air saturation to 1% of air saturation. The fermentations were stopped 48 hours after inoculation.

The broth of each fermentor was maintained at a pH of 7.4 by the controlled addition of a 50:50 mixture of concentrated ammonium hydroxide and water. Dissolved oxygen was maintained at the desired levels by sparging with sterile-filtered air. Samples were taken for optical density measurements as well as HPLC analysis for 3-HP concentration. The maximum biomass concentration and the biomass concentration at harvest as well as the maximum 3-HP titer in each fermentor are summarized in the Table 20 below.

**Table 20**

| **Fermentor No.** | **Maximum Biomass Conc. (g DCW/L)** | **Biomass Conc. at Harvest (g DCW/L)** | **Maximum 3HP Titer (g/L)** |
|---|---|---|---|
| 1 | 9.6 | 8.4 | 23.7 |
| 2 | 11.3 | 11.3 | 27.8 |
| 3 | 14.8 | 12.9 | 39.8 |
| 4 | 12.3 | 10.9 | 44.1 |

### Example 10D: 3-HP production in 1 L fermentations

Two 1 L fed batch fermentation experiments were carried out using the strain BX3_0240. Seed culture was started from 1 mL of glycerol stock of the strain inoculated into 100 mL of TB medium (Terrific Broth) in a shake flask and incubated at 30°C until the OD600 was between 5 and 6. The shake flask culture was used to aseptically inoculate (5% volume/volume) each 1 L volume bioreactor so that the post-inoculation volume was 800 mL in each vessel. The fermentors used in this experiment were Das Gip fed-batch pro parallel fermentation system (DASGIP AG, Julich, Germany, model SR07000DLS). The fermentation system included real-time monitoring and control of dissolved oxygen (%D0), pH, temperature, agitation, and feeding. Fermentors 1 and 2 contained defined FM5 medium, made as shown in the Common Methods Section except that Citric Acid was added at 2.0 g/L and MgS04 was added at 0.40 g/L. In each fermentor, the initial temperature was 30°C. Induction was effected by adding IPTG to a final concentration of 2 mM at OD600 values of 17-19, which corresponded to a time post-inoculation of 14.5 hr. Glucose feed (consisting of a 500 g/L glucose solution) was initiated when the residual glucose in the fermentor was about 1 g/L. The feed rate was adjusted to maintain the residual glucose >3 g/L. Three hours after induction, the temperature was shifted to 37°C over 1 hour. At the time the temperature shift was initiated, the OTR was set to 40mmol/L-hr by setting airflow and agitation to 1.08 vvm and 1000 rpm respectively. Compressed air at 2 bar was used as the air feed. The broth of each fermentor was maintained at a pH of approximately 7.4 by the controlled addition of a pH titrant. Two hours subsequent to IPTG induction, the pH titrant was changed from 50% NH₄(OH) to 7.4 M NaOH. Samples were taken for optical density measurements as well as HPLC analysis for 3-HP concentration. The maximum biomass concentration and the biomass concentration at harvest as well as the maximum 3-HP titer in each fermentor are summarized in the Table 21 below.

**Table 21**

| **Fermentor No.** | **Maximum Biomass Conc. (g DCW/L)** | **Biomass Conc. at Harvest (g DCW/L)** | **Total 3-HP (g) at 69hrs** | **Yield of 3-HP At (g3-HP/g glucose)** |
|---|---|---|---|---|
| 1 | 10.5 | 8.7 | 49.0 | 0.46 |
| 2 | 10.5 | 8.7 | 47.8 | 0.46 |

The following Table 22 provides a summary of concentrations of metabolic products obtained in the fermentation broth at the indicated time in hours.

| **Replicate** | **Time (hrs)** | **3-HP (g/L)** | **Pyruvate (g/L)** | **Succinate (g/L)** | **Lactate (g/L)** |
|---|---|---|---|---|---|
| 1 | 0 | 0 | 0.341 | 0.328 | 0 |
| 1 | 45 | 35.128 | 5.596 | 0 | 0 |
| 1 | 69 | 36.05 | 9.179 | 0 | 0 |
| 2 | 0 | 0 | 0.346 | 0.376 | 0 |
| 2 | 45 | 31.188 | 8.407 | 0 | 0 |
| 2 | 69 | 35.139 | 13.143 | 0 | 0 |

**Table 22 (continued)**

| **Fumarate (g/L)** | **Glutamate (g/L)** | **Glutamine (g/L)** | **Glycerol (g/L)** | **Alanine (g/L)** | |
|---|---|---|---|---|---|
| 0.002 | 0.006 | 0 | 0.563 | 0.139 | |
| 0.013 | 0.959 | 0 | 0.160 | 0.104 | |
| 0.003 | 1.77 | 0 | 0.244 | 0.075 | |
| 0.002 | 0.893 | 0.075 | 0.471 | 0.109 | |
| 0.004 | 0.796 | 0 | 0.347 | 0.084 | |
| 0.011 | 1.23 | 0 | 0.481 | 0.077 | |

### Example 10E: 3-HP production in 1 L fermentations

Four 1 L fed batch fermentation experiments were carried out using the strain BX3_0240. Seed culture was started from 1 ml of glycerol stock of the strain inoculated into 100 mL of TB medium (Terrific Broth) in a shake flask and incubated at 30°C until the OD₆₀₀ was between 5 and 6. The shake flask culture was used to aseptically inoculate (5% volume/volume) each 1 L volume bioreactor so that the post-inoculation volume was 800 ml in each vessel. The fermentors used in this experiment were Das Gip fed-batch pro parallel fermentation system (DASGIP AG, Julich, Germany, model SR07000DLS). The fermentation system included real-time monitoring and control of dissolved oxygen (%D0), pH, temperature, agitation, and feeding. All fermentors contained defined FM5 medium, made as shown in the Common Methods Section except that Citric Acid was added at 2.0 g/L and MgS04 was added at 0.40 g/L. In each fermentor, the initial temperature was 30°C. Induction was effected by adding IPTG to a final concentration of 2 mM at OD600 values of 15-19, which corresponded to a time post-inoculation of 15.75 hr. Glucose feed (consisting of a 500 g/L glucose solution) was initiated when the residual glucose in the fermentor was about 3 g/L. The feed rate was adjusted to maintain the residual glucose >3 g/L. Three hours after induction, the temperature was shifted to 37°C over 1 hour.The broth of each fermentor was maintained at a pH of approximately 7.4 by the controlled addition of a pH titrant 50% NH4(OH). At the time the temperature shift was initiated, the OTR was changed for each fermentor by varying the agitation and airflow according to Table 23. Compressed air at 2 bar was used as the air feed) Samples were taken for optical density measurements as well as HPLC analysis for 3-HP concentration. The maximum biomass concentration and the biomass concentration at harvest as well as the maximum 3-HP titer in each fermentor are summarized in the Table 23 below.

**Table 23**

| **Fermentor No.** | **Airflow (vvm)** | **Agitation during Production (rpm)** | **Biomass Conc. at Harvest (g DCW/L)** | **3HP Titer (g/L) at 37 hrs** |
|---|---|---|---|---|
| 1 | 1.08 | 1000 | 8.6 | 14.9 |
| 2 | 1.08 | 800 | 9.0 | 7.9 |
| 3 | 1.08 | 600 | 8.2 | 0.5 |
| 4 | 1.08 | 400 | 5.9 | 0.5 |

### Example 10F: 3-HP production in 1.8 L fermentation

A 1.8 L fed batch fermentation experiment was carried out using the strain BX3_0240. Seed culture was started from 1 ml of glycerol stock of the strain inoculated into 105 ml of TB medium (Terrific Broth) in a shake flask and incubated at 30°C until the OD600 was between 5 and 7.90 ml of the shake flask culture was used to aseptically inoculate 1.71 L of FM5 growth medium, except that the phosphate concentrations were 0.33 g/L K2HPO4 and 0.17 g/L KH2PO4 in batch medium. The other ingredients in the FM5 media formulation are as listed in the Common Methods Section. The initial temperature in the fermentor was 30°C. Induction was effected by adding IPTG to a final concentration of 2 mM when the OD600 value was at 15.46. Glucose feed (consisting of a 500 g/L glucose solution) was initiated when the residual glucose in the fermentor was about 10 g/L. The feed rate was adjusted to maintain the residual glucose >6.5 g/L. Three hours after induction, the temperature was shifted to 37°C over 1 hour. At the time the temperature shift was initiated, the dissolved oxygen (DO) set point was changed from 20% of air saturation to 1% of air saturation. The broth of each fermentor was maintained at a pH of 7.4 by the controlled addition of a 50:50 mixture of concentrated ammonium hydroxide and water. Dissolved oxygen was maintained at the desired levels by sparging with sterile-filtered air. Samples were taken for optical density measurements as well as HPLC analysis for 3-HP concentration. The maximum final biomass concentration was 9.84 g/L, the maximum 3-HP titer was 48.4g/L with a final yield from glucose of 0.53g 3-HP/ g glucose.

### Example 11

### Part 1: Strain Construction for Further Evaluations of 3-HP Production

According to the respective combinations indicated in Table 24 below, the plasmids described herein were introduced into the respective strains. All plasmids were introduced at the same time via electroporation using standard methods. Transformed cells were grown on the appropriate media with antibiotic supplementation and colonies were selected based on their appropriate growth on the selective media. As summarized in Table 24, the mcr expression plasmids pTrc-ptrc-mcr or pACYC(kan)-ptalA-mcr were transformed into two strains derived from E. coli BW25113 (F-, A(araD-araB)567, AlacZ4787(::rrnB-3), lamba-, rph-1, A(rhaD-rhaB)568, hsdR514), these strains comprising additional chromosomal modifications introduced using Gene Bridges technology as described in the Common Methods Section. Strain BX_0590 comprises additional deletions of the ldhA, pflB, mgsA, and poxB genes. Strain BX_0591 comprises the additional deletions of Strain BX_0590 and an additional deletion of the ack_pta genes. Transformants were subsequently selected for on media containing the appropriate combination of antibiotics.

**Table 24**

| **Strain name** | **Host** | **Plasmids** |
|---|---|---|
| BX3_ 0194 | BX_ 0590 | PTrc-ptrc-mcr |
| BX3_ 0195 | BX_ 0591 | PTrc-ptrc-mcr |
| BX3_ 0206 | BX_ 0590 | pACYC(kan)-ptalA-mcr |

### Example 11A: Construction of Additional Strains for Evaluation

### Part 1: Gene Deletions

The homologous recombination method using Red/ET recombination, as described elsewhere herein, was employed for gene deletion in E. coli strains. This method is known to those of ordinary skill in the art and described in U.S. Patent Nos. 6,355,412 and 6,509,156, issued to Stewart et al. and incorporated by reference herein for its teachings of this method. Material and kits for such method are available from Gene Bridges (Gene Bridges GmbH, Heidelberg (formerly Dresden), Germany, <<www.genebridges.com>>), and the method proceeded by following the manufacturer's instructions. The method replaces the target gene by a selectable marker via homologous recombination performed by the recombinase from X-phage. The host organism expressing k-red recombinase is transformed with a linear DNA product coding for a selectable marker flanked by the terminal regions (generally -50 bp, and alternatively up to about -300 bp) homologous with the target gene or promoter sequence. The marker is thereafter removed by another recombination step performed by a plasmid vector carrying the FLP-recombinase, or another recombinase, such as Cre.

Specific deletions were constructed by amplification using PCR from the Keio strains carrying particular deletions using primers as specified below. The Keio collection was obtained from Open Biosystems (Huntsville, AL USA 35806). Individual clones may be purchased from the Yale Genetic Stock Center (New Haven, CT USA 06520). These strains each contain a kanamycin marker in place of the deleted gene. In cases where the desired deletion was not in a Keio strain, for example ackA-pta, the deletion was constructed by the above-noted recombination method using the kanamycin resistance marker to replace the deleted sequence, followed by selection of a kanamycin resistance clone having the deletion. The PCR products were introduced into targeted strains using the above-noted recombination method. Combinations of deletions were generated sequentially to obtain strains as described in the following parts of this example.

**Table 25**

| **Plasmid template** | **Keio Clone Number** | **Gene Deletion** | **Forward Primer SEQ ID NO:** | **Reverse Primer SEQ ID NO:** |
|---|---|---|---|---|
| | JW1375 | ldhA | | |
| | JW0886 | pflB | 86 | 99 |
| | JW5129 | mgsA | 87 | 100 |
| | JW0855 | poxB | 88 | 101 |
| | JW2880 | serA | 89 | 102 |
| | JW4364 | arcA | 90 | 103 |
| | JW4356 | trpR | 91 | 104 |
| | JW3561 | aldB | 92 | 105 |
| | JW1412 | aldA | 93 | 106 |
| | JW1293 | puuC | 94 | 107 |
| | JW2755 | relA | 95 | 108 |
| pKD4 | | spoT | 96 | 109 |
| pKD4 | | ackA-pta | 97 | 110 |
| | JW1228 | adhE | 98 | 111 |

### Part 2: Construction of strains BW_595 and BW_651 having a fabl mutation

The fads mutation (Ser241->Phe) in E. coli strain JP1111 significantly increases the malonyl-CoA concentration when cells are grown at the nonpermissive temperature (37°C) and thus produces more 3-HP at this temperature. However, JP1111 is not an ideal strain for transitioning into pilot and commercial scale, since it is the product of NTG mutagenesis and thus may harbor unknown mutations, carries mutations in the stringency regulatory factors relA and spoT, and has enhanced conjugation propensity due to the presence of an Hfr factor. Thus the fads mutation was moved into strain BX_591, a strain developed from the well-characterized BW23115 carrying the additional mutations AldhA, ApflB, AmgsA, ApoxB, Apta-ack. These mutations were generated by the sequential application of the gene deletion method described in Part 1 above. The fads gene with 600 by of upstream and downstream DNA sequence was isolated from JP1111 genomic DNA by PCR using primers:
SEQ ID NO: 112 FW056: 5'-CCAGTGGGGAGCTACATTCTC; and
SEQ ID NO: 113 FW057: 5'-CGTCATTCAGATGCTGGCGCGATC.

The FRT::kan::FRT cassette was then inserted at a Smal site downstream of the fads to generate plasmid pSMART(HC)amp_fabr_FRT::kan::FRT. This plasmid was used as template DNA and the region between primers:
SEQ ID NO: 114 FW043: 5'- ATGGGTTTTCTTTCCGG and
FW057 (SEQ ID NO: 113)
was amplified in a PCR using KOD HS DNA polymerase (Novagen). The reaction was treated with Dpnl to fragment the plasmid template and the amplification fragment was gel-purified and recovered using the DNA Clean and Concentrator kit (Zymo Research, Orange, CA). Strain BX_591 was transformed with pSIM5 (Datta, S., et al., Gene 379:109-115, 2006) and expression of the lambda red genes carried on this plasmid were induced by incubation at 42°C for 15 min.

Electrocompetent cells were made by standard methods. These cells were transformed with the amplification fragment bearing the fabr_FRT::kan::FRT cassette and transformant colonies isolated on LB plates containing 35 jig/mlkanamycin at 30°C. Individual colonies were purified by restreaking, and tested for temperature sensitivity by growth in liquid medium at 30°C and 42°C. Compared to wildtype parental strain, the strain bearing the fabI allele grows poorly at 42°C but exhibited comparable growth at 30°C. Correct insertion of the FRT::kan::FRT marker was verified by colony PCR, and the fabr kanR strain was designated BX_594.

To allow use of the kanR marker on plasmids, the marker incorporated in the chromosome adjacent to fabr was replaced with a DNA fragment encoding resistance to zeocin. The zeoR gene was amplified by PCR from plasmid pJ402 (DNA 2.0, Menlo Park, CA) using primers:
SEQ ID NO: 115 HL018: 
5'-CAGGTTTGCGGCGTCCAGCGGTTATGTAACTACTATTCGGC
GCGACTTACGCCGCTCCCCGCTCGCGATAATGTGGTAGC; and
SEQ ID NO: 116 HL019:

5'-AATAAAACCAATGATTTGGCTAATGATCACACAGTC
CCAGGCAGTAAGACCGACGTCATTCTATCATGCCATACCGCGAA.

The reaction was treated with Dpnl and gel-purified as above. Strain BX_594 was transformed with pKD46 (Datsenko and Wanner, Proc. Natl. Acad. Sci. USA 96: 6640-6645, 2000) and the lambda red genes carried on this plasmid were induced by the addition of L-arabinose to 1 mM for 2 hr. Electrocompetent cells were made by standard methods (e.g, Sambrook and Russell, 2001). These cells were transformed with the zeoR fragment and transformants selected for on LB plates formulated without NaC1 and with 25 jig/ml zeocin. Plates were kept in the dark by wrapping in aluminum foil, and incubated at 30°C. A zeocin-resistant, kanamycin-sensitive strain isolated by this method was designated BX_595. Retention of the fablts allele was confirmed by growth as above.

Strain BX_651 was constructed by transferring the fabr -zeoR cassette from BX_595 to strain BW25113 which does not carry mutations in metabolic genes. A DNA fragment carrying this cassette was obtained by PCR using BX 595 chromosomal DNA and primers FW043 (see above) and SEQ ID NO:117 FW65: 5'-GAGATAAGCCTGAAATGTCGC. The PCR product was purified and concentrated using the DNA Clean and Concentrator kit (Zymo Research, Orange, CA). Strain BW25113 was transformed with pRedD/ET (Gene Bridges GmBH, Heidelberg, Germany) and the lambda red genes carried on this plasmid were induced by the addition of L-arabinose to 5 mM for 2 hr. Electrocompetent cells were made by standard methods, and transformed with the fabr-zeoR DNA fragment. Transformants were plated as above on zeocin, and clones bearing the temperature-sensitive allele verified by growth at 30°C and 42°C as described above.

### Part 3: Promoter Replacement for Selected Genes in Chromosome

The homologous recombination method described elsewhere herein was employed to replace promoters of various genes. As noted, use of Red/ET recombination is known to those of ordinary skill in the art and described in U.S. Patent Nos. 6,355,412 and 6,509,156, issued to Stewart et al. and incorporated by reference herein for its teachings of this method. Material and kits for such method are available from Gene Bridges (Gene Bridges GmbH, Heidelberg, Germany, <<www.genebridges.com>>), and the method may proceed by following the manufacturer's instructions. The method involves replacement of the target gene (or, in this case, a promoter region) by a selectable marker via homologous recombination performed by the recombinase from X-phage. The host organism expressing X-red recombinase is transformed with a linear DNA product coding for a selectable marker flanked by the terminal regions (generally -50 bp, and alternatively up to about -300 bp) homologous with the target gene or promoter sequence. The marker can then be removed by another recombination step performed by a plasmid vector carrying the FLP-recombinase, or another recombinase, such as Cre. This method was used according to manufacturer's instructions. Template sequences, each comprising end sequences to achieve the recombination to replace a native promoter for the indicated gene of interest, the desired replacement promoter, and an antibiotic marker sequence, were synthesized by an outside manufacturer (Integrated DNA Technologies, Coralville, IA). These sequences are designed to replace the native promoter in front of these genes with a T5 promoter. The T5-aceEF cassette (SEQ ID NO:120) also includes a zeocin resistance cassette flanked by loxP sites. The T5-pntAB (SEQ ID NO:121), T5-udhA (SEQ ID NO:122) and T5-cynTS (SEQ ID NO:123) cassettes each include a blasticidin resistance cassette flanked by loxP sites. Also, T5-cynTS (SEQ ID NO:123) comprises modified loxP sites in accordance with Lambert et al., AEM 73(4) p1126-1135.

Each cassette first is used as a template for PCR amplification to generate a PCR product using the primers CAGTCCAGTTACGCTGGAGTC (SEQ ID NO:118), and ACTGACCATTTAAATCATACCTGACC (SEQ ID NO:119). This PCR product is used for electroporation (using standard methods such as described elsewhere herein) and recombination into the genome following the Red/ET recombination method of Gene Bridges described above. After transformation positive recombinants are selected on media containing zeocin or blasticidin antibiotics. Curing of the resistance marker is accomplished by expression of the Cre-recombinase according to standard methods. Table 27 shows strains having genotypes that comprise replaced promoters. These are shown as "T5" followed by the affected gene(s).

### Part 4: Construction of Plasmids

The following table summarizes the construction of plasmids that were used in strains described below. To make the plasmids, a respective gene or gene region of interest was isolated by either PCR amplification and restriction enzyme (RE) digestion or direct restriction enzyme digestion of an appropriate source carrying the gene. The isolated gene was then ligated into the desired vector, transformed into E. coli 10G (Lucigen, Middleton, WI) competent cells, screened by restriction mapping and confirmed by DNA sequencing using standard molecular biology procedures (e.g., Sambrook and Russell, 2001).

It is noted that among these plasmids are those that comprise mono-functional malonyl-CoA reductase activity. Particularly, truncated portions of malonyl-CoA reductase from C. aurantiacus were constructed by use of PCR primers adjacent, respectively, to nucleotide bases encoding amino acid residues 366 and 1220, and 496 and 1220, of the codon-optimized malonyl-CoA reductase from pTRC-ptrc-mcr-amp. Also, a malonyl-CoA reductase from Erythrobacter sp. was incorporated into another plasmid. As for other plasmids, these were incorporated into strains and evaluated as described below.

**Table 26**

| **Gene(s) or Region Name** | **Vector and *Supplier** | **Catalog Number** | **Cloning Method/Gene(s) Source** | **Plasmid Name** | **Plasmid SEQ ID NO:** |
|---|---|---|---|---|---|
| *Erythrobacter sp* MCR | pTRCHisA *A | V360-20 | RE (Ncol/BgIII) / pUC *57-Eb* mcr (SEQ ID NO:905 | pTrc-ptrc-Ebmcr-amp | 128 |
| Truncated C. *aurantiacus mcr* (366-1220) | pTRCHisA *A | V360-20 | PCR, RE (Ncol/HindIII)/ pTRC-ptrc mcr-amp | pTrc-ptrc-(366-1220)mcr-ptrc-ydfG-kan | 129 |
| Truncated C. *aurantiacus mcr* (496-1220) | pTRCHisA *A | V360-20 | PCR, RE (Ncol/HindIII)/ pTRC-ptrc mcr-amp | pTrc-ptrc-ydfG-ptrc-(496-1220)mcr-amp | 130 |
| Mcr | pTRCHisA *A | V360-20 | PCR, RE (Ncol/HindIII)/ SEQ ID No. 003 | pTrc-ptrc-mcr-amp | 131 |
| Mcr | pTRCHisA *A | V360-20 | RE (Ahdl, blunted) for Kan insertion/pTRC-ptrc mcr-amp | pTrc-ptrc-mcr-kan | 3 |
| mcr/cynTS | pTRCHisA *A | V360-20 | RE/ (Ndel, blunted: pTRC ptrc-mcr ) kan, (EcoRV: pSMARTHC am pcynTS) | pTrc-ptrc-mcr-kan-cynTS | 132 |
| AccABCD | pJ251 *C | N/A | RE (EcoNI, Asel, blunted) for Cat insertion/ SEQ ID No 820 | pJ251-cat-PtpiA-accAD-PrpiA-accBC | 2 |
| PntAB | pACYC184 cat *B | E4152S | RE (Nrul, Pcil, blunted) self-ligate/ pACYC184-cat-PtpiA-accAD- PrpiA-accBCptalA-pntAB | pACYC184-cat-PtalA-pntAB | 133 |
| acccABCD/pntAB | pACYC184 cat *B | E41525 | RE/( EcoRV,Aval, BseB1, blunted: pACYC184), (BamHI, blunted: pJ244-pntAB-accABCD) | pACYC184-cat-PtpiA-accAD-PrpiA-accBC-ptalA-pntAB | 4 |
| accABCD/udhA | pACYC184 cat *B | E4152S | RE (Swal, Apal)/ pJ244-pTaI-udhA | pACYC184-cat-PtpiA-accAD-PrpiA-accBC-ptaIA-udhA | 5 |
| accABCD/T5- | pACYC184 | E4152S | RE (Swal, Ndel)/ | pACYC184- | 134 |
| udhA | cat *B | | pACYC184-cat-PtpiA-accAD-PrpiA-accBC PCR, RE (Pmel, Ndel)/ BX_00635 | cat-PtpiA-accADPrpiA-accBC-T5-udhA | |
| mcr/serA | pTRCHisA *A | V360-20 | RE (Pcil, blunted) for pTpiA serA insertion/ SEQ ID No. 0047 | pTrc-ptrc-mcr-kan-PtpiA-serA | 135 |
| FabF | pTRCHisA *A | V360-20 | PCR, RE (Ncol/Pstl)/ *E.coli* K12 genome | pTrc-ptrc-fabF-amp | 136 |
| Mcr | pACYC177 kan *B | E41515 | PCR (blunt)/ pTRC-ptrc mcr-amp | pACYC177-kan-ptrc-mcr | 137 |
| mcr/accABCD | pACYC 177 kan *B | E41515 | RE / (Swal, Xbal: pACYC 177 kan ptrc-mcr), (Pmel Xbal: pJ251-cat-PtpiA-accAD-PrpiA-accBC | pACYC 177-kan-ptrc-mcr-ptPIA accAD-PrpiA-accBC | 138 |

| | | | | | |
|---|---|---|---|---|---|
| *A: Invitrogen, Carlsbad, CA *B: New England Biolabs, Ipswich, MA *C: DNA 2.0, Menlo Park | | | | | |

### Part 5: Cloning of pACYC-cat-accABCD-PT5-udhA.

The Pfau promoter driving expression of udhA in pACYC-cat-accABCD-udhA was replaced with the stronger T5 promoter. The genomic PT5-udhA construct from strain BX_00635 - was amplified using primer AS 1170 (udhA 300 by upstream). See SEQ ID NO:139 for sequence of udhA). PCR fragments of PT5-udhA obtained above were digested with PmeI and NdeI (New England BioLabs, Ipswich, MA). Vector pACYC-cat¬accABCD-P,rudhA was similarly digested with SwaI and NdeI (New England BioLabs). The two digested DNA fragments were ligated and transformed to create pACYC-cat-accABCD-PT5-udhA (SEQ ID NO:140). Plasmid digests were used to confirm the correct sequence. This plasmid is incorporated into strains shown in Table 27.

### Part 6: Strain Construction

Using constructs made by the above methods, strains shown in Table 27, given the indicated Strain Names, were produced providing the genotypes. This is not meant to be limiting, and other strains may be made using these methods and following the teachings provided in this application, including providing different genes and gene regions for tolerance, and/or 3-HP production and modifications to modulate the fatty acid synthase system. Further to the latter, such strains may be produced by chromosomal modifications and/or introduction of non-chromosomal introductions, such as plasmids.

As to the latter, according to the respective combinations indicated in Table 27 below, the plasmids described above were introduced into the respective strains. All plasmids were introduced at the same time via electroporation using standard methods. Transformed cells were grown on the appropriate media with antibiotic supplementation and colonies were selected based on their appropriate growth on the selective media.

**Table 27**

| **Strain Name** | **Strain Genotvpe** |
|---|---|
| BW25113 | F-, A(araD-araB)567, AlacZ4787(::rrnB-3), LAM-, rph-1, A(rhaD-rhaB)568, hsdR514 |
| BX 0591 - | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt |
| 6X_0595 | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt, fablts (S241F)-zeoR |
| BX_0619 | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt, fablts (S241F)-zeoR, T5-pntAB¬BSD |
| BX_0634 | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt, fablts (S241F)-zeoR, T5-pntAB, T5-aceEF |
| BX_0635 | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt, fablts (S241F)-zeoR, T5-pntAB, T5-aceEF, T5-udhA-BSD |
| 6X_0636 | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt, fablts (S241F)-zeoR, T5-aceEF |
| BX_0637 | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt, fablts (S241F)-zeoR, T5-aceEF, T5-udhA-BSD |
| BX_0638 | F-, A(araD-araB)567, AlacZ4787(::rrnB-3), LAM-, rph-1, A(rhaD-rhaB)568, AldhA::frt, ApfIB::frt, AmgsA::frt, ApoxB::frt, Apta-ack::frt, fablts (S241F)-zeoR, T5-pntAB, T5-aceEF, AaldB::frt |
| BX_0639 | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt, fablts (S241F)-zeoR, T5-pntAB, T5-aceEF, AtrpR::kan |
| BX 0651 - | F-, A(araD-araB)567, AlacZ4787(::rrnB-3), LAM-, rph-1, A(rhaD-rhaB)568, hsdR514, fablts (S241F)-zeoR |
| BX_0652 | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt, fablts (S241F)-zeoR, T5-pntAB, T5-aceEF, T5-udhA, AarcA::kan |
| BX_0653 | F-, A(araD-araB)567, AlacZ4787(::rrnB-3), LAM-, rph-1, A(rhaD-rhaB)568, AldhA::frt, ApfIB::frt, AmgsA::frt, ApoxB::frt, Apta-ack::frt, fablts (S241F)-zeoR, T5-pntAB, T5-aceEF, T5-udhA, ApuuC::kan |
| BX_0654 | F-, A(araD-araB)567, AlacZ4787(::rrnB-3), LAM-, rph-1, A(rhaD-rhaB)568, hsdR514, AldhA::frt, ApfIB::frt, AmgsA::frt, ApoxB::frt, Apta-ack::frt, fablts (S241F)-zeoR, T5-pntAB, T5-aceEF, T5-udhA, AaldA::kan |

### Example 11B: Preparing a Genetically Modified E. coli Host Cell Comprising malonyl-CoA-reductase (Mcr) in Combination with Other Genetic Modifications to Increase 3-HP Production Relative to a Control E. coli Cell

Genetic modifications are made to introduce a vector comprising mmsB such as from Pseudomonas auruginos, which further is codon-optimized for E. coli. Vectors comprising galP and a native or mutated ppc also may be introduced by methods known to those skilled in the art (see, e.g., Sambrook and Russell, Molecular Cloning: A Laboratory Manual, Third Edition 2001 (volumes 1-3), Cold Spring Harbor Laboratory Press, Cold Spring Harbor, N.Y., "Sambrook and Russell, 2001"), additionally recognizing that mutations may be made by a method using the XL1-Red mutator strain, using appropriate materials following a manufacturer's instructions (Stratagene QuikChange Mutagenesis Kit, Stratagene, La Jolla, CA USA) and selected for or screened under standard protocols.

Also, genetic modifications are made to reduce or eliminate the enzymatic activities of E.coli genes as desired. These genetic modifications are achieved by using the RED/ET homologous recombination method with kits supplied by Gene Bridges (Gene Bridges GmbH, Dresden, Germany, <<www.genebridges.com>>) according to manufacturer's instructions.

Also, in some embodiments genetic modifications are made to increase the NADPH cellular pool. Non-limiting examples of some targets for genetic modification are provided herein. These are pgi (in a mutated form), pntAB, overexpressed, gapA:gapN substitution/replacement, and disrupting or modifying a soluble transhydrogenase such as sthA, and genetic modifications of one or more of zwf, gnd, and edd.

The so-genetically modified microorganism of any such engineered embodiment is evaluated and found to exhibit higher productivity of 3-HP compared with a control E. coli lacking said genetic modifications. Productivity is measured by standard metrics, such as volumetric productivity (grams of 3-HP/hour) under similar culture conditions.

### Example 11C: Mutational Development of Selected Polynucleotides

A selected gene sequence, such as a nucleic acid sequence that encodes for any of SEQ ID NOs:15 and 42-49, is subjected to a mutation development protocol, starting by constructing a mutant library of a native or previously evolved and/or codon-optimized polynucleotide by use of an error-inducing PCR site-directed mutagenesis method.

A polynucleotide exhibiting enzymatic activity of the selected gene (which may be any disclosed herein, e.g., an aminotransferase or mmsB) is cloned into an appropriate expression system for E. coli. This sequence may be codon optimized Cloning of a codon-optimized polynucleotide and its adequate expression will be accomplished via gene synthesis supplied from a commercial supplier using standard techniques. The gene will be synthesized with an eight amino acid C-terminal tag to enable affinity based protein purification. Once obtained using standard methodology, the gene will be cloned into an expression system using standard techniques.

The plasmid containing the above-described polynucleotide will be mutated by standard methods resulting in a large library of mutants (>106). The mutant sequences will be excised from these plasmids and again cloned into an expression vector, generating a final library of greater than 106 clones for subsequent screening. These numbers ensure a greater than 99% probability that the library will contain a mutation in every amino acid encoded by sequence. It is acknowledged that each method of creating a mutational library has its own biases, including transformation into mutator strains of E. coli, error prone PCR, and in addition more site directed muagenesis.

In some embodiments, various methods may be considered and possibly several explored in parallel. One such method is the use of the XL1-Red mutator strain, which is deficient in several repair mechanisms necessary for accurate DNA replication and generates mutations in plasmids at a rate 5,000 times that of the wild-type mutation rate, may be employed using appropriate materials following a manufacturer's instructions (See Stratagene QuikChange Mutagenesis Kit, Stratagene, La Jolla, CA USA). This technique or other techniques known to those skilled in the art, may be employed and then a population of such mutants, e.g., in a library, is evaluated, such as by a screening or selection method, to identify clones having a suitable or favorable mutation.

With the successful construction of a mutant library, it will be possible to screen this library for increased activity, such as increased malonyl-CoA reductase activity. The screening process will be designed to screen the entire library of greater than 106 mutants. This is done by screening methods suited to the particular enzymatic reaction.

### Example 12A: Evaluation of 3-HP Production

3-HP production by BX3_0194 was demonstrated at 100-mL scale in SM3 (minimal salts) media. Cultures were started from freezer stocks by standard practice (Sambrook and Russell, 2001) into 50 mL of LB media plus 100 µg/mL ampicillin and grown to stationary phase overnight at 37°C with rotation at 225 rpm. Five ml of this culture were transferred to 100 ml of SM3 media plus 40 g/L glucose, 100 kg/ml ampicillin, and 1 mM IPTG in triplicate 250-ml baffled flasks and incubated at 37°C, 225 rpm. To monitor cell growth and 3-HP production by these cultures, samples (2 ml) were withdrawn at designated time points for optical density measurements at 600nm (OD600, 1 cm pathlength) and pelleted by centrifugation at 12,000rpm for 5 min and the supernatant collected for analysis of 3-HP production as described under "Analysis of cultures for 3-HP production" in the Common Methods section. Dry cell weight (DCW) is calculated as 0.33 times the measured OD600 value, based on baseline DCW to OD600 determinations. All data are the average of triplicate cultures. For comparison purposes, the specific productivity is calculated from the averaged data at the 24-h time point and expressed as g 3-HP produced per gDCW. Under these conditions, no 3HP is produced after 24 hours in a culture growing to an OD600 that corresponds to approximately 1.0 g DCW. Production of 3-HP by strain BX3_0194 in SM3 medium is shown in Table 28.

**Table 28. Production of 3-HP by BX3_0194 in SM3 medium**

| **Time (hr)** | **3HP (gIL)** | **OD600** |
|---|---|---|
| 4 | 0 | 1.3 |
| 6 | 0 | 2.3 |
| 8 | 0 | 2.8 |
| 24 | 0 | 3.4 |

Production by strain BX3_0194 in SM3 medium in the presence of 10 .1,g/ml cerulenin is shown in Table 29. In the presence of cerulenin, an inhibitor of the fatty acid synthase system, internal pools of the malonyl-CoA precursor are proposed to increase thus leading to increased production of 3-HP. As may be seen by comparison to the results without cerulenin (Table 28), substantially more 3-HP is produced at every time point. Under these conditions, the specific productivity after 24 hours is 1.3 g 3HP per gDCW.

**Table 29. Production of 3-HP by BX3_0194 in SM3 medium and the presence of 10 .1,g/m1 cerulenin**

| **Time (hr)** | **3HP (gIL)** | **OD600** |
|---|---|---|
| 4 | 0.003 | 1.3 |
| 6 | 0.14 | 2.6 |
| 8 | 0.43 | 3.1 |
| 24 | 1.43 | 3.3 |

3-HP production by BX3_0195 was demonstrated at 100-mL scale in SM3 (minimal salts) media. Cultures were started from freezer stocks by standard practice (Sambrook and Russell, 2001) into 50 mL of LB media plus 100 µg/mL ampicillin and grown to stationary phase overnight at 37°C with rotation at 225 rpm. Five ml of this culture were transferred to 100 ml of SM3 media plus 40 g/L glucose, 100 .1,g/m1 ampicillin, and 1 mM IPTG in triplicate 250-ml baffled flasks and incubated at 37°C, 225 rpm. To monitor cell growth and 3-HP production by these cultures, samples (2 ml) were withdrawn at designated time points for optical density measurements at 600nm (OD600, 1 cm pathlength) and pelleted by centrifugation at 12,000rpm for 5 mM and the supernatant collected for analysis of 3-HP production as described under "Analysis of cultures for 3-HP production" in the Common Methods section. Dry cell weight (DCW) is calculated as 0.33 times the measured OD600 value, based on baseline DCW to OD600 determinations. All data are the average of triplicate cultures. For comparison purposes, the specific productivity is calculated from the averaged data at the 24-h time point and expressed as g 3-HP produced per gDCW. Under these conditions, no 3HP is produced after 24 hours in a culture growing to and OD 600 that corresponds to approximately 1.65 g DCW. Production of 3-HP by strain BX3_0195 in SM3 medium is shown in Table 30.

**Table 30. Production of 3-HP by BX3_0195 in SM3 medium**

| **Time (hr)** | **3HP (gIL)** | **OD600** |
|---|---|---|
| 4 | 0 | 0.92 |
| 6 | 0 | 1.35 |
| 8 | 0 | 2.36 |
| 24 | 0 | 5.00 |

Production by strain BX3_0195 in SM3 medium in the presence of 10 u.g/ml cerulenin is shown in Table 31. In the presence of cerulenin, an inhibitor of the fatty acid synthase system, internal pools of the malonyl-CoA precursor are proposed to increase thus leading to increased production of 3-HP. As may be seen by comparison to the results without cerulenin (Table 30), substantially more 3-HP is produced at every time point. Under these conditions, the specific productivity after 24 hours is 0.54 g 3HP per gDCW.

**Table 31. Production of 3-HP by BX3_0195 in SM3 medium and the presence of 10 .1,g/m1 cerulenin**

| **Time (hr)** | **3HP (gIL)** | **OD600** |
|---|---|---|
| 4 | 0.003 | 0.97 |
| 6 | 0.07 | 1.57 |
| 8 | 0.31 | 2.36 |
| 24 | 1.17 | 6.59 |

3-HP production by BX3_0206 was demonstrated at 100-mL scale in SM3 (minimal salts) media. Cultures were started from freezer stocks by standard practice (Sambrook and Russell, 2001) into 50 mL of LB media plus 35 µg/mL kanamycin and grown to stationary phase overnight at 37°C with rotation at 225 rpm. Five ml of this culture were transferred to 100 ml of SM3 media plus 40 g/L glucose and 35 .1,g/m1 kanamycin in triplicate 250-ml baffled flasks and incubated at 37°C, 225 rpm. To monitor cell growth and 3-HP production by these cultures, samples (2 ml) were withdrawn at designated time points for optical density measurements at 600nm (OD600, 1 cm pathlength) and pelleted by centrifugation at 12,000rpm for 5 min and the supernatant collected for analysis of 3-HP production as described under "Analysis of cultures for 3-HP production" in the Common Methods section. Dry cell weight (DCW) is calculated as 0.33 times the measured OD600 value, based on baseline DCW to OD600 determinations. All data are the average of triplicate cultures. For comparison purposes, the specific productivity is calculated from the averaged data at the 24-h time point and expressed as g 3-HP produced per gDCW. Under these conditions, the specific productivity after 24 hours is 0.05 g 3HP per gDCW. Production of 3-HP by strain BX3_0206 in SM3 medium is shown in Table 32.

**Table 32. Production of 3-HP by BX3_0206 in SM3 medium**

| **Time (hr)** | **3HP (gIL)** | **OD600** |
|---|---|---|
| 24 | 0.01 | 6.5 |

Production by strain BX3_0206 in SM3 medium in the presence of 10 .1,g/ml cerulenin is shown in Table 33. In the presence of cerulenin, an inhibitor of the fatty acid synthase system internal pools of the malonyl-CoA precursor are proposed to increase thus leading to increased production of 3-HP. As may be seen by comparison to the results without cerulenin (Table 32), substantially more 3-HP is produced after 24 hours. Under these conditions, the specific productivity after 24 hours is 0.20 g 3HP per gDCW, an approximately 40-fold increase relative to the results without cerulenin.

**Table 33. Production of 3-HP by BX3_0195 in SM3 medium and the presence of 10 ug/ml cerulenin**

| **Time (hr)** | **3HP (gIL)** | **OD600** |
|---|---|---|
| 24 | 0.43 | 6.4 |

### Example 12B: Evaluation of Strains for 3-HP Production

3-HP production in biocatalysts (strains) listed in the following table was demonstrated at 100-mL scale in SM3 (minimal salts) media. SM3 used is described under the Common Methods Section, but was supplemented with 200 mM MOPS. Cultures were started from LB plates containing antibiotics by standard practice (Sambrook and Russell, 2001) into 50 mL of TB media plus the appropriate antibiotic as indicated and grown to stationary phase overnight at 30°C with rotation at 250 rpm. Five ml of this culture were transferred to 100 ml of SM3 media plus 30 g/L glucose, antibiotic, and 1 mM IPTG (identified as "yes" under the "Induced" column) in triplicate 250-ml baffled flasks and incubated at 30°C, 250 rpm. Flasks were shifted to 37°C, 250 rpm after 4 hours. To monitor cell growth and 3-HP production by these cultures, samples (2 ml) were withdrawn at 24 hours for optical density measurements at 600nm (OD600, 1 cm pathlength) and pelleted by centrifugation at 14000 rpm for 5 mins and the supernatant collected for analysis of 3-HP production. 3-HP titer and standard deviation is expressed as g/L. Dry cell weight (DCW) is calculated as 0.33 times the measured OD600 value, based on baseline DCW per OD600 determinations. All data are the average of triplicate cultures. For comparison purposes, product to cell ratio is calculated from the averaged data over 24 hours and is expressed as g 3-HP produced per gDCW. The specific productivity is calculated from the cell/product ratio obtained over the 20 hours of production and expressed as g 3-HP produced per gDCW per hour.

**Table 34**

| **Strain Name** | **Strain Host** | **Plasmids** | **Induced** | **Average 24 Hour Titer** | **Standard Deviation** | **20 Hour Specific Productivit y** | **24 Hour Product/ Cell Ratio** |
|---|---|---|---|---|---|---|---|
| BX3 027 | BW2511 3 | 1) pTrc-ptrc-mcr-kan | yes | <0.001 | 0.000 | <0.001 | <0.001 |
| BX3_028 2 | BW2511 3 | 1)pTrc-ptrc-mcr-kan 2)pJ251-cat-PtpiA-accAD-PrpiA-accBC | yes | <0.001 | 0.000 | <0.001 | <0.001 |
| BX3_028 3 | BW2511 3 | 1)pTrc-ptrc-mcr-kan 2)pACYC18 4-cat-Pta1A-pntAB | yes | <0.001 | 0.000 | <0.001 | <0.001 |
| BX3_027 5 | BW2511 3 | 1)pTrc-ptrc-mcr-kan 2)pACYC18 4-cat¬PtpiA-accAD-PrpiA¬accB C-ptalA-pntAB | yes | <0.001 | 0.000 | <0.001 | <0.001 |
| BX3_028 4 | BW2511 3 | 1)pTrc-ptrc-mcr-kan 2)pACYC18 4-cat¬PtpiA-accAD-PrpiA¬accB C-ptalA-udhA | yes | <0.001 | 0.000 | <0.001 | <0.001 |
| BX3_028 5 | BX_005 91 | 1) pTrc-ptrc-mcr-kan | yes | <0.001 | 0.000 | <0.001 | <0.001 |
| BX3_028 6 | BX_005 91 | 1)pTrc-ptrc-mcr-kan 2) pJ251-cat-PtpiA-accAD-PrpiA-accBC | yes | <0.001 | <0.001 | 0.000 | <0.001 |
| BX3_028 7 | BX_005 91 | 1)pTrc-ptrc-mcr-kan 2)pACYC18 4-cat-PtalA-pntAB | yes | <0.001 | 0.000 | <0.001 | <0.001 |
| BX3_028 8 | BX_005 91 | 1)pTrc-ptrc-mcr-kan 2)pACYC18 4-cat¬PtpiA-accAD-PrpiA¬accB C-ptalA-pntAB | yes | <0.001 | 0.000 | <0.001 | <0.001 |
| BX3_028 9 | BX_005 91 | 1)pTrc-ptrc-mcr-kan 2)pACYC18 4-cat¬PtpiA-accAD-PrpiA¬accB C-ptalA-udhA | yes | <0.001 | 0.000 | <0.001 | <0.001 |
| BX3_023 9 | BX_005 95 | 1) pTrc-ptrc-mcr-kan | yes | 2.317 | 0.001 | 0.067 | 1.335 |
| BX3_026 1 | BX_005 95 | 1)pTrc-ptrc-mcr-kan 2)pJ251-cat-PtpiA | yes | 4.576 | 0.327 | 0.187 | 3.748 |
| | | accAD-PrpiA-accBC | | | | | |
| BX3_029 0 | BX_005 95 | 1) pIrc-ptrc-mcr-kan 2) pACYC184-cat-PtalA-pntAB | yes | 1.706 | 0.396 | 0.060 | 1.194 |
| BX3_024 0 | BX_005 95 | 1) pIrc-ptrc-mcr-kan 2) pACYC184-cat¬PtpiA-accAD-PrpiA¬accB C-ptalA-pntAB | yes | 5.878 | 0.684 | 0.228 | 4.563 |
| BX3_026 7 | BX_005 95 | 1) pIrc-ptrc-mcr-kan, 2)pACYC18 4-cat-PtpiA-accAD-PrpiA-accBC-ptalA-udhA | yes | 3.440 | 0.205 | 0.160 | 2.912 |
| BX3_025 3 | BX_006 19 | 1) pIrc-ptrc-mcr-kan | yes | 1.327 | 0.575 | 0.034 | 0.670 |
| BX3_025 4 | BX_006 19 | 1) pIrc-ptrc-mcr-kan 2)pJ251-cat-PtpiA-accAD-PrpiA-accBC | yes | 3.131 | 0.058 | 0.136 | 2.711 |
| BX3_026 3 | BX_006 19 | 1)pIrc-ptrc-mcr-kan | | | | | |
| 2) pACYC18 4-cat¬PtpiA-accAD-PrpiA¬acc BC-ptalA-pntAB | yes | 2.376 | 0.717 | 0.060 | 1.200 | | |
| BX3_026 8 | BX_006 19 | 1)pIrc-ptrc-mcr-kan 2) pACYC184-cat¬PtpiA-accAD-PrpiA ¬accB C-ptalA-udhA | yes | 5.555 | 0.265 | 0.240 | 4.809 |
| BX3_027 9 | BX_006 37 | 1) pIrc-ptrc-mcr-kan 2) pJ251-cat-PtpiA-accAD-PrpiA-accBC | yes | 3.640 | 0.210 | 0.154 | 3.073 |
| BX3_030 3 | BX_006 37 | 1) pIrc-ptrc-mcr-kan 2) pACYC184-cat-PtalA-pntAB | yes | 2.620 | 0.085 | 0.065 | 1.297 |
| BX3_028 1 | BX_006 37 | 1) pIrc-ptrc-mcr-kan 2) pACYC184-cat¬PtpiA-accAD-PrpiA¬accB C-ptalA-pntAB | yes | 4.700 | 0.271 | 0.209 | 4.177 |
| BX3_028 0 | BX_006 37 | 1) pIrc-ptrc-mcr-kan 2) pACYC184-cat-PtpiA-accAD-PrpiA¬accB C-ptalA-udhA | yes | 4.270 | 0.314 | 0.175 | 3.507 |
| BX3_027 6 | BX_006 35 | 1) pIrc-ptrc-mcr-kan 2) pJ251-cat-PtpiA-accAD-PrpiA-accBC | yes | 5.110 | 0.542 | 0.210 | 4.196 |
| BX3_030 4 | BX_006 35 | 1) pIrc-ptrc-mcr-kan 2) pACYC184-cat-PtalA-pntAB | yes | 2.430 | 0.147 | 0.076 | 1.512 |
| BX3_027 8 | BX_006 35 | 1) pIrc-ptrc-mcr-kan 2) pACYC184-cat-PtpiA-accAD-PrpiA¬accB C-ptalA-pntAB | yes | 0.790 | 0.015 | 0.034 | 0.672 |
| BX3_027 7 | BX_006 35 | 1) plrc-ptrc-mcr-kan 2) pACYC184-cat¬PtpiA-accAD-PrpiA¬accB C-ptalA-udhA | yes | 6.340 | 0.580 | 0.260 | 5.207 |
| BX3_029 | BX_006 | 1) pIrc-ptrc-mcr-kan | yes | 3.400 | 0.139 | 0.102 | 2.032 |
| BX3_029 7 | BX_006 BX 36 | 1) pIrc-ptrc-mcr-kan 2) pJ251-cat-PtpiA-accAD-PrpiA-accBC | yes | 1.830 | 0.144 | 0.069 | 1.376 |
| BX3_029 8 | BX_006 BX 36 | 1) pIrc-ptrc-mcr-kan 2) pACYC184-cat-PtalA-pntAB | yes | 2.670 | 0.065 | 0.081 | 1.628 |
| BX3_029 9 | BX_006 36 | 1) pIrc-ptrc-mcr-kan 2) pACYC184-cat¬PtpiA-accAD-PrpiA-accBC-pta1A-pntAB | yes | 3.200 | 0.418 | 0.121 | 2.412 |
| BX3_030 0 | BX_006 36 | 1) pIrc-ptrc-mcr-kan 2) pACYC184-cat¬PtpiA-accAD-PrpiA-accBC-pta1A-udhA | yes | 4.930 | 0.638 | 0.184 | 3.671 |
| BX3_029 1 | BX_006 34 | 1) pIrc-ptrc-mcr-kan | yes | 1.330 | 0.138 | 0.039 | 0.783 |
| BX3_029 2 | BX_006 34 | 1) pIrc-ptrc-mcr-kan 2) pJ251-cat-PtpiA-accAD-PrpiA-accBC | yes | 1.209 | 0.087 | 0.030 | 0.599 |
| BX3_029 3 | BX_006 34 | 1) pIrc-ptrc-mcr-kan 2) pACYC184-cat-PtalA-pntAB | yes | 0.269 | 0.035 | 0.006 | 0.124 |
| BX3_029 4 | BX_006 34 | 1) pIrc-ptrc-mcr-kan 2) pACYC184-cat¬PtpiA-accAD-PrpiA-accBC-ptalA-pntAB | yes | 1.588 | 0.136 | 0.046 | 0.927 |
| BX3_ 029 5 | BX_006 34 | 1) plrc-ptrc-mcr-kan 2) pACYC184-cat¬PtpiA-accAD-PrpiA-accBC-ptalA-udhA | yes | 1.054 | 0.048 | 0.028 | 0.552 |
| BX3_030 2 | BX_006 37 | 1) pIrc-ptrc-mcr-kan | yes | 3.710 | 0.221 | 0.118 | 2.352 |
| BX3_030 1 | BX_006 35 | 1) plrc-ptrc-mcr-kan | yes | 3.150 | 0.576 | 0.101 | 2.027 |
| BX3 030 5 | BW2511 3 | 1) plrc-ptrc-mcr-kan-cynTS 2) pACYC184-cat-PtpiA-accAD-PrpiA-accBC-ptalA-pntAB | yes | 0.006 | 0.006 | 0.000 | 0.003 |
| BX3 030 6 | BX 005 91 | 1) pIrc-ptrc-mcr-kan-cynTS 2) pACYC184-cat-PtpiA-accAD-PrpiA-accBC-ptalA-pntAB | yes | 0.035 | 0.035 | 0.001 | 0.014 |
| BX3_025 8 | BX 005 9-5 | 1) plrc-ptrc-mcr-kan-cynTS 2) pACYC184-cat-PtpiA-accAD-PrpiA-accBC-ptalA-pntAB | yes | 1.190 | 0.046 | 0.039 | 0.771 |
| BX3_030 8 | BX_006 34 | 1) pIrc-ptrc-mcr-kan-cynTS 2) pACYC184-cat-PtpiA-accAD-PrpiA accBC-ptalA-udhA | yes | 0.401 | 0.006 | 0.011 | 0.211 |
| BX3 031 0 | BX_006 37 | 1)pIrc-ptrc-mcr-kan-cynTS 2)pACYC18 4-cat-PtpiA-accAD-PrpiA-accBC-ptalA-udhA | yes | 1.450 | 0.072 | 0.045 | 0.897 |
| BX3 030 9 | BX_006 35 | 1) pIrc-ptrc-mcr-kan-cynTS 2) pACYC184-cat-PtpiA-accAD-PrpiA-accBC-ptalA-udhA | yes | 4.079 | 0.054 | 0.155 | 3.098 |
| BX3_031 1 | BX_006 38 | 1) pIrc-ptrc-mcr-kan 2) pACYC184-cat¬PtpiA-accAD-PrpiA¬accB C-ptalA-udhA | yes | 3.040 | 0.227 | 0.119 | 2.387 |
| BX3_ 031 2 | BX_ 006 39 | 1) pIrc-ptrc-mcr-amp 2) pACYC184-cat¬PtpiA-accAD-PrpiA¬accB C-ptalA-udhA | yes | 2.850 | 0.071 | 0.152 | 3.030 |
| BX3_035 2 | BX_065 1 | 1) pIrc-ptrc-mcr-kan | yes | <0.001 | 0.000 | <0.001 | NA |
| BX3_035 3 | BX_065 1 | 1) pIrc-ptrc-mcr-kan 2) pJ251-cat-PtpiA-accAD-PrpiA-accBC | yes | <0.001 | 0.000 | <0.001 | NA |
| BX3_031 3 | BX_006 35 | 1) pACYC177-kan-ptrc-mcr | no | 0.037 | 0.009 | 0.001 | 0.027 |
| BX3_031 3 | BX_006 35 | 1) pACYC177-kan-ptrc-mcr | yes | 0.031 | 0.009 | 0.001 | 0.023 |
| BX3 033 5 | BX BX_006 35 | 1) pACYC177-kan-ptrc-mcr-PtpiA-accAD-PrpiA - accBC | no | 0.037 | 0.021 | 0.001 | 0.020 |
| BX3 033 5 | BX_006 BX 35 | 1) pACYC177-kan-ptrc-mcr-PtpiA-accAD-PrpiA-accBC | yes | 0.037 | 0.021 | 0.001 | 0.020 |
| BX3_034 9 | BX_005 91 | 1) pIrc-ptrc-(366-1220)mcr-ptrc-ydfG-kan | yes | 0.057 | 0.006 | 0.001 | 0.025 |
| BX3_035 0 | BX_005 95 | 1) pIrc-ptrc-(366-1220)mcr-ptrc-ydfG-kan | yes | 1.163 | 0.045 | 0.023 | 0.457 |
| BX3_035 1 | BX_006 35 | 1) pTrc-ptrc-(366-1220)mcr-ptrc-ydfG-kan 2) pACYC184-cat-PtpiA-accAD-PrpiA-accBC-ptalA-udhA | yes | 0.658 | 0.060 | 0.020 | 0.390 |
| BX3_035 8 | BX_005 91 | 1) pIrc-ptrc-ydfG-ptrc-(496-1220)mcr-amp | yes | 0.040 | 0.000 | 0.001 | 0.015 |
| BX3_036 0 | BX_006 35 | 1) pTrc-ptrc-ydfG-ptrc-(496-1220)mcr-amp 2) pACYC184-cat- PtpiA-accAD-PrpiA - accBC-ptalA-udhA | yes | 4.027 | 0.185 | 0.138 | 2.761 |
| BX3_031 | BX_006 | 1) pIrc-ptrc-mcr-kan- | yes | 1.170 | 0.118 | 0.055 | 1.101 |
| 4 | 35 | PtpiA-serA 2) pACYC184-cat-PtpiA-accAD-PrpiA-accBC-ptalA-udhA | | | | | |
| BX3_031 5 | BX_005 91 | 1) pACYC177-kan-ptrc-mcr | no | 0.013 | 0.006 | 0.000 | 0.008 |
| BX3_031 6 | BX_005 95 | 1) pACYC177-kan-ptrc-mcr | no | 0.010 | 0.012 | 0.000 | 0.007 |
| BX3_033 3 | BX_005 91 | 1) pACYC177-kan¬ptrc-mcr-PtpiA-accAD-PrpiA-accBC | no | 0.005 | 0.004 | 0.000 | 0.002 |
| BX3_033 4 | BX_005 BX 9-5 | 1) pACYC177-kan-ptrc-mcr-PtpiA-accAD-PrpiA-accBC | no | 0.300 | 0.013 | 0.007 | 0.134 |
| BX3_031 7 | BX_005 91 | 1) pACYC177-kan-ptrc-mcr 2) pIrc-ptrc-fabF-amp | no | <0.001 | 0.000 | <0.2 | <0.2 |
| BX3_031 7 | BX_005 91 | 1) pACYC177-kan-ptrc-mcr 2) pIrc-ptrc-fabF-amp | yes | 0.033 | 0.024 | 0.001 | 0.021 |
| BX3_033 8 | BX_005 91 | 1) pACYC177-kan¬ptrc-mcr-PtpiA-accAD¬Prpi A-accBC 2) pIrc-ptrc-fabF-amp | no | 0.010 | 0.005 | 0.000 | 0.004 |
| BX3_033 8 | BX_005 91 | 1) pACYC177-kan¬ptrc-mcr-PtpiA-accAD¬Prpi A-accBC 2) pIrc-ptrc-fabF-amp | yes | 1.580 | 0.142 | 0.006 | 0.116 |
| BX3_031 8 | BX_005 95 | 1) pACYC177-kan-ptrc-mcr 2) pIrc-ptrc-fabF-amp | no | 0.161 | 0.013 | 0.005 | 0.097 |
| BX3_031 8 | BX_005 95 | 1) pACYC177-kan-ptrc-mcr 2) pIrc-ptrc-fabF-amp | yes | 1.330 | 0.101 | 0.049 | 0.976 |
| BX3_033 9 | BX_005 95 | 1) pACYC177-kan¬ptrc-mcr-PtpiA-accAD¬Prpi A-accBC 2) pIrc-ptrc-fabF-amp | no | 0.083 | 0.015 | 0.007 | 0.149 |
| BX3_033 9 | BX_005 95 | 1) pACYC177-kan¬ptrc-mcr-PtpiA-accAD¬Prpi A-accBC 2) pIrc-ptrc-fabF-amp | yes | 0.010 | 0.009 | 0.000 | 0.007 |
| BX3_031 9 | BX_006 35 | 1) pACYC177-kan-ptrc-mcr 2) pIrc-ptrc-fabF-amp | no | 0.120 | 0.008 | 0.005 | 0.094 |
| BX3_031 9 | BX BX_006 3-5 | 1) pACYC177-kan-ptrc-mcr 2) pIrc-ptrc-fabF-amp | yes | 1.068 | 0.450 | 0.043 | 0.854 |
| BX3 034 1 | BX_006 35 | 1) pACYC177-kan¬ptrc-mcr-PtpiA-accAD¬Prpi A-accBC 2) pIrc-ptrc-fabF-amp | no | 0.327 | 0.021 | 0.009 | 0.171 |
| BX3_ 034 | BX_ 006 | 1) | yes | 0.140 | 0.017 | 0.015 | 0.293 |
| 1 | 35 | pACYC177-kan-ptrc-mcr-PtpiA-accAD PrpiA - accBC 2) pTrc-ptrc-fabF-amp | | | | | |
| BX3_034 2 | BX_006 35 | 1) pTrc-ptrc-mcr-kan 2) pACYC18 4-cat¬PtpiA-accAD-PrpiA¬acc BC-T5-udhA | yes | 0.341 | 0.055 | 0.009 | 0.188 |
| BX3 034 3 | BX_006 35 | 1) pTrc-ptrc-mcr-kan-cynTS 2) pACYC18 4-cat-PtpiA-accAD-PrpiA-accBC-T5-udhA | yes | 1.927 | 0.047 | 0.077 | 1.536 |
| BX3_034 4 | BX_006 52 | 1) pTrc-ptrc-mcr-amp | yes | 1.562 | 0.280 | 0.040 | 0.797 |
| BX3_034 5 | BX_006 52 | 1) pTrc-ptrc-mcr-amp 2) | yes | 5.195 | 0.229 | 0.184 | 3.678 |
| | | pJ251-cat-PtpiA-accAD-PrpiA-accBC | | | | | |
| BX3_034 6 | BX_006 52 | 1) pTrc-ptrc-mcr-amp 2) pACYC18 4-cat¬PtpiA-accAD-PrpiA ¬acc BC-ptalA-udhA | yes | 1.781 | 0.132 | 0.056 | 1.119 |
| BX3_034 7 | BX_006 53 | 1) pTrc-ptrc-mcr-amp 2) pACYC18 4-cat¬PtpiA-accAD-PrpiA¬acc BC-ptalA-udhA | yes | 1.370 | 0.307 | 0.049 | 0.977 |
| BX3_034 8 | BX_006 54 | 1) pTrc-ptrc-mcr-amp 2) pACYC18 4-cat¬PtpiA-accAD-PrpiA¬acc BC-ptalA-udhA | yes | 1.387 | 0.184 | 0.049 | 0.982 |
| BX3_032 4 | BX_005 91 | 1) pTrc-ptrc-Ebmcr-amp | yes | 0.009 | 0.002 | 0.000 | 0.004 |
| BX3_032 8 | BX_005 95 | 1) pTrc-ptrc-Ebmcr-amp | yes | 0.011 | 0.005 | 0.000 | 0.006 |

### Example 12C: Evaluation of BX3_240 Strain with Carbonate Addition

3-HP production in E. coli BX3_240 (made by methods above) was evaluated at 100-mL scale in SM3 (minimal salts) media having added sodium carbonate. SM3 used is described under the Common Methods Section, to which was added 10mM, 20mM and 50mM Na2CO3 as treatments. Cultures were started from LB plates containing antibiotics by standard practice (Sambrook and Russell, 2001) into 50 mL of TB media plus the appropriate antibiotics kan and cat and grown to stationary phase overnight at 30°C with rotation at 250 rpm. Five ml of this culture were transferred to 100 ml of SM3 media plus 30 g/L glucose, antibiotic, the indicated sodium carbonate, 0.1% yeast extract and 1 mM IPTG in triplicate 250-ml baffled flasks and incubated at 30°C, 250 rpm. Flasks were shifted to 37°C, 250 rpm after 4 hours. To monitor cell growth and 3-HP production by these cultures, samples (2 ml) were withdrawn at 24, 48 and 60 hours for optical density measurements at 600nm (0D600, 1 cm path length) and pelleted by centrifugation at 14000 rpm for 5 min and the supernatant collected for analysis of 3-HP production as described under "Analysis of cultures for 3-HP production" in the Common Methods section. 3-HP titer and standard deviation is expressed as g/L. Dry cell weight (DCW) is calculated as 0.33 times the measured OD₆₀₀ value, based on baseline DCW per OD₆₀₀ determinations. All data are the average of triplicate cultures. For comparison purposes, product to cell ratio is calculated from the averaged data over 60 hours and is expressed as g 3-HP produced per gDCW.

3-HP titer were 0.32 (+/- 0.03), 0.87 (+/- 0.10), 2.24 (+/- 0.03), 4.15 (+/- 0.27), 6.24 (+/-0.51), 7.50 (+/- 0.55) and 8.03 (+/-0.14) g/L at 9, 11, 15, 19, 24, 48 and 60 hr, respectively. Biomass concentrations were 0.54 (+/- 0.02), 0.79 (+/- 0.03), 1.03 (+/- 0.06), 1.18 (+/- 0.04), 1.20 (+/- 0.12), 1.74 (+/- 0.30) and 1.84 (+/¬0.22) at 9, 11, 15, 19, 24, 48 and 60 hr, respectively. Maximum product to cell ratio was 4.6 g 3-HP/g DCW.

### Example 13A: General example of genetic modification to a host cell

In addition to the above specific examples, this example is meant to describe a non-limiting approach to genetic modification of a selected microorganism to introduce a nucleic acid sequence of interest. Alternatives and variations are provided within this general example. The methods of this example are conducted to achieve a combination of desired genetic modifications in a selected microorganism species, such as a combination of genetic modifications as described in sections herein, and their functional equivalents, such as in other bacterial and other microorganism species.

A gene or other nucleic acid sequence segment of interest is identified in a particular species (such as E. coli as described herein) and a nucleic acid sequence comprising that gene or segment is obtained.

Based on the nucleic acid sequences at the ends of or adjacent the ends of the segment of interest, 5' and 3' nucleic acid primers are prepared. Each primer is designed to have a sufficient overlap section that hybridizes with such ends or adjacent regions. Such primers may include enzyme recognition sites for restriction digest of transposase insertion that could be used for subsequent vector incorporation or genomic insertion. These sites are typically designed to be outward of the hybridizing overlap sections. Numerous contract services are known that prepare primer sequences to order (e.g., Integrated DNA Technologies, Coralville, IA USA).

Once primers are designed and prepared, polymerase chain reaction (PCR) is conducted to specifically amplify the desired segment of interest. This method results in multiple copies of the region of interest separated from the microorganism's genome. The microorganism's DNA, the primers, and a thermophilic polymerase are combined in a buffer solution with potassium and divalent cations (e.g., Mg or Mn) and with sufficient quantities of deoxynucleoside triphosphate molecules. This mixture is exposed to a standard regimen of temperature increases and decreases. However, temperatures, components, concentrations, and cycle times may vary according to the reaction according to length of the sequence to be copied, annealing temperature approximations and other factors known or readily learned through routine experimentation by one skilled in the art.

In an alternative embodiment the segment of interest may be synthesized, such as by a commercial vendor, and prepared via PCR, rather than obtaining from a microorganism or other natural source of DNA.

The nucleic acid sequences then are purified and separated, such as on an agarose gel via electrophoresis. Optionally, once the region is purified it can be validated by standard DNA sequencing methodology and may be introduced into a vector. Any of a number of vectors may be used, which generally comprise markers known to those skilled in the art, and standard methodologies are routinely employed for such introduction. Commonly used vector systems are pSMART (Lucigen, Middleton, WI), pET E. coli EXPRESSION SYSTEM (Stratagene, La Jolla, CA), pSC-B StrataClone Vector (Stratagene, La Jolla, CA), pRANGER-BTB vectors (Lucigen, Middleton, WI), and TOPO vector (Invitrogen Corp, Carlsbad, CA, USA). Similarly, the vector then is introduced into any of a number of host cells. Commonly used host cells are E. cloni 100 (Lucigen, Middleton, WI), E. cloni 10GF' (Lucigen, Middleton, WI), StrataClone Competent cells (Stratagene, La Jolla, CA), E. coli BL21, E. coli BW25113, and E. coli K12 MG1655. Some of these vectors possess promoters, such as inducible promoters, adjacent the region into which the sequence of interest is inserted (such as into a multiple cloning site), while other vectors, such as pSMART vectors (Lucigen, Middleton, WI), are provided without promoters and with dephosporylated blunt ends. The culturing of such plasmid-laden cells permits plasmid replication and thus replication of the segment of interest, which often corresponds to expression of the segment of interest.

Various vector systems comprise a selectable marker, such as an expressible gene encoding a protein needed for growth or survival under defined conditions. Common selectable markers contained on backbone vector sequences include genes that encode for one or more proteins required for antibiotic resistance as well as genes required to complement auxotrophic deficiencies or supply critical nutrients not present or available in a particular culture media. Vectors also comprise a replication system suitable for a host cell of interest.

The plasmids containing the segment of interest can then be isolated by routine methods and are available for introduction into other microorganism host cells of interest. Various methods of introduction are known in the art and can include vector introduction or genomic integration. In various alternative embodiments the DNA segment of interest may be separated from other plasmid DNA if the former will be introduced into a host cell of interest by means other than such plasmid.

While steps of the general example involve use of plasmids, other vectors known in the art may be used instead. These include cosmids, viruses (e.g., bacteriophage, animal viruses, plant viruses), and artificial chromosomes (e.g., yeast artificial chromosomes (YAC) and bacteria artificial chromosomes (BAC).

Host cells into which the segment of interest is introduced may be evaluated for performance as to a particular enzymatic step, and/or tolerance or bio-production of a chemical compound of interest. Selections of better performing genetically modified host cells may be made, selecting for overall performance, tolerance, or production or accumulation of the chemical of interest.

It is noted that this procedure may incorporate a nucleic acid sequence for a single gene (or other nucleic acid sequence segment of interest), or multiple genes (under control of separate promoters or a single promoter), and the procedure may be repeated to create the desired heterologous nucleic acid sequences in expression vectors, which are then supplied to a selected microorganism so as to have, for example, a desired complement of enzymatic conversion step functionality for any of the herein-disclosed metabolic pathways. However, it is noted that although many approaches rely on expression via transcription of all or part of the sequence of interest, and then translation of the transcribed mRNA to yield a polypeptide such as an enzyme, certain sequences of interest may exert an effect by means other than such expression.

The specific laboratory methods used for these approaches are well-known in the art and may be found in various references known to those skilled in the art, such as Sambrook and Russell, Molecular Cloning: A Laboratory Manual, Third Edition 2001 (volumes 1-3), Cold Spring Harbor Laboratory Press, Cold Spring Harbor, N.Y. (hereinafter, Sambrook and Russell, 2001).

As an alternative to the above, other genetic modifications may also be practiced, such as a deletion of a nucleic acid sequence of the host cell's genome. One non-limiting method to achieve this is by use of Red/ET recombination, known to those of ordinary skill in the art and described in U.S. Patent Nos. 6,355,412 and 6,509,156, issued to Stewart et al. and incorporated by reference herein for its teachings of this method. Material and kits for such method are available from Gene Bridges (Gene Bridges GmbH, Dresden, Germany, <<www.genebridges.com>>), and the method may proceed by following the manufacturer's instructions. Targeted deletion of genomic DNA may be practiced to alter a host cell's metabolism so as to reduce or eliminate production of undesired metabolic products. This may be used in combination with other genetic modifications such as described herein in this general example.

### Example 13B. Utilization of sucrose as the feedstock for production of 3-HP and other products

Common laboratory and industrial strains of E. coli, such as the strains described herein, are not capable of utilizing sucrose as the sole carbon source, although this property is found in a number of wild strains, including pathogenic E. coli strains. Sucrose, and sucrose-containing feedstocks such as molasses, are abundant and often used as feedstocks for the production by microbial fermentation of organic acids, amino acids, vitamins, and other products. Thus further derivatives of the 3-HP-producing strains that are capable of utilizing sucrose would expand the range of feedstocks that can be utilized to produce 3-HP.

Various sucrose uptake and metabolism systems are known in the art (for example, U.S. Pat. No. 6,960,455), incorporated by reference for such teachings. We describe the construction of E. coli strains that harbor the csc genes confering the ability to utilize sucrose via a non-phosphotransferase system, wherein the csc genes constitute cscA, encoding a sucrose hydrolase, cscB, encoding a sucrose permease, cscK, encoding a fructokinase, and cscR, encoding a repressor. The sequences of these genes are annotated in the NCBI database as accession No. X81461 AF473544. To allow efficient expression utilizing codons that are highly abundant in E. coli genes, an operon containing cscB, cscK, and cscA was designed and synthesized using the services of a commercial synthetic DNA provider (DNA 2.0, Menlo Park, CA). The amino acid sequences of the genes are set forth as, respectively, cscB - SEQ. ID. No. 141; cscA - SEQ. ID. No. 142; csck - SEQ. ID. No. 143. The synthetic operon consisted of 60 base pairs of the region of the E. coli genome immediately 5' (upstream) of the adhE gene, a consensus strong promoter to drive expression of the csc genes, the coding regions for cscB, cscK, and cscA with short intergenic regions containing ribosome binding sites but no promoters, and 60 by immediately 3' (downstream) of the adhE gene. The segments homologous to sequences flanking the adhE gene will be used to target insertion of the csc operon genes into the E. coli chromosome, with the concomittent deletion of adhE. The nucleotide sequence of the entire synthetic construct is shown as SEQ. ID. No. 144. The synthetic csc operon is constructed in plasmid pJ214 (DNA 2.0, Menlo Park, CA) that provides an origin of replication derived from plasmid p15A and a gene conferring resistance to ampicillin. This plasmid is denoted pSUCR. A suitable host cell, such as E. coli strain BX_595, is transformed simultaneously with pSUCR and with plasmid pTrc_kan_mcr or other suitable plasmid, and transformed strains selected for on LB medium plates containing ampicillin and kanamycin. Transformants carrying both plasmids are grown and evaluated for 3-HP production in shake flasks as described, except that the glucose in SM3 medium is replaced with an equal concentration of sucrose.

Genes that confer functions to enable utilization of sucrose by E. coli can also be obtained from the natural isolate pUR400 (Cowan, P.J., et al. J. Bacteriol. 173:7464-7470, 1991) which carries genes for the phosphoenolpyruvate-dependent carbohydrate uptake phosphotransferase system (PTS). These genes consist of scrA, encoding the enzyme II component of the PTS transport complex, scrB, encoding sucrose-6 phosphate hydrolase, scrK, encoding fructokinase, and scrY, encoding a porin. These genes may be isolated or synthesized as described above, incorporated on a plasmid, and transformed into a suitable host cell, such as E. coli strain BX_595, simultaneously with plasmid pTrc_kan_mcr or other suitable plasmid, and transformed strains selected for on LB medium plates containing the appropriate antibiotics. Transformants carrying both plasmids are grown and evaluated for 3-HP production in shake flasks as described, except that the glucose in SM3 medium is replaced with an equal concentration of sucrose.

### Example 13C: Construction and Evaluation of Additional Strains

Other strains are produced that comprise various combinations of the genetic elements (additions, deletions and modifications) described herein are evaluated for and used for 3-HP production, including commercial-scale production. The following table illustrates a number of these strains.

Additionally, a further deletion or other modification to reduce enzymatic activity, of multifunctional 2-keto-3-deoxygluconate 6-phosphate aldolase and 2-keto-4-hydroxyglutarate aldolase and oxaloacetate decarboxylase (eda in E. coli), may be provided to various strains. Further to the latter, in various embodiments combined with such reduction of enzymatic activity of multifunctional 2-keto-3-deoxygluconate 6-phosphate aldolase and 2-keto-4-hydroxyglutarate aldolase and oxaloacetate decarboxylase (eda in E. coli), further genetic modifications may be made to increase a glucose transporter (e.g. galP in E. coli) and/or to decrease activity of one or more of heat stable, histidyl phosphorylatable protein (of PTS) (ptsH (HPr) in E. coli), phosphoryl transfer protein (of PTS) (ptsl in E. coli), and the polypeptide chain of PTS (Crr in E. coli).

These strains are evaluated in either flasks, or fermentors, using the methods described above. Also, it is noted that after a given extent of evaluation of strains that comprise introduced plasmids, the genetic elements in the plasmids may be introduced into the microorganism genome, such as by methods described herein as well as other methods known to those skilled in the art.

**Table 35**

| **Strain** | **Host** | **Plasmids** |
|---|---|---|
| BX3P_001 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoR T5 aceEF, T5-pntAB, T5-udhA, fabB-tS | 1) ptrc-mcr |
| BX3P_002 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoR T5 aceEF, T5-pntAB, T5-udhA, fabB-tS | 1) ptrc-mcr, 2)accABCD |
| BX3P_003 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoR T5 aceEF, T5-pntAB, T5-udhA, fabB-tS | 1) ptrc-mcr, 2)accABCD-udhA |
| BX3P-004 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, relA, spoT | 1) ptrc-mcr |
| BX3P-005 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta ack::frt, fablts (S241F)-zeoRT5 aceEF, T5-pntAB, T5-udhA, relA, spoT | 1) ptrc-mcr, 2)accABCD |
| BX3P_006 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, relA, spoT | 1) ptrc-mcr, 2)accABCD udhA |
| BX3P_007 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, del-arcA:kan | 1) ptrc-mcr |
| BX3P_008 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, del-arcA:kan | 1) ptrc-mcr, 2)accABCD |
| BX3P_009 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, del-arcA:kan | 1) ptrc-mcr, 2)accABCD-udhA |
| BX3P-010 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, del-aldA, del puuC, del arcA, del aldB, spoT, relA, T5-cynTS | 1) ptrc-mcr |
| BX3P-011 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, del-aldA, del puuC, del arcA, del aldB, spoT, relA, T5-cynTS | 1) ptrc-mcr, 2)accABCD |
| BX3P-012 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta ack::frt, fablts (S241F)-zeoRT5 aceEF, T5-pntAB, T5-udhA, del-aldA, del puuC, del arcA, del aldB, spoT, relA, T5-cynTS | 1) ptrc-mcr, 2)accABCD-udhA |
| BX3P_013 | F-, A(araD-araB)567, AlacZ4787(::rrnB-3), LAM-, rph-1, A(rhaDrhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, del-aldA, del puuC, del arcA, del aldB, spoT, relA, T5-cynTS, fabBts | 1) ptrc-mcr |
| BX3P_014 | F-, A(araD-araB)567, AlacZ4787(::rrnB-3), LAM-, rph-1, A(rhaDrhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, del-aldA, del puuC, del arcA, del aldB, spoT, relA, T5-cynTS, fabB- is | 1) ptrc-mcr, 2)accABCD |
| BX3P_015 | F-, A(araD-araB)567, AlacZ4787(::rrnB-3), LAM-, rph-1, A(rhaDrhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, del-aldA, del puuC, del arcA, del aldB, spoT, relA, T5-cynTS, fabB- is | 1) ptrc-mcr, 2)accABCD-udhA |
| BX3P-016 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, T5-cynTS | 1) ptrc-mcr |
| BX3P-017 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, T5-cynTS | 1) ptrc-mcr, 2)accABCD |
| BX3P_018 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, T5-cynTS | 1) ptrc-mcr, 2)accABCD-udhA |
| BX3P_019 | F-, A(araD-araB)567, AlacZ4787(::rrnB-3), LAM-, rph-1, A(rhaD-rhaB)568, hsdR514, AldhA::frt, ApflB::frt, AnagsA::frt, ApoxB::frt, Apta-ack::frt, fablts (S241F)-zeoRT5 aceEF, T5-pntAB, T5-udhA, del puuC, del arcA, del aldB, spoT, relA, T5-cynTS | 1) ptrc-mcr |
| BX3P_ 020 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA, del puuC, del arcA, del aldB, spoT, relA, T5-cynTS | 1) ptrc-mcr, 2)accABCD |
| BX3P_021 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, Apta-ack::frt, fablts (S241F)-zeoRT5 aceEF, T5-pntAB, T5-udhA del puuC, del arcA, del aldB, spoT, relA, T5-cynTS | 1) ptrc-mcr, 2)accABCD-udhA |
| BX3P_022 | F-, A(araD-araB)567, AlacZ4787(::rrnB-3), LAM-, rph-1, A(rhaD-rhaB)568, hsdR514, AldhA::frt, ApflB::frt, AnagsA::frt, ApoxB::frt, Apta-ack::frt, fablts (S241F)-zeoRT5 aceEF, T5-pntAB, T5-udhA, del-aldA, del puuC, del aldB, spoT, relA, T5-cynTS, fabB-ts | 1) ptrc-mcr |
| BX3P_023 | F-, A(araD-araB)567, AlacZ4787(::rrnB-3), LAM-, rph-1, A(rhaD-rhaB)568, hsdR514, AldhA::frt, ApflB::frt, AmgsA::frt, ApoxB::frt, | 1) ptrc-mcr, 2)accABCD |
| | Apta-ack::frt, fablts (S241F)-zeoRT5 aceEF, T5-pntAB, T5-udhA, del-aldA, del puuC, del aldB, spoT, relA, T5-cynTS, fabB-ts | |
| BX3P_024 | F-, A(araD-araB)567, AlacZ4787(::rrnB-3), LAM-, rph-1, A(rhaD-rhaB)568, hsdR514, AldhA::frt, ApflB::frt, AnagsA::frt, ApoxB::frt, Apta-ack::frt, fablts (S241F)-zeoRT5 aceEF, T5-pntAB, T5-udhA del-aldA, del puuC, del aldB, spoT, relA, T5-cynTS, fabB-ts | 1) ptrc-mcr, 2)accABCD-udhA |
| BX3P_025 | F-, A(araD-araB)567, AlacZ4787(::rrnB-3), LAM-, rph-1, A(rhaD-rhaB)568, hsdR514, AldhA::frt, ApflB::frt, AnagsA::frt, ApoxB::frt, Apta-ack::frt, fablts (S241F)-zeoR, T5-pntAB, T5-aceEF, T5-udhABSD | 1) pACYCmcr-accABCD, 2) pKK223- metE C645A |
| BX3P_026 | F-, A(araD-araB)567, AlacZ4787(::rrnB-3), LAM-, rph-1, A(rhaD-rhaB)568, hsdR514, AldhA::frt, ApflB::frt, AnagsA::frt, ApoxB::frt, Apta-ack::frt, fablts (S241F)-zeoR, T5-pntAB, T5-aceEF, T5-udhABSD | 1) pACYCmcr-accABCD, 2) pKK223-ct his-thrA |
| BX3P_027 | F-, A(araD-araB)567, AlacZ4787(::rrnB-3), LAM-, rph-1, A(rhaD-rhaB)568, hsdR514, AldhA::frt, ApflB::frt, AnagsA::frt, ApoxB::frt Apta-ack::frt, fablts (S241F)-zeoR, T5-pntAB, T5-aceEF, T5-udhA-BSD | 1) pACYCmcr-accABCD, 2) pKK223-aroH*457 |
| BX3P_028 | F-, A(araD-araB)567, AlacZ4787(::rrnB-3), LAM-, rph-1, A(rhaD-rhaB)568, hsdR514, AldhA::frt, ApflB::frt, AnagsA::frt, ApoxB::frt Apta-ack::frt, fablts (S241F)-zeoR, T5-pntAB, T5-aceEF, T5-udhA-BSD | 1) pACYCmcr-accABCD, 2) psmart-hcamp-cadA |
| BX3P_ 029 | F-, A(araD-araB)567, AlacZ4787(::rrnB-3), LAM-, rph-1, A(rhaD-rhaB)568, hsdR514, AldhA::frt, ApflB::frt, AnagsA::frt, ApoxB::frt Apta-ack::frt, fablts (S241F)-zeoR, T5-pntAB, T5-aceEF, T5-udhA-BSD | 1) pACYCmcr-accABCD, 2) psmart-hcamp-metC |
| BX3P_ 030 | F-, A(araD-araB)567, AlacZ4787(::rrnB-3), LAM-, rph-1, A(rhaD-rhaB)568, hsdR514, AldhA::frt, ApflB::frt, AnagsA::frt, ApoxB::frt, Apta-ack::frt, fablts (S241F)-zeoR, T5-pntAB, T5-aceEF, T5-udhA- BSD | 1) pACYCmcr-accABCD, 2) psmart-hcampnrdAB |
| BX3P_031 | F-, A(araD-araB)567, AlacZ4787(::rrnB-3), LAM-, rph-1, A(rhaD-rhaB)568, hsdR514, AldhA::frt, ApflB::frt, AnagsA::frt, ApoxB::frt Apta-ack::frt, fablts (S241F)-zeoR, T5-pntAB, T5-aceEF, BSD | 1) pACYCmcr-accABCD, 2) psmart-hcamp-prs |

### Example 14: 3-HP Production

An inoculum of a genetically modified microorganism that possesses a 3-HP production pathway and other genetic modifications as described above is provided to a culture vessel to which also is provided a liquid media comprising nutrients at concentrations sufficient for a desired bio-process culture period.

The final broth (comprising microorganism cells, largely 'spent' media and 3-HP, the latter at concentrations, in various embodiments, exceeding 1, 2, 5, 10, 30, 50, 75 or 100 grams/liter) is collected and subjected to separation and purification steps so that 3-HP is obtained in a relatively purified state. Separation and purification steps may proceed by any of a number of approaches combining various methodologies, which may include centrifugation, concentration, filtration, reduced pressure evaporation, liquid/liquid phase separation (including after forming a polyamine-3-HP complex, such as with a tertiary amine such as CAS#68814-95-9, Alamine® 336, a triC8-10 alkyl amine (Cognis, Cincinnati, OH or Henkel Corp.), membranes, distillation, and/or other methodologies recited in this patent application, incorporated herein. Principles and details of standard separation and purification steps are known in the art, for example in "Bioseparations Science and Engineering," Roger G. Harrison et al., Oxford University Press (2003), and Membrane Separations in the Recovery of Biofuels and Biochemicals-An Update Review, Stephen A. Leeper, pp. 99-194, in Separation and Purification Technology, Norman N. Li and Joseph M. Calo, Eds., Marcel Dekker (1992), incorporated herein for such teachings. The particular combination of methodologies is selected from those described herein, and in part is based on the concentration of 3-HP and other components in the final broth.

### Example 15: Genetic modification/introduction of Malonyl-CoA reductase for 3-HP production in Bacillus subtilis

For creation of a 3-HP production pathway in Bacillus Subtilis the codon optimized nucleotide sequence for the malonyl-CoA reductase gene from Chloroflexus aurantiacus that was constructed by the gene synthesis service from DNA 2.0 (Menlo Park, CA USA), a commercial DNA gene synthesis provider, was added to a Bacillus Subtilis shuttle vector. This shuttle vector, pHT08 (SEQ ID NO:17), was obtained from Boca Scientific (Boca Raton, FL USA) and carries an inducible Pgrac IPTG-inducible promoter.

This mcr gene sequence was prepared for insertion into the pHT08 shuttle vector by polymerase chain reaction amplification with primer 1 (5'GGAAGGATCCATGTCCGGTACGGGTCG-3') (SEQ ID NO:18), which contains homology to the start site of the mcr gene and a BamHI restriction site, and primer 2 (5'-Phos-GGGATTAGACGGTAATCGCACGACCG-3') (SEQ ID NO:19), which contains the stop codon of the mcr gene and a phosphorylated 5' terminus for blunt ligation cloning. The polymerase chain reaction product was purified using a PCR purification kit obtained from Qiagen Corporation (Valencia, CA USA) according to manufacturer's instructions. Next, the purified product was digested with BamHI obtained from New England BioLabs (Ipswich, MA USA) according to manufacturer's instructions. The digestion mixture was separated by agarose gel electrophoresis, and visualized under UV transillumination as described in Subsection II of the Common Methods Section. An agarose gel slice containing a DNA piece corresponding to the mcr gene was cut from the gel and the DNA recovered with a standard gel extraction protocol and components from Qiagen (Valencia, CA USA) according to manufacturer's instructions.

This pHT08 shuttle vector DNA was isolated using a standard miniprep DNA purification kit from Qiagen (Valencia, CA USA) according to manufacturer's instructions. The resulting DNA was restriction digested with BamHI and Smal obtained from New England BioLabs (Ipswich, MA USA) according to manufacturer's instructions. The digestion mixture was separated by agarose gel electrophoresis, and visualized under UV transillumination as described in Subsection II of the Common Methods Section. An agarose gel slice containing a DNA piece corresponding to digested pHT08 backbone product was cut from the gel and the DNA recovered with a standard gel extraction protocol and components from Qiagen (Valencia, CA USA) according to manufacturer's instructions.

Both the digested and purified mcr and pHT08 products were ligated together using T4 ligase obtained from New England BioLabs (Ipswich, MA USA) according to manufacturer's instructions. The ligation mixture was then transformed into chemically competent 100 E. coli cells obtained from Lucigen Corporation (Middleton WI, USA) according to the manufacturer's instructions and plated LB plates augmented with ampicillin for selection. Several of the resulting colonies were cultured and their DNA was isolated using a standard miniprep DNA purification kit from Qiagen (Valencia, CA USA) according to manufacturer's instructions. The recovered DNA was checked by restriction digest followed by agarose gel electrophoresis. DNA samples showing the correct banding pattern were further verified by DNA sequencing. The sequence verified DNA was designated as pHT08-mcr, and was then transformed into chemically competent Bacillus subtilis cells using directions obtained from Boca Scientific (Boca Raton, FL USA). Bacillus subtilis cells carrying the pHT08-mcr plasmid were selected for on LB plates augmented with chloramphenicol.

Bacillus subtilis cells carrying the pHT08-mcr, were grown overnight in 5 ml of LB media supplemented with 20ug/mL chloramphenicol, shaking at 225 rpm and incubated at 37 degrees Celsius. These cultures were used to inoculate 1% v/v, 75 mL of M9 minimal media supplemented with 1.47 g/L glutamate, 0.021 g/L tryptophan, 20 ug/mL chloramphenicol and 1mM IPTG. These cultures were then grown for 18 hours in a 250mL baffled Erlenmeyer flask at 25 rpm, incubated at 37 degrees Celsius. After 18 hours, cells were pelleted and supernatants subjected to GCMS detection of 3-HP (described in Common Methods Section Mb)). Trace amounts of 3-HP were detected with qualifier ions.

### Example 16: Bacillus subtilis strain construction

Plasmids may be prepared and transformed into B. subtilis using a modified protocol developed from Anagnostopoulos and Spizizen (Requirements for transformation in Bacillus subtilis. J. Bacteriol. 81:741-746 (1961) as provided with the instructions for the pHT08 shuttle vector by Boca Scientific (Boca Raton, FL USA).

### Example 17: Yeast aerobic pathway for 3HP production

The following construct (SEQ ID NO:20) containing: 200 by 5' homology to ACC1,His3 gene for selection, Adhl yeast promoter, BamHI and Spel sites for cloning of MCR, cyc 1 terminator, Tefl promoter from yeast and the first 200 by of homology to the yeast ACC1 open reading frame will be constructed using gene synthesis (DNA 2.0). The MCR open reading frame (SEQ ID NO:21) will be cloned into the BamHI and Spel sites, this will allow for constitutive transcription by the adhl promoter. Following the cloning of MCR into the construct the genetic element (SEQ ID NO:22) will be isolated from the plasmid by restriction digestion and transformed into relevant yeast strains. The genetic element will knock out the native promoter of yeast ACC1 and replace it with MCR expressed from the adhl promoter and the Tefl promoter will now drive yeast ACC1 expression. The integration will be selected for by growth in the absence of histidine. Positive colonies will be confirmed by PCR. Expression of MCR and increased expression of ACC1 will be confirmed by RT-PCR.

An alternative approach that could be utilized to express MCR in yeast is expression of MCR from a plasmid. The genetic element containing MCR under the control of the ADH1 promoter (SEQ ID 4) could be cloned into a yeast vector such as pRS421 (SEQ ID NO:23) using standard molecular biology techniques creating a plasmid containing MCR (SEQ ID NO:24). A plasmid based MCR could then be transformed into different yeast strains. Based on the present disclosure, it is noted that, in addition to introducing a nucleic acid construct that comprises a sequence encoding for malonyl-CoA reductase activity in a yeast cell, in some embodiments additional genetic modifications are made to decrease enoyl-CoA reductase activity and/or other fatty acid synthase activity.

### Example 18: Yeast Strain construction

Yeast strains were constructed using standard yeast transformation and selected for by complementation of auxotrophic markers. All strains are S288C background. For general yeast transformation methods, see Gietz, R.D. and R.A. Woods. (2002) "Transformation of Yeast by the Liac/SS Carrier DNA/PEG Method." Methods in Enzymology 350: 87-96.

### Example 19: Production of Flaviolin Polyketide

This example provides data and analysis from strains to which plasmids were added in various combinations. One such plasmid comprises a gene for 1,3,6,8-tetrahydronapthalene synthase (rppA from Streptomyces coelicolor A3(2)), which was codon-optimized for E. coli (DNA2.0, Menlo Park, CA USA). Below this is referred to as THNS, which converts 5 malonyl-CoA to one molecule of 1,3,6,8-naphthalenetetrol, 5 CO2, and 5 coenzyme A. The 1,3,6,8-naphthalenetetrol product of THNS is reported to convert spontaneously to the polyketide flaviolin (CAS No. 479-05-0), which is readily detected spectrometrically at 340 nm. Another plasmid comprises acetyl-CoA carboxylase genes ABCD, which as described elsewhere herein may increase supply of malonyl-CoA from acetyl-CoA.

Two of the strains comprise mutant forms of one or more genes of the fatty acid synthase pathway. These forms are temperature-sensitive and have lower activity at 37 C. These strains are designated as BX595, comprising a temperature-sensitive mutant fabl, and BX660, comprising both temperature-sensitive fabl and fabB genes.

The results herein generally demonstrate that polyketide synthesis is increased when a genetically modified microorganism comprises both at least one heterologous nucleic acid sequence of a polyketide synthesis pathway and at least one modification to decrease activity, such as transiently, of one or more enzymatic conversion steps of the fatty acid synthase pathway. This is considered to reduce enzymatic activity in the microorganism's fatty acid synthase pathway providing for reduced conversion of malonyl-CoA to fatty acids, and in this case lead to increased polyketide synthesis.

The following strains and plasmids were obtained or made using common genetic/molecular biology methods, such as described elsewhere herein, and also in Sambrook and Russell, "Molecular Cloning: A Laboratory Manual," Third Edition 2001 (volumes 1-3), Cold Spring Harbor Laboratory Press, Cold Spring Harbor, N.Y.. Respective genotypes follow strain identifications.
BW25113 (F-, A(araD-araB)567, AlacZ4787(::rrnB-3), LAM-, rph-1, A(rhaD-rhaB)568, hsdR514)
BX595 (F-, A(araD-araB)567, AlacZ4787(::rrnB-3), LAM-, rph-1, A(rhaD-rhaB)568, hsdR514,, AldhA:frt, ApflB:frt, mgsA:frt,ApoxB:frt, Apta-ack:frt, fablts (S241F)-zeoR)
BX660 (F-, A(araD-araB)567, AlacZ4787(::rrnB-3), LAM-, rph-1, A(rhaD-rhaB)568, hsdR514,, AldhA:frt, ApflB:frt, mgsA:frt,ApoxB:frt, Apta-ack:frt, fablts (S241F)-zeoR, fabBts-BSD), pTRC-ptrc_THNS (SEQ ID NO:1) (developed from Invitrogen's ptrc-HisA plasmid, Invitrogen, Carlsbad, CA USA, with TENS under control of ptrc promoter in this plasmid pJ251-accABCD.

Using the above-listed E. coli strains and plasmids, the following were prepared by standard introduction of plasmids to E. coli strains:
1. BW25113 + pIRC-ptrc_THNS
2. BW25113 + pTRC-ptrc_THNS ; pJ251+accABCD
3. BX595 + pTRC-ptrc_THNS
4. BX595 + pTRC-ptrc THNS ; pJ251 +accABCD
5. BX660 + pTRC-ptrc_THNS
6. BX660 + pTRC-ptrc THNS ; pJ251+accABCD

These then were evaluated as described below by following the protocols summarized for each respective evaluation.

### A: 96 Deep Well Plate Screen:

The BW25113 and BX595 strains above were run in triplicate. lmL SM3 or LB media with amp and IPTG was added to the appropriate number of wells. The wells were inoculated with single colonies picked from plates. The 96 well plate was put at 30C for -6-8 hours then shifted to 37°C overnight. After 24 hours, a 200uL aliquot was removed and transferred to a 96 well flat bottom plate used for measuring absorbance on the spectrometer. The first read was done at OD600 to quantify cell growth and to use for normalizing the flaviolin reading. The plate was then spun at 4000rpm for 10 minutes. A 150uL aliquot was removed and read at OD340 to quantify the amount of flaviolin produced. The data is reported as both OD340/0D600 and just OD340.

### B: Shake Flask Screen #1:

25mL cultures of BW25113 and BX595 strains were grown overnight in TB medium with appropriate antibiotics. 50mL shake flask cultures were set up in SM3 with a 5% inoculation from the TB overnight cultures. The shake flasks were induced at time of inoculation. The cultures were grown for 48 hours and samples were taken for flaviolin readings throughout the experiment. Again, data is reported as both OD340/0D600 and just OD340.

### C: Shake Flask Screen #2:

The shake flask experiment above was repeated for 24 hours only and with all three background strains mentioned above. Samples were taken at the 24 hour time point only. Data is shown in the figures. ANOVA tests were run when necessary to compare data and find statistically significant results. The amounts of malonyl-CoA produced by the different strains should be evident by flaviolin levels.

### Example 20: Production of Polyketides

The following example generally describes polyketide synthesis in E. coli by expression of polyketide synthases (PKS). Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. Genes encoding any of numerous PKS enzymes, including type I, type II and type III PKS can be used to introduce these activities into genetically modified organisms that can then convert malonyl-CoA with or without other substrates and reductants including NADPH and NADH into numerous chemical products, for a nonlimiting list of PKS systems refer to the various PKS examples provided in Tables 1A-1H.

### Example 21: Production of Phloroglucinol

This example describes phloroglucinol production in E. coli by expression of phloroglucinol synthases. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. Genes encoding phloroglucinol synthase such as that encoded by the phlD gene of P. fluorescens Pf-5 (or known mutants thereof) can be used to introduce this activity into genetically modified organisms that can then convert 3 molecules of malonyl-CoA into one molecule of phloroglucinol. The sequence of the expressed protein (SEQ ID NO:161) is provided below:

MSTLCLPHVMFPQHKITQQQMVDHLENLHADHPRMALAKRMIANTEVNERHLVLPIDE
LAVHTGFTHRSIVYEREARQMSSAAARQAIENAGLQISDIRMVIVTSCTGFMMPSLTAHL
INDLALPTSTVQLPIAQLGCVAGAAAINRANDFARLDARNHVLIVSLEFSSLCYQPDDTK
LHAFISAALFGDAVSACVLRADDQAGGFKIKKTESYFLPKSEHYIKYDVKDTGFHFTLD
KAVMNSIKDVAPVMERLNYESFEQNCAHNDFFIFHTGGRKILDELVMHLDLASNRVSQS

RSSLSEAGNIASW VFDVLKRQFD SNLNRGDIGL LAAFGPGFTA EMAVGEWTA (SEQ
ID NO:161)

### Example 22: Production of Resorcinol

This example describes resorcinol production in E. coli by expression of phloroglucinol synthases and phloroglucinol reductases. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. Genes encoding phloroglucinol synthase such as that encoded by the ph1D gene of P. fluorescens Pf-5 (or known mutants thereof) can be used to introduce this activity into genetically modified organisms that can then convert 3 molecules of malonyl-CoA into one molecule of phloroglucinol. Phloroglucinol can then serve as a substrate for phloroglucinol reductase, which acts to reduce phloroglucinol to dihydrophloroglucinol. Dihydro can then be abiotically converted to resorcinol by acidic conditions such as by acidicd extraction into solvents such as ethyl acetate as described by Armstrong & Patel, "Ablotic conversion of dihydrophloroglucinol to resorcinol" Canadian Journal of -Microbiology, 1993. 39:(9) 899-902., 10.1139/m93-1:35.

### Example 23: Production of Alkylresorcinol(s)

This example describes alkylresorcinol production in E. coli by expression of alkylresorcinol synthases (polyketide synthases). Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. Genes encoding alkylresorinol synthases can be used to introduce this activity into genetically modified organisms that can then convert malonyl-CoA into alkylresorcinols, generally 3 molecules of malonyl-CoA and fatty acyl-CoA of differing chain lengths are condensed to form alkylresoricnols of differing chain lengths.

### Example 24: Production of Triacetic lactone

The following example details triacetic lactone (TAL) production in E. coli by expression of TAL synthases (polyketide synthases). Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. Genes encoding TAL synthases can be used to introduce this activity into genetically modified organisms that can then convert malonyl-CoA and acetyl-CoA into TAL. Specifically Penicillium patulum 6-methylsalycilic acid synthase (6- MSAS) and its mutant (Y1572F) may be used as a TAL synthase for TAL production. (Biotechnol Bioeng. 2006 Mar 5;93(4):727-36.Microbial synthesis of triacetic acid lactone.Xie D, Shao Z, Achkar J, Zha W, Frost JW, Zhao H. Department of Chemistry, Michigan State University, East Lansing, Michigan 48824, USA.)

### Example 25: C10 fatty acid Production in E. coli with fatty acid synthase inhibition.

This example describes C10 fatty acid production in E. coli by expression of malonyl-CoA specific elongases. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. First genes required for fatty acid beta oxidation may be deleted to remove the cells ability to degrade product. In E.coli this can be accomplished by deleting either the fadD or fade genes, in addition to other possible targets in the beta-oxidation pathway. Genes encoding acetoacetyl-CoA thiolase activity such as the phaA genes from ralstonia species or rhodobacter species, may be used to produce acetoacetyl-CoA from 2 molecules of acetyl-CoA. Acetoacetyl-CoA can in turn be converted to (S)-3-hydroxybutyryl-CoA via the action of an NADH dependent (S)-3-hydroxybutyryl-CoA dehydrogenase, such as that encoded by the hbd gene of C. beijerinckii. (S)-3-hydroxybutyryl-CoA can be dehydrated by the actions of crotonase or enoyl-CoA hydratase enzymes such as those encoded by the crt gene from C. acetobutylicum and the ech gene from P.
putida respectively to produce crotonyl-CoA. Crotonyl-CoA can then be reduced to butyryl-CoA by the actions of either the NADPH dependent crotonyl-CoA reductase encoded by the crr gene of S. colinus, or the NADH dependent crotonyl-CoA reductase encoded by the ter gene of T. denticola. The result of these enzymatic steps is the production of buyryl-CoA from acetyl-CoA. Butyryl-CoA then serves as the primer unit for the action of the elongase enzymes. The elol gene from T brucei (Genbank accession no. AAX70671) may be expressed to convert butyryl-CoA to C10-fatty acyl-CoA (decanoyl-CoA) by the iterative condensation, dehydration and reduction of 3 molecules of malonyl-CoA, requiring electrons from either NADPH or NADH. Once decanoyl¬CoA is produced the action of a thioesterase such as encoded by the tesA gene of E. coli thioesterase I (known to act on C10-fatty-acyl-CoA , Bonner & Block, Journal of Biological Chemistry, Vol 247., No. 10, 1972,p3123-3133) to produce the free fatty acid, in this case C10 free fatty acid decanoate.

### Example 26: C10 fatty acid Production in E. coli with fatty acid synthase inhibition.

This example describes C10 fatty acid production in E. coli by expression of malonyl-CoA specific elongases. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. First genes required for fatty acid beta oxidation may be deleted to remove the cells ability to degrade product. In E.coli this can be accomplished by deleting either the fadD or fade genes, in addition to other possible targets in the beta-oxidation pathway. A gene encoding acetoacetyl-CoA synthase activity such as the nphT7 gene from Streptomyces species may be used to produce acetoacetyl-CoA from 1 molecule of acetyl-CoA and 1 molecule of malonyl-CoA. This irreversible reaction can be ensured to accumulate acetoacetyl-CoA pools in E. coli with the additional deletion of the atoB gene encoding an aceto-acetyl-CoA thiolase, which can degrade acetoacetyl-CoA into two molecules of acetyl-CoA. Acetoacetyl-CoA can in turn be converted to (S)-3-hydroxybutyryl-CoA via the action of an NADH dependent (S)-3-hydroxybutyryl-CoA dehydrogenase, such as that encoded by the hbd gene of C. beijerinckii. (S)-3-hydroxybutyryl-CoA can be dehydrated by the actions of crotonase or enoyl-CoA hydratase enzymes such as those encoded by the crt gene from C. acetobutylicum and the ech gene from P. putida respectively to produce crotonyl-CoA. Crotonyl-CoA can then be reduced to butyryl-CoA by the actions of either the NADPH dependent crotonyl-CoA reductase encoded by the crr gene of S. colinus, or the NADH dependent crotonyl-CoA reductase encoded by the ter gene of T. denticola. The result of these enzymatic steps is the production of buyryl-CoA from acetyl-CoA. Butyryl-CoA then serves as the primer unit for the action of the elongase enzymes. The elol gene from T brucei (Genbank accession no. AAX70671) may be expressed to convert butyryl-CoA to C10-fatty acyl-CoA (decanoyl-CoA) by the iterative condensation, dehydration and reduction of 3 molecules of malonyl-CoA, requiring electrons from either NADPH or NADH. Once decanoyl-CoA is produced the action of a thioesterase such as encoded by the tesA gene of E. coli thioesterase I(known to act on C10-fatty-acyl-CoA , Bonner & Block, Journal of Biological Chemistry, Vol 247., No. 10, 1972,p3123-3133) to produce the free fatty acid, in this case C10 free fatty acid decanoate.

### Example 27: C10 fatty acid Production in E. coli with fatty acid synthase inhibition.

This example describes C10 fatty acid production in E. coli by expression of malonyl-CoA specific elongases. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. First genes required for fatty acid beta oxidation may be deleted to remove the cells ability to degrade product. In E.coli this can be accomplished by deleting either the fadD or fade genes, in addition to other possible targets in the beta-oxidation pathway. Genes encoding acetoacetyl-CoA thiolase activity such as the phaA genes from ralstonia species or rhodobacter species, may be used to produce acetoacetyl-CoA from 2 molecules of acetyl-CoA. Acetoacetyl-CoA can in turn be converted to (R)-3- hydroxybutyryl-CoA via the action of an NADH or NADPH dependent (R)-3-hydroxybutyryl-CoA dehydrogenase, such as that encoded by the phaB gene of C. necator. (R)-3-hydroxybutyryl-CoA can subsequently epimerized to (S) - 3 hydroxybutyryl-CoA by any number of epimerases including those encoded by the fadll genes of E. coli. (S) -3 hydroxybutyryl-CoA be dehydrated by the actions of crotonase or enoyl¬CoA hydratase enzymes such as those encoded by the crt gene from C. acetobutylicum and the ech gene from P. putida respectively to produce crotonyl-CoA. Crotonyl-CoA can then be reduced to butyryl-CoA by the actions of either the NADPH dependent crotonyl-CoA reductase encoded by the crr gene of S. colinus, or the NADH dependent crotonyl-CoA reductase encoded by the ter gene of T. denticola. The result of these enzymatic steps is the production of buyryl-CoA from acetyl-CoA. Butyryl-CoA then serves as the primer unit for the action of the elongase enzymes. The elol gene (Genbank accession no. AAX70671) from T brucei may be expressed to convert butyryl-CoA to C10-fatty acyl-CoA (decanoyl-CoA) by the iterative condensation, dehydration and reduction of 3 molecules of malonyl-CoA, requiring electrons from either NADPH or NADH. Once decanoyl-CoA is produced the action of a thioesterase such as encoded by the tesA gene of E. coli thioesterase I(known to act on C10-fatty-acyl-CoA , Bonner & Block, Journal of Biological Chemistry, Vol 247., No. 10, 1972,p3123-3133) to produce the free fatty acid, in this case C10 free fatty acid decanoate.

### Example 28: C10 fatty acid Production in E. coli with fatty acid synthase inhibition.

This example describes C10 fatty acid production in E. coli by expression of malonyl-CoA specific elongases. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. First genes required for fatty acid beta oxidation may be deleted to remove the cells ability to degrade product. In E.coli this can be accomplished by deleting either the fadD or fade genes, in addition to other possible targets in the beta-oxidation pathway. A gene encoding acetoacetyl-CoA synthase activity such as the nphT7 gene from Streptomyces species may be used to produce acetoacetyl-CoA from 1 molecule of acetyl-CoA and 1 molecule of malonyl-CoA. This irreversible reaction can be ensured to accumulate acetoacetyl-CoA pools in E. coli with the additional deletion of the atoB gene encoding an aceto-acetyl-CoA thiolase, which can degrade acetoacetyl-CoA into two molecules of acetyl-CoA. Acetoacetyl-CoA can in turn be converted to (R)-3-hydroxybutyryl-CoA via the action of an NADH or NADPH dependent (R)-3- hydroxybutyryl-CoA dehydrogenase, such as that encoded by the phaB gene of C. necator. (R)-3- hydroxybutyryl-CoA can subsequently epimerized to (S) -3 hydroxybutyryl-CoA by any number of epimerases including those encoded by the fadll genes of E. coli. (S)-3-hydroxybutyryl-CoA can be dehydrated by the actions of crotonase or enoyl-CoA hydratase enzymes such as those encoded by the crt gene from C. acetobutylicum and the ech gene from P. putida respectively to produce crotonyl-CoA. Crotonyl-CoA can then be reduced to butyryl-CoA by the actions of either the NADPH dependent crotonyl-CoA reductase encoded by the crr gene of S. colinus, or the NADH dependent crotonyl-CoA reductase encoded by the ter gene of T. denticola. The result of these enzymatic steps is the production of buyryl-CoA from acetyl-CoA. Butyryl-CoA then serves as the primer unit for the action of the elongase enzymes. The elol gene from T brucei (Genbank accession no. AAX70671) may be expressed to convert butyr yl-CoA to C10-fatty acyl-CoA (decanoyl-CoA)
by the iterative condensation, dehydration and reduction of 3 molecules of malonyl-CoA, requiring electrons from either NADPH or NADH. Once decanoyl-CoA is produced the action of a thioesterase such as encoded by the tesA gene of E. coli thioesterase I(known to act on C10-fatty-acyl-CoA , Bonner & Block, Journal of Biological Chemistry, Vol 247., No. 10, 1972,p3123-3133) to produce the free fatty acid, in this case C10 free fatty acid decanoate.

### Example 29: C14 fatty acid (Myristic Acid) Production in E. coli with fatty acid synthase inhibition.

This example describes C14 fatty acid production in E. coli by expression of malonyl-CoA specific elongases. Any of the genetically modified mircoorganisms described above that produce C10-fatty-acyl-CoA (decanoyl-CoA) can be further genetically modified to produce C14 fatty acid or myristic acid. Vectors and tools are well known in the art for introducing further genetic modifications, as are promoter systems allowing for controlled or constitutive gene expression. In these microorganisms, decanoyl-CoA can serve as the primer unit for the action of the elongase enzymes. The elo2 gene from T brucei (Genbank accession no. AAX70672) may be expressed to convert decanoyl - CoA to C14-fatty acyl-CoA (myristyl-CoA) by the iterative condensation, dehydration and reduction of 2 molecules of malonyl-CoA, requiring electrons from either NADPH or NADH. Once myristyl-CoA is produced the action of a thioesterase such as encoded by the tesA gene of E. coli thioesterase I(known to act on C14- fatty-acyl-CoA , Bonner & Block, Journal of Biological Chemistry, Vol 247., No. 10, 1972,p3123-3133) to produce the free fatty acid, in this case C14 free fatty acid myristic acid.

### Example 30: C18 fatty acid (Stearic Acid) Production in E. coli with fatty acid synthase inhibition.

This example describes C18 fatty acid production in E. coli by expression of malonyl-CoA specific elongases. Any of the genetically modified mircoorganisms described above that produce C14-fatty-acyl-CoA (myristyl-CoA) can be further genetically modified to produce C18 fatty acid or stearic acid. Vectors and tools are well known in the art for introducing further genetic modifications, as are promoter systems allowing for controlled or constitutive gene expression. In these microorganisms, myristyl-CoA can serve as the primer unit for the action of the elongase enzymes. The elo3 gene from T brucei (Genbank accession no. AAX70673) may be expressed to convert myristyl-CoA to C18-fatty acyl-CoA (stearoyl-CoA) by the iterative condensation, dehydration and reduction of 2 molecules of malonyl-CoA, requiring electrons from either NADPH or NADH. Once stearoyl-CoA is produced the action of a thioesterase such as encoded by the tesA gene of E. coli thioesterase I(known to act on C14-fatty-acyl-CoA , Bonner & Block, Journal of Biological Chemistry, Vol 247., No. 10, 1972,p3123-3133) to produce the free fatty acid, in this case C18 free fatty acid stearic acid.

### Example 31: Fatty acid production in yeast with fatty acid synthase inhibition.

Any of the above examples can be ported to any number of other hosts for chemicals production, using tools and techniques well known in the art for genetic modification of a multitude of yeast systems. Briefly, to produce fatty acids in yeast the elongase systems, metabolic pathways to produce fatty acyl-CoAs can be introduced as described above, in addition to genetic modifications to reduce or eliminate native host fatty acid beta-oxidation and inhibit the host fatty acid synthase.

### Example 32: Fatty acid production in bacillus with fatty acid synthase inhibition.

Any of the above examples can be ported to any number of other hosts for chemicals production, using tools and techniques well known in the art for genetic modification of a multitude of gram positive systems such as bacillus subtilis. Briefly, to produce fatty acids in bacillus or other gram positive systems the elongase systems, metabolic pathways to produce fatty acyl-CoAs can be introduced as described above, in addition to genetic modifications to reduce or eliminate native host fatty acid beta-oxidation and inhibit the host fatty acid synthase.

### Example 33: Fatty acid production in C. necator with fatty acid synthase inhibition.

Any of the above examples can be ported to any number of other hosts for chemicals production, using tools and techniques well known in the art for genetic modification of a multitude of gram negative systems such as C. necator. Briefly, to produce fatty acids in C. necatot the elongase systems, metabolic pathways to produce fatty acyl-CoAs can be introduced as described above, in addition to genetic modifications to reduce or eliminate native host fatty acid beta-oxidation and inhibit the host fatty acid synthase. Additionally, in C. necator modifications to remove polyhydroxybutyrate (PHB) synthesis can be incorporated. C. necator has an additional advatnge of producing fatty acids via the feedstocks, hydrogen and carbon dioxide.

### Example 34: Diacid production with fatty acid synthase inhibition.

This example generally describes diacid synthesis in E. coli by expression of polyketide syntheses (PKS) that have been modified for diacid (dicarboxylic acid) production. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. Genes encoding any of numerous modified PKS enzymes, including type I, type II and type III PKS can be used to introduce these activities into genetically modified organisms that can then convert malonyl-CoA with or without other substrates and reductants including NADPH and NADH into diacids. Specifically PCT Patent Publication No. WO2009/121066 is incorporated by reference for its teachings of diacid production by PKS systems.

### Example 35: Diene production with fatty acid synthase inhibition.

This example generally describes diene synthesis in E. coli by expression of polyketide syntheses (PKS) that have been modified for diene production. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. Genes encoding any of numerous modified PKS enzymes, including type I, type II and type III PKS can be used to introduce these activities into genetically modified organisms that can then convert malonyl-CoA with or without other substrates and reductants including NADPH and NADH into dienes.

### Example 36: n-butanol production with fatty acid synthase inhibition from malonyl-CoA .

This example describes n-butanol production in E. coli by expression of malonyl-CoA dependent acetoacetyl-CoA synthetase. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. A gene encoding acetoacetyl-CoA synthase activity such as the nphT7 gene from Streptomyces species may be used to produce acetoacetyl-CoA from 1 molecule of acetyl¬CoA and 1 molecule of malonyl-CoA. This irreversible reaction can be ensured to accumulate acetoacetyl-CoA pools in E. coli with the additional deletion of the atoB gene encoding an aceto-acetyl-CoA thiolase, which can degrade acetoacetyl-CoA into two molecules of acetyl-CoA. Acetoacetyl-CoA can in turn be converted to (S)- 3-hydroxybutyryl-CoA via the action of an NADH dependent (S)-3-hydroxybutyryl-CoA dehydrogenase, such as that encoded by the hbd gene of C. beijerinckii. (S)-3-hydroxybutyryl-CoA can be dehydrated by the actions of crotonase or enoyl-CoA hydratase enzymes such as those encoded by the crt gene from C. acetobutylicum and the ech gene from P. putida respectively to produce crotonyl-CoA. Alternatively, acetoacetyl-CoA can in turn be converted to (R)-3-hydroxybutyryl-CoA via the action of an NADH or NADPH dependent (R)-3- hydroxybutyryl-CoA dehydrogenase, such as that encoded by the phaB gene of C. necator. (R)-3- hydroxybutyryl-CoA can subsequently epimerized to (S) -3 hydroxybutyryl-CoA by any number of epimerases including those encoded by the fadll genes of E. coli. (S)-3-hydroxybutyryl-CoA can be dehydrated by the actions of crotonase or enoyl-CoA hydratase enzymes such as those encoded by the crt gene from C. acetobutylicum and the ech gene from P. putida respectively to produce crotonyl-CoA. Crotonyl-CoA can then be reduced to butyryl-CoA by the actions of either the NADPH dependent crotonyl-CoA reductase encoded by the crr gene of S. colinus, or the NADH dependent crotonyl-CoA reductase encoded by the ter gene of T. denticola. Butyryl-CoA can then serve as the substrate for the NADH dependent butanol dehydrogenase adhE2 gene from Clostridia acetobutylicum. This enzyme carries out the two step reduction of butyryl-CoA to butanol.

### Example 37: Isobutanol production with fatty acid synthase inhibition from malonyl-CoA.

This example describes n-butanol production in E. coli by expression of malonyl-CoA dependent acetoacetyl-CoA synthetase. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. A gene encoding acetoacetyl-CoA synthase activity such as the nphT7 gene from Streptomyces species may be used to produce acetoacetyl-CoA from 1 molecule of acetyl-CoA and 1 molecule of malonyl-CoA. This irreversible reaction can be ensured to accumulate acetoacetyl-CoA pools in E. coli with the additional deletion of the atoB gene encoding an aceto-acetyl-CoA thiolase, which can degrade acetoacetyl-CoA into two molecules of acetyl-CoA. Acetoacetyl-CoA can in turn be converted to (S)- 3-hydroxybutyryl-CoA via the action of an NADH dependent (S)-3-hydroxybutyryl-CoA dehydrogenase, such as that encoded by the hbd gene of C. beijerinckii. (S)-3-hydroxybutyryl-CoA can be dehydrated by the actions of crotonase or enoyl-CoA hydratase enzymes such as those encoded by the crt gene from C. acetobutylicum and the ech gene from P. putida respectively to produce crotonyl-CoA. Alternatively, acetoacetyl-CoA can in turn be converted to (R)-3-hydroxybutyryl-CoA via the action of an NADH or NADPH dependent (R)-3- hydroxybutyryl-CoA dehydrogenase, such as that encoded by the phaB gene of C. necator. (R)-3- hydroxybutyryl-CoA can subsequently epimerized to (S)-3 hydroxybutyryl-CoA by any number of epimerases including those encoded by the fadll genes of E. coli. (S)-3-hydroxybutyryl-CoA can be dehydrated by the actions of crotonase or enoyl-CoA hydratase enzymes such as those encoded by the crt gene from C. acetobutylicum and the ech gene from P. putida respectively to produce crotonyl-CoA. Crotonyl-CoA can then be reduced to butyryl-CoA by the actions of either the NADPH dependent crotonyl-CoA reductase encoded by the crr gene of S. colinus, or the NADH dependent crotonyl-CoA reductase encoded by the ter gene of T. denticola. Butyryl-CoA can then serve as the substrate for an isomerase capable of producing isobutyryl-CoA from butyryl-CoA, suh as that endoded by the icmA and icmB genes of Streptoriisi-e

Isobutyryl-CoA is then the substrate for the NADH dependent dehydrogenases adhE2 that can carry out the two step reduction of isobutyryl-CoA to isobutanol, such as the adhE genes from Giardia species.

### Example 38: Chemical production from butyryl-CoA as an intermediate.

Similarly to the production of butanol, described above, the following example details any chemical production stemming from butyryl-CoA as an intermediate by expression of malonyl-CoA dependent acetoacetyl-CoA synthetase. As discussed above, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. A gene encoding acetoacetyl-CoA synthase activity such as the nphT7 gene from Streptomyces species may be used to produce acetoacetyl-CoA from 1 molecule of acetyl-CoA and 1 molecule of malonyl-CoA. This irreversible reaction can be ensured to accumulate acetoacetyl-CoA pools with the additional deletion of the atoB gene encoding an aceto-acetyl-CoA thiolase, which can degrade acetoacetyl-CoA into two molecules of acetyl-CoA.

Acetoacetyl-CoA can in turn be converted to (S)-3-hydroxybutyryl-CoA via the action of an NADH dependent (S)-3-hydroxybutyryl-CoA dehydrogenase, such as that encoded by the hbd gene of C. beijerinckii. (S)-3¬hydroxybutyryl-CoA can be dehydrated by the actions of crotonase or enoyl-CoA hydratase enzymes such as those encoded by the crt gene from C. acetobutylicum and the ech gene from P. putida respectively to produce crotonyl-CoA. Alternatively, acetoacetyl-CoA can in turn be converted to (R)-3-hydroxybutyryl-CoA via the action of an NADH or NADPH dependent (R)-3-hydroxybutyryl-CoA dehydrogenase, such as that encoded by the phaB gene of C. necator. (R)-3-hydroxybutyryl-CoA can subsequently epimerized to (S) -3 hydroxybutyryl¬CoA by any number of epimerases including those encoded by the fadll genes of E. coli. (S)-3-hydroxybutyryl¬CoA can be dehydrated by the actions of crotonase or enoyl-CoA hydratase enzymes such as those encoded by the crt gene from C. acetobutylicum and the ech gene from P. putida respectively to produce crotonyl-CoA. Crotonyl-CoA can then be reduced to butyryl-CoA by the actions of either the NADPH dependent crotonyl¬CoA reductase encoded by the crr gene of S. colinus, or the NADH dependent crotonyl-CoA reductase encoded by the ter gene of T denticola. Butyryl-CoA can then serve as the substrate for numerous other chemical products, such as butyrate with activity of a butyryl-CoA hydrolase, or alternatively from the actions of a phosphotranbutyrylase and butyrate kinase such as those encoded by the ptb and bukl genes of Clostridia sp. Additionally, other chemical products may be produced via adding additional enzymes to further convert butyryl-CoA to these products from malonyl-CoA.

### Example 39: Chemical production from isobutyryl-CoA as an intermediate.

Similarly to the production of butanol, described above, the following example describes any chemical production stemming from isobutyryl-CoA as an intermediate by expression of malonyl-CoA dependent acetoacetyl-CoA synthetase. As discussed above, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. A gene encoding acetoacetyl-CoA synthase activity such as the nphT7 gene from Streptomyces species may be used to produce acetoacetyl-CoA from 1 molecule of acetyl-CoA and 1 molecule of malonyl-CoA. This irreversible reaction can be ensured to accumulate acetoacetyl-CoA pools with the additional deletion of the atoB gene encoding an aceto-acetyl-CoA thiolase, which can degrade acetoacetyl-CoA into two molecules of acetyl-CoA. Acetoacetyl-CoA can in turn be converted to (S)-3-hydroxybutyryl-CoA via the action of an NADH dependent (S)-3-hydroxybutyryl-CoA dehydrogenase, such as that encoded by the hbd gene of C. beijerinckii. (S)-3-hydroxybutyryl-CoA can be dehydrated by the actions of crotonase or enoyl-CoA hydratase enzymes such as those encoded by the crt gene from C. acetobutylicum and the ech gene from P. putida respectively to produce crotonyl-CoA. Alternatively, acetoacetyl-CoA can in turn be converted to (R)-3- hydroxybutyryl-CoA via the action of an NADH or NADPH dependent (R)-3-hydroxybutyryl-CoA dehydrogenase, such as that encoded by the phaB gene of C. necator. (R)-3-hydroxybutyryl-CoA can subsequently epimerized to (S) -3 hydroxybutyryl-CoA by any number of epimerases including those encoded by the fadll genes of E. coli. (S)-3-hydroxybutyryl-CoA can be dehydrated by the actions of crotonase or enoyl¬CoA hydratase enzymes such as those encoded by the crt gene from C. acetobutylicum and the ech gene from P. putida respectively to produce crotonyl-CoA. Crotonyl-CoA can then be reduced to butyryl-CoA by the actions of either the NADPH dependent crotonyl-CoA reductase encoded by the crr gene of S. colinus, or the NADH dependent crotonyl-CoA reductase encoded by the ter gene of T. denticola. Butyryl-CoA can then serve as the substrate for an isomerase capable of producing isobutyryl-CoA from butyryl-CoA, suh as that endoded by the icmA and icmB genes of S.frt'pit)rilyt'e, Isobutyryl-CoA can then serve as the substrate for numerous other chemical products, such as isobutyrate with activity of a isobutyryl-CoA hydrolase, or alternatively from the actions of a phosphotransisobutyrylase and isobutyrate kinase. Additionally, other chemical products may be produced via adding additional enzymes to further convert isobutyryl-CoA to these products from malonyl-CoA, such as phlorisobutyrophenone via the actions of either isobutyrophenone synthase form Hypericum calycinum or phlorisobutyrophenone synthase / phlorisovalerophenone synthase encoded by the VPS gene from Humulus lupulus. Additionally, other chemical products may be produced via adding additional enzymes to further convert isobutyryl-CoA to these products from malonyl-CoA.

### Example 40: Chemical production from methacrylyl-CoA as an intermediate.

Similarly to the production of chemicals in the examples described above, the following example describes any chemical production stemming from methacrylyl-CoA as an intermediate by expression of malonyl-CoA dependent acetoacetyl-CoA synthetase. As discussed above, genetically modififed organisms can be engineered to produce butyryl-CoA and the subsequently isobutyryl-CoA as intermediates. Isobutyryl-CoA can then be further converted to methacrylyl-CoA by the actions of a methylacyl-CoA dehydrogenase such as those encoded by the acdH or Acadsb genes from Streptomyces avermitilis and Rattus norvegicus, respectively. Any of these strains may be used as starting points for further genetic modifications. Other chemical products may be produced via adding additional enzymes to further convert methacrylyl-CoA to these products from malonyl¬CoA, such as methylacrylate via the actions of a methacrylyl-CoA hydrolase. Alternatively, 3- hydroxyisobutyrate can be made from methylacrylyl-CoA via the actions of first a short chain enoyl-CoA hydratase such as those encoded by the ECHS1 or ech genes of Bos Taurus Pseudomonas fluorescens, respectively, converting methylacrylyl-CoA to 3-hydroxyisobutyryl-CoA. 3- hydroxyisobutyryl-CoA can then be converted to 3-hydroxyisobutyrate via the actions of a 3- hydroxyisobutyryl-CoA hydrolase, such as that encoded by the Hibch gene of Rattus norvegicus. Additionally, other chemical products may be produced via adding additional enzymes to further convert methylacryryl-CoA to these products from malonyl-CoA.

### Example 41: Chemical production from 3-hydroxy-3-methylglutaryl-CoA, as an intermediate.

Similarly to the production of chemicals in the examples described above, this example describes any chemical production stemming from acetoacetyl-CoA as an intermediate by expression of malonyl-CoA dependent acetoacetyl-CoA synthetase. As discussed above, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. A gene encoding acetoacetyl-CoA synthase activity such as the nphT7 gene from Streptomyces species may be used to produce acetoacetyl-CoA from 1 molecule of acetyl-CoA and 1 molecule of malonyl-CoA. This irreversible reaction can be ensured to accumulate acetoacetyl-CoA pools, with the additional deletion of the atoB gene encoding an aceto-acetyl-CoA thiolase, which can degrade acetoacetyl-CoA into two molecules of acetyl-CoA. Acetoacetyl-CoA can further be converted to 3-hydroxy-3-methylglutaryl-CoA, by the actions of numerous hydroxymethylglutaryl-CoA syntheses such as that encoded by the hmgS gene of Saccharomyces species. 3-hydroxy-3-methylglutaryl-CoA can further be converted to numerous products via the mevalonate pathway, wherein mevalonate is first produced from 3-hydroxy-3-methylglutaryl-CoA (hmg-CoA) by reduction using enzymes such as the hmg-CoA reductases encoded by numerous genes including the HMG1 and HMG2 genes of Saccharomyces species. The mevalonate pathway is well known as a metabolic pathway allowing the production of numerous products, such as farnesene and other isoprenoids. Additionally, other chemical products may be produced via adding additional enzymes to further convert 3-hydroxy-3-methylglutaryl-CoA or mevalonate to these products from malonyl-CoA.

### Example 42: Chemical production in yeast

Any of the above examples can be ported to any number of other hosts for chemicals production, using tools and techniques well known in the art for genetic modification of a multitude of yeast systems. Briefly, metabolic pathways to produce products can be introduced as described above.

### Example 43: Chemical production in bacillus

Any of the above examples can be ported to any number of other hosts for chemicals production, using tools and techniques well known in the art for genetic modification of a multitude of gram positive systems, such as bacillus. Briefly, metabolic pathways to produce products can be introduced as described above.

### Example 44: Chemical production in C. necator

Any of the above examples can be ported to any number of other hosts for chemicals production, using tools and techniques well known in the art for genetic modification of a multitude of gram negative systems, such as C. necatot. Briefly, metabolic pathways to produce products can be introduced as described above. Additionally, in C. necator modifications to remove polyhydroxybutyrate (PHB) synthesis can be incorporated. C. necator has an additional advatnge of producing fatty acids via the feedstocks, hydrogen and carbon dioxide.

### Example 45: Production of Phloroglucinol

This example describes phloroglucinol production in E. coli by expression of phloroglucinol synthases. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. Genes encoding phloroglucinol synthase such as that encoded by the ph1D gene of P. fluorescens Pf-5 (or known mutants thereof) can be used to introduce this activity into genetically modified organisms that can then convert 3 molecules of malonyl-CoA into one molecule of phloroglucinol. Additionally, as phloroglucinol is more oxidized than dextrose and other sugars additional modifications may be made to reduce acitivity and flux through the citric acid (TCA) cycle. When more reduced feedstocks are used and more oxidized products are made, more than enough electrons are generated through glycolysis for maintenance energy and production needs. Flux through the citric acid cycle can lead to wasted carbon and lower yields. Genetic modifications to reduce flux through the TCA cycle can include genetic modifications to key steps such as those aimed to reduce activity or expression of the citrate synthase enzyme ecoded by the gltA gene, alternatively enzymes that lead to oxaloacetate production can be reduced or eiliminated such as phophoenolpyruvate carboxylase (such as encoded by the ppc gene) or phosphoenolpyruvate carboxykinase such as encoded by the pck genes. Alternatively genetic modifications may be introduced to cause temperature sensitive ("ts") activity in these enzymes (such as in the genes gltA, ppc, pck) to low activity at a permissive temeperature such as 30 degrees Celsius, but no activity at a nonpermissive temperature such as 37 degrees Celsius. These "ts" mutants enable a controllable decrease in TCA flux upon temperature change. Numerous temperature sensitive alleles are known for many enzymes such as gltA, and in general methods for screening mutant libraries for such mutations are known in the art, as are more directed approaches for engineering these ts mutations denovo. (Ben-Aroya, S., Coombes, C., Kwok, T., O'Donnell, K.A., Boeke, J.D., and Hieter, P. (2008) Molecular Cell 30:248-258.)

### Example 46: Production of more oxidized products from malonyl-CoA.

This example describes chemical product production in E. coli from sugar feedstocks for chemicals more oxidized than sugar. Briefly, genetically modified E. coli with controlled inhibition of fatty acid production can be constructed as described in any of the above examples. Any of these strains may be used as starting points for further genetic modifications. Vectors and tools are well known in the art for introducing further genetic modifications, as are promoter systems allowing for controlled or constitutive gene expression. Genes encoding pathway enzymes can be used to introduce activities into genetically modified organisms that can then convert molecules of malonyl-CoA into numerous chemical products. Additionally, as these products are more oxidized than dextrose and other sugars additional modifications may be made to reduce acitivty and flux through the citric acid (TCA) cycle. When more reduced feedstocks are used and more oxidized products are made, more than enough electrons are generated through glycolysis for maintenance energy and production needs. Flux through the citric acid cycle can lead to wasted carbon and lower yields. Genetic modifications to reduce flux through the TCA cycle can include genetic modifications to key steps such as those aimed to reduce activity or expression of the citrate synthase enzyme such as encoded by the gltA gene, alternatively enzymes that lead to oxaloacetate production can be reduced or eliminated such as by disruption of phophoenolpyruvate carboxylase (such as encoded by the ppc) gene or phosphoenolpyruvate carboxykinase such as encoded by the pck genes. Alternatively genetic modifications may be introduced to cause temperature sensitive activity in these enzymes (such as gltA, ppc, pck) to low activity at a permissive temeperature such as 30 degrees Celsius, but no activity at a nonpermissive temperature such as 37 degrees Celsius. These temperature sensitive ("ts") mutants enable a controllable decrease in TCA flux upon temperature change.

### Example 47: Polyhydroxybutyrate production with fatty acid synthase inhibition from malonyl-CoA.

This example describes polyhydroxybutyrate production in E. coli by expression of malonyl-CoA dependent acetoacetyl-CoA synthetase. Briefly, genetically modified E. coli with controlled fatty acid inhibition can be constructed as described in any of the above examples. Any of these strains may be used as starting points for further genetic modifications. Vectors and tools are well known in the art for introducing further genetic modifications, as are promoter systems allowing for controlled or constitutive gene expression. A gene encoding acetoacetyl-CoA synthase activity such as the nphT7 gene from Streptomyces species may be used to produce acetoacetyl-CoA from 1 molecule of acetyl-CoA and 1 molecule of malonyl-CoA. This irreversible reaction can be ensured to accumulate acetoacetyl-CoA pools in E. coli with the additional deletion of the atoB gene encoding an aceto-acetyl-CoA thiolase, which can degrade acetoacetyl-CoA into two molecules of acetyl-CoA. Acetoacetyl-CoA can in turn be converted to (R)-3-hydroxybutyryl-CoA via the action of an NADPH dependent (R)-3-hydroxybutyryl-CoA dehydrogenase, such as that encoded by the phaB gene of Rhodobacter spaeroides or Cuprivdus Necator. (R)- 3-hydroxybutyryl-CoA can be polymerized by the actions of a polyhydroxybutyrate polymerase, such as those encoded by the phaC gene from Cupriavidus necator, to form polyhydroxybutyrate.

### Example 48: General example of genetic modification to a host cell.

In addition to the above specific examples, this example is meant to describe a non-limiting approach to genetic modification of a selected microorganism to introduce a nucleic acid sequence of interest. Alternatives and variations are provided within this general example. The methods of this example are conducted to achieve a combination of desired genetic modifications in a selected microorganism species, such as a combination of genetic modifications as described in sections herein, and their functional equivalents, such as in other bacterial and other microorganism species.

A gene or other nucleic acid sequence segment of interest is identified in a particular species (such as *E. coli* as described herein) and a nucleic acid sequence comprising that gene or segment is obtained.

Based on the nucleic acid sequences at the ends of or adjacent the ends of the segment of interest, 5' and 3' nucleic acid primers are prepared. Each primer is designed to have a sufficient overlap section that hybridizes with such ends or adjacent regions. Such primers may include enzyme recognition sites for restriction digest of transposase insertion that could be used for subsequent vector incorporation or genomic insertion. These sites are typically designed to be outward of the hybridizing overlap sections. Numerous contract services are known that prepare primer sequences to order (e.g., Integrated DNA Technologies, Coralville, IA USA).

Once primers are designed and prepared, polymerase chain reaction (PCRn) is conducted to specifically amplify the desired segment of interest. This method results in multiple copies of the region of interest separated from the microorganism's genome. The microorganism's DNA, the primers, and a thermophilic polymerase are combined in a buffer solution with potassium and divalent cations (e.g., Mg or Mn) and with sufficient quantities of deoxynucleoside triphosphate molecules. This mixture is exposed to a standard regimen of temperature increases and decreases. However, temperatures, components, concentrations, and cycle times may vary according to the reaction according to length of the sequence to be copied, annealing temperature approximations and other factors known or readily learned through routine experimentation by one skilled in the art.

In an alternative embodiment the segment of interest may be synthesized, such as by a commercial vendor, and prepared via PCRn, rather than obtaining from a microorganism or other natural source of DNA.

The nucleic acid sequences then are purified and separated, such as on an agarose gel via electrophoresis. Optionally, once the region is purified it can be validated by standard DNA sequencing methodology and may be introduced into a vector. Any of a number of vectors may be used, which generally comprise markers known to those skilled in the art, and standard methodologies are routinely employed for such introduction. Commonly used vector systems are pSMART (Lucigen, Middleton, WI USA), pET *E*. *coli* EXPRESSION SYSTEM (Stratagene, La Jolla, CA USA), pSC-B StrataClone Vector (Stratagene, La Jolla, CA USA), pRANGER-BTB vectors (Lucigen, Middleton, WI USA), and TOPO vector (Invitrogen Corp, Carlsbad, CA, USA). Similarly, the vector then is introduced into any of a number of host cells. Commonly used host cells are *E. coli* 10G (Lucigen, Middleton, WI USA), *E. coli* 10GF' (Lucigen, Middleton, WI USA), StrataClone Competent cells (Stratagene, La Jolla, CA USA), *E. coli* BL21, *E. coli* BW25113, and *E. coli* K12 MG1655. Some of these vectors possess promoters, such as inducible promoters, adjacent the region into which the sequence of interest is inserted (such as into a multiple cloning site), while other vectors, such as pSMART vectors (Lucigen, Middleton, WI USA), are provided without promoters and with dephosporylated blunt ends. The culturing of such plasmid-laden cells permits plasmid replication and thus replication of the segment of interest, which often corresponds to expression of the segment of interest.

Various vector systems comprise a selectable marker, such as an expressible gene encoding a protein needed for growth or survival under defined conditions. Common selectable markers contained on backbone vector sequences include genes that encode for one or more proteins required for antibiotic resistance as well as genes required to complement auxotrophic deficiencies or supply critical nutrients not present or available in a particular culture media. Vectors also comprise a replication system suitable for a host cell of interest.

The plasmids containing the segment of interest can then be isolated by routine methods and are available for introduction into other microorganism host cells of interest. Various methods of introduction are known in the art and can include vector introduction or genomic integration. In various alternative embodiments the DNA segment of interest may be separated from other plasmid DNA if the former will be introduced into a host cell of interest by means other than such plasmid.

While steps of this general example involve use of plasmids, other vectors known in the art may be used instead. These include cosmids, viruses (e.g., bacteriophage, animal viruses, plant viruses), and artificial chromosomes (e.g., yeast artificial chromosomes (YAC) and bacteria artificial chromosomes (BAC)).

Host cells into which the segment of interest is introduced may be evaluated for performance as to a particular enzymatic step such as regarding biosynthesis of a chemical compound of interest. Selections of better performing genetically modified host cells may be made, selecting for overall performance, tolerance, or production or accumulation of the chemical of interest.

It is noted that this procedure may incorporate a nucleic acid sequence for a single gene (or other nucleic acid sequence segment of interest), or multiple genes (under control of separate promoters or a single promoter), and the procedure may be repeated to create the desired heterologous nucleic acid sequences in expression vectors, which are then supplied to a selected microorganism so as to have, for example, a desired complement of enzymatic conversion step functionality for any of the herein-disclosed metabolic pathways. However, it is noted that although many approaches rely on expression via transcription of all or part of the sequence of interest, and then translation of the transcribed mRNA to yield a polypeptide such as an enzyme, certain sequences of interest may exert an effect by means other than such expression.

The specific laboratory methods used for these approaches are well-known in the art and may be found in various references known to those skilled in the art, such as Sambrook and Russell, Molecular Cloning: A Laboratory Manual, 3rd Ed., (2001) (Volumes 1-3), Cold Spring Harbor Laboratory Press, Cold Spring Harbor, N.Y. (hereinafter, Sambrook and Russell, 2001).

As an alternative to the above, other genetic modifications may also be practiced, such as a deletion of a nucleic acid sequence of the host cell's genome. One non-limiting method to achieve this is by use of Red/ET recombination, known to those of ordinary skill in the art and described in U.S. Patent Nos. 6,355,412 and 6,509,156, issued to Stewart et al. and incorporated by reference herein for its teachings of this method. Material and kits for such method are available from Gene Bridges (Gene Bridges GmbH, Heidelberg, Germany), and the method may proceed by following the manufacturer's instructions. Targeted deletion of genomic DNA may be practiced to alter a host cell's metabolism so as to reduce or eliminate production of undesired metabolic products. This may be used in combination with other genetic modifications such as described herein in this general example.

### Example 49: Preparing a Genetically Modified E. coli Host Cell Comprising malonyl-CoA-reductase (Mcr) in Combination with Other Genetic Modifications to Increase 3-HP Production Relative to a Control E. coli Cell.

Genetic modifications are made to introduce a vector comprising mmsB such as from *Pseudomonas auruginos,* which further is codon-optimized for *E. coli.* Vectors comprising galP and a native or mutated ppc also may be introduced by methods known to those skilled in the art (see, e.g., Sambrook and Russell, 2001), additionally recognizing that mutations may be made by a method using the XLI-Red mutator strain, using appropriate materials following a manufacturer's instructions (Stratagene QuikChange Mutagenesis Kit, Stratagene, La Jolla, CA USA) and selected for or screened under standard protocols.

Also, genetic modifications are made to reduce or eliminate the enzymatic activities of *E. coli* genes as desired. These genetic modifications are achieved by using the RED/ET homologous recombination method with kits supplied by Gene Bridges (Gene Bridges GmbH, Heidelberg, Germany) according to manufacturer's instructions.

Also, in some embodiments genetic modifications are made to increase the NADPH cellular pool. Non-limiting examples of some targets for genetic modification are provided herein. These are pgi (in a mutated form), pntAB, overexpressed, gapA:gapN substitution/replacement, and disrupting or modifying a soluble transhydrogenase such as sthA, and genetic modifications of one or more of zwf, gnd, and edd.

The so-genetically modified microorganism of any such engineered embodiment is evaluated and found to exhibit higher productivity of 3-HP compared with a control *E. coli l*acking said genetic modifications. Productivity is measured by standard metrics, such as volumetric productivity (grams of 3-HP/hour) under similar culture conditions.

### Example 50: Polyketide production via malonyl-coA in strains with combinations of FAS mutations.

The following genetically modified *E. coli* strains (listed in Table 36) were constructed form a wild type BW25113 starting host. *E. coli* BW25113 was obtained from the Yale genetic stock center (New Haven, CT, USA). These strains were constructed by standard methods such as discussed in the Common Methods Section and also known in the art as referenced above. Briefly, chromosomal modifications were constructed via homologous recombination.

**Table 36: Strain List 1**

| **Strain** | **Chromosomal genotype** | **Plasmid 1** | **Plasmid 2** |
|---|---|---|---|
| 1 | F-, Δ(araD-araB)567, ΔlacZ4787(::rrnB-3), LAM-, rph-1, Δ(rhaD-rhaB)568, hsdR514 | Ptrc- THNS | NA |
| 2 | F-, Δ(araD-araB)567, ΔlacZ4787(::rrnB-3), LAM-, rph-1, Δ(rhaD-rhaB)568, hsdR514 | Ptrc-THNS | pACYC-accABCD |
| 3 | F-, Δ(araD-araB)567, ΔlacZ4787(::rrnB-3), LAM-, rph-1, A(rhaD-rhaB)568, hsdR514, ΔldhA:frt, ΔpflB:frt, mgsA:frt, ΔpoxB:frt, F-, Apta-ack:frt | Ptrc-THNS | NA |
| 4 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt | Ptrc- THNS | pACYC-accABCD |
| 5 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt, fabIts (S241F)-zeoR | Ptrc-THNS | NA |
| 6 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt, fabIts (S241F)-zeoR | Ptrc-THNS | pACYC-accABCD |
| 7 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt, fabIts (S241F)-zeoR, fabBts (A329V) | Ptrc-THNS | NA |
| 8 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt Δpta-ack:frt, fabIts (S241F)-zeoR, fabBts (A329V) | Ptrc-THNS | pACYC-accABCD |
| 9 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt, fabIts (S241F)-zeoR ,MabF | Ptrc-THNS | NA |
| 10 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt, fabIts (S241F)-zeoR ,MabF | Ptrc-THNS | pACYC-accABCD |
| 11 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt, fabIts (S241F)-zeoR, ΔfabF, fabBts (A329V) | Ptrc-THNS | NA |
| 12 | F-, Δ(araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fabIts (S241F)-zeoR, ΔfabF, fabBts (A329V) | Ptrc-THNS | pACYC-accABCD |

The above strains were evaluated in shake flasks for the production of flaviolin. Triplicate evaluations were performed. Briefly, overnight starter cultures were made in 50 mL of Luria Broth including the appropriate antibiotics and incubated 16-24 hours are 30°C, while shaking at 225 rpm. These cultures were used to inoculate 3 x 50 mL cultures of each strain in SM8 minimal medium with 5% culture as starting inoculum, antibiotics, and 1 mM IPTG. Flasks were grown in the 30°C in a shaking incubator. At 24 hours, samples were taken for analyses of OD at 600nm and absorbance at 340 nm, the latter an indicator of the presence of flaviolin.

### Example 51: 3-HP production via malonyl-coA in strains with additional combinations of FAS mutations.

The following genetically modified *E. coli* strains (listed in Table 37) were constructed from a wild type BW25113 starting host. *E. coli* BW25113 was obtained from the Yale genetic stock center (New Haven, CT USA). These strains were constructed by standard methods such as discussed in the Common Methods Section and also known in the art as referenced above. Briefly, chromosomal modifications were constructed via homologous recombination.

**Table 37: Strain List 2**

| **Strain** | **Chromosomal genotype** | **Plasmid 1** | **Plasmid 2** |
|---|---|---|---|
| 240 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Apta-ack:frt, fabIts (S241F)-zeoR | Ptrc-mcr | pACYC-accABCD-pntAB |
| 470 | F-, Δ (araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Apta-ack:frt, fabIts (S241F)-zeoR, ΔfabF, fabB(ts) | Ptrc-mcr | NA |
| 471 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Apta-ack:frt, fablts (S241F)-zeoR, ΔfabF, fabB(ts) | Ptrc-mcr | pACYC-accABCD |
| 472 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Apta-ack:frt, fabIts (S241F)-zeoR, ΔfabF, fabB(ts) | Ptrc-mcr | pACYC-accABCD-pntAB |
| 473 | F-, Δ(araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fabIts (S241F)-zeoR, ΔfabF, fabB(ts) | Ptrc-mcr | pACYC-CAT-accADBC-udhA |
| 474 | F-, Δ(araD-araB)567, ΔlacZ4787(::rrnB-3), LAM-, rph-1, Δ (rhaD-rhaB)568, hsdR514, ΔldhA:frt, ΔpflB:frt, mgsA:frt, ΔpoxB:frt, Δpta-ack:frt, fabIts (S241F)-zeoR, ΔfabF, fabB(ts) | Ptrc-mcr | pBT3-tpiA-pntAB |

The above strains were evaluated in shake flasks for the production of 3-HP. Triplicate evaluations were performed. Briefly, overnight starter cultures were made in 50 mL of Luria Broth including the appropriate antibiotics and incubated 16-24 hours are 30°C, while shaking at 225 rpm. These cultures were used to inoculate 3 x 50 mL cultures of each strain in SM8 minimal medium with 5% culture as the starting inoculum, antibiotics, and 1 mM IPTG. Flasks were grown at 30°C in a shaking incubator. At 4, 8, 10, 12 and 24 hours, samples were taken for analyses of OD at 600nm and 3-HP production using the 3-HP bioassay described in the Common Methods Section.

**Table 38. Specific 3-HP Production of FAS mutants**

| | **Average Specific Productivity** (g 3-HP/ gDCW-hr) | | | |
|---|---|---|---|---|
| Strain | 8 hrs | 10 hrs | 12 hrs | 24 hrs |
| 240 | 0.0622887 | 0.10102 | 0.2004337 | 0.147062 |
| 472 | 0.0264916 | 0.17765 | 0.2737726 | 0.166645 |

### Example 52: 3-HP production via malonyl-coA in strains with additional combinations of FAS mutations

The following genetically modified *E. coli* strains (listed in Table 39) were constructed form a wild type BW25113 starting host. *E. coli* BW25113 was obtained from the Yale genetic stock center. These strains were constructed by standard methods discussed in the Common Methods Section and also known in the art as referenced above. Briefly, chromosomal modifications were constructed via homologous recombination.

**Table 39: Strain List 3**

| **Strain** | **Chromosomal genotype** | **Plasmid 1** | **Plasmid 2** |
|---|---|---|---|
| 240 | F-, Δ(araD-araB)567, ΔlacZ4787(::rrnB-3), LAM-, rph-1, Δ (rhaD-rhaB)568, hsdR514, ΔldhA:frt, ΔpflB:frt, mgsA:frt, ΔpoxB:frt, Δpta-ack:frt, fabIts (S241F)-zeoR | Ptrc-mcr | pACYC-accABCD-pntAB |
| 465 | F-, Δ (araD-araB)567, ΔlacZ4787(::rrnB-3), LAM-, rph-1, Δ(rhaD-rhaB)568, hsdR514 ΔldhA:frt, ΔpflB:frt, mgsA:frt, ΔpoxB:frt, Δpta-ack:frt, fabIts (S241F)-zeoR, ΔfabF | Ptrc-mcr | NA |
| 466 | F-, Δ (araD-araB)567, ΔlacZ4787(::rrnB-3), LAM-, rph-1, Δ (rhaD- rhaB)568, hsdR514,, ΔldhA:frt, ΔpflB:frt, mgsA:frt, ΔpoxB:frt, Δpta-ack:frt, fablts (S241F)-zeoR, ΔfabF | Ptrc-mcr | pACYC-accABCD |
| 467 | F-, Δ (araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fablts (S241F)-zeoR, ΔfabF | Ptrc-mcr | pACYC-accABCD-pntAB |
| 468 | F-, Δ (araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fablts (S241F)-zeoR, ΔfabF | Ptrc-mcr | pACYC-CAT-accADBC-udhA |
| 469 | F-, Δ (araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fabIts (S241F)-zeoR, ΔfabF | Ptrc-mcr | pBT3-tpiA-pntAB |

The above strains were evaluated in shake flasks for the production of 3-HP. Triplicate evaluations were performed. Briefly, overnight starter cultures were made in 50 mL of Luria Broth including the appropriate antibiotics and incubated 16-24 hours are 30°C, while shaking at 225 rpm. These cultures were used to inoculate 3 x 50 mL cultures of each strain in SM8 minimal medium with 5% culture, antibiotics, and 1 mM IPTG. Flasks were grown in the 30°C in shaking incubator, at 4, 8, 10, 12 and 24 hours, samples were taken for analyses of OD at 600nm and 3-HP production using the 3-HP bioassay described in the general methods section.

**Table 40: Specific 3-HP Production of FAS mutants.**

| | **Average Specific Productivity** (g 3-HP/gDCW-hr) | | | |
|---|---|---|---|---|
| Strain | 8 hrs | 10 hrs | 12 hrs | 24 hrs |
| 240 | 0.0622887 | 0.10102 | 0.2004337 | 0.147062 |
| 467 | 0.0741601 | 0.17242 | 0.2229593 | 0.126147 |

### Example 53: Polyketide Production via a FAS and CoA synthesis mutant strains

The following genetically modified *E. coli* strains (listed in Table 41) were constructed form a wild type BW25113 starting host. *E. coli* BW25113 was obtained from the Yale genetic stock center (New Haven, CT USA). These strains were constructed by standard methods discussed in the Common Methods Section and also known in the art as referenced above. Briefly, chromosomal modifications were constructed via homologous recombination.

**Table 41: Strain List 4**

| **Strain** | **Chromosomal genotype** | **Plasmid 1** | **Plasmid 2** |
|---|---|---|---|
| 6 | F-, Δ(araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fabIts (S241F)-zeoR | Ptrc-THNS | pACYC-accABCD |
| M1 | F-, Δ(araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fabIts (S241F)-zeoR, coaA(R106A) | Ptrc-THNS | pACYC-accABCD |
| M2 | F-, Δ(araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fabIts (S241F)-zeoR, coaA(R106A) | Ptrc-THNS | pACYC-accABCD |

The above strains were evaluated in shake flasks for the production of flaviolin. Triplicate evaluations were performed. Briefly, overnight starter cultures were made in 50 mL of Luria Broth including the appropriate antibiotics and incubated 16-24 hours are 30°C, while shaking at 225 rpm. These cultures were used to inoculate 3 x 50 mL cultures of each strain in SM8 minimal medium with 5% culture, antibiotics, and 1 mM IPTG with or without 40uM pantothenic acid. Flasks were grown in the 30°C in shaking incubator; at 24 hours, samples were taken for analyses of OD at 600nm and absorbance at 340 nm.

### Example 54: 3-HP Production via FAS mutant strains.

The following genetically modified *E. coli* strains (listed in Table 42) were constructed form a wild type BW25113 starting host. *E. coli* BW25113 was obtained from the Yale genetic stock center. These strains were constructed by standard methods discussed in the Common Methods Section and also known in the art as referenced above. Briefly, chromosomal modifications were constructed via recombination. Alternatively strains including any combination of fabI, fabB, fabF and or fabD mutations may be constructed and or evaluated.

**Table 42: Strain List 5**

| **Strain** | **Chromosomal genotype** | **Plasmid 1** | **Plasmid 2** |
|---|---|---|---|
| 240 | F-, Δ(araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fabIts (S241F)-zeoR | Ptrc-mcr | pACYC-accABCD-pntAB |
| M5 | F-, Δ(araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fabIts (S241F)-zeoR, fabD(ts) | Ptrc-mcr | NA |
| M6 | F-, Δ(araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fablts (S241F)-zeoR, fabD(ts) | Ptrc-mcr | pACYC-accABCD |
| M7 | F-, Δ(araD-araB)567, ΔlacZ4787(::rrnB-3), LAM-, rph-1, Δ(rhaD-rhaB)568, hsdR514,, ΔldhA:frt, ΔpflB:frt, mgsA:frt, ΔpoxB:frt, Δpta-ack:frt, fabIts (S241F)-zeoR, fabD(ts) | Ptrc-mcr | pACYC-accABCD-pntAB |

Each of the above strain are evaluated in shake flask experiments for the production of 3-HP using the methods discussed in the above examples.

### Example 55: Product production via malonyl-coA in strains with combinations of FAS mutations

In general strains are constructed from a wild type starting host. These strains are constructed by standard methods discussed in the Common Methods Section and also known in the art as referenced above. Chromosomal modifications are constructed via recombination. Strains including any combination of the mutations and modifications disclosed above may be combined, particularly including any combination of enoyl-acyl carrier protein (ACP) reductase (such as fabIi), β-ketoacyl-acyl carrier protein synthase I (such as fabB), β-ketoacyl-acyl carrier protein synthase II (such as fabF), and malonyl-CoA-acyl carrier protein transacylase (such as fabD) and or pantothenate kinase (such as coaA) mutations may be constructed and/or evaluated with or without the supplementation of pantothenate to the media.

### Example 56: Example of 3-HP Production

An inoculum of a genetically modified microorganism that possesses a 3-HP production pathway and other genetic modifications as described above is provided to a culture vessel to which also is provided a liquid media comprising nutrients at concentrations sufficient for a desired bio-process culture period.

The final broth (comprising microorganism cells, largely 'spent' media and 3-HP, the latter at concentrations, in various embodiments, exceeding 1, 2, 5, 10, 30, 50, 75 or 100 grams/liter) is collected and subjected to separation and purification steps so that 3-HP is obtained in a relatively purified state. Separation and purification steps may proceed by any of a number of approaches combining various methodologies, which may include centrifugation, concentration, filtration, reduced pressure evaporation, liquid/liquid phase separation (including after forming a polyamine-3-HP complex, such as with a tertiary amine such as CAS#68814-95-9, Alamine® 336, a triC8-10 alkyl amine (Cognis, Cincinnati, OH USA), membranes, distillation, evaporation, and/or other methodologies. Principles and details of standard separation and purification steps are known in the art, for example in *"*Bioseparations Science and Engineering," Roger G. Harrison et al., Oxford University Press (2003), and Membrane Separations in the Recovery of Biofuels and Biochemicals - An Update Review, Stephen A. Leeper, pp. 99-194, in Separation and Purification Technology, Norman N. Li and Joseph M. Calo, Eds., Marcel Dekker (1992), incorporated herein for such teachings. The particular combination of methodologies is selected from those described herein, and in part is based on the concentration of 3-HP and other components in the final broth.

Similar culture procedures may be applied for other chemical products disclosed herein.

### Example 57: Induction of malonyl-CoA reductase by fermentation under low-phosphate conditions

Plasmid maps are shown in the Figures. Plasmid 1 was digested with NcoI/Bst1107. A fragment size of 7059 bases was excised from a gel and purified (SEQ ID NO:168). The target promoter sequence was ordered (Integrated DNA Technologies, Coralville, Iowa USA) including with modifications to the native ribosome binding site and subsequently changed to be compatible with existing expression vectors and to accommodate expression of key downstream gene(s) within the vector(s), in this example malonyl CoA reductase (MCR, mcr). Plasmid 2, synthesized by (Integrated DNA Technologies, Coralville, IA USA) to comprise this low-phosphate promoter (see discussion above regarding SEQ ID NOs: 210 and 211), was digested with NcoI/PmlI. A fragment size of 156 bases was excised from a gel and purified, this fragment is the pYibD promoter (SEQ ID NO:210). Fragments were ligated overnight using T4 DNA ligase to create Plasmid 3, identified as pTrc-P_{yibD}-mcr (SEQ ID NO:170).

The pTrc_PyibD-mcr plasmid was evaluated by the standard shake flask protocol with variable phosphate levels.

### Example 58: Fermentation Events Using Strain 547

Strain 547 was prepared using molecular biology techniques described elsewhere herein, including use of homologous recombination to introduce and/or replace portions of the genome and construction and introduction of plasmids.

**Table 43: Strain List 6**

| **Strain** | **Chromosomal genotype** | **Plasmid 1** | **Plasmid 2** |
|---|---|---|---|
| 547 | F-, Δ(araD-araB)567, ΔlacZ4787(::rrnB-3), LAM-, rph-1, Δ(rhaD-rhaB)568, hsdR514, ΔldhA:frt, ΔpflB:frt, ΔmgsA:frt, ΔpoxB:frt, Δpta-ack:frt, fabIts (S241F)-zeoR, fabB(ts), ΔfabF:frt, coaA*, fabD(ts), ΔaceBAK:frt | pTRC-PyibD-mcr | pACYC-Ptal-pntAB-Ptpia-accAD-PrpiA-accCD |

This strain was then evaluated under various fermentations conditions that modulated or otherwise controlled temperature, pH, oxygen concentration, glucose feed rate and concentration, and other media conditions. Among the parameters used to determine other process steps, low ambient phosphate concentration was used as a control point that lead to temperature shift from approximately 30°C to approximately 37°C. As noted elsewhere, a promoter sensitive to low ambient phosphate was utilized to control expression of the gene encoding malonyl-CoA reductase in the plasmid identified as pTrc-P_{yibD}-mcr. Also, during one or more evaluations, any one or more of dissolved oxygen, redox potential, aeration rate, agitation rate, oxygen transfer rate, and oxygen utilization rate was/were used to control the system and/or measured.

Strain BX3_547 was evaluated for 36 fermentation events that were conducted over an 8 week period using FM11 medium (described in the Common Methods Section). The duration of the fermentation events were all less than approximately 80 hours, of which a portion was after temperature increase to effectively reduce enzymatic activity of fabI(ts), fabB(ts), and fabD(ts).

Overall the results demonstrated microbial performance over a range of reduced oxygen conditions, with final 3-HP titers ranging between 50 and 62 grams of 3-HP/liter of final culture media volume. It is appreciated that the P_{yibD} promoter, or a similar low-phosphate induction promoter, could be utilized in a genetic construct to induce any one or more of the sequences described and/or taught herein, so as to enable production of any other of the chemical products disclosed herein, including in the other examples above.

### Example 59: 3HP production via malonyl-coA in strains with combinations of changes to increase pyruvate dehydrogenase activity and reduce pathway inhibition.

A strong constitutive promoter, PT5 from the bacteriophage T5 (SEQ ID NO:171), was cloned upstream of the pyruvate dehydrogenase (PDH) operon (aceEF, lpd) on a plasmid using standard molecular biology techniques described elsewhere herein. The entire operon (T5_aceEF-lpd) (SEQ ID NO: 172) was inserted into the puuC locus *of E. coli* deleting the native puuC coding sequence by using homologous recombination techniques to introduce and/or replace portions of the genome described elsewhere herein. This insertion creates a strain with two chromosomal copies of the PDH operon: one of which is transcribed by the native PDH promoter and one that is transcribed by the strong T5 promoter. In addition a mutation was made in some strains (see strain list below) in the lpdA (E354K) protein to cause the pyruvate dehydrogenase complex to be less sensitive to NADH inhibition and active during anaerobic growth (Kim et al. (J. Bacterial. 190:3851-3858, 2008). This mutation was made in either copy of lpd and in both copies creating the strains in Table 44.

**Table 44: Strain List 7**

| **Strain** | **Chromosomal genotype** | **PDH Status** |
|---|---|---|
| 775 | F-, Δ(araD-araB)567, ΔlacZ4787(::rrnB-3), LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, fabB(ts)-(A329V), ΔfabF:frt, coaA(R106A), fabD(ts)-(W257Q), ΔaceBAK:frt, | aceEF_ lpdA= native promoter and protein sequence |
| 770 | F-, Δ(araD-araB)567, ΔlacZ4787(::rrnB-3),LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts)-(A329V), ΔfabF::frt, coaA(R106A), fabD(ts)-(W257Q), ΔaceBAK:frt, lpd(E354K):loxP | aceEF_ lpda (E354K) = native promoter and mutated lpd |
| 801 | F-, Δ(araD-araB)567, ΔlacZ4787(::rrnB-3),LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts)-(A329V), ΔfabF::frt, coaA(R106A), fabD(ts)-(W257Q), ΔaceBAK:frt, ΔpuuC:T5-aceEF-lpd(E354K):loxP | aceEF_ lpdA = native promoter and protein sequence plus-ΔpuuC:T5-aceEF-lpd(E354K) |
| 803 | F-, Δ(araD-araB)567, ΔlacZ4787(::rrnB-3),LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts)-(A329V), ΔfabF::frt, coaA(R106A), fabD(ts)-(W257Q), ΔaceBAK:frt, lpd(E354K):loxP, ΔpuuC:T5-aceEF-lpd(E354K):loxP | aceEF_lpdA (E354K) = native promoter and mutated lpd plus-ΔpuuC:T5-aceEF-lpd(E354K) |

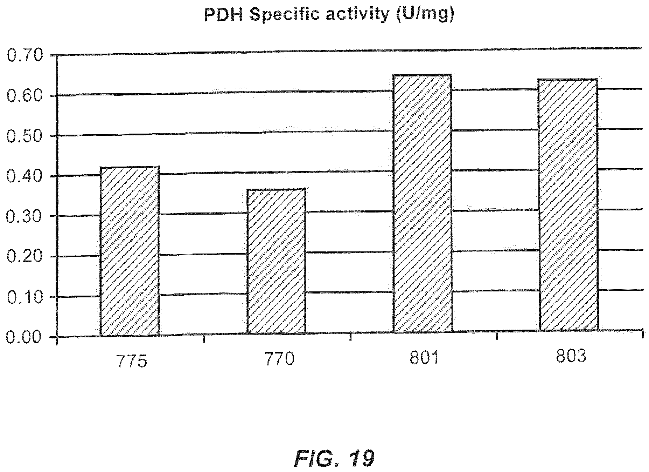

The above strains were evaluated in shake flasks for pyruvate dehydrogenase activity (assay is described in the Common Methods Section). The results in Figure 19 show an increase of PDH activity from ∼0.4 U/mg to >0.6U/mg in strains expressing the PT5-PDH operon over wild type strains.

### Example 60: Construction of a 3HP production strain via malonyl-CoA with combinations of genetic changes to reduce degradation of 3HP, reduce byproduct formation, and utilize sucrose feedstocks.

Strains were designed to reduce or eliminate enzymatic activity through the 3HP degradation pathway or competitive byproduct formation. Modifications were optionally further selected from one or more of the following gene deletions: lactate dehydrogenase (ldhA), pyruvate formate lyase (pflB), methylglyoxyl synthase (mgsA), pyruvate oxidase (poxB), phosphate acetyltransferase (pta), acetate kinase (ackA), aldehyde dehydrogenase A (aldA), acetaldehyde dehydrogenase B (aldB), alcohol dehydrogenase (adhE), γ-glutamyl-γ-aminobutyraldehyde dehydrogenase (puuC), malate synthase (aceB), isocitrate lyase (aceA), isocitrate dehydrogenase (aceK), and KASII (fabF). Additional modifications may include incorporation of additional FAS mutations described in other examples herein, incorporation of the sucrose utilization operon (cscBKA genes) into the aldA locus, incorporation of the PT5-aceEF-lpd operon into the puuC locus described in other examples herein, incorporation of a mutation in pantothenate kinase which is refractory to feedback inhibition (coaA*) (SEQ ID NO 173), and an additional insertion of the PyibD-T7PolI gene into the aldB locus.

The following genetically modified *E. coli* strains (listed in Table 45) were constructed from a wild type BW25113 starting host. *E. coli* BW25113 was obtained from the Yale genetic stock center (New Haven, CT USA). These strains were constructed by standard methods such as discussed in the Common Methods Section and also known in the art as referenced above.

**Table 45: Strain List 8**

| **Strain** | **Chromosomal genotype** | **Plasmid 1** | **Plasmid 2** |
|---|---|---|---|
| 547 | F-, Δ(araD-araB)567, ΔlacZ4787(::rrnB-3),LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, fabB(ts)-(A329V), ΔfabF:frt, coaA(R106A), fabD(ts)-(W257Q) ΔaceBAK:frt, | pTRC-kan-PyibD-mcr, | pACYC-Ptpia-accAD-PrpiA- |
| 571 | F-, Δ(araD-araB)567, ΔlacZ4787(::rrnB-3),LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, fabB(ts)-(A329V), ΔfabF:frt, coaA(R106A), fabD(ts)-(W257Q), ΔlacI:frt, ΔpuuC::T5-aceEF -lpd(E354K):loxP, ΔaceBAK:frt, lpd(E354K):loxP, ΔaldB:PyibD-T7pol:loxP, ΔadhE:frt, ΔaldA:cscBKA | pTRC-PyibD-mcr | pACYC-PyibD-accADBC |

### Example 61: Construction of a 3HP production strain via malonyl-CoA that does not require the addition of antibiotics.

Strains were designed to eliminate the need for antibiotic supplementation to the medium to maintain plasmid stability. Modifications to maintain plasmid stability without antibiotics may include auxotrophic markers (gapA) or toxin/ antitoxin systems (ccdAB). The following genetically modified *E. coli* strains (listed in Table 46) were constructed from a wild type BW25113 starting host. *E. coli* BW25113 was obtained from the Yale genetic stock center (New Haven, CT USA) using standard methods as described in other examples herein.

**Table 46: Strain List 9**

| **Strain** | **Chromosomal genotype** | **Plasmid 1** | **Plasmid 2** |
|---|---|---|---|
| 240 | F-, Δ(araD-araB)567, ΔlacZ4787(::rrnB-3),LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR | pTRC-Ptrc-mcr | pACYC-Ptal-pntAB-Ptpia-accAD-PrpiA-accCD |
| 501 | F-, Δ(araD-araB)567, ΔlacZ4787(::rrnB-3), LAM-, rph-1, Δ(rhaD-rhaB)568, hsdR514, ΔldhA:frt, ΔpflB:frt, ΔmgsA:frt, ΔpoxB:frt, Δpta-ack:frt, fabIts (S241F)-zeoR, ΔgapA | pTRC-Ptrc-mcr-Pkan-gapA | pACYC-Ptal-pntAB-Ptpia-accAD-PrpiA-accCD-Pkan-ccdAB |

The above strains were evaluated for production in the absence of antibiotic addition in 1L fermentation systems.

**Table 47: Fermentation metrics for strains with and without antibiotic additions**

| | BX3_ 240 (with antibiotics) | BX3_240 (without antibiotics) BX3_501 (without antibiotics) | |
|---|---|---|---|
| Max Specific Growth (1/hr) | 0.28 | 0.29 | 0.3 |
| Avg Specific Growth (1/hr) | 0.31 | 0.22 | 0.22 |
| Max Volumetric 3-HP Rate (g/L/hr) | 3.43 | 3.1 | 7.04 |
| Max Specific 3-HP Rate (g/gDCW/hr) | 0.25 | 0.26 | 0.57 |
| Avg Volumetric 3-HP Rate (g/L/hr) | 1.73 | 1.96 | 1.94 |
| Avg Specific 3-HP Rate (g/gDCW/hr) | 0.14 | 0.17 | 0.18 |
| Max Titer (g/L) | | 47.3 | 48.2 |
| Yield During Production | 57 | 55.9 | 56.1 |

### Example 62: Utilization of sucrose as the feedstock for production of 3-HP and other products

### Cloning of csc genes

Common laboratory and industrial strains *of E. coli,* such as the strains described herein, are not capable of utilizing sucrose as the sole carbon source, although this property is found in a number of wild strains, including pathogenic *E. coli* strains. Sucrose, and sucrose-containing feedstocks such as molasses, are abundant and often used as feedstocks for the production by microbial fermentation of organic acids, amino acids, vitamins, and other products. Thus further derivatives of the strains described herein that are capable of utilizing sucrose would expand the range of feedstocks that can be utilized to produce 3-HP and other products.

Various sucrose uptake and metabolism systems are known in the art (for example, U.S. Pat. No. 6,960,455), incorporated by reference for such teachings. We describe the construction of *E. coli* strains that harbor the *csc* genes conferring the ability to utilize sucrose via a non-phosphotransferase system, wherein the *csc* genes constitute *cscA*, encoding a sucrose hydrolase, *cscB*, encoding a sucrose permease, *cscK*, encoding a fructokinase, and *cscR,* encoding a repressor. The sequences of these genes are annotated in the NCBI database as accession No. X81461 AF473544. To allow efficient expression utilizing codons that are highly abundant in *E*. *coli* genes, an operon containing *cscB*, *cscK*, and *cscA* was designed and synthesized using the services of a commercial synthetic DNA provider (DNA 2.0, Menlo Park, CA). The sequences of the genes are set forth as, respectively, cscB - SEQ. ID. No. 176; cscA - SEQ. ID. No. 177; csck - SEQ. ID. No. 178. The synthetic operon consisted of 60 base pairs of the region of the *E. coli* genome immediately 5' (upstream) of the *adhE* gene, a consensus strong promoter to drive expression of the *csc* genes, the coding regions for *cscB*, *cscK*, and *cscA* with short intergenic regions containing ribosome binding sites but no promoters, and 60 bp immediately 3' (downstream) of the *adhE* gene. The segments homologous to sequences flanking the *adhE* gene may be used to target insertion of the *csc* operon genes into the *E. coli* chromosome, with the concomittent deletion of *adhE.* The nucleotide sequence of the entire synthetic construct is shown as SEQ. ID. No. 179.

The synthetic *csc* operon was constructed in plasmid pJ214 (DNA 2.0, Menlo Park, CA) that provides an origin of replication derived from plasmid p15A and a gene conferring resistance to ampicillin. This plasmid is denoted pSUCR and shown as SEQ. ID No. 213. Transformation of a suitable host cell, such as *E. coli* strain 595, with pSUCR rendered it capable of growth on sucrose as the sole carbon source, where the host bearing a control plasmid without the csc gene cluster was not able to utilize this feedstock.

### Chromosomal integration of csc genes

The *csc* gene cluster was integrated into into the *aldA* locus *of E. coli* to generate strains that stably carried the sucrose utilization trait. The promoter - *csc* region of pSUCR was amplified using primers:
(SEQ ID NO: 214)HL021: 
ATTTCTGCCTTTTATTCCTTTTACACTTGTTTTTATGAAGCCCTTCACAGAATTGTCCT
TTCACGAAAACATTGACATCCCTATCAGTGA
(SEQ ID NO:215)HL022:

CACTCATTAAGACTGTAAATAAACCACCTGGGTCTGCAGATATTCATGCAAGCCATG
TTTACCATAAGCTTAACCCAGTTGCCACAGTGC

### Improving sucrose utilization rates

To increase the utilization of sucrose, the sucrose permease encoded by the *cscB* gene, known to be the slowest step in sucrose utilization, was mutagenized and subjected to selection for increased sucrose growth rates. Mutagenic PCR was used to generate a number of libraries, each of ∼20,000 individuals, with average mutation rates ranging from 2.7 to 17.8 changes per 1000 bases, using the Diversity PCR Random Mutagenesis Kit (Clontech Laboratories, Mountain View, CA). Each library was digested with *Nco*I and *Bgl*II and ligated to pSUCR similarly digested and purified by agarose gel electrophoresis to recover the plasmid fragment from which the parental *cscB* gene was removed. The mutagenic plasmid library was then transformed into Ecloni 10G Elite electrocompetent cells (Lucigen, Madison WI). The mutagenic frequency was determined by sequencing 6 unselected clones from each transformation. Each library was recovered by scraping the colonies off transformation plates and extracting the plasmids with multiple minipreps (Qiagen). The library with the lowest number of changes per 1000 bases was transformed into 595 and the entire transformation mixture passaged repeatedly in SM8 medium containing 30g/L sucrose for 11 transfers. Twelve individual colonies were isolated from this enriched culture and sequenced. Seven (mutants designated A, B, D, E, G, H, I) of the 12 have the identical *cscB* sequence, and carry 3 coding mutations (N234D, I312V, I369V). In addition, mutant J has only the N234D change, while mutant C carries the N234D and I313V changes. In this nomenclature, the first letter denotes the amino acid present in the wildtype protein (using the one-letter code where N is asparagine), the numbers represent the position of the residue in the protein chain, and the second letter denotes the amino acid present in the mutant (where D is aspartic acid). The other clones isolated from this enrichment procedure, although carrying changes in the *cscB* gene, did not grow when re-inoculated into sucrose medium. The sequence of cscB mutant 1A is included as SEQ. ID No. 180. Strains 535 and 536 were constructed from hosts carrying mutant 1A as shown in Table 48 below.

**Table 48: Strain List 10**

| **Strain** | **Chromosomal genotype** | **Plasmid 1** | **Plasmid 2** |
|---|---|---|---|
| 535 | F-, Δ(araD-araB)567, ΔlacZ4787(::rrnB-3),LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, ΔaldB:cscB(1A)AK | pTRC-Ptrc-mcr | pACYC-Ptal-pntAB-Ptpia-accAD-PrpiA-accCD |
| 536 | F-, Δ(araD-araB)567, ΔlacZ4787(::rrnB-3),LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, ΔaldB:escB(1A)AK | pTRC-Ptrc-mcr-glk | pACYC-Ptal-pntAB-Ptpia-accAD-PrpiA-accCD |

### Example 63: 3-HP production via malonyl-CoA in strains with improved acetyl-CoA carboxylase activity

Plasmid map 4 shown below (pACYC-T7-rbs accADBC) was constructed by subcloning the gene synthesized accADBC construct with optimized ribosome binding sites for each subunit (GenScript) (SEQ ID NO: 182) into pACYC-DUET (Novagen) at the EcoICRI and EcorV restriction sites to make the final pACYC-T7-rbs accADBC construct (SEQ ID NO: 183).

Plasmid pACYC-pyibD-rbsaccADBC (SEQ ID NO: 184) shown in Plasmid Map 5 was constructed by replacement of the T7 promoter with the pyibD promoter by conventional cloning methods as described elsewhere herein.

The strains described in Table 49 were evaluated in shake flasks for increased acetyl-CoA carboxylase (AccAse) activity. Triplicate evaluations were performed. Briefly, overnight starter cultures were made in 50 mL of Luria Broth including the appropriate antibiotics and incubated 16-24 hours are 30°C, while shaking at 225 rpm. These cultures were used to inoculate 3 x 100 mL cultures of each strain in SM11 minimal medium and antibiotics to an OD 600nm = 0.2. Flasks were grown in the 30°C in a shaking incubator. When the cultures reach an OD600 nm = 0.5, the strains were induced by either phosphate depletion or 0.3 mM IPTG and further incubated at 30C. After 3 hours, the cultures were shifted to 37C. Samples were taken for analyses of AccAse enzyme activities after 24 hours as described in the Common Methods Section.

**Table 49: Strain List 11**

| **Strain** | **Chromosomal genotype** | **Plasmid 1** | **Plasmid 2** |
|---|---|---|---|
| 1 | F-, Δ(araD-araB)567, ΔlacZ4787(::rrnB-3), LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, fabB(ts)-(A329V), ΔfabF:frt, coaA(R106A), fabD(ts)-(W257Q), ΔaceBAK:frt, DE3 | pTRC-Ptrc-mcr | pACYC (empty vector) |
| 2 | F-, Δ(araD-araB)567, ΔlacZ4787(::rrnB-3), LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, fabB(ts)-(A329V), ΔfabF:frt, coaA(R106A), fabD(ts)-(W257Q), ΔaceBAK:frt, DE3 | pTRC-Ptrc-mcr | pACYC-Ptpia-accAD-PrpiA-accBC |
| 3 | F-, Δ(araD-araB)567, ΔlacZ4787(::rrnB-3), LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, fabB(ts)-(A329V), ΔfabF:frt, coaA(R106A), fabD(ts)-(W257Q), ΔaceBAK:frt, DE3 | pTRC-Ptrc-mcr | pACYC-PT7-accADBC |
| 4 | F-, Δ(araD-araB)567, ΔlacZ4787(::rrnB-3), LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, fabB(ts)-(A329V), ΔfabF:frt, coaA(R106A), fabD(ts)-(W257Q), ΔlacI:frt, ΔpuuC:T5-aceEF-lpd*, ΔaceBAK:frt, lpd*:loxP, ΔaldB:PyibD-T7poI:BSD, ΔadhE:frt | pTRC-PyibD-mcr | pACYC-Ptpia-accAD-PrpiA-accBC |
| 5 | F-, Δ(araD-araB)567, ΔlacZ4787(::rrnB-3), LAM-, rph-1, Δ(rhaD-rhaB)568, hsdR514, ΔldhA:frt, ΔpflB:frt, ΔmgsA:frt, ΔpoxB:frt, Δpta-ack:frt, fabI(ts)-(S241F)-zeoR, fabB(ts)-(A329V), ΔfabF:frt, coaA(R106A), fabD(ts)-(W257Q), Δlacl:frt, ΔpuuC:T5-aceEF-lpd*, ΔaceBAK:frt, lpd*:loxP, ΔaldB:PyibD-T7pol:BSD, ΔadhE:frt | pTRC-PyibD-mcr | pACYC-PyibD-accADBC |

| | | ACCase activity (umoles/min/mg) | |
|---|---|---|---|
| | | | |
| 1 | | 0.003 | |
| 2 | | 0.109 | |
| 3 | | 0.206 | |
| 4 | | 0.09 | |
| 5 | | 0.22 | |

### Example 64: 3-HP production via malonyl-coA in strains with additional combinations of FAS mutations by 1L fermentation

Strains 472 and 479 (Table 50) were constructed by standard molecular biology techniques as described elsewhere herein. Two 1 L fed batch fermentation experiments were carried out using strains 472 and 494. Seed culture was started from 1 ml of glycerol stock for each strain inoculated into 100 mL of TB medium (Terrific Broth) in a corresponding shake flask and incubated at 30°C until the OD₆₀₀ was between 5 and 6. The shake flask culture was used to aseptically inoculate (5% volume/volume) the corresponding 1 L volume bioreactor so that the post-inoculation volume was 800 ml in each vessel. The fermentors used in this experiment were Das Gip fed-batch pro parallel fermentation systems (DASGIP AG, Julich, Germany, model SR0700ODLS). The fermentation system included real-time monitoring and control of dissolved oxygen (%DO), pH, temperature, agitation, and feeding. All fermentors contained defined FM8 medium, made as shown in the Common Methods Section. In each fermentor, the initial temperature was 30°C. Induction was effected by adding IPTG to a final concentration of 2 mM when phosphate is depleted. Glucose feed (consisting of a 500 g/L glucose solution) was initiated to maintain glucose levels between 1-10 g/L. At induction, the temperature was shifted to 37°C over 1 hour. The broth of each fermentor was maintained at a pH of approximately 7.4 by the controlled addition of a pH titrant 50% NH4(OH). At the time the temperature shift was initiated, the DO was changed to 1%. Samples were taken for optical density measurements as well as HPLC analysis for 3-HP concentration. The biomass concentration at harvest as well as the maximum 3-HP titer and specific 3HP rate during production are summarized in the Table 50 below.

**Table 50: Strain List 12**

| **Strain** | **Chromosomal genotype** | **Plasmid 1** | **Plasmid 2** |
|---|---|---|---|
| 472 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts), ΔfabF::frt | pTRC-Ptrc-mcr | pACYC-Ptal-pntAB-Ptpia-accAD-PrpiA-accCD |
| 479 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, ΔarcA, lpd*,fabB(ts), | pTRC-Ptrc-mcr-glk | pACYC-Ptal-pntAB-Ptpia-accAD-PrpiA-accCD |

**Table 51**

| Strain | Airflow (vvm) | Agitation During Production (rpm) | Biomass Conc. at Harvest (g DCW/L) | 3HP Titer (g/L) at 40 hrs | Specific 3HP Rate (g/gDCW/hr) over 40 hours |
|---|---|---|---|---|---|
| BX3 472 | 1.2 | 600 | 6.6 | 39.2 | 0.15 |
| BX3 494 | 1.2 | 670 | 10.0 | 55.9 | 0.16 |

### Example 65: 3-HP production via malonyl-CoA using strain 571

Strain 571 was constructed by standard molecular biology techniques as described elsewhere herein. The genotype and corresponding plasmids used to construct 571 are listed in Table 52. 3-HP production by strain 571 is demonstrated at 100-mL scale in SM11 (minimal salts) media. Cultures were started from freezer stocks by standard practice (Sambrook and Russell, 2001) into 50 mL of SM11 media plus 35 µg/mL kanamycin and 20 µg/mL chloramphenicol and grown to stationary phase overnight at 30°C with rotation at 250 rpm. Three mL of this culture was transferred to 100 ml of SM11 media without phosphate plus 30 g/L glucose, 35 µg/ml kanamycin, and 20 µg/mL chloramphenicol in triplicate 250-ml baffled flasks and incubated at 30°C, 250 rpm. The flasks are shifted to 37°C six hours post inoculation. To monitor growth rate, samples (2 ml) are withdrawn at designated time points for optical density measurements at 600nm (OD₆₀₀, 1 cm path length). To monitor 3-HP production by these cultures, samples (10 mL) are pelleted by centrifugation at 12,000rpm for 5 min and the supernatant collected for analysis of 3-HP titer as described under "Analysis of cultures for 3-HP production" in the Common Methods section. Dry cell weight (DCW) is calculated as 0.40 times the measured OD₆₀₀ value, based on baseline DCW to OD₆₀₀ determinations. All data are the average of triplicate cultures. The average specific rate is calculated from the averaged data at the 24-h time point and expressed as g 3-HP produced per gDCW over 16 hours. Production of 3-HP by strain 571 in SM11 medium is shown in Table 52.

**Table 52: Strain List 13**

| **Strain** | **Chromosomal genotype** | **Plasmid 1** | **Plasmid 2** |
|---|---|---|---|
| 571 | F-, Δ(araD-araB)567, ΔlacZ4787(::rrnB-3),LAM-, rph-1, Δ(rhaD-rhaB)568, hsdR514, ΔldhA:frt, ΔpflB:frt, ΔmgsA:frt, ΔpοxB:frt, Δpta-ack:frt, fabI(ts)-(S241F)-zeoR, fabB(ts)-(A329V), ΔfabF:frt, coaA(R106A), fabD(ts)-(W257Q), ΔlacI:frt, ΔpuuC::T5-aceEF-lpd(E354K):loxP, ΔaceBAK:frt, lpd(E354K):loxP, ΔaldB:PyibD-T7pol:loxP, ΔadhE:frt, ΔaldA:cscBKA | pTRC-PyibD-mcr | pACYC-PyibD-accADBC |

**Table 53. Production of 3-HP by 571 in SM11 medium**

| Time (hr) | 3HP (g/L) | Specific Rate g 3HP/gDCW/hr |
|---|---|---|
| 6 | 0 | 0 |
| 10 | 0 | 0 |
| 24 | 1.8 | 0.37 |

### Example 66: Construction of strains carrying the glutamate dehydrogenase from the Antarctic psychrotolerant bacterium Psychrobacter sp. TAD1 and deletion of glutamate synthase function.

Deletions and replacements of genes within strains were carried out using the Red/ET Recombination system commercially available from Gene Bridges (Heidelberg, Germany) by following the manufactures instructions. For deletion of the gdhA gene within strains, a kanamycin carrying cassette for the deletion of gdhA was created by polymerase chain reaction using genomic DNA from the Keio collection (Baba et al, 2000) knockout strain for gdhA as template, using the forward primer (SEQ ID NO: 185) and reverse primer (SEQ ID NO: 186) for the reaction. For deletion of the gltB gene within strains, a kanamycin carrying cassette for the deletion of gltB was created by polymerase chain reaction using genomic DNA from the Keio collection (Baba et al, 2000) knockout strain for gltB as template, using the forward primer (SEQ ID NO: 187) and reverse primer (SEQ ID NO: 188) for the reaction. For deletion of the gltD gene within strains, a kanamycin carrying cassette for the deletion of gltD was created by polymerase chain reaction using genomic DNA from the Keio collection (Baba et al, 2000) knockout strain for gltD as template, using the forward primer (SEQ ID NO: 189) and reverse primer (SEQ ID NO: 190) for the reaction. After insertion of any of these deletion cassettes, the antibiotic marker was removed from the genome-integrated strain using FLP-mediated site specific recombination via the 708-FLPe, cm expression plasmid with chloramphenicol resistance marker from Gene Bridges (Heidelberg, Germany) using the manufactures instructions.

Replacement of the *E.coli* gdhA gene with the glutamate dehydrogenase gdh gene from the Antarctic psychrotolerant bacterium *Psychrobacter sp. TAD1* was performed using the using the Red/ET Recombination system commercially available from Gene Bridges (Heidelberg, Germany) by following the manufactures instructions. For these replacements, the *E.coli* gdhA gene was replaced with the *Psychrobacter sp. TAD1* gdh gene using a replacement cassette produced via a polymerase chain reaction. The template for the polymerase chain reaction was produced using the gene synthesis servicers of GenScript (Piscataway, NJ). Additional, the gdh gene from the Antarctic psychrotolerant bacterium *Psychrobacter sp. TAD1* was codon optimized using methods developed by GenScript. The gene synthesized cassette contained the codon optimized gdh gene, a blasticidin resistance selection marker, and homology regions targeted toward the upstream and downstream regions of *the E.coli* gdhA genomic region. The sequence of the plasmid provided by GenScript carrying this replacement cassette is provided as (SEQ ID NO: 191). This replacement cassette was amplified with that forward primer (SEQ ID NO: 185) and reverse primer (SEQ ID NO: 186) for the polymerase chain reaction and inserted into strains using the Gene Bridges method.

Using these various deletion and replacement cassettes, the several strains were created as shown in the Table 54 below using strain 822 as the parent strain.

**Table 54: Strain List 14**

| **Strain** | **Chromosomal genotype** |
|---|---|
| 822 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts), ΔfabF::frt, coaA*, fabD(ts), ΔlacI::frt, ΔpuuC::T5-aceEF-lpd*::loxP, ΔaceBAK::frt, lpd*::loxP, ΔaldB::PyibD- |
| 841 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts), ΔfabF::frt, coaA*, fabD(ts), ΔlacI::frt, ΔpuuC::T5-aceEF-lpd*::loxP, ΔaceBAK::frt, lpd*::loxP, ΔaldB::PyibD- |
| 842 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts), ΔfabF::frt, coaA*, fabD(ts), ΔlacI:frt, ΔpuuC::T5-aceEF-lpd*::loxP, ΔaceBAK::frt, lpd*::loxP, ΔaldB::PyibD- |
| 853 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts), ΔfabF::frt, coaA*, fabD(ts), ΔlacI::frt, ΔpuuC::T5-aceEF-lpd*::loxP, ΔaceBAK::frt, lpd*::loxP, ΔaldB::PyibD-T7pol::loxP, ΔadhE::frt, ΔaldA::CSC, gdhA(ts)::BSD |
| 844 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts), ΔfabF::frt, coaA*, fabD(ts), ΔlacI::frt, ΔpuuC::T5-aceEF-lpd*::loxP, ΔaceBAK::frt, lpd*::loxP, ΔaldB::PyibD- |

### Example 67: Evaluation of strains with controllable glutamate production using the Antarctic psychrotolerant bacterium Psychrobacter sp. TAD1

In order to evaluate the ability to control glutamate production, cultures of various strains were grown at 30 degrees Celsius for 8 hours and then shifted to 37 degrees Celsius and 40 degrees Celsius and grown for an additional 16hr. At these increased temperatures, the activity of the Antarctic psychrotolerant bacterium *Psychrobacter sp. TAD1* gdh gene should lower significantly as compared to the levels detected at 37C. Activities were measured as described in the Common Methods Section. The result of this experiment is shown in Figures 26-28. Specific activities were calculated from cultures of strains carrying the *Psychrobacter sp. TAD1* gdh gene alone (strain 853) or the gltB deletion and *Psychrobacter sp. TAD1* gdh gene in combination (strain 842). Upon the shift and growth at 37 degrees Celsius and 40 degrees Celsius, this activity decreased significantly. This result shows that the *Psychrobacter sp. TAD1* gdh gene product is inhibited at these temperatures.

### Example 68: Increased production rates, titers, and yields using strains carrying glutamate dehydrogenase from the Antarctic psychrotolerant bacterium Psychrohacter sp. TAD1 and deletion of glutamate synthase function.

The strains listed in Table 55 below were created to evaluate glutamate/glutamine production in a 3-HP production strain.

**Table 55: Strain List 15**

| **Strain** | **Chromosomal genotype** | **Plasmid 1** | **Plasmid 2** |
|---|---|---|---|
| 579 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts), ΔfabF::frt, coaA*, fabD(ts), ΔlacI::frt, ΔpuuC::T5-aceEF-lpd*::loxP, ΔaceBAK::frt, lpd*::loxP, ΔaldB::PyibD-T7pol::loxP, ΔadhE::frt, ΔaldA::CSC | pTRC-PyibD-mcr(st)-PyibD-mmsB | pACYC-PyibD-accADBC |
| 593 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts), ΔfabF::frt, coaA*, fabD(ts), ΔlacI::frt, ΔpuuC::T5-aceEF-lpd*::loxP, ΔaceBAK::frt, lpd*::loxP, ΔaldB::PyibD-T7pol::loxP, ΔadhE::frt, ΔaldA::CSC, ΔgltB::frt, gdhA(ts)::BSD | pTRC-PyibD-mcr(st)-PyibD-mmsB | pACYC-PyibD-accADBC |

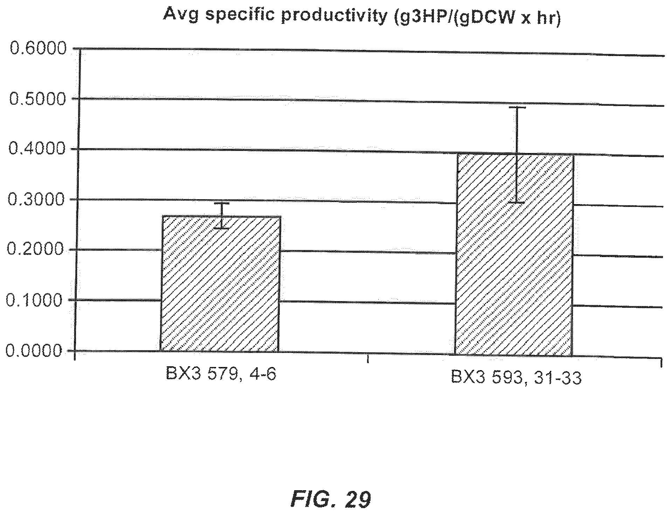

These strains were grown in shake flasks at 30 degree Celsius for 6 hours in SM11 media without phosphate to induce protein production for the production pathway. At 6 hours, the cultures were shifted to 37 degrees Celsius to shut down fatty acid production and lower the glutamate dehydrogenase activity. The results for these shake flasks are shown Figure 29. This experiment shows that strain 593 showed an average specific productivity that was greater strain 579.

### Example 69: Alternative method for limiting or controlling glutamate production.

Other routes for limiting glutamate production include 1) deleting the glutamate dehydrogenase gene or 2) deleting the glutamate dehydrogenase gene and regulating expression of glutamate synthase genes or gene products . In this embodiment, the regulation of gltB or gltD could be controlled altering expression as is common in the art or alternatively by using a temperature sensitive version of the glutamate synthase as described above. Such temperature sensitive versions of the gltB and gltD could be isolated in a manner similar to those used by Dendinger et al. (1980) to isolate temperature-sensitive versions of the Salmonella gdhA gene. Alternatively, other methods using sequence and structural-based information could be used to create variants of gltB, gltD, or gdhA genes similar to those described in Chakshusmathi et al (2004).

### Example 70: Construction of the Sulfolobus tokodaii mcr gene for expression

The MCR gene from *Sulfolobus tokodaii* was synthesized using the services of GenScript (Piscataway, NJ) using GenScripts' codon optimization methods for expression in E. coli. This gene was synthesized with a Ptrc promoter and was designated pUC57-Ptrc-StMCR (SEQ ID NO: 192).

### Example 71: Biochemical assays to measure Sulfolobus tokodaii MCR activity and combining malonyl-CoA reductase and 3-HP dehydrogenase activities

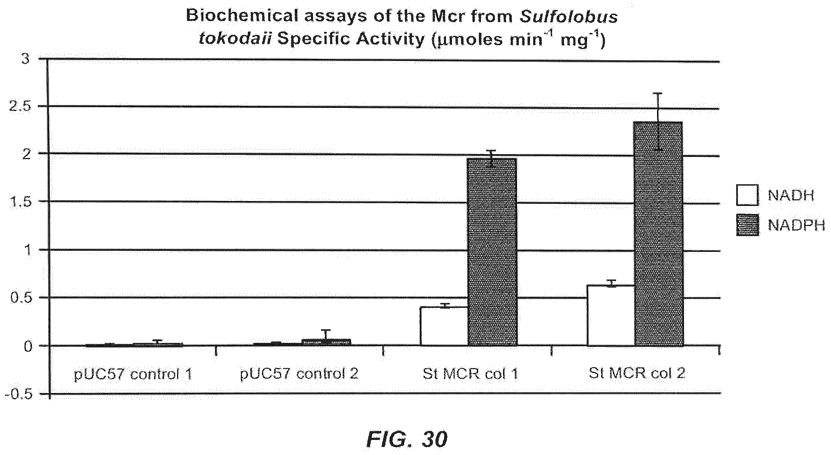

In order to evaluate the use *Sulfolobus tokodaii* MCR, lysates for assaying the specific activities were prepared from over expressed cultures. The results for the specific activity of *Sulfolobus tokodaii* MCR using NADH and NADPH cofactors, independently, are shown in Figure 30. In this experiment, culture begun from 2 different colonies for E. coli cells carrying either the pUC57 control plasmid or the pUC57-Ptrc-StMCR plasmid able to overexpress *Sulfolobus tokodaii* MCR. This experiment show no detectable malonyl CoA reductase activity in the two control colonies tested, and shows robust malonyl CoA specific activity in the culture overexpressing *Sulfolobus tokodaii* MCR. The activity with NADPH in these lysates was at least 1.9 units per mg lysate in these experiments, and agrees well for the reported specific activity of 44 units per mg for the purified proteins as evaluated by Alber et al. (2006). In addition to NADPH cofactor, NADH was also assessed as a potential cofactor even though no such activity was previous reported. The activity of *Sulfolobus tokodaii* MCR was only 4.2 less active than the specific activity with NADPH. Accordingly, this activity and this only mild preference for NADPH potentially make this enzyme more suitable than other malonyl CoA reductases depending on the fermentation process used.

The results of these experiments are shown in Figure 30. These results demonstrate that *Sulfolobus tokodaii* MCR can utilize NADH as a cofactor as well as the previously reported NADPH cofactor. None of the samples evaluating the dehydrogenases or *Sulfolobus tokodaii* MCR, independently, show significant 3HP formation. Conversely, reactions contain the combination *Sulfolobus tokodaii* MCR and any of the dehydrogenase were able to make significant amounts of 3HP. The reaction containing *Sulfolobus tokodaii* MCR and the E.coli ydfG overexpressing lysate showed less production of 3HP. Since *E.coli* ydfG has a strong preference for NADPH, this result shows how different combinations of malonyl coA reductase domains and dehydrogenase domains with various preferences for either NADPH or NADH could be exploited to yield better production characteristics depending on the fermentation conditions which are known to influence the ratios and amounts of NADPH and NADH within cells.

Production of 3-HP from malonyl-CoA can in addition be achieved with a NADH-dependent malonyl-CoA reductase activity and an activity that converts malonyl semialdehyde to 3-HP using a biological reductant other than NADPH or NADH, such as the activity encoded by the *rutE* gene of *E. coli* or by the *nemA* gene *of E. coli* which are reductases that utilize a flavin derivative as the reductant and which further require the activity of a function such as the *fre* gene product encoding FMN reductase to regenerate the reductant. See, for example, Kim et al., 2010, J. Bacteriol. 192(16): 4089-4102.

Combinations of a gene encoding malonyl-CoA reductase activity and a gene or genes encoding 3-HP dehydrogenase can be achieved by cloning the respective genes behind promoters such that the genes are operably expressed in the microorganism under conditions that induce expression. The genes may be cloned in plasmid vectors, such as plasmids based on the ColE1 replication or the p15A replication, or may be inserted into the chromosome of the microorganism, such as at a locus which encodes a dispensable function, for example the *aldA* locus of *E. coli.* Expression of these genes can be driven by regulated promoters, such as the *lac* promoter or derivatives thereof, or by the T7 bacteriophage promoter, or by the arabinose promoter, or other DNA sequences known or found to drive expression in *E. coli.* These examples of constructs and promoters are not meant to be limiting.

### Example 72: Production of 3HP and other products from Xylose

3HP production using xylose by strain 240, described elsewhere herein, is demonstrated at 100-mL scale in SM11 (minimal salts) media made without glucose. Cultures are started from freezer stocks by standard practice (Sambrook and Russell, 2001) into 50 mL of TB media plus 35 µg/mL kanamycin and 20 µg/mL chloramphenicol and grown to stationary phase overnight at 30°C with rotation at 250 rpm. 5 mL of this culture is transferred to 100 ml of SM11 media made without glucose but with 30 g/L xylose, 35 µg/ml kanamycin, 20 µg/mL chloramphenicol, and 1 mM IPTG in triplicate 250-ml baffled flasks and incubated at 30°C, 250 rpm. The flasks are shifted to 37°C four hours post inoculation. To monitor growth rate, samples (2 ml) are withdrawn at designated time points for optical density measurements at 600nm (OD₆₀₀, 1 cm path length). To monitor 3HP production by these cultures, samples are pelleted by centrifugation at 12,000rpm for 5 min and the supernatant collected for analysis of 3-HP titer as described under "Analysis of cultures for 3-HP production" in the Common Methods section. Dry cell weight (DCW) is calculated as 0.40 times the measured OD₆₀₀ value, based on baseline DCW to OD₆₀₀ determinations. All data are the average of triplicate cultures. The average specific productivity is calculated from the averaged data at the 24-h time point and expressed as g 3-HP produced per gDCW. Glucose or xylose concentrations can be determined in g/L using appropriate sensors such as those from YSI Incorporated, and the yield is calculated using the averaged data at the 24-hour time point.

### Example 73: Production of 3HP and other products via malonyl-CoA in C. necator hosts with combinations of FAS mutations from Syngas

The following homologues were identified for E. coli FAS enzymes in C. necator.

**Table 56: FAS homologues in C. necator**

| Enzyme Function | Homologue in C. necator | % Identity | e value |
|---|---|---|---|
| MALONYL-COA-ACP-TRANSACYL-MONOMER | gi\|113868530\|ref\|YP_727019.1\| | 59.61 | 2.00E-95 |
| FABH-MONOMER | gi\|113868531\|ref\|YP_727020.1\| | 51.7 | 6.00E-93 |
| FABB-MONOMER | gi\|113868527\|ref\|YP_727016.1\| | 38.2 | 3.00E-62 |
| FABB-MONOMER | gi\|116695606\|ref\|YP_841182.1\| | 30.96 | 1.00E-14 |
| 3-OXOACYL-ACP-SYNTHII-MONOMER | gi\|113868527\|ref\|YP_727016.1\| | 63.11 | 2.00E-151 |
| 3-OXOACYL-ACP-SYNTHII-MONOMER | gi\|116695606\|ref\|YP_841182.1\| | 38.7 | 5.00E-28 |
| ENOYL-ACP-REDUCT-NADH-MONOMER | gi\|113868381\|ref\|YP_726870.1\| | 62.06 | 6.00E-91 |
| ENOYL-ACP-REDUCT-NADH-MONOMER | gi\|38637922\|ref\|NP_942896.1\| | 57.37 | 5.00E-83 |
| ENOYL-ACP-REDUCT-NADH-MONOMER | gi\|116695568\|ref\|YP_841144.1\| | 44.71 | 2.00E-57 |
| ENOYL-ACP-REDUCT-NADH-MONOMER | gi\|113866900\|ref\|YP_725389.1\| | 28.52 | 5.00E-17 |
| ENOYL-ACP-REDUCT-NADH-MONOMER | gi\|113869529\|ref\|YP_728018.1\| | 27.95 | 4.00E-14 |
| ENOYL-ACP-REDUCT-NADH-MONOMER | gi\|116694061\|ref\|YP_728272.1\| | 29.3 | 5.00E-14 |
| ENOYL-ACP-REDUCT-NADH-MONOMER | gi\|113866064\|ref\|YP_724553.1\| | 25.87 | 1.00E-11 |
| ENOYL-ACP-REDUCT-NADH-MONOMER | gil\|116694602\|ref\|YP_728813.1\| | 24.16 | 4.00E-11 |
| ENOYL-ACP-REDUCT-NADH-MONOMER | gi\|116695241\|ref\|YP_840817.1\| | 26.36 | 6.00E-11 |
| ENOYL-ACP-REDUCT-NADH-MONOMER | gi\|116695184\|ref\|YP_840760.1\| | 24.32 | 2.00E-10 |
| ENOYL-ACP-REDUCT-NADH-MONOMER | gi\|113869434\|ref\|YP_727923.1\| | 27.41 | 6.00E-10 |
| ENOYL-ACP-REDUCT-NADH-MONOMER | gi\|116696275\|ref\|YP_841851.1\| | 24.52 | 9.00E-10 |
| ENOYL-ACP-REDUCT-NADH-MONOMER | gi\|116695085\|ref\|YP_840661.1\| | 30.7 | 1.00E-08 |
| ENOYL-ACP-REDUCT-NADH-MONOMER | gi\|116694581\|ref\|YP_728792.1\| | 22.1 | 2.00E-08 |
| ENOYL-ACP-REDUCT-NADH-MONOMER | gi\|116695770\|ref\|YP_841346.1\| | 26.62 | 5.00E-08 |
| ENOYL-ACP-REDUCT-NADH-MONOMER | gi\|113867286\|ref\|YP_725775.1\| | 26.72 | 9.00E-08 |
| ACYLCOASYN-MONOMER | gi\|113869241\|ref\|YP_727730.1\| | 56.96 | 0 |
| ACYLCOASYN-MONOMER | gi\|113868752\|ref\|YP_727241.1\| | 33.87 | 2.00E-77 |
| ACYLCOASYN-MONOMER | gi\|113867951\|ref\|YP_726440.1\| | 32.18 | 6.00E-76 |
| ACYLCOASYN-MONOMER | gi\|113869452\|ref\|YP_727941.1\| | 33.27 | 1.00E-71 |
| ACYLCOASYN-MONOMER | gi\|113867314\|ref\|YP_725803.1\| | 31.54 | 1.00E-68 |
| ACYLCOASYN-MONOMER | gi\|113868228\|ref\|YP_726717.1\| | 30.87 | 1.00E-62 |
| ACYLCOASYN-MONOMER | gi\|116694647\|ref\|YP_728858.1\| | 30.05 | 4.00E-62 |
| ACYLCOASYN-MONOMER | gi\|113868902\|ref\|YP_727391.1\| | 29.93 | 9.00E-62 |
| ACYLCOASYN-MONOMER | gi\|113866897\|ref\|YP_725386.1\| | 31.14 | 2.00E-60 |
| ACYLCOASYN-MONOMER | gi\|113868933\|ref\|YP_727422.1\| | 31.07 | 2.00E-59 |
| ACYLCOASYN-MONOMER | gi\|116695183\|ref\|YP_840759.1\| | 31.07 | 1.00E-58 |
| ACYLCOASYN-MONOMER | gi\|116694856\|ref\|YP_729067.1\| | 31.91 | 2.00E-58 |
| ACYLCOASYN-MONOMER | gi\|113866865\|ref\|YP_725354.1\| | 30.11 | 4.00E-58 |
| ACYLCOASYN-MONOMER | gi\|116694595\|ref\|YP_728806.1\| | 28.73 | 1.00E-56 |
| ACYLCOASYN-MONOMER | gi\|116694129\|ref\|YP_728340.1\| | 30.91 | 2.00E-53 |
| ACYLCOASYN-MONOMER | gi\|116695208\|ref\|YP_840784.1\| | 31.88 | 2.00E-52 |
| ACYLCOASYN-MONOMER | gi\|113868706\|ref\|YP_727195.1\| | 28.86 | 1.00E-50 |
| ACYLCOASYN-MONOMER | gi\|116695648\|ref\|YP_841224.1\| | 30.71 | 2.00E-50 |
| ACYLCOASYN-MONOMER | gi\|116694665\|ref\|YP_728876.1\| | 29.49 | 4.00E-50 |
| ACYLCOASYN-MONOMER | gi\|113867426\|ref\|YP_725915.1\| | 27.46 | 2.00E-49 |
| ACYLCOASYN-MONOMER | gi\|116695854\|ref\|YP_841430.1\| | 28.35 | 4.00E-48 |
| ACYLCOASYN-MONOMER | gi\|116694628\|ref\|YP_728839.1\| | 28.86 | 8.00E-48 |
| ACYLCOASYN-MONOMER | gi\|113868764\|ref\|YP_727253.1\| | 29.61 | 2.00E-47 |
| ACYLCOASYN-MONOMER | gi\|116695316\|ref\|YP_840892.1\| | 29.1 | 2.00E-46 |
| ACYLCOASYN-MONOMER | gi\|116695341\|ref\|YP_840917.1\| | 28.8 | 5.00E-46 |
| ACYLCOASYN-MONOMER | gi\|113866892\|ref\|YP_725381.1\| | 32.96 | 4.00E-45 |
| ACYLCOASYN-MONOMER | gi\|113868055\|ref\|YP_726544.1\| | 26.24 | 1.00E-44 |
| ACYLCOASYN-MONOMER | gi\|113867721\|ref\|YP_726210.1\| | 28.71 | 3.00E-44 |
| ACYLCOASYN-MONOMER | gi\|116696458\|ref\|YP_842034.1\| | 30.25 | 7.00E-43 |
| ACYLCOASYN-MONOMER | gi\|113867704\|ref\|YP_726193.1\| | 28.46 | 2.00E-42 |
| ACYLCOASYN-MONOMER | gi\|113868126\|ref\|YP_726615.1\| | 26.92 | 1.00E-41 |
| ACYLCOASYN-MONOMER | gi\|116695632\|ref\|YP_841208.1\| | 27.15 | 3.00E-40 |
| ACYLCOASYN-MONOMER | gi\|113868425\|ref\|YP_726914.1\| | 26.74 | 2.00E-39 |
| ACYLCOASYN-MONOMER | gi\|38638060\|ref\|NP_943034.1\| | 28.21 | 4.00E-39 |
| ACYLCOASYN-MONOMER | gi\|116694704\|ref\|YP_728915.1\| | 27.64 | 5.00E-39 |
| ACYLCOASYN-MONOMER | gi\|113869299\|ref\|YP_727788.1\| | 26.4 | 5.00E-38 |
| ACYLCOASYN-MONOMER | gi\|116695047\|ref\|YP_840623.1\| | 27.19 | 3.00E-35 |
| ACYLCOASYN-MONOMER | gi\|116695279\|ref\|YP_840855.1\| | 25.14 | 3.00E-35 |
| ACYLCOASYN-MONOMER | gi\|113867249\|ref\|YP_725738.1\| | 31.28 | 5.00E-35 |
| ACYLCOASYN-MONOMER | gi\|113867217\|ref\|YP_725706.1\| | 25.5 | 9.00E-35 |
| ACYLCOASYN-MONOMER | gi\|113867530\|ref\|YP_726019.1\| | 25.05 | 6.00E-33 |
| ACYLCOASYN-MONOMER | gi\|116695093\|ref\|YP_840669.1\| | 27.74 | 2.00E-32 |
| ACYLCOASYN-MONOMER | gi\|38638059\|ref\|NP_943033.1\| | 25.54 | 2.00E-31 |
| ACYLCOASYN-MONOMER | gi\|113867503\|ref\|YP_725992.1\| | 44.52 | 4.00E-28 |
| ACYLCOASYN-MONOMER | gi\|116696036\|ref\|YP_841612.1\| | 28.86 | 8.00E-28 |
| ACYLCOASYN-MONOMER | gi\|116694780\|ref\|YP_728991.1\| | 25.66 | 8.00E-27 |
| ACYLCOASYN-MONOMER | gi\|113868491\|ref\|YP_726980.1\| | 25.66 | 9.00E-27 |
| ACYLCOASYN-MONOMER | gi\|116695672\|ref\|YP_841248.1\| | 24.91 | 9.00E-25 |
| ACYLCOASYN-MONOMER | gi\|113867627\|ref\|YP_726116.1\| | 25.04 | 2.00E-22 |
| ACYLCOASYN-MONOMER | gi\|116695626\|ref\|YP_841202.1\| | 25.5 | 4.00E-21 |
| ACYLCOASYN-MONOMER | gi\|116695626\|ref\|YP_841202.1\| | 23.85 | 4.00E-15 |
| ACYLCOASYN-MONOMER | gi\|113868430\|ref\|YP_726919.1\| | 24.06 | 6.00E-20 |
| ACYLCOASYN-MONOMER | gi\|113866315\|ref\|YP_724804.1\| | 23.54 | 3.00E-15 |
| ACYLCOASYN-MONOMER | gi\|116695622\|ref\|YP_841198.1\| | 23.26 | 1.00E-13 |
| ACYLCOASYN-MONOMER | gi\|116695624\|ref\|YP_841200.1\| | 27.13 | 1.00E-12 |
| ACYLCOASYN-MONOMER | gi\|116695625\|ref\|YP_841201.1\| | 23.55 | 4.00E-10 |
| 3-OXOACYL-ACP-REDUCT-MONOMER | gi\|113868529\|ref\|YP_727018.1\| | 63.52 | 9.00E-86 |
| 3-OXOACYL-ACP-REDUCT-MONOMER | gi\|113867453\|ref\|YP_725942.1\| | 41.7 | 5.00E-51 |
| 3-OXOACYL-ACP-REDUCT-MONOMER | gi\|113867981\|ref\|YP_726470.1\| | 40.41 | 2.00E-50 |
| 3-OXOACYL-ACP-REDUCT-MONOMER | gi\|113868147\|ref\|YP_726636.1\| | 43.4 | 1.00E-48 |
| 3-OXOACYL-ACP-REDUCT-MONOMER | gi\|116695840\|ref\|YP_841416.1\| | 40.66 | 3.00E-47 |
| 3-OXOACYL-ACP-REDUCT-MONOMER | gi\|113869118\|ref\|YP_727607.1\| | 38.62 | 2.00E-43 |
| 3-OXOACYL-ACP-REDUCT-MONOMER | gi\|116696446\|ref\|YP_842022.1\| | 43.1 | 4.00E-42 |
| 3-OXOACYL-ACP-REDUCT-MONOMER | gi\|116694315\|ref\|YP_728526.1\| | 38.89 | 2.00E-41 |
| 3-OXOACYL-ACP-REDUCT-MONOMER | gi\|116694338\|ref\|YP_728549.1\| | 36.8 | 3.00E-39 |
| 3-OXOACYL-ACP-REDUCT-MONOMER | gi\|113867286\|ref\|YP_725775.1\| | 38.62 | 5.00E-38 |
| 3-OXOACYL-ACP-REDUCT-MONOMER | gi\|116695278\|ref\|YP_840854.1\| | 37.55 | 9.00E-36 |
| 3-OXOACYL-ACP-REDUCT-MONOMER | gi\|113867797\|ref\|YP_726286.1\| | 36.86 | 2.00E-35 |
| 3-OXOACYL-ACP-REDUCT-MONOMER | gi\|116695770\|ref\|YP_841346.1\| | 35.27 | 7.00E-35 |
| 3-OXOACYL-ACP-REDUCT-MONOMER | gi\|116694022\|ref\|YP_728233.1\| | 33.88 | 7.00E-35 |
| 3-OXOACYL-ACP-REDUCT-MONOMER | gi\|116695384\|ref\|YP_840960.1\| | 36.96 | 1.00E-34 |
| 3-OXOACYL-ACP-REDUCT-MONOMER | gi\|113867306\|ref\|YP_725795.1\| | 35.06 | 1.00E-34 |
| 3-OXOACYL-ACP-REDUCT-MONOMER | gi\|116695635\|ref\|YP_841211.1\| | 40.68 | 1.00E-32 |
| 3-OXOACYL-ACP-REDUCT-MONOMER | gi\|113867542\|ref\|YP_726031.1\| | 34.12 | 1.00E-32 |
| 3-OXOACYL-ACP-REDUCT-MONOMER | gi\|113867353\|ref\|YP_725842.1\| | 31.64 | 1.0OE-31 |
| 3-OXOACYL-ACP-REDUCT-MONOMER | gi\|116695184\|ref\|YP_840760.1\| | 33.33 | 5.00E-31 |
| 3-OXOACYL-ACP-REDUCT-MONOMER | gi\|116694602\|ref\|YP_728813.1\| | 32.66 | 9.00E-31 |
| 3-OXOACYL-ACP-REDUCT-MONOMER | gi\|113868128\|ref\|YP_726617.1\| | 33.46 | 2.00E-30 |
| 3-OXOACYL-ACP-REDUCT-MONOMER | gi\|116695020\|ref\|YP_840596.1\| | 34.17 | 7.00E-30 |
| 3-OXOACYL-ACP-REDUCT-MONOMER | gi\|116695668\|ref\|YP_841244.1\| | 30.95 | 3.00E-29 |
| 3-OXOACYL-ACP-REDUCT-MONOMER | gi\|116694617\|ref\|YP_728828.1\| | 33.78 | 4.00E-29 |
| 3-OXOACYL-ACP-REDUCT-MONOMER | gi\|116694552\|ref\|YP_728763.1\| | 32.8 | 4.00E-29 |
| 3-OXOACYL-ACP-REDUCT-MONOMER | gi\|116696275\|ref\|YP_841851.1\| | 35.25 | 5.00E-29 |
| 3-OXOACYL-ACP-REDUCT-MONOMER | gi\|113868428\|ref\|YP_726917.1\| | 34.18 | 9.00E-29 |
| 3-OXOACYL-ACP-REDUCT-MONOMER | gi\|113867344\|ref\|YP_725833.1\| | 30.86 | 1.00E-28 |
| 3-OXOACYL-ACP-REDUCT-MONOMER | gi\|116695241\|ref\|YP_840817.1\| | 33.74 | 3.00E-28 |
| 3-OXOACYL-ACP-REDUCT-MONOMER | gi\|116695722\|ref\|YP_841298.1\| | 33.88 | 5.00E-28 |
| 3-OXOACYL-ACP-REDUCT-MONOMER | gi\|116694061\|ref\|YP_728272.1\| | 37.6 | 2.00E-27 |
| 3-OXOACYL-ACP-REDUCT-MONOMER | gi\|113866956\|ref\|YP_725445.1\| | 33.59 | 7.00E-27 |
| 3-OXOACYL-ACP-REDUCT-MONOMER | gi\|113868440\|ref\|YP_726929.1\| | 36.14 | 1.00E-26 |
| 3-OXOACYL-ACP-REDUCT-MONOMER | gi\|116694682\|ref\|YP_728893.1\| | 32.94 | 1.00E-26 |
| 3-OXOACYL-ACP-REDUCT-MONOMER | gi\|113866770\|ref\|YP_725259.1\| | 33.2 | 4.00E-26 |
| 3-OXOACYL-ACP-REDUCT-MONOMER | gi\|116694156\|ref\|YP_728367.1\| | 33.05 | 1.00E-25 |
| 3-OXOACYL-ACP-REDUCT-MONOMER | gi\|113867502\|ref\|YP_725991.1\| | 31.02 | 5.00E-24 |
| 3-OXOACYL-ACP-REDUCT-MONOMER | gi\|113866875\|ref\|YP_725364.1\| | 34.93 | 7.00E-24 |
| 3-OXOACYL-ACP-REDUCT-MONOMER | gi\|116694685\|ref\|YP_728896.1\| | 30.59 | 2.00E-23 |
| 3-OXOACYL-ACP-REDUCT-MONOMER | gi\|116695830\|ref\|YP_841406.1\| | 31.45 | 5.00E-23 |
| 3-OXOACYL-ACP-REDUCT-MONOMER | gi\|116694347\|ref\|YP_728558.1\| | 30.52 | 7.00E-23 |
| 3-OXOACYL-ACP-REDUCT-MONOMER | gi\|116695926\|ref\|YP_841502.1\| | 31.2 | 8.00E-23 |
| 3-OXOACYL-ACP-REDUCT-MONOMER | gi\|116694593\|ref\|YP_728804.1\| | 31.15 | 2.00E-22 |
| 3-OXOACYL-ACP-REDUCT-MONOMER | gi\|116694550\|ref\|YP_728761.1\| | 30.36 | 2.00E-22 |
| 3-OXOACYL-ACP-REDUCT-MONOMER | gi\|113869434\|ref\|YP_727923.1\| | 30.04 | 9.00E-22 |
| 3-OXOACYL-ACP-REDUCT-MONOMER | gi\|116694600\|ref\|YP_728811.1\| | 28.99 | 2.00E-21 |
| 3-OXOACYL-ACP-REDUCT-MONOMER | gi\|113866064\|ref\|YP_724553.1\| | 27.2 | 4.00E-21 |
| 3-OXOACYL-ACP-REDUCT-MONOMER | gi\|116694638\|ref\|YP_728849.1\| | 33.13 | 2.00E-20 |
| 3-OXOACYL-ACP-REDUCT-MONOMER | gi\|116694259\|ref\|YP_728470.1\| | 35.18 | 3.00E-20 |
| 3-OXOACYL-ACP-REDUCT-MONOMER | gi\|116694664\|ref\|YP_728875.1\| | 30.43 | 9.00E-20 |
| 3-OXOACYL-ACP-REDUCT-MONOMER | gi\|113867940\|ref\|YP_726429.1\| | 32.56 | 1.00E-19 |
| 3-OXOACYL-ACP-REDUCT-MONOMER | gi\|116695703\|ref\|YP_841279.1\| | 30.92 | 1.00E-19 |
| 3-OXOACYL-ACP-REDUCT-MONOMER | gi\|116695910\|ref\|YP_841486.1\| | 31.44 | 2.00E-19 |
| 3-OXOACYL-ACP-REDUCT-MONOMER | gi\|116695451\|ref\|YP_841027.1\| | 31.77 | 4.00E-19 |
| 3-OXOACYL-ACP-REDUCT-MONOMER | gi\|116695846\|ref\|YP_841422.1\| | 34.36 | 4.00E-19 |
| 3-OXOACYL-ACP-REDUCT-MONOMER | gi\|116694614\|ref\|YP_728825.1\| | 31.15 | 6.00E-19 |
| 3-OXOACYL-ACP-REDUCT-MONOMER | gi\|116696432\|ref\|YP_842008.1\| | 27.73 | 1.00E-18 |
| 3-OXOACYL-ACP-REDUCT-MONOMER | gi\|113867868\|ref\|YP_726357.1\| | 29.8 | 1.00E-18 |
| 3-OXOACYL-ACP-REDUCT-MONOMER | gi\|113866900\|ref\|YP_725389.1\| | 30 | 7.00E-18 |
| 3-OXOACYL-ACP-REDUCT-MONOMER | gi\|113869529\|ref\|YP_728018.1\| | 31.02 | 1.00E-17 |
| 3-OXOACYL-ACP-REDUCT-MONOMER | gi\|116695085\|ref\|YP_840661.1\| | 29.03 | 1.00E-16 |
| 3-OXOACYL-ACP-REDUCT-MONOMER | gi\|116695734\|ref\|YP_841310.1\| | 31.95 | 1.00E-16 |
| 3-OXOACYL-ACP-REDUCT-MONOMER | gi\|113866629\|ref\|YP_725118.1\| | 27.95 | 6.00E-16 |
| 3-OXOACYL-ACP-REDUCT-MONOMER | gi\|116695726\|ref\|YP_841302.1\| | 31.89 | 1.00E-15 |
| 3-OXOACYL-ACP-REDUCT-MONOMER | gi\|116694599\|ref\|YP_728810.1\| | 31.84 | 1.00E-15 |
| 3-OXOACYL-ACP-REDUCT-MONOMER | gi\|116694585\|ref\|YP_728796.1\| | 30.42 | 2.00E-15 |
| 3-OXOACYL-ACP-REDUCT-MONOMER | gi\|116694581\|ref\|YP_728792.1\| | 28.14 | 2.00E-15 |
| 3-OXOACYL-ACP-REDUCT-MONOMER | gi\|116695741\|ref\|YP_841317.1\| | 30.9 | 4.00E-14 |
| 3-OXOACYL-ACP-REDUCT-MONOMER | gi\|116694341\|ref\|YP_728552.1\| | 28.35 | 7.00E-14 |
| 3-OXOACYL-ACP-REDUCT-MONOMER | gi\|113866193\|ref\|YP_724682.1\| | 28.95 | 3.00E-13 |
| 3-OXOACYL-ACP-REDUCT-MONOMER | gi\|116695568\|ref\|YP_841144.1\| | 29.03 | 3.00E-13 |
| 3-OXOACYL-ACP-REDUCT-MONOMER | gi\|116695287\|ref\|YP_840863.1\| | 30.22 | 5.00E-13 |
| 3-OXOACYL-ACP-REDUCT-MONOMER | gi\|113869676\|ref\|YP_728165.1\| | 28.14 | 1.00E-12 |
| 3-OXOACYL-ACP-REDUCT-MONOMER | gi\|113867547\|ref\|YP_726036.1\| | 22.48 | 1.00E-12 |
| 3-OXOACYL-ACP-REDUCT-MONOMER | gi\|113866289\|ref\|YP_724778.1\| | 28 | 4.00E-12 |
| 3-OXOACYL-ACP-REDUCT-MONOMER | gi\|113867750\|ref\|YP_726239.1\| | 29.59 | 3.00E-11 |
| 3-OXOACYL-ACP-REDUCT-MONOMER | gi\|116696287\|ref\|YP_841863.1\| | 27.6 | 4.00E-11 |
| 3-OXOACYL-ACP-REDUCT-MONOMER | gi\|116694597\|ref\|YP_728808.1\| | 31.69 | 2.00E-10 |
| 3-OXOACYL-ACP-REDUCT-MONOMER | gi\|116694624\|ref\|YP_728835.1\| | 33.01 | 7.00E-08 |
| FABZ-MONOMER | gi\|113868023\|ref\|YP_726512.1\| | 57.25 | 3.00E-45 |

The following homologues have been annotated as homologues by <<www.metacyc.org>>.

**Table 57:**

| **E. coli gene** | **C. necator gene** |
|---|---|
| fabD | fabD |
| fabH | fabH |
| fabB | fabB |
| fabF | fabF |
| fabG | h16_B1904, h16_B0361, h16_B0385 |
| fabZ | |
| fabI | PHG261, fabI1, fabI2 |

Strains can be made with equivalent combinations of fatty acid synthesis mutations to those described elsewhere herein for improved production of malonyl-CoA derived products in *E. coli.* Deletions and temperature sensitive mutations in equivalent homologues can be made by standard recombineering techniques previously described. Identification of relevant homologues for mutation/deletion can be completed by complementation of *E. coli* strains with FAS mutations. For example, in the case of fabI, the three fabI homologues annotated for C. necator were cloned into standard E. coli expression vectors and transformed using standard techniques into *E. coli* strains with and without temperature-sensitive fabI mutations. Strains constructed for this study are listed in Table 58 below:

| **Strain** | **Chromosomal genotype** | **Plasmid 1** | **Plasmid 2** |
|---|---|---|---|
| 595 | F-, Δ (araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt, fabIts (S241F)-zeoR | N/A | |
| 1 | F-, Δ (araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt, fabIts (S241F)-zeoR | pTRC empty vector | |
| 591 | F-, Δ(araD-araB)567, Δ lacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt | N/A | |
| 2 | F-, Δ(araD-araB)567, Δ lacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt | pTRC_fabI1 | |
| 3 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack:frt | pTRC_fabI2 | |
| 4 | F-, Δ(araD-araB)567, ΔlacZ4787(::rrnB-3), LAM-, rph-1, Δ(rhaD-rhaB)568, hsdR514, ΔldhA:frt, ΔpflB:frt, mgsA:frt, ΔpoxB:frt, Δpta-ack: frt | pTRC_PHG261 | |

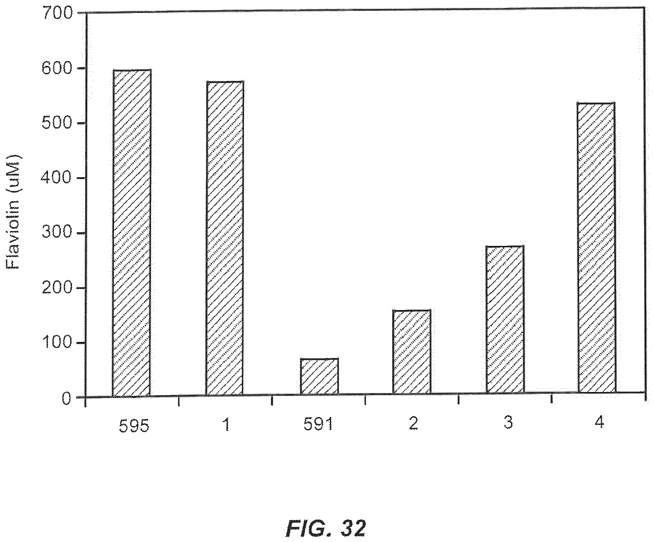

These strains were grown in shake flasks at 37 degrees Celsius in SM11 media for 24 hours. Samples were taken for OD600 and flaviolin measurements (see Common Methods section for detailed protocol). Results from flaviolin quantification are shown Figure 32. This complementation study suggests that fabI1, which shows the lowest production of flaviolin, is primarily responsible for fabI activity as seen by the diminished production and is followed by partial complementation observed for fabI2.

Strains with all combinations of fabI deletions/mutations are made by standard techniques. The genes encoding malonyl coA reductase (mcr) with combinations of other genes to enhance 3HP production described elsewhere herein are cloned into appropriate expression vectors for high level expression in *C*. *necator* using standard molecular biology techniques and transformed into *C*. *necator* hosts with FAS inhibition. Vectors were constructed on a broad host range plasmid (pBMT3) with the Ptrc induction system that drives expression of mcr and accAse. A subset of *C*. *necator* strains are constructed are listed in Table 59 below:

**Table 59:**

| **Strain** | **Modifications made to *C. necator H16* Chromosomal genotype** | **Plasmid 1** | **Plasmid 2** |
|---|---|---|---|
| C. necator H16 | | pBMT3-Ptrc-mcr-accABCD | N/A |
| C. necator H16-1 | fabI1(ts)-(S241F) | pBMT3-Ptrc-mcr-accABCD | N/A |
| C. necator H16-2 | fabI2(ts)-(S241F) | pBMT3-Ptrc-mcr-accABCD | N/A |
| C. necator H16-3 | fabI1(ts)-(S241F), fabI2(ts)-(S241F) | pBMT3-Ptrc-mcr-accABCD | N/A |
| C. necator H16-4 | fabI1(ts)-(S241F), fabI2(ts)-(S241F), ΔphaCAB | pBMT3-Ptrc-mcr-accABCD | N/A |

3HP production using syngas feedstocks is demonstrated at 0.6 L scale in SM11 (minimal salts) media. Cultures of *C*. *necator* strains listed in Table 59 are started from freezer stocks by standard practice (Sambrook and Russell, 2001) into 49 mL of FGN30 medium supplemented with appropriate antibiotics and incubated at 30°C for 24 hours with rotation at 250 rpm. 5 mL of this culture is transferred to 45 ml of FGN30 HN with appropriate antibiotics and is incubated at 30°C for 24 hours with rotation at 250 rpm. Cultures are used to inoculate gas-fed columns maintained at 30C as described under "Syngas Fermentation Method" in the Common Methods Section.

### Example 74: Butyrate production via malonyl-coA in strains with combinations of FAS mutations.

The following genetically modified *E. coli* strains (listed in Table 60) were constructed form a wild type BW25113 starting host. *E*. *coli* BW25113 was obtained from the Yale genetic stock center (New Haven, CT, USA). These strains were constructed by standard methods such as discussed in the Common Methods Section and also known in the art as referenced above. Briefly, chromosomal modifications were constructed via homologous recombination.

The following plasmids can be constructed by gene synthesis (Genscript, Piscataway, NJ). The target gene sequences were ordered (Genscript, Piscataway, NJ) including with modifications to the native ribosome binding site and subsequently changed to be compatible with existing expression vectors and to accommodate expression of key downstream gene(s) within the vector(s).

**Table 60: Strain List 16**

| **Strain** | **Chromosomal genotype** | **Plasmid 1** | **Plasmid 2** |
|---|---|---|---|
| 1 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, ΔfadD::frt, lambda-DE3; ΔatoDAEB::frt | pACYC (empty) | pET28B (empty) |
| 2 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, ΔfadD::frt, lambda-DE3; ΔatoDAEB::frt | pACYC (empty) | pET28B (ptb-buk) |
| 3 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, ΔfadD::frt, lambda-DE3; ΔatoDAEB::frt | pACYC(phaA-hbd-crt-ter) | pET28B (ptb-buk) |
| 4 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, ΔfadD::frt, lambda-DE3; ΔatoDAEB::frt | pACYC(npht7-hbd-crt-ter) | pET28B (ptb-buk) |
| 5 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, fabB(ts), ΔfabF::frt, coaA*, fabD(ts), ΔlacI::frt, ΔpuuC::T5-aceEF-lpd*::loxP, ΔaceBAK::frt, lpd*::loxP, ΔaldB::PyibD-T7pol::loxP, ΔadhE::frt, ΔaldA::CSC, lambda-DE3, **ΔatoDAEB** | pACYC(npht7-hbd-crt-ter) | pET28B (ptb-buk) |

The above strains were evaluated in shake flasks for the production of butyrate. Triplicate evaluations were performed. Briefly, overnight starter cultures were made in 50 mL of Luria Broth including the appropriate antibiotics and incubated 16-24 hours are 30°C, while shaking at 225 rpm. These cultures were used to inoculate 3 x 50 mL cultures of each strain in SM11 minimal medium with 5% culture as starting inoculum, and antibiotics. The cultures are grown at 30°C for approximately 4h to an OD of 0.4-0.6 and then induced with IPTG (0.5mM), after 1h cells were shifted to 37°C and monitored for 28 hours. Samples were taken at 18h and 28h and the supernatant was analyzed for the presence of Butyrate, and cells were saved to measure enzyme activity.

**Table 61. Butyrate Production.**

| **Strain:** | **Plasmid(s)** | **mg/L produced** |
|---|---|---|
| 1 | pACYC (Empty vector), pET28B (empty vector) | 0 |
| 2 | pACYC (Empty vector), pET28B (ptb-buk) | 0 |
| 3 | pACYC (pT7But4 (phaA, hbd, crt, ter), pET28B (ptb-buk) | 0 |
| 4 | pACYC (pT7But7 (nphT7, hbd, crt, ter), pET28B (ptb-buk) | 8-10mg/L in 18h 10mg/L - 14mg/L in 24h (OD=3-6) |
| 5 | pACYC (pT7But7 (nphT07, hbd, crt, ter), pET28B (ptb-buk) | 528 mg/L in 20 h (OD∼5) |

### Example 75: NADPH dependent Butyrate production via malonyl-coA in strains with combinations of FAS mutations.

The following genetically modified *E. coli* strains (listed in Table 62) were constructed form a wild type BW25113 starting host. *E. coli* BW25113 was obtained from the Yale genetic stock center (New Haven, CT, USA). These strains were constructed by standard methods such as discussed in the Common Methods Section and also known in the art as referenced above. Briefly, chromosomal modifications were constructed via homologous recombination.

The following plasmids can be constructed by gene synthesis (Genscript, Piscataway, NJ). The target gene sequences were ordered (Genscript, Piscataway, NJ) including with modifications to the native ribosome binding site and subsequently changed to be compatible with existing expression vectors and to accommodate expression of key downstream gene(s) within the vector(s).

**Table 62: Strain List 17**

| **Strain** | **Chromosomal genotype** | **Plasmid 1** | **Plasmid 2** |
|---|---|---|---|
| 1 | F-, Δ(araD-araB)567, ΔlacZ4787(::rrnB-3), LAM-, rph-1, Δ(rhaD-rhaB)568, hsdR514, ΔldhA::frt, Δρfl::frt, ΔmgsA::frt, ΔpoxB::frt, Δpta-ack::frt, fabI(ts)-(S241F)-zeoR, ΔfadD::frt, lambda-DE3; ΔatoDAEB::frt | pACYC (empty) | pET28B (empty) |
| 2 | F-, Δ(araD-araB)567, ΔlacZ4787(::rrnB-3), LAM-, rph-1, Δ(rhaD-rhaB)568, hsdR514, ΔldhA::frt, ΔpflB::frt, ΔmgsA::frt, ΔpoxB::frt, Δpta-ack::frt, fabI(ts)-(S241F)-zeoR, ΔfadD::frt, lambda-DE3; ΔatoDAEB::frt | pACYC(npht07-phaB-ech2-crr) | pET28B (ptb-buk) |

The above strains can be evaluated in shake flasks for the production of butyrate. Briefly, overnight starter cultures can be made in 50 mL of Luria Broth including the appropriate antibiotics and incubated 16-24 hours are 30°C, while shaking at 225 rpm. These cultures are used to inoculate 3 x 50 mL cultures of each strain in SM11 minimal medium with 5% culture as starting inoculum, and antibiotics. The cultures are grown at 30°C for approximately 4h to an OD of 0.4-0.6 and then induced with IPTG (0.5mM), after 1h cells are shifted to 37°C and monitored for 28 hours. Samples are taken at 18h and 28h and the supernatant is analyzed for the presence of Butyrate, and cells are saved to measure enzyme activity.

### Example 76: C10- and C14-free fatty acids production by using Trypanosoma brucei elongases in E. coli host cells that are genetically engineered for production of butyryl-CoA and malonyl-CoA.

*E. coli* host cells that are genetically engineered for production of butyryl-CoA and malonyl-CoA have been described above. The genes encoding *Trypanosoma brucei* elongases, ELO (SEQ ID NO: 199), ELO2 (SEQ ID NO: 200), and ELO3 (SEQ ID NO: 201), can be amplified from *T. brucei* genomic DNA and cloned into appropriate *E. coli* expression vectors. Alternatively, *ELO1*, *ELO2*, *and ELO3* can be synthesized chemically, codon-optimized for expression in *E. coli,* and cloned into appropriate *E. coli* expression vectors.

Genetically modified *E. coli* host cells with *T. brucei* ELO1, ELO2, and ELO3 alone will not be able to produce C10- and C14-free fatty acids, since accessory enzymes are needed. First, enzymes know as β-ketoacyl-CoA reductase, β-hydroxyacyl- CoA dehydratase, and enoyl-acyl-CoA reductase are required to work together with *T. brucei* ELO1 and/or ELO2 for biotransformation of butyryl-CoA and malonyl-CoA into C10- and C14-CoAs. *Saccharomyces cerevisiae* possess β-ketoacyl-CoA reductase, β-hydroxyacyl- CoA dehydratase, and enoyl-acyl-CoA reductase (YBR159W (SEQ ID NO: 202), PHS1 (SEQ ID NO: 203), TSC13 (SEQ ID NO: 204), respectively) that will function together with *T. brucei* ELO1 and ELO2. Genes encoding β-ketoacyl-CoA reductase (SEQ ID NO: 205), β-hydroxyacyl- CoA dehydratase (SEQ ID NO: 206), and enoyl-acyl-CoA reductase (SEQ ID NO: 207) are also present in *T. brucei* genomes. Genes encoding these 3 enzymes can be PCR-amplified from *S. cerevisiae* or *T. brucei* genomic DNA.

Alternatively, these genes can be synthesized chemically and codon-optimized for expression in *E. coli.* Genes encoding these 3 accessory enzymes can be cloned into a different *E. coli* expression vector that is compatible with the *E. coli* expression vector carrying *T. brucei* ELO1 and ELO2 genes. Alternatively, genes encoding these 3 accessory enzymes can be cloned into the same expression vector carrying *T. brucei* ELO1 and ELO2 genes. In the latter case, genes of ELO1, ELO2, β-ketoacyl-CoA reductase, β-hydroxyacyl- CoA dehydratase, and enoyl-acyl-CoA reductase will be under regulation of a single promoter and transcribed together in a polycistronic mRNA.

Second, a thioesterase is needed to convert C10- and C14-CoAs produced by ELO1, ELO2, β-ketoacyl-CoA reductase, β-hydroxyacyl- CoA dehydratase, and enoyl-acyl-CoA reductase into C10- and C14-free fatty acids. *E. coli tesA* encodes a thioesterase that is naturally expressed in the periplasm. A truncated version of *tesA* in which the secretion signal is removed (designated as *'tesA*) will allow expression of a TesA protein in *E. coli* cytoplasm (SEQ ID NO: 208). Alternatively, a second *E. coli* thioesterase gene known as *tesB* (SEQ ID NO: 209) can also be co-expressed with ELO1, ELO2, β-ketoacyl-CoA reductase, β-hydroxyacyl- CoA dehydratase, and enoyl-acyl-CoA reductase. Genes of either *tesA* or *tesB* are amplified by PCR from *E. coli* genome or chemically synthesized, and cloned into an expression vector that is compatible with the plasmids carrying ELO1, ELO2, β-ketoacyl-CoA reductase, β-hydroxyacyl-CoA dehydratase, and enoyl-acyl-CoA reductase. Alternatively, *tesA* or *tesB* can be cloned into the same plasmid carrying ELO1, ELO2, β-ketoacyl-CoA reductase, β-hydroxyacyl- CoA dehydratase, and enoyl-acyl-CoA reductase. In the latter case, all genes will be under regulation of a single promoter and transcribed together in a polycistronic mRNA.

Expression plasmids carrying *T. brucei* ELO1, β-ketoacyl-CoA reductase, β-hydroxyacyl- CoA dehydratase, enoyl-acyl-CoA reductase, and either *tesA* or *tesB* are transformed into the butyryl- and malonyl-CoA-producing *E. coli* host cells described above, giving rise to transformants that will produce C10-free fatty acid. In a separate experiment, expression plasmids carrying *T. brucei* ELO1 plus ELO2 with and without ELO3, β-ketoacyl-CoA reductase, β-hydroxyacyl- CoA dehydratase, enoyl-acyl-CoA reductase, and either *tesA* or *tesB* are transformed into the butyryl- and malonyl-CoA-producing *E. coli* host cells described above, giving rise to transformants that will produce C10-, C14-, and C-18free fatty acids.

The two different types of transformants are cultured independently in a suitable medium, such as LB broth at 37°C. Appropriate concentrations of antibiotics are included in the medium to keep the expression plasmids inside *E. coli* host cells. Expression of ELO1, ELO2, ELO3, and all accessory enzymes are induced by adding the appropriate inducers, such as IPTG, to the culture. Induced cultures are incubated at temperature between 15°C to 37°C, for 1-7 days.

Transformants expressing ELO1, β-ketoacyl-CoA reductase, β-hydroxyacyl- CoA dehydratase, enoyl-acyl-CoA reductase, and either *tesA* or *tesB* are capable of secreting C10-fatty acid into the medium. Transformants expressing ELO1, ELO2, β-ketoacyl-CoA reductase, β-hydroxyacyl- CoA dehydratase, enoyl-acyl-CoA reductase, and either *tesA* or *tesB* are capable of secreting C10- and C14-fatty acids into the medium. Transformants expressing ELO1, ELO2, ELO3, β-ketoacyl-CoA reductase, β-hydroxyacyl- CoA dehydratase, enoyl-acyl-CoA reductase, and either *tesA* or *tesB* are capable of secreting C10-, C14-, and C-18 fatty acids into the medium.

While preferred embodiments of the present invention have been shown and described herein, such embodiments are provided by way of example only. Numerous variations, changes, and substitutions may be made without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

The present invention will now be described by way of reference to the following clauses:
1. A method for producing a chemical, said method comprising combining a carbon source, a microorganism, and a cell culture to produce the chemical, wherein
   a) said cell culture comprises an inhibitor of fatty acid synthase and/or the microorganism is genetically modified for reduced enzymatic activity in at least two of the microorganism's fatty acid synthase pathway enzymes, providing for reduced conversion of malonyl-CoA to fatty acids; and
   b) the microorganism additionally has one or more genetic modifications relating to a metabolic production pathway from malonyl-CoA to the chemical.
2. The method of clause 1, wherein the at least two fatty acid synthase pathway enzymes with reduced enzymatic activity are an enoyl-coA reductase and a beta-ketoacyl-ACP synthase.
3. The method of clause 2, wherein the enoyl-coA reductase is fabI or a peptide of 80% or more homology to SEQ ID NO: 14.
4. The method of clause 2, wherein the beta-ketoacyl-ACP synthase is selected from the group consisting of fabB, fabF, a peptide of 80% or more homology to SEQ ID NO: 9, and a peptide of 80% or more homology to SEQ ID NO: 8.
5. The method of clause 2, wherein a third fatty acid synthase pathway enzyme is modified for reduced enzymatic activity.
6. The method of clause 5, wherein the enoyl-coA reductase is fabI or a peptide of 80% or more homology to SEQ ID NO: 14, the beta-ketoacyl-ACP synthase is fabB or a peptide of 80% homology or more to SEQ ID NO: 9, and the third fatty acid synthase pathway enzyme is fabF or a peptide of 80% homology or more to SEQ ID NO: 8.
7. The method of clause 5, wherein the third fatty acid synthase pathway enzyme is a malonyl-coA-ACP transacylase.
8. The method of clause 7, wherein the malonyl-coA-ACP transacylase is modified fabD or a peptide of 80% or more homology to SEQ ID NO: 7.
9. The method of clause 7, wherein the enoyl-coA reductase is fabI or a peptide of 80% or more homology to SEQ ID NO: 14, the beta-ketoacyl-ACP synthase is selected from the group consisting of fabB or a peptide of 80% homology or more to SEQ ID NO: 9, and fabF or a peptide of 80% homology or more to SEQ ID NO: 8, and the malonyl-coA-ACP transacylase is fabD or a peptide of 80% or more homology to SEQ ID NO: 7.
10. The method of clause 1, wherein the at least two fatty acid synthase pathway enzymes with reduced enzymatic activity are an enoyl-coA reductase and a malonyl-coA-ACP transacylase.
11. The method of clause 10, wherein the enoyl-coA reductase is fabI or a peptide of 80% or more homology to SEQ ID NO: 14.
12. The method of clause 10, wherein the malonyl-coA-ACP transacylase is fabD or a peptide of 80% or more homology to SEQ ID NO: 7.
13. The method of any of clauses 1-12, wherein the microorganism is E. coli.
14. The method of any of clauses 1-13, wherein the method is performed at above room temperature.
15. The method of any of clauses 1-13, wherein the method is performed at a temperature between 25°C and 50°C.
16. The method of any of clauses 1-15, wherein the microorganism has one or more genetic modifications to increase levels of pantothenate.
17. The method of clause 16, wherein the one or more genetic modifications to increase levels of pantothenate is a modification of panE or a peptide of 80% or more homology to panE.
18. The method of any one of clauses 1-17, wherein the microorganism has one or more genetic modifications to increase pyruvate dehydrogenase activity.
19. The method of clause 18, wherein the one or more genetic modifications to increase pyruvate dehydrogenase activity is a modification of aceE or a peptide of 80% or more homology to SEQ ID NO: 172.
20. The method of clause 18, wherein the increased pyruvate dehydrogenase activity is resistant to inhibition by elevated NADH levels.
21. The method of any one of clauses 1-14, wherein the microorganism is further genetically modified to have reduced alcohol dehydrogenase activity.
22. The method of clause 21, wherein the microorganism is genetically modified to have a modification of adhE or a peptide of 80% or more homology to adhE.
23. The method of any one of clauses 1-22, wherein the microorganism is further genetically modified to have one or more genetic modifications relating to a metabolic production pathway from malonyl-CoA to the chemical, wherein the pathway uses NADH as a reducing agent and wherein there is a mutation or deletion of an alcohol dehydrogenase gene.
24. The method of any one of clauses 1-22, wherein the microorganism is further genetically modified to have modified malonyl-coA dependant acetoacetyl-coA synthase activity.
25. The method of clause 24, wherein the modified malonyl-coA dependant acetoacetyl-coA synthase is npth07 or a peptide of 80% or more homology to npth07.
26. The method of any one of clauses 1-25, wherein the microorganism is further genetically modified to have altered elongase activity.
27. The method of clause 26, wherein the microorganism has one or more modifications in the group consisting of elo1, elo2, and elo3, or a peptide of 80% or more homology to a peptide from SEQ ID NOs: 199-201.
28. The method of any one of clauses 1-27, wherein acetoacetyl-coA is converted to (S)-3-hydroxybutyryl-coA, (R)-3-hydroxybutyryl-coA, 3-hydroxymethylglutaryl-coA, crotonyl-coA, butyryl-coA, isobutyryl-coA, methacrylyl-coA, or 2-hydroxyisobutyryl-coA.
29. The method of clause 28, wherein conversion of acetoacetyl-coA to (S)-3-hydroxybutyryl-coA, (R)-3-hydroxybutyryl-coA, 3-hydroxymethylglutaryl-coA, crotonyl-coA, butyryl-coA, isobutyryl-coA, methacrylyl-coA, or 2-hydroxyisobutyryl-coA occurs in more than one step.
30. The method of any one of clauses 1-27, wherein acetoacetyl-coA is converted to a linear acyl-coA with a chain length from 4-18 carbons.
31. The method of any of clauses 1-27, wherein the chemical is selected from the group consisting of butanol, isobutanol, 3-hydroxypropionic acid, methacrylic acid, 2-hydroxyisobutyrate, butyrate, isobutyrate, acetoacetate, polyhydroxybutyrate, (S)-3-hydroxybutyrate, (R)-3-hydroxybutyrate, mevalonate, an isoprenoid, a fatty acid, a fatty aldehyde, a fatty alcohol, a fatty acid ester, phloroglucinol, resorcinol, an alkylresorcinol, tetracycline, erythromycin, avermectin, macrolides, Vancomycin-group antibiotics, and Type II polyketides.
32. The method of clause 31, wherein the chemical is produced at a concentration higher than a concentration of said chemical produced by a wild type microorganism.
33. A method for producing a C4-C18 fatty acid, said method comprising combining a carbon source, a microorganism, and a cell culture to produce the C4-C18 fatty acid, wherein
   a) said cell culture comprises an inhibitor of fatty acid synthase and/or the microorganism is genetically modified for reduced enzymatic activity in at least one of the microorganism's native fatty acid synthase pathway enzymes, providing for reduced conversion of malonyl-CoA to fatty acyl-ACPs; and
   b) the microorganism additionally has one or more genetic modifications increasing fatty acid production.
34. The method of clause 33, wherein the C4-C18 fatty acid is selected from the group consisting of C4, C6, C8, C10, C12, C14, C16 and C18 fatty acids.
35. The method of clause 33, further comprising isolating a C4-C18 fatty acid from said cell culture.
36. The method of clause 33, wherein the microorganism synthesizes fatty acyl-coAs of 4-18 carbon chain length.
37. The method of clause 36, wherein the microorganism expresses at least one heterologous elongase enzyme.
38. The method of clause 37, wherein the elongase enzyme is a peptide with a sequence corresponding to the group consisting of elo1, elo2, and elo3, or a peptide of 80% or more homology to a peptide from SEQ ID NOs: 199-201.
39. A genetically modified microorganism for use according to any one of clauses 1-38.
40. The genetically modified microorganism of clause 39, wherein said microorganism is a yeast or a bacteria.
41. The genetically modified microorganism of clause 40, wherein said microorganism is E. coli.
42. The geneically modified microorganism of clause 39, wherein said microorganism comprises a sequence selected from group consisting of SEQ ID NO: 1-215 or a sequence of 80% or more homology to SEQ ID NO: 1-215.
43. The geneically modified microorganism of clause 39, wherein said microorganism comprises at least one, two, three, four, five, six, seven, eight, nine, ten, or more sequences selected from group consisting of SEQ ID NO: 1-215 or sequences of 80% or more homology to SEQ ID NO: 1-215.
44. The method of any one of clauses 1-38, wherein the microorganism is further genetically modified to induce enzyme expression from the yibD gene promoter with a decreased environmental phosphate concentration.
45. The method of any one of clauses 1-38, wherein the microorganism is further genetically modified to maintain plasmids with expression of a gapA gene on the plasmid and a deletion of the gapA gene in the chromosome.

## Claims

1. A method for producing a mixture of C4-C18 fatty acids, fatty alcohols, fatty acid esters, fatty aldehydes, fatty amines, fatty amides, or fatty acid salts, said method comprising combining a carbon source, a microorganism, and a cell culture to produce the mixture, wherein
a) the microorganism is genetically modified for reduced enzymatic activity in at least one of beta-ketoacyl-ACP synthase (fabB), beta-ketoacyl-ACP synthase III (FabH), enoyl-ACP reductase (fabI), malonyl-CoA-ACP transacylase (fabD), β-ketoacyl-ACP reductase (fabG), and β-hydroxyacyl-ACP dehydratase (fabZ/fabA) such that enzymatic activity is reduced by at least 10, at least 20, at least 30, at least 40, at least 50, at least 60, at least 70, at least 80, or at least 90% compared with the activity of the native enzyme under standard conditions; and
b) the microorganism additionally has one or more genetic modifications increasing production of the mixture, wherein the microorganism synthesizes fatty acyl-CoAs of 4-18 carbon chain length; and
c) the mixture is further processed as a mixture of fatty alcohols, fatty acid esters, fatty aldehydes, fatty amines, fatty amides, or fatty acid salts.

2. The method of claim 1, wherein the C4-C18 fatty acids, fatty alcohols, fatty acid esters, fatty aldehydes, fatty amines, fatty amides, or fatty acid salts are selected from the group consisting of C4, C6, C8, C10, C12, C14, C16 and C18 fatty acids, fatty alcohols, fatty acid esters, fatty aldehydes, fatty amines, fatty amides, or fatty acid salts.

3. The method of any of claims 1 to 2, wherein the microorganism expresses at least one heterologous elongase enzyme.

4. The method of claim 3, wherein the elongase enzyme is a peptide with a sequence corresponding to the group consisting of elo1, elo2, and elo3, or a peptide of 80% or more sequence identity with a peptide from SEQ ID NOs: 199-201.

5. The method of any one of claims 1 to 4, wherein the microorganism is E. coli.

6. The method of any one of claims 1-5, wherein the microorganism is further genetically modified to induce enzyme expression from the yibD gene promoter with a decreased environmental phosphate concentration.

7. The method of claim 1, wherein the mixture is processed to remove cellular debris.

8. The method of claim 1, wherein the mixture consists essentially of C4-C18 compounds.

9. The method of claim 1, wherein the mixture is essentially free of one or more additional components selected from the group consisting of opacifying agents, pearlescent aids, astringets anti-acne agents, anti-caking agents, antimicrobial agents, antioxidants, biocides, pH modifiers, skin treating agents, vitamins, and humectants.

10. The method of claim 1, wherein the mixture is essentially free of one or more essential oils selected from the group consisting of almond, anise, basil, bay, bergamot, bitter almond, camphor, cassia, cedarwood, chamomile, cinnamon, citronella, clove, clove bud, coriander, cypress, eucalyptus, fennel, fir, frankincense, geranium, ginger, grapefruit, jasmine, lavender, lemon, lemongrass, lime, litsea, marigold, marjoram, myrtle, neroli, nutmeg, oakmoss, orange, palmorosa, patchouli, pennyroyal, peppermint, pine, rose, rosemary, sage, sandalwood, spearmint, spruce, sweet birch, tangerine, tea tree, vanilla, vetivert, white thyme, and wintergreen.

11. A genetically modified microorganism for use according to any one of claims 1-10, wherein said microorganism:
a) is genetically modified for reduced enzymatic activity in at least one of beta-ketoacyl-ACP synthase (fabB), beta-ketoacyl-ACP synthase III (FabH), enoyl-ACP reductase (fabI), malonyl-CoA-ACP transacylase (fabD), β-ketoacyl-ACP reductase (fabG), and β-hydroxyacyl-ACP dehydratase (fabZ/fabA) such that enzymatic activity is reduced by at least 10, at least 20, at least 30, at least 40, at least 50, at least 60, at least 70, at least 80, or at least 90% compared with the activity of the native enzyme under standard conditions; and
b) additionally has one or more genetic modifications increasing production of the mixture, wherein the microorganism synthesizes fatty acyl-CoAs of 4-18 carbon chain length, fatty alcohols, fatty acid esters, fatty aldehydes, fatty amines, fatty amides, or fatty acid salts.

12. The genetically modified microorganism of claim 11, wherein said microorganism is a yeast or a bacteria.

13. The genetically modified microorganism of claim 12, wherein said microorganism is E. coli.

14. The genetically modified microorganism of any one of claim 12 to claim 13, wherein said microorganism comprises at least two, three, four, five, six, seven, eight, nine, ten, or more sequences selected from group consisting of SEQ ID NO: 1-215 or sequences of 80% or more sequence identity with SEQ ID NO: 1-215.
